# EUROPEAN PATENT APPLICATION

(11) **EP 4 563 580 A1**
(43) Date of publication of application: **04.06.2025**
(21) Application number: 23845651.1
(22) Date of filing: 27.07.2023
(51) Int. Cl.: C07D 498/22, C07D 498/16, C07D 519/00, A61K 31/553, A61P 35/00

(54) **FUSED RING COMPOUND, PREPARATION METHOD THEREFOR AND MEDICINAL APPLICATION THEREOF**

(30) Priority: 27.07.2022 CN 202210891331; 22.11.2022 CN 202211467809; 27.12.2022 CN 202211724856; 30.12.2022 CN 202211724115; 15.02.2023 CN 202310120312; 10.03.2023 CN 202310234194; 08.06.2023 CN 202310676437
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: LI, Xin, Shanghai 200245 (CN); CAI, Guodong, Shanghai 200245 (CN); SHEN, Feng, Shanghai 200245 (CN); HE, Feng, Shanghai 200245 (CN)
(74) Representative: Potter Clarkson
(86) International application number: PCT/CN2023/109598
(87) International publication number: WO 2024/022444

(57) **Abstract**

The present disclosure relates to a fused ring compound, a preparation method therefor and a medicinal application thereof. Specifically, the present disclosure relates to a fused ring compound represented by general formula (IN), a preparation method therefor, a pharmaceutical composition containing the compound, and a use thereof as a therapeutic agent, in particular, the use thereof in the preparation of a drug for inhibiting KRAS G12D. Each group in the general formula (IN) is as defined in the description.

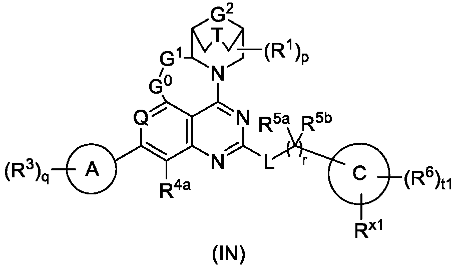

## Description

### TECHNICAL FIELD

The present disclosure pertains to the field of pharmaceutics and relates to a fused cyclic compound, a preparation method therefor, and pharmaceutical use thereof. In particular, the present disclosure relates to a fused cyclic compound represented by general formula (IN), a preparation method therefor, a pharmaceutical composition comprising the compound, and use thereof in the preparation of a medicament for inhibiting KRAS G12D.

### BACKGROUND

RAS is one of the oncogenes with the highest mutation rate in tumors, and about 30% of human malignancies are associated with mutations of the RAS gene. The RAS family includes KRAS, NRAS, and HRAS, and KRAS mutations are the most common and account for approximately 85%. KRAS mutations are common in solid tumors, with highfrequency mutations in the three fatal cancers in humans: lung cancer (17%), colorectal cancer (33%), and pancreatic cancer (61%). A total of 97% of the KRAS gene mutations involve mutation of amino acid residue No. 12 or 13, and G12D is an important mutation. Data analysis of Western populations shows that G12D mutation is found in 36%, 12%, and 4% of pancreatic cancer, colorectal cancer, and non-small cell lung cancer patients, respectively.

After being activated, KRAS regulates multiple functions such as cell proliferation, survival, migration, and metabolism through a plurality of downstream signaling pathways represented by RAF-MEK-ERK, PI3K-AKT-mTOR, and TIAM1-RAc. After mutation of the KRAS gene, the protein is continuously activated, resulting in continuous activation of downstream signaling pathways and thereby promoting tumorigenesis. KRAS protein has long been considered as an undruggable drug target because it lacks conventional small molecule binding sites on its surface and it is extremely difficult to inhibit due to its ultrahigh affinity for guanylic acid. However, based on the importance and prevalence of abnormal KRAS activation in cancer progression, KRAS has been and remains a target of high interest for drug development. Currently, except for inhibitors against KRAS G12C, there is a lack of KRAS inhibitors that are effective against other mutations, such that most patients with KRAS mutation remain non-medicated. G12D is a mutant widely expressed in multiple tumors, and it is of important clinical significance to develop inhibitors against it.

Currently, published related patent applications include WO2023030385A1, WO2023274383A1, WO2021041671A1, WO2020146613A1, WO2017172979A1, WO2020238791A1, and WO2021000885A1.

### SUMMARY

The present disclosure aims to provide a compound represented by general formula (IN) or a pharmaceutically acceptable salt thereof: 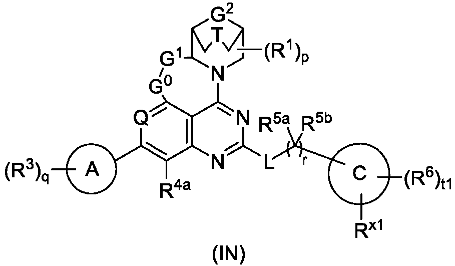 wherein:
ring C is heterocyclyl;
R^{x1} is a hydrogen atom or R^{x}; preferably, R^{x1} is R^{x}
R^{x} is selected from the group consisting of =N-O-R⁶¹, =CR⁶²R⁶³, and =N-R⁶⁴;
G⁰ is selected from the group consisting of O, S, S(O), S(O)₂, CR^{G0a}R^{G0b}, and NR^{G0c};
G¹ is selected from the group consisting of CR^{G1a}R^{G1b}, CR^{G1a}R^{G1b}CR^{G1c}R^{G1d}, C=O, and C(O)CR^{G1a}R^{G1b};
G² is NR^{d};
T is a chemical bond or is selected from the group consisting of CR^{a}R^{b}, NR^{T}, and O;
Q is N or CR^{2a};
ring A is aryl or heteroaryl;
L is selected from the group consisting of a single bond, O, and NR^{e};
R^{a}, R^{b}, R^{G0a}, R^{G0b}, R^{G1a}, R^{G1b}, R^{G1c}, and R^{G1d} are identical or different and are each independently selected from the group consisting of a hydrogen atom, halogen, alkyl, alkoxy, haloalkyl, haloalkoxy, cyano, amino, nitro, hydroxy, hydroxyalkyl, cycloalkyl, and heterocyclyl; or, R^{G1a} and R^{G1b}, together with the carbon atom to which they are attached, form cycloalkyl; or, R^{G1c} and R^{G1d}, together with the carbon atom to which they are attached, form cycloalkyl;
each R¹ is identical or different and is independently selected from the group consisting of halogen, alkyl, alkenyl, alkynyl, alkoxy, haloalkyl, haloalkoxy, cyano, amino, -(CH₂)ᵤ-NR^{f}R^{g}, hydroxy, and hydroxyalkyl;
R^{2a} and R^{4a} are identical or different and are each independently selected from the group consisting of a hydrogen atom, halogen, alkyl, alkenyl, alkynyl, alkoxy, haloalkyl, haloalkoxy, cyano, amino, -(CH₂)ᵥ-NR^{h}Rⁱ, hydroxy, hydroxyalkyl, and cycloalkyl;
each R³ is identical or different and is independently selected from the group consisting of halogen, alkyl, alkenyl, alkynyl, alkoxy, haloalkyl, haloalkoxy, cyano, amino, -(CH₂)_{w}-NR^{j}R^{k}, -(CH₂)_{w1}-(O)ₓ₁-C(O)NR^{j1}R^{k1}, -(CH₂)_{w2}-(O)ₓ₂-C(O)OR^{j2}, nitro, hydroxy, hydroxyalkyl, cycloalkyl, heterocyclyl, aryl, and heteroaryl;
each R⁶ is identical or different and is independently selected from the group consisting of a hydrogen atom, halogen, alkyl, alkenyl, alkynyl, alkoxy, haloalkyl, haloalkoxy, cyano, amino, -(CH₂)_{y}-NR^{m}Rⁿ, -(CH₂)_{y1}-(O)_{z1}-C(O)NR^{m1}Rⁿ¹, -(CH₂)_{y2}-(O)_{z2}-C(O)OR^{m2}, =N-O-R⁶¹, =CR⁶²R⁶³, =N-R⁶⁴, nitro, hydroxy, hydroxyalkyl, oxo, cycloalkyl, heterocyclyl, aryl, and heteroaryl;
R⁶¹, R⁶², R⁶³, and R⁶⁴ are identical or different and are each independently selected from the group consisting of a hydrogen atom, halogen, alkyl, haloalkyl, hydroxyalkyl, and cycloalkyl;
R^{5a} and R^{5b} are identical or different and are each independently selected from the group consisting of a hydrogen atom, halogen, alkyl, haloalkyl, cyano, hydroxy, and hydroxyalkyl; or
R^{5a} and R^{5b}, together with the carbon atom to which they are attached, form cycloalkyl or heterocyclyl, and the cycloalkyl or heterocyclyl is independently and optionally substituted with one or more identical or different substituents selected from the group consisting of halogen, alkyl, haloalkyl, alkoxy, haloalkoxy, cyano, amino, hydroxy, and hydroxyalkyl;
R^{G0c}, R^{T}, R^{d}, R^{e}, R^{f}, R^{g}, R^{h}, Rⁱ, R^{j}, R^{k}, R^{j1}, R^{k1}, R^{j2}, R^{m}, Rⁿ, R^{m1}, Rⁿ¹, and R^{m2} are identical or different and are each independently selected from the group consisting of a hydrogen atom, alkyl, alkenyl, alkynyl, haloalkyl, hydroxyalkyl, cycloalkyl, heterocyclyl, aryl, and heteroaryl;
u, v, w, w1, w2, y, y1, and y2 are identical or different and are each independently selected from the group consisting of 0, 1, 2, and 3;
x1, x2, z1, and z2 are identical or different and are each independently selected from the group consisting of 0 and 1;
r is 0, 1, 2, or 3;
p is 0, 1, 2, 3, 4, or 5;
q is 0, 1, 2, 3, 4, or 5; and
t1 is 0, 1, 2, 3, 4, or 5.

In some embodiments of the present disclosure, the compound represented by general formula (IN) or the pharmaceutically acceptable salt thereof is a compound represented by general formula (IN') or a pharmaceutically acceptable salt thereof: 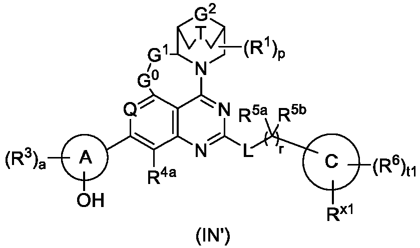 wherein:
a is 0, 1, 2, 3, or 4;
G⁰, G¹, G², T, ring A, ring C, Q, L, R¹, R³, R^{4a}, R^{5a}, R^{5b}, R⁶, R^{x1}, p, t1, and r are as defined in general formula (IN).

The present disclosure provides a compound represented by general formula (IM) or a pharmaceutically acceptable salt thereof: 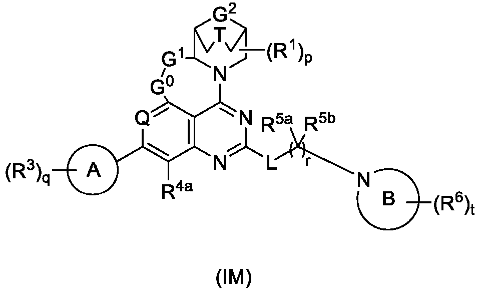 wherein:
ring B is heterocyclyl;
G⁰ is selected from the group consisting of O, S, S(O), S(O)₂, CR^{G0a}R^{G0b}, and NR^{G0c};
G¹ is selected from the group consisting of CR^{G1a}R^{G1b}, CR^{G1a}R^{G1b}CR^{G1c}R^{G1d}, C=O, and C(O)CR^{G1a}R^{G1b};
G² is NR^{d};
T is a chemical bond or is selected from the group consisting of CR^{a}R^{b}, NR^{T}, and O;
Q is N or CR^{2a};
ring A is aryl or heteroaryl;
L is selected from the group consisting of a single bond, O, and NR^{e};
R^{a}, R^{b}, R^{G0a}, R^{G0b}, R^{G1a}, R^{G1b}, R^{G1c}, and R^{G1d} are identical or different and are each independently selected from the group consisting of a hydrogen atom, halogen, alkyl, alkoxy, haloalkyl, haloalkoxy, cyano, amino, nitro, hydroxy, hydroxyalkyl, cycloalkyl, and heterocyclyl; or, R^{G1a} and R^{G1b}, together with the carbon atom to which they are attached, form cycloalkyl; or, R^{G1c} and R^{G1d}, together with the carbon atom to which they are attached, form cycloalkyl;
each R¹ is identical or different and is independently selected from the group consisting of halogen, alkyl, alkenyl, alkynyl, alkoxy, haloalkyl, haloalkoxy, cyano, amino, -(CH₂)ᵤ-NR^{f}R^{g}, hydroxy, and hydroxyalkyl;
R^{2a} and R^{4a} are identical or different and are each independently selected from the group consisting of a hydrogen atom, halogen, alkyl, alkenyl, alkynyl, alkoxy, haloalkyl, haloalkoxy, cyano, amino, -(CH₂)ᵥ-NR^{h}Rⁱ, hydroxy, hydroxyalkyl, and cycloalkyl;
each R³ is identical or different and is independently selected from the group consisting of halogen, alkyl, alkenyl, alkynyl, alkoxy, haloalkyl, haloalkoxy, cyano, amino, -(CH₂)_{w}-NR^{j}R^{k}, -(CH₂)_{w1}-(O)ₓ₁-C(O)NR^{j1}R^{k1}, -(CH₂)_{w2}-(O)ₓ₂-C(O)OR^{j2}, nitro, hydroxy, hydroxyalkyl, cycloalkyl, heterocyclyl, aryl, and heteroaryl;
each R⁶ is identical or different and is independently selected from the group consisting of a hydrogen atom, halogen, alkyl, alkenyl, alkynyl, alkoxy, haloalkyl, haloalkoxy, cyano, amino, -(CH₂)_{y}-NR^{m}Rⁿ, -(CH₂)_{y1}-(O)_{z1}-C(O)NR^{m1}Rⁿ¹, -(CH₂)_{y2}-(O)_{z2}-C(O)OR^{m2}, =N-O-R⁶¹, =CR⁶²R⁶³, =N-R⁶⁴, nitro, hydroxy, hydroxyalkyl, cycloalkyl, heterocyclyl, aryl, and heteroaryl;
R⁶¹, R⁶², R⁶³, and R⁶⁴ are identical or different and are each independently selected from the group consisting of a hydrogen atom, halogen, alkyl, haloalkyl, hydroxyalkyl, and cycloalkyl;
R^{5a} and R^{5b} are identical or different and are each independently selected from the group consisting of a hydrogen atom, halogen, alkyl, haloalkyl, cyano, hydroxy, and hydroxyalkyl; or
R^{5a} and R^{5b}, together with the carbon atom to which they are attached, form cycloalkyl or heterocyclyl, and the cycloalkyl or heterocyclyl is independently and optionally substituted with one or more identical or different substituents selected from the group consisting of halogen, alkyl, haloalkyl, alkoxy, haloalkoxy, cyano, amino, hydroxy, and hydroxyalkyl;
R^{G0c}, R^{T}, R^{d}, R^{e}, R^{f}, R^{g}, R^{h}, Rⁱ, R^{j}, R^{k}, R^{j1}, R^{k1}, R^{j2}, R^{m}, Rⁿ R^{m1}, Rⁿ¹, and R^{m2} are identical or different and are each independently selected from the group consisting of a hydrogen atom, alkyl, alkenyl, alkynyl, haloalkyl, hydroxyalkyl, cycloalkyl, heterocyclyl, aryl, and heteroaryl;
u, v, w, w1, w2, y, y1, and y2 are identical or different and are each independently selected from the group consisting of 0, 1, 2, and 3;
x1, x2, z1, and z2 are identical or different and are each independently selected from the group consisting of 0 and 1;
r is 0, 1, 2, or 3;
p is 0, 1, 2, 3, 4, or 5; and
q is 0, 1, 2, 3, 4, or 5; and
t is 0, 1, 2, 3, 4, or 5.

In some embodiments of the present disclosure, the compound represented by general formula (IN), (IN'), or (IM) or the pharmaceutically acceptable salt thereof is a compound represented by general formula (IM') or a pharmaceutically acceptable salt thereof: 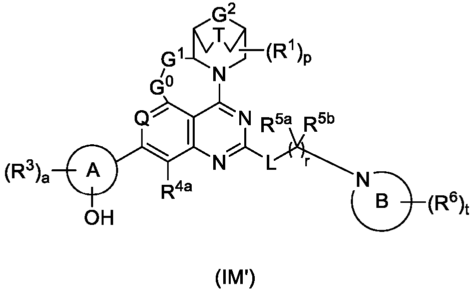 wherein:
a is 0, 1, 2, 3, or 4;
G⁰, G¹, G², T, ring A, ring B, Q, L, R¹, R³, R^{4a}, R^{5a}, R^{5b}, R⁶, p, t, and r are as defined in general formula (IM).

In some embodiments of the present disclosure, the compound represented by general formula (IN) or (IM) or the pharmaceutically acceptable salt thereof is a compound represented by general formula (I) or a pharmaceutically acceptable salt thereof: 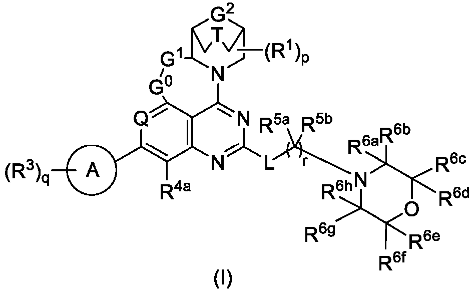 wherein:
G⁰ is selected from the group consisting of O, S, S(O), S(O)₂, CR^{G0a}R^{G0b}, and NR^{G0c};
G¹ is selected from the group consisting of CR^{G1a}R^{G1b}, CR^{G1a}R^{G1b}CR^{G1c}R^{G1d}, C=O, and C(O)CR^{G1a}R^{G1b};
G² is NR^{d};
T is a chemical bond or is selected from the group consisting of CR^{a}R^{b}, NR^{T}, and O;
Q is N or CR^{2a};
ring A is aryl or heteroaryl;
L is selected from the group consisting of a single bond, O, and NR^{e};
R^{a}, R^{b}, R^{G0a}, R^{G0b}, R^{G1a}, R^{G1b}, R^{G1c}, and R^{G1d} are identical or different and are each independently selected from the group consisting of a hydrogen atom, halogen, alkyl, alkoxy, haloalkyl, haloalkoxy, cyano, amino, nitro, hydroxy, hydroxyalkyl, cycloalkyl, and heterocyclyl; or, R^{G1a} and R^{G1b}, together with the carbon atom to which they are attached, form cycloalkyl; or, R^{G1c} and R^{G1d}, together with the carbon atom to which they are attached, form cycloalkyl;
each R¹ is identical or different and is independently selected from the group consisting of halogen, alkyl, alkenyl, alkynyl, alkoxy, haloalkyl, haloalkoxy, cyano, amino, -(CH₂)ᵤ-NR^{f}R^{g}, hydroxy, and hydroxyalkyl;
R^{2a} and R^{4a} are identical or different and are each independently selected from the group consisting of a hydrogen atom, halogen, alkyl, alkenyl, alkynyl, alkoxy, haloalkyl, haloalkoxy, cyano, amino, -(CH₂)ᵥ-NR^{h}Rⁱ, hydroxy, hydroxyalkyl, and cycloalkyl;
each R³ is identical or different and is independently selected from the group consisting of halogen, alkyl, alkenyl, alkynyl, alkoxy, haloalkyl, haloalkoxy, cyano, amino, -(CH₂)_{w}-NR^{j}R^{k}, -(CH₂)_{w1}-(O)ₓ₁-C(O)NR^{j1}R^{k1}, -(CH₂)_{w2}-(O)ₓ₂-C(O)OR^{j2}, nitro, hydroxy, hydroxyalkyl, cycloalkyl, heterocyclyl, aryl, and heteroaryl;
R^{6a}, R^{6b}, R^{6c}, R^{6d}, R^{6e}, R^{6f}, R^{6g}, and R^{6h} are identical or different and are each independently selected from the group consisting of a hydrogen atom, halogen, alkyl, alkenyl, alkynyl, alkoxy, haloalkyl, haloalkoxy, cyano, amino, -(CH₂)_{y}-NR^{m}Rⁿ, -(CH₂)_{y1}-(O)_{z1}-C(O)NR^{m1}Rⁿ¹, -(CH₂)_{y2}-(O)_{z2}-C(O)OR^{m2}, nitro, hydroxy, hydroxyalkyl, cycloalkyl, heterocyclyl, aryl, and heteroaryl; or
R^{6a} and R^{6e}, together with the carbon atoms to which they are attached, R^{6a} and R^{6g}, together with the carbon atoms to which they are attached, R^{6c} and R^{6e}, together with the carbon atoms to which they are attached, or R^{6c} and R^{6g}, together with the carbon atoms to which they are attached, form a bridge; the bridge has 1, 2, 3, or 4 CH₂, any one of which may be optionally replaced with O, S, or NH, and the bridge is optionally substituted with one or more identical or different substituents selected from the group consisting of halogen, alkyl, haloalkyl, alkoxy, haloalkoxy, cyano, amino, hydroxy, and hydroxyalkyl; or
R^{6a} and R^{6b}, together with the carbon atom to which they are attached, R^{6c} and R^{6a}, together with the carbon atom to which they are attached, R^{6e} and R^{6f}, together with the carbon atom to which they are attached, or R^{6g} and R^{6h}, together with the carbon atom to which they are attached, form cycloalkyl or heterocyclyl, and the cycloalkyl or heterocyclyl is optionally substituted with one or more identical or different substituents selected from the group consisting of halogen, alkyl, haloalkyl, alkoxy, haloalkoxy, cyano, amino, hydroxy, and hydroxyalkyl;
R^{5a} and R^{5b} are identical or different and are each independently selected from the group consisting of a hydrogen atom, halogen, alkyl, haloalkyl, cyano, hydroxy, and hydroxyalkyl; or
R^{5a} and R^{5b}, together with the carbon atom to which they are attached, form cycloalkyl or heterocyclyl, and the cycloalkyl or heterocyclyl is independently and optionally substituted with one or more identical or different substituents selected from the group consisting of halogen, alkyl, haloalkyl, alkoxy, haloalkoxy, cyano, amino, hydroxy, and hydroxyalkyl;
R^{G0c}, R^{T}, R^{d}, R^{e}, R^{f}, R^{g}, R^{h}, Rⁱ, R^{j}, R^{k}, R^{j1}, R^{k1}, R^{j2}, R^{m}, Rⁿ, R^{m1}, Rⁿ¹, and R^{m2} are identical or different and are each independently selected from the group consisting of a hydrogen atom, alkyl, alkenyl, alkynyl, haloalkyl, hydroxyalkyl, cycloalkyl, heterocyclyl, aryl, and heteroaryl;
u, v, w, w1, w2, y, y1, and y2 are identical or different and are each independently selected from the group consisting of 0, 1, 2, and 3;
x1, x2, z1, and z2 are identical or different and are each independently selected from the group consisting of 0 and 1;
r is 0, 1, 2, or 3;
p is 0, 1, 2, 3, 4, or 5; and
q is 0, 1, 2, 3, 4, or 5.

In some embodiments of the present disclosure, the compound represented by general formula (IN), (IN'), (IM), (IM'), or (I) or the pharmaceutically acceptable salt thereof is a compound represented by general formula (I') or a pharmaceutically acceptable salt thereof: 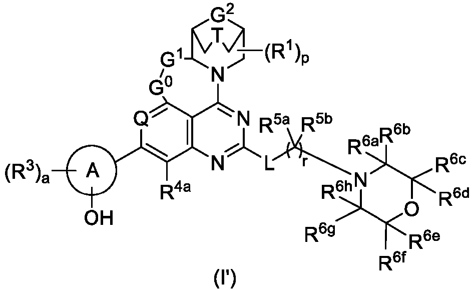 wherein:
a is 0, 1, 2, 3, or 4;
G⁰, G¹, G², T, ring A, Q, L, R¹, R³, R^{4a}, R^{5a}, R^{5b}, R^{6a}, R^{6b}, R^{6c}, R^{6d}, R^{6e}, R^{6f}, R^{6g}, R^{6h}, p, and r are as defined in general formula (I).

In some embodiments of the present disclosure, provided is the compound represented by formula (IN), (IN'), (IM), (IM'), (I), or (I') or the pharmaceutically acceptable salt thereof, wherein 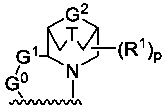 is selected from the group consisting of 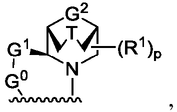 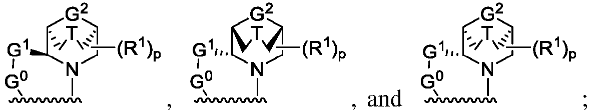 preferably, 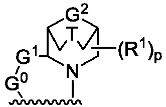 is 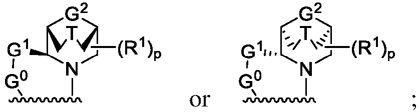 more preferably, 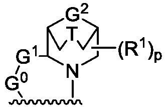 is 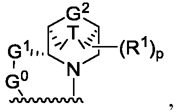 and G⁰, G¹, G², R¹, and p are as defined in general formula (I).

In some embodiments of the present disclosure, provided is the compound represented by general formula (IN), (IN'), (IM), (IM'), (I), or (I') or the pharmaceutically acceptable salt thereof, wherein G⁰ is selected from the group consisting of O, CR^{G0a}R^{G0b}, and NR^{G0c}; R^{G0a} and R^{G0b} are identical or different and are each independently selected from the group consisting of a hydrogen atom, halogen, C₁₋₆ alkyl, and C₁₋₆ haloalkyl; R^{G0c} is a hydrogen atom or C₁₋₆ alkyl; preferably, G⁰ is selected from the group consisting of O, CH₂, and NH; more preferably, G⁰ is O.

In some embodiments of the present disclosure, provided is the compound represented by general formula (IN), (IN'), (IM), (IM'), (I), or (I') or the pharmaceutically acceptable salt thereof, wherein G¹ is CR^{G1a}R^{G1b}, CR^{G1a}R^{G1b}CR^{G1c}R^{G1d}, or C=O; R^{G1a}, R^{G1b}, R^{G1c}, and R^{G1d} are identical or different and are each independently selected from the group consisting of a hydrogen atom, halogen, C₁₋₆ alkyl, and C₁₋₆ haloalkyl; preferably, G¹ is CH₂ or C=O; more preferably, G¹ is CH₂.

In some embodiments of the present disclosure, provided is the compound represented by general formula (IN), (IN'), (IM), (IM'), (I), or (I') or the pharmaceutically acceptable salt thereof, wherein G¹ is CR^{G1a}H, and R^{G1a} is as defined in general formula (IN); preferably, G¹ is CR^{G1a}H, and R^{G1a} is selected from the group consisting of a hydrogen atom, halogen, C₁₋₆ alkyl, and C₁₋₆ haloalkyl; more preferably, G¹ is CR^{G1a}H, and R^{G1a} is a hydrogen atom or C₁₋₆ alkyl; yet more preferably, G¹ is CR^{G1a}H, and R^{G1a} is C₁₋₆ alkyl; still more preferably, G¹ is CR^{G1a}H, and R^{G1a} is methyl.

In some embodiments of the present disclosure, provided is the compound represented by general formula (IN), (IN'), (IM), (IM'), (I), or (I') or the pharmaceutically acceptable salt thereof, wherein -G⁰-G¹- is selected from the group consisting of -O-CH₂-, -NH-C(O)-,-NH-CH₂-, -CH₂-CH₂-, and -O-CH₂-CH₂-; preferably, -G⁰-G¹- is -O-CH₂- or -NH-C(O)-; more preferably, -G⁰-G¹- is -O-CH₂-; in some embodiments, -G⁰-G¹- is -O-CH(CH₃)-. In some embodiments of the present disclosure, provided is the compound represented by general formula (IN), (IN'), (IM), (IM'), (I), or (I') or the pharmaceutically acceptable salt thereof, wherein T is a chemical bond.

In some embodiments of the present disclosure, provided is the compound represented by general formula (IN), (IN'), (IM), (IM'), (I), or (I') or the pharmaceutically acceptable salt thereof, wherein R^{5a} and R^{5b} are identical or different and are each independently selected from the group consisting of a hydrogen atom, halogen, C₁₋₆ alkyl, C₁₋₆ haloalkyl, hydroxy, and C₁₋₆ hydroxyalkyl, or R^{5a} and R^{5b}, together with the carbon atom to which they are attached, form 3- to 8-membered cycloalkyl; preferably, R^{5a} and R^{5b} are hydrogen atoms, or R^{5a} and R^{5b}, together with the carbon atom to which they are attached, form 3-to 6-membered cycloalkyl; more preferably, R^{5a} and R^{5b} are hydrogen atoms, or R^{5a} and R^{5b}, together with the carbon atom to which they are attached, form cyclopropyl.

In some embodiments of the present disclosure, provided is the compound represented by general formula (IN), (IN'), (IM), (IM'), (I), or (I') or the pharmaceutically acceptable salt thereof, wherein r is 1 or 3.

In some embodiments of the present disclosure, provided is the compound represented by general formula (IN), (IN'), (IM), (IM'), (I), or (I') or the pharmaceutically acceptable salt thereof, wherein 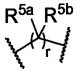 is -CH₂- or 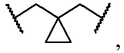 preferably

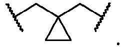

In some embodiments of the present disclosure, the compound represented by general formula (IN), (IM), or (I) or the pharmaceutically acceptable salt thereof is a compound represented by general formula (II') or a pharmaceutically acceptable salt thereof: 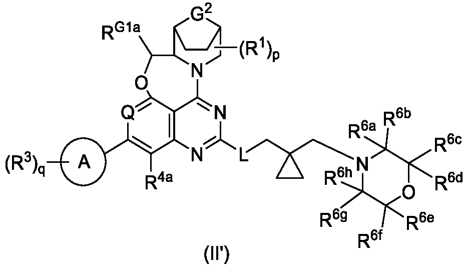 wherein:
ring A, G², Q, L, R^{G1a}, R¹, R³, R^{4a}, R^{6a}, R^{6b}, R^{6c}, R^{6d}, R^{6e}, R^{6f}, R^{6g}, R^{6h}, p, and q are as defined in general formula (I); preferably, R^{G1a} is C₁₋₆ alkyl.

In some embodiments of the present disclosure, the compound represented by general formula (IN), (IM), (I), or (II') or the pharmaceutically acceptable salt thereof is a compound represented by general formula (II) or a pharmaceutically acceptable salt thereof: 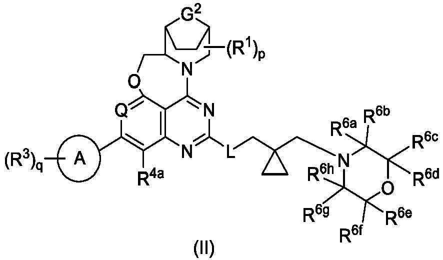 wherein:
ring A, G², Q, L, R¹, R³, R^{4a}, R^{6a}, R^{6b}, R^{6c}, R^{6d}, R^{6e}, R^{6f}, R^{6g}, R^{6h}, p, and q are as defined in general formula (I).

In some embodiments of the present disclosure, the compound represented by general formula (IN), (IN'), (IM), (IM'), (I), (I'), (II'), or (IVV') or the pharmaceutically acceptable salt thereof is a compound represented by general formula (III') or a pharmaceutically acceptable salt thereof: 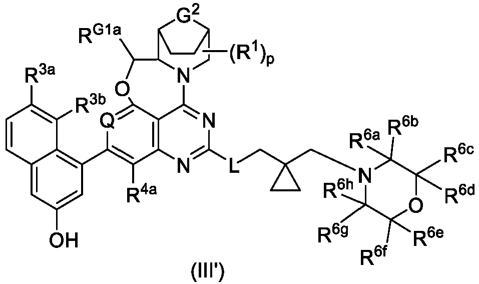 wherein:
R^{3a} and R^{3b} are identical or different and are each independently selected from the group consisting of a hydrogen atom, halogen, alkyl, alkenyl, alkynyl, alkoxy, haloalkyl, haloalkoxy, cyano, amino, hydroxy, hydroxyalkyl, cycloalkyl, and heterocyclyl;
G², Q, L, R^{G1a}, R¹, R^{4a}, R^{6a}, R^{6b}, R^{6c}, R^{6d}, R^{6e}, R^{6f}, R^{6g}, R^{6h}, and p are as defined in general formula (I); preferably, R^{G1a} is C₁₋₆ alkyl.

In some embodiments of the present disclosure, the compound represented by general formula (IN), (IN'), (IM), (IM'), (I), (I'), (II'), (II), (III'), or (IVV') or the pharmaceutically acceptable salt thereof is a compound represented by general formula (III) or a pharmaceutically acceptable salt thereof: 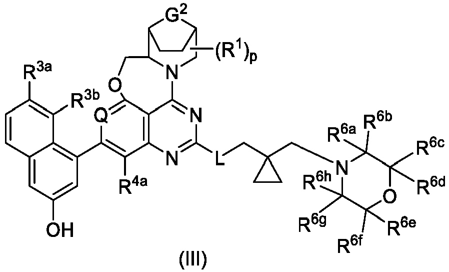 wherein:
R^{3a} and R^{3b} are identical or different and are each independently selected from the group consisting of a hydrogen atom, halogen, alkyl, alkenyl, alkynyl, alkoxy, haloalkyl, haloalkoxy, cyano, amino, hydroxy, hydroxyalkyl, cycloalkyl, and heterocyclyl;
G², Q, L, R¹, R^{4a}, R^{6a}, R^{6b}, R^{6c}, R^{6d}, R^{6e}, R^{6f}, R^{6g}, R^{6h}, and p are as defined in general formula (I).

In some embodiments of the present disclosure, the compound represented by general formula (IN) or (IN') or the pharmaceutically acceptable salt thereof is a compound represented by general formula (IVV') or a pharmaceutically acceptable salt thereof: 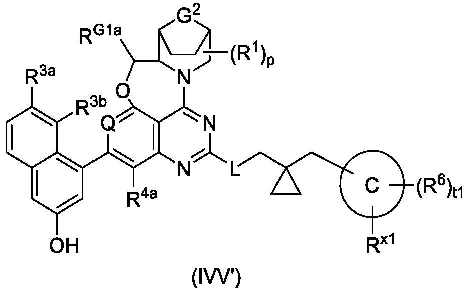 wherein:
R^{3a} and R^{3b} are identical or different and are each independently selected from the group consisting of a hydrogen atom, halogen, alkyl, alkenyl, alkynyl, alkoxy, haloalkyl, haloalkoxy, cyano, amino, hydroxy, hydroxyalkyl, cycloalkyl, and heterocyclyl;
ring C, G², Q, L, R^{G1a}, R¹, R^{4a}, R⁶, R^{x1}, t1, and p are as defined in general formula (IN); preferably, R^{G1a} is C₁₋₆ alkyl.

In some embodiments of the present disclosure, the compound represented by general formula (IN), (IN'), (IM), (IM'), or (IVV') or the pharmaceutically acceptable salt thereof is a compound represented by general formula (IV') or (VV') or a pharmaceutically acceptable salt thereof: 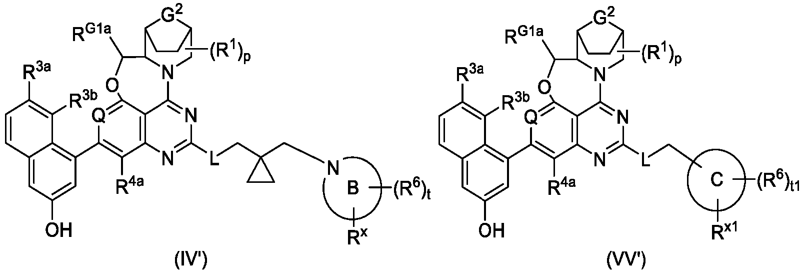 wherein:
R^{x} is selected from the group consisting of =N-O-R⁶¹, =CR⁶²R⁶³, and =N-R⁶⁴;
R⁶¹, R⁶², R⁶³, and R⁶⁴ are identical or different and are each independently selected from the group consisting of a hydrogen atom, halogen, alkyl, haloalkyl, hydroxyalkyl, and cycloalkyl;
t is 0, 1, 2, 3, or 4;
R^{3a} and R^{3b} are identical or different and are each independently selected from the group consisting of a hydrogen atom, halogen, alkyl, alkenyl, alkynyl, alkoxy, haloalkyl, haloalkoxy, cyano, amino, hydroxy, hydroxyalkyl, cycloalkyl, and heterocyclyl;
ring B is heterocyclyl;
Rⁿ¹, t1, ring C, G², Q, L, R^{G1a}, R¹, R^{4a}, R⁶, and p are as defined in general formula (IN); preferably, R^{G1a} is C₁₋₆ alkyl.

In some embodiments of the present disclosure, the compound represented by general formula (IN), (IN'), (IM), (IM'), or (IVV') or the pharmaceutically acceptable salt thereof is a compound represented by general formula (IV') or a pharmaceutically acceptable salt thereof: 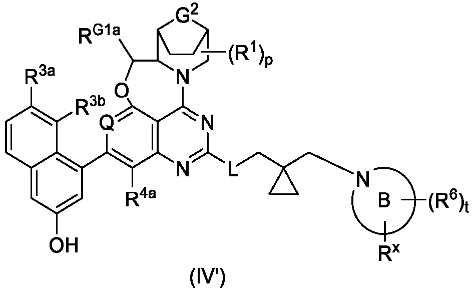 wherein:
R^{x} is selected from the group consisting of =N-O-R⁶¹, =CR⁶²R⁶³, and =N-R⁶⁴;
R⁶¹, R⁶², R⁶³, and R⁶⁴ are identical or different and are each independently selected from the group consisting of a hydrogen atom, halogen, alkyl, haloalkyl, hydroxyalkyl, and cycloalkyl;
t is 0, 1, 2, 3, or 4;
R^{3a} and R^{3b} are identical or different and are each independently selected from the group consisting of a hydrogen atom, halogen, alkyl, alkenyl, alkynyl, alkoxy, haloalkyl, haloalkoxy, cyano, amino, hydroxy, hydroxyalkyl, cycloalkyl, and heterocyclyl;
ring B, G², Q, L, R^{G1a}, R¹, R^{4a}, R⁶, and p are as defined in general formula (IM); preferably, R^{G1a} is C₁₋₆ alkyl.

In some embodiments of the present disclosure, the compound represented by general formula (IN) or (IN') or the pharmaceutically acceptable salt thereof is a compound represented by general formula (VV') or a pharmaceutically acceptable salt thereof: 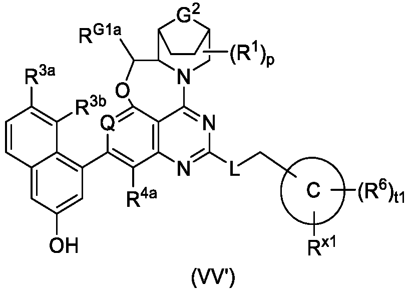 wherein:
R^{3a} and R^{3b} are identical or different and are each independently selected from the group consisting of a hydrogen atom, halogen, alkyl, alkenyl, alkynyl, alkoxy, haloalkyl, haloalkoxy, cyano, amino, hydroxy, hydroxyalkyl, cycloalkyl, and heterocyclyl;
ring C, G², Q, L, R^{G1a}, R¹, R^{4a}, R⁶, R^{x1}, t1, and p are as defined in general formula (IN); preferably, R^{G1a} is C₁₋₆ alkyl.

In some embodiments of the present disclosure, the compound represented by general formula (IN), (IN'), (IM), (IM'), or (VV') or the pharmaceutically acceptable salt thereof is a compound represented by general formula (V') or a pharmaceutically acceptable salt thereof: 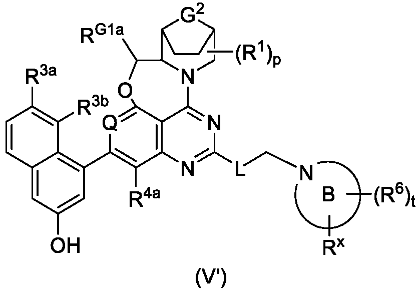 wherein:
R^{x} is selected from the group consisting of =N-O-R⁶¹, =CR⁶²R⁶³, and =N-R⁶⁴;
R⁶¹, R⁶², R⁶³, and R⁶⁴ are identical or different and are each independently selected from the group consisting of a hydrogen atom, halogen, alkyl, haloalkyl, hydroxyalkyl, and cycloalkyl;
t is 0, 1, 2, 3, or 4;
R^{3a} and R^{3b} are identical or different and are each independently selected from the group consisting of a hydrogen atom, halogen, alkyl, alkenyl, alkynyl, alkoxy, haloalkyl, haloalkoxy, cyano, amino, hydroxy, hydroxyalkyl, cycloalkyl, and heterocyclyl;
ring B, G², Q, L, R^{G1a}, R¹, R^{4a}, R⁶, and p are as defined in general formula (IM); preferably, R^{G1a} is C₁₋₆ alkyl.

In some embodiments of the present disclosure, provided is the compound of general formula (II) or (III) or the pharmaceutically acceptable salt thereof, wherein 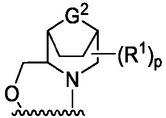 is selected from the group consisting of 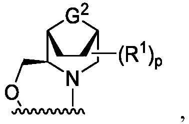 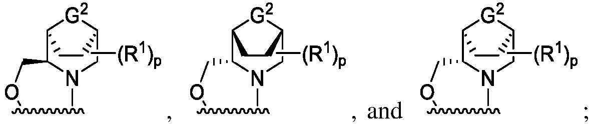 preferably, 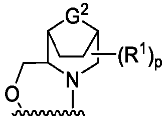 is 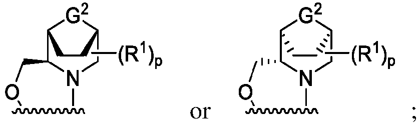 more preferably, 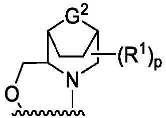 is 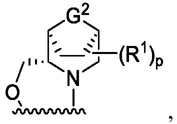 and G², R¹, and p are as defined in general formula (I).

In some embodiments of the present disclosure, provided is the compound represented by general formula (II'), (III'), (IV'), (V'), (IVV'), or (VV') or the pharmaceutically acceptable salt thereof, wherein 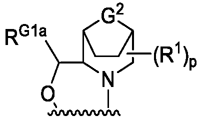 is selected from the group consisting of 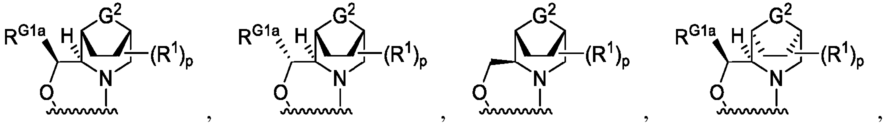 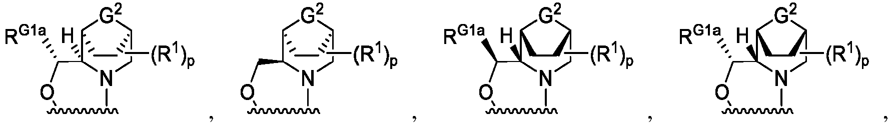 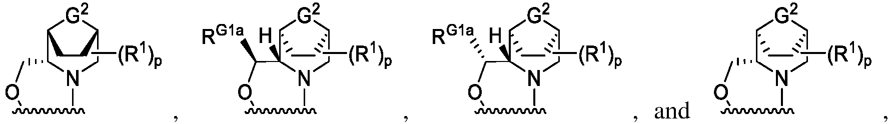 preferably from the group consisting of 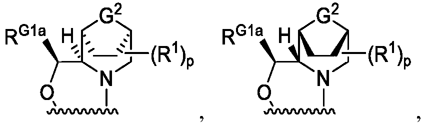 and 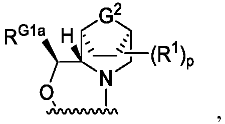 and more preferably from the group consisting of 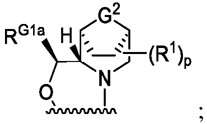 in this paragraph, R^{G1a} is selected from the group consisting of halogen, alkyl, alkoxy, haloalkyl, haloalkoxy, cyano, amino, nitro, hydroxy, hydroxyalkyl, cycloalkyl, and heterocyclyl, and G², R¹, and p are as defined in general formula (I).

In some embodiments of the present disclosure, the compound represented by general formula (IN), (IN'), (IM), (IM'), (I), (I'), (II'), (III'), (IV'), or (IVV') or the pharmaceutically acceptable salt thereof is a compound represented by general formula (III'-1) or a pharmaceutically acceptable salt thereof: 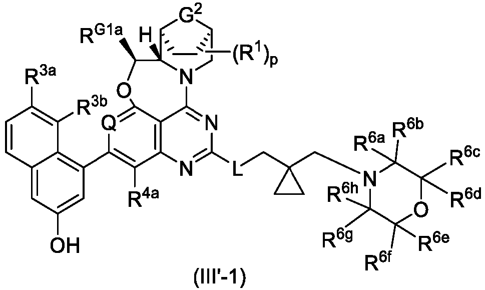 wherein:
R^{G1a} is selected from the group consisting of halogen, alkyl, alkoxy, haloalkyl, haloalkoxy, cyano, amino, nitro, hydroxy, hydroxyalkyl, cycloalkyl, and heterocyclyl; preferably, R^{G1a} is C₁₋₆ alkyl;
G², Q, L, R¹, R^{3a}, R^{3b}, R^{4a}, R^{6a}, R^{6b}, R^{6c}, R^{6d}, R^{6e}, R^{6f}, R^{6g}, R^{6h}, and p are as defined in general formula (III').

In some embodiments of the present disclosure, the compound represented by general formula (IN), (IN'), (IM), (IM'), (IV'), or (IVV') or the pharmaceutically acceptable salt thereof is a compound represented by general formula (IV'-1) or a pharmaceutically acceptable salt thereof: 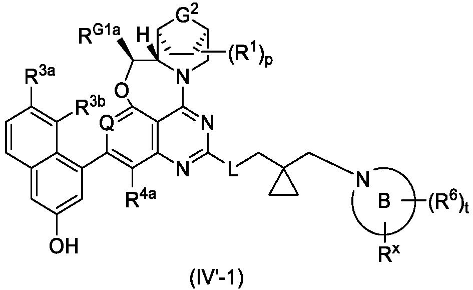 wherein:
R^{G1a} is selected from the group consisting of halogen, alkyl, alkoxy, haloalkyl, haloalkoxy, cyano, amino, nitro, hydroxy, hydroxyalkyl, cycloalkyl, and heterocyclyl;
preferably, R^{G1a} is C₁₋₆ alkyl;
ring B, G², Q, L, R¹, R^{3a}, R^{3b}, R^{4a}, R⁶, R^{x}, t, and p are as defined in general formula (IV').

In some embodiments of the present disclosure, the compound represented by general formula (IN), (IN'), or (VV') or the pharmaceutically acceptable salt thereof is a compound represented by general formula (VV'-1) or a pharmaceutically acceptable salt thereof: 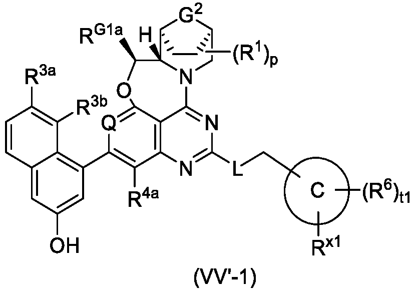 wherein:
R^{G1a} is selected from the group consisting of halogen, alkyl, alkoxy, haloalkyl, haloalkoxy, cyano, amino, nitro, hydroxy, hydroxyalkyl, cycloalkyl, and heterocyclyl;
preferably, R^{G1a} is C₁₋₆ alkyl;
ring C, G², Q, L, R¹, R^{3a}, R^{3b}, R^{4a}, R⁶, R^{x1}, t1, and p are as defined in general formula (VV').

In some embodiments of the present disclosure, the compound represented by general formula (IN), (IN'), (IM), (IM'), (V'), or (VV') or the pharmaceutically acceptable salt thereof is a compound represented by general formula (V'-1) or a pharmaceutically acceptable salt thereof: 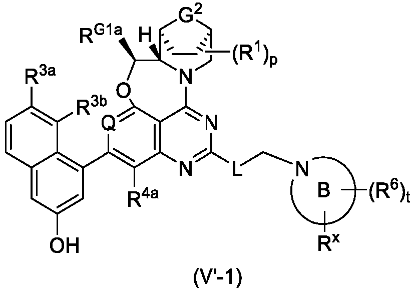 wherein:
R^{G1a} is selected from the group consisting of halogen, alkyl, alkoxy, haloalkyl, haloalkoxy, cyano, amino, nitro, hydroxy, hydroxyalkyl, cycloalkyl, and heterocyclyl;
preferably, R^{G1a} is C₁₋₆ alkyl;
G², Q, L, R¹, R^{3a}, R^{3b}, R^{4a}, R⁶, R^{x}, t, and p are as defined in general formula (V').

In some embodiments of the present disclosure, provided is the compound represented by general formula (IM), (IM'), (IV'), (IV'-1), (V'), or (V'-1) or the pharmaceutically acceptable salt thereof, wherein ring B is 3- to 8-membered heterocyclyl; preferably, ring B is selected from the group consisting of morpholinyl, 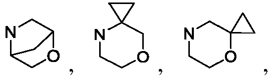 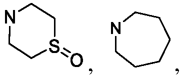 tetrahydropyrrolyl, piperidinyl, 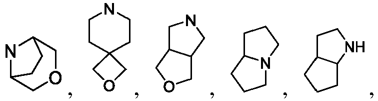 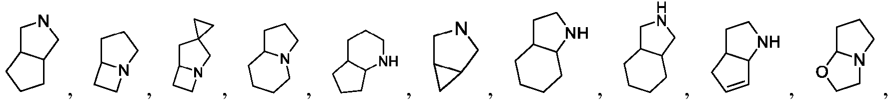 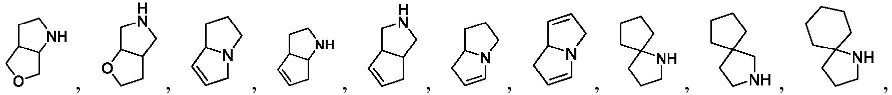 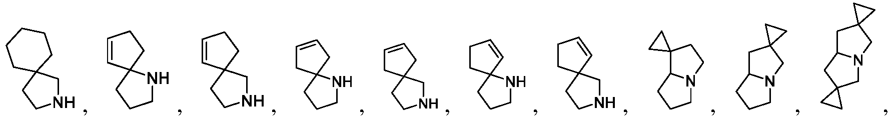 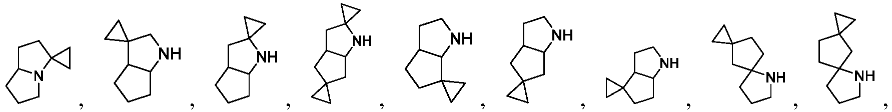 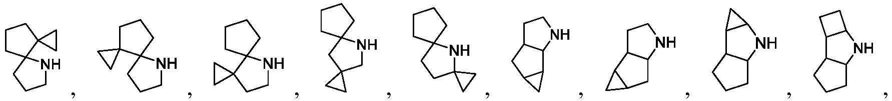 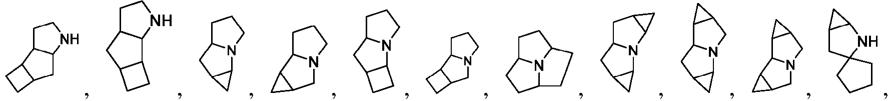 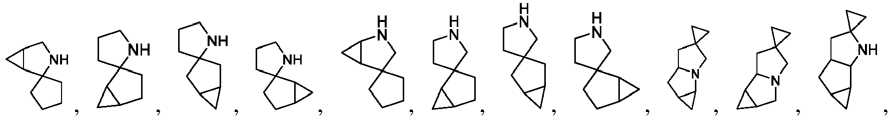 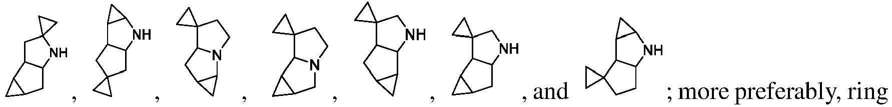 B is selected from the group consisting of piperidinyl, tetrahydropyrrolyl, 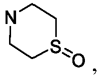 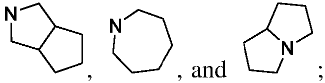 yet more preferably, ring B is selected from the group consisting of piperidinyl, tetrahydropyrrolyl,

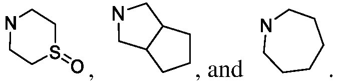

In some embodiments of the present disclosure, provided is the compound represented by general formula (IN), (IN'), (IV'), (IV'-1), (V'), (V'-1), (IVV'), (VV'), or (VV'-1) or the pharmaceutically acceptable salt thereof, wherein ring C is 3- to 8-membered heterocyclyl; preferably, ring C is selected from the group consisting of azetidinyl, morpholinyl, 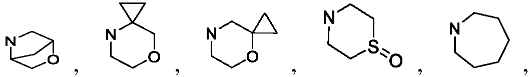 tetrahydropyrrolyl, piperidinyl,

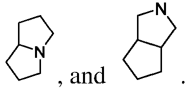

In some embodiments of the present disclosure, provided is the compound represented by general formula (IM) or (IM') or the pharmaceutically acceptable salt thereof, wherein 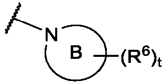 is selected from the group consisting of 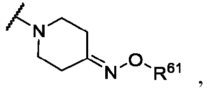 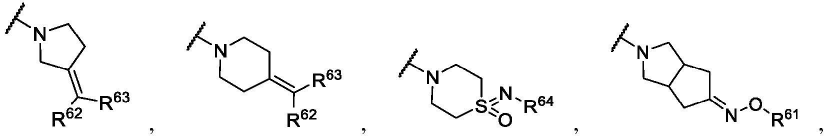 and 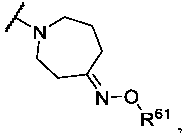 and R⁶¹, R⁶², R⁶³, and R⁶⁴ are as defined in general formula (IV'); preferably, 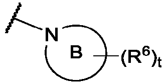 is selected from the group consisting of 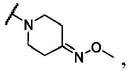 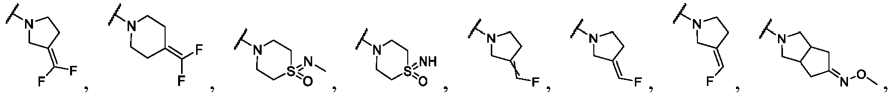 and

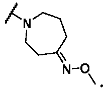

In some embodiments of the present disclosure, provided is the compound represented by general formula (IM) or (IM') or the pharmaceutically acceptable salt thereof, wherein 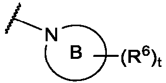 is selected from the group consisting of 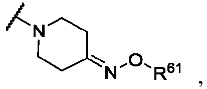 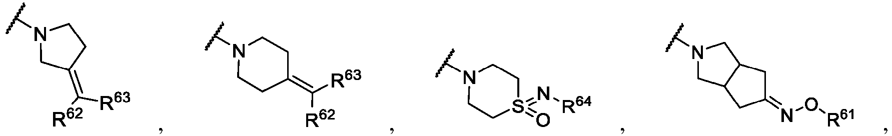 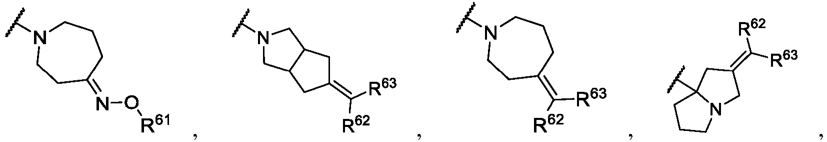 and 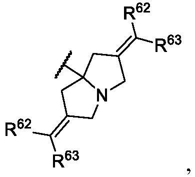 and R⁶¹, R⁶², R⁶³, and R⁶⁴ are as defined in general formula (IV'); preferably, 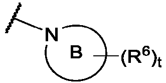 is selected from the group consisting of 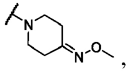 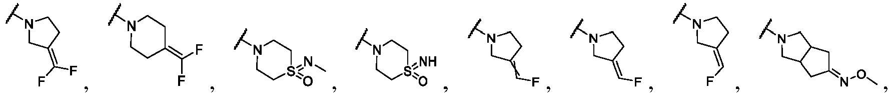 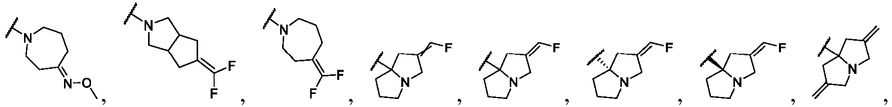 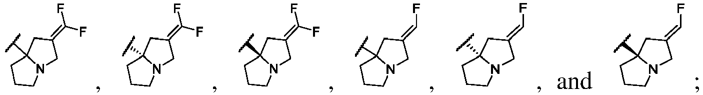 more preferably, 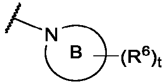 is selected from the group consisting of

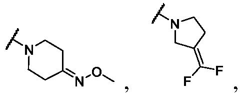

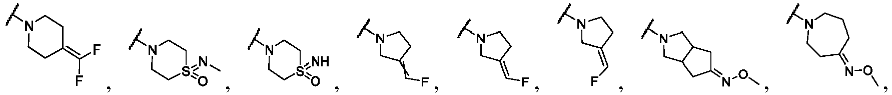

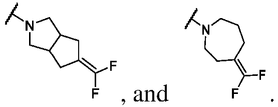

In some embodiments of the present disclosure, provided is the compound represented by general formula (IM) or (IM') or the pharmaceutically acceptable salt thereof, wherein 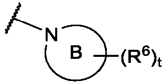 is selected from the group consisting of 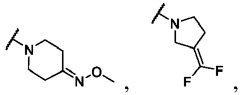 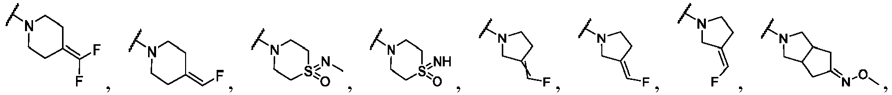 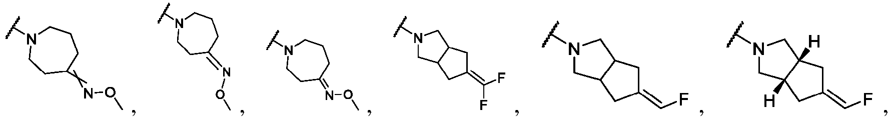 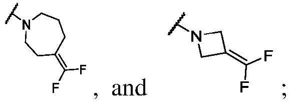 preferably, 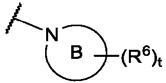 is selected from the group consisting of 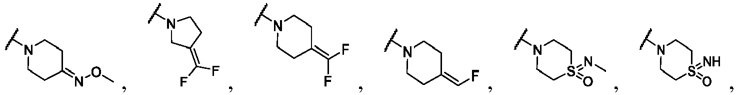 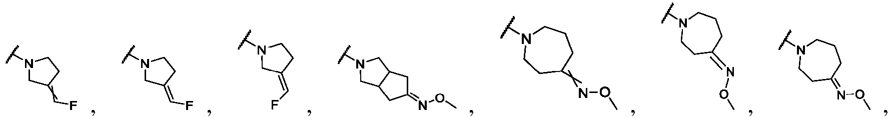 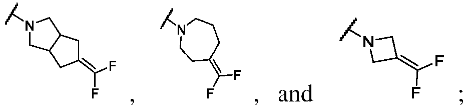 more preferably, 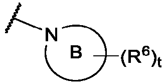 is

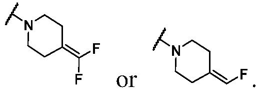

In some embodiments of the present disclosure, provided is the compound represented by general formula (IM) or (IM') or the pharmaceutically acceptable salt thereof, wherein 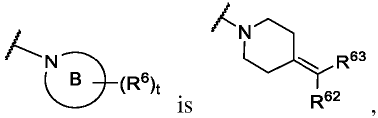 and R⁶² and R⁶³ are identical or different and are each independently a hydrogen atom or halogen; in some embodiments, 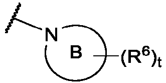 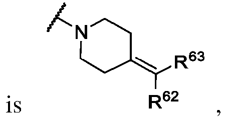 and both R⁶² and R⁶³ are halogens; in some embodiments, 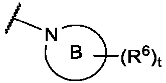 is 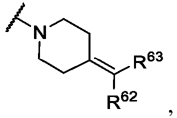 R⁶² is halogen, and R⁶³ is a hydrogen atom.

In some embodiments of the present disclosure, provided is the compound represented by general formula (IV'), (IV'-1), (V'), or (V'-1) or the pharmaceutically acceptable salt thereof, wherein 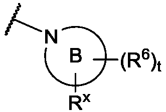 is selected from the group consisting of 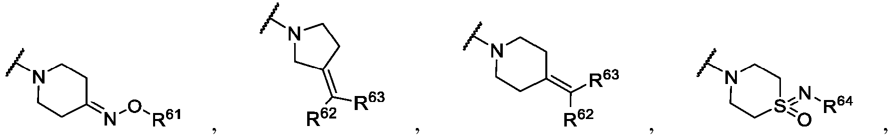 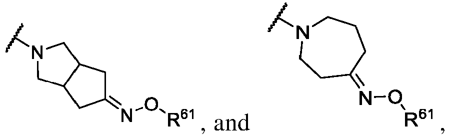 and R⁶¹, R⁶², R⁶³, and R⁶⁴ are as defined in general formula (IV'); preferably, 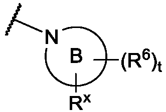 is selected from the group consisting of 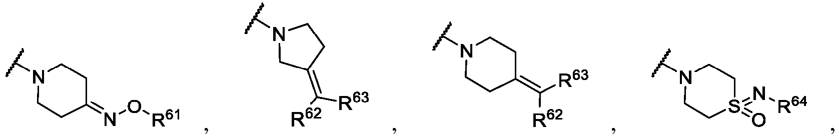 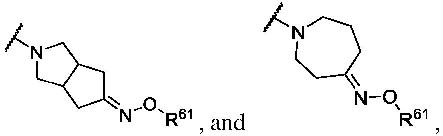 R⁶¹ is C₁₋₆ alkyl, R⁶² and R⁶³ are identical or different and are each independently a hydrogen atom or halogen, and R⁶⁴ is a hydrogen atom or C₁₋₆ alkyl; more preferably, 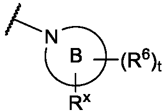 is selected from the group consisting of

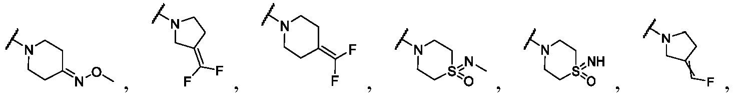

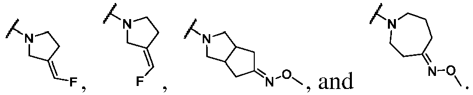

In some embodiments of the present disclosure, provided is the compound represented by general formula (IV'), (IV'-1), (V'), or (V'-1) or the pharmaceutically acceptable salt thereof, wherein 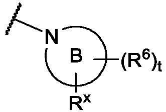 is selected from the group consisting of 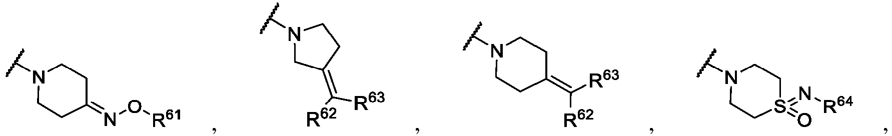 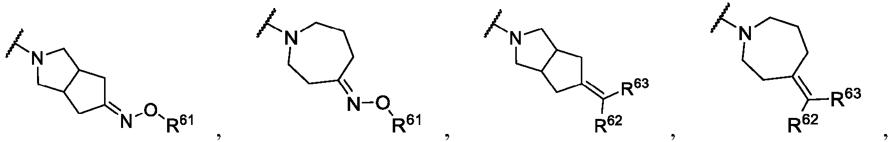 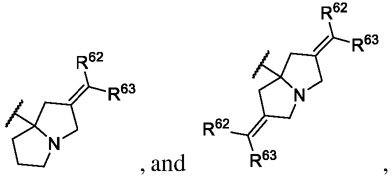 and R⁶¹, R⁶², R⁶³, and R⁶⁴ are as defined in general formula (IV'); preferably, 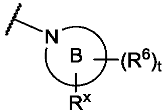 is selected from the group consisting of 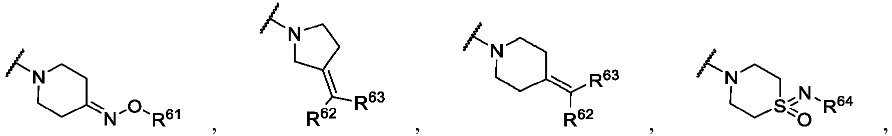 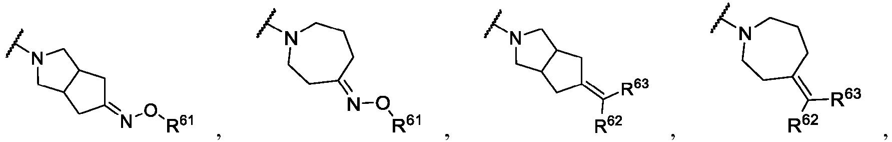 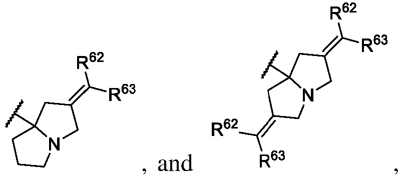 R⁶¹ is C₁₋₆ alkyl, R⁶² and R⁶³ are identical or different and are each independently a hydrogen atom or halogen, and R⁶⁴ is a hydrogen atom or C₁₋₆ alkyl; more preferably, 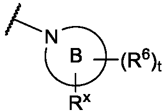 is selected from the group consisting of 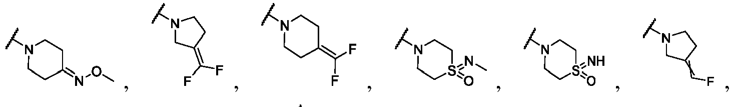 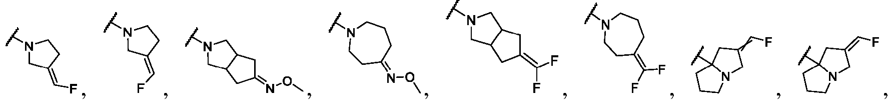 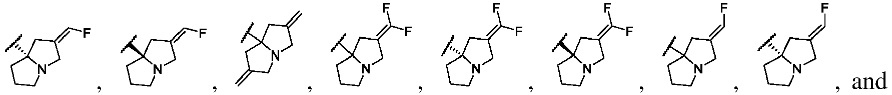 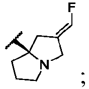 yet more preferably, 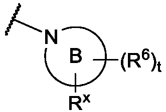 is selected from the group consisting of

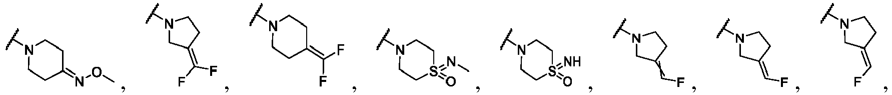

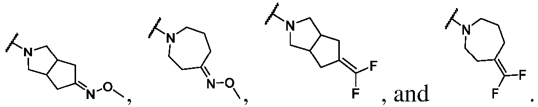

In some embodiments of the present disclosure, provided is the compound represented by general formula (IV'), (IV'-1), (V'), or (V'-1) or the pharmaceutically acceptable salt thereof, wherein 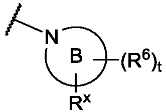 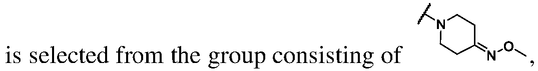 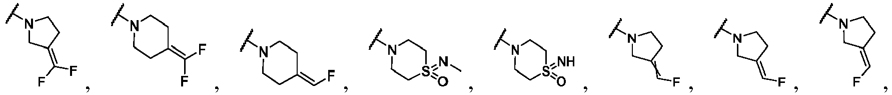 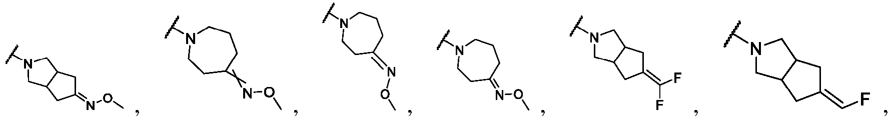 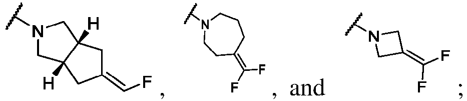 preferably, 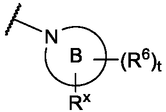 is selected from the group consisting of 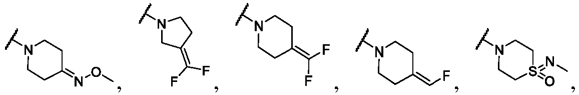 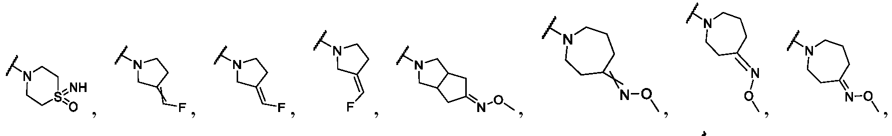 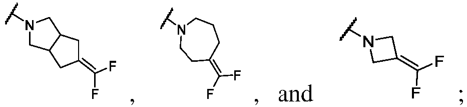 more preferably, 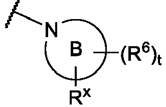 is 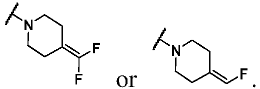 In some embodiments of the present disclosure, provided is the compound represented by general formula (IV'), (IV'-1), (V'), or (V'-1) or the pharmaceutically acceptable salt thereof, wherein 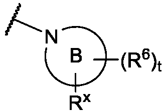 is 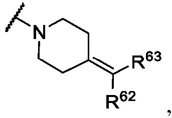 and R⁶² and R⁶³ are identical or different and are each independently a hydrogen atom or halogen; in some embodiments, 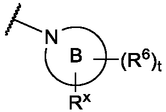 is 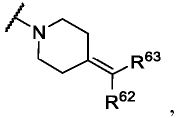 and both R⁶² and R⁶³ are halogens; in some embodiments, 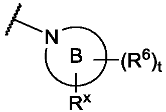 is 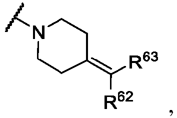 R⁶² is halogen, and R⁶³ is a hydrogen atom.

In some embodiments of the present disclosure, provided is the compound represented by general formula (IN), (IN'), (IVV'), (VV'), or (VV'-1) or the pharmaceutically acceptable salt thereof, wherein 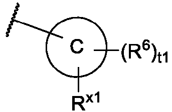 is selected from the group consisting of 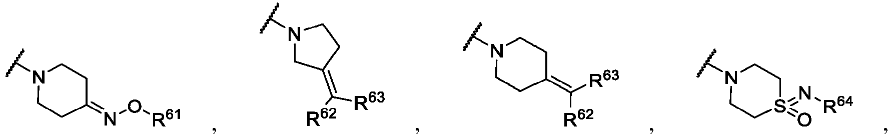 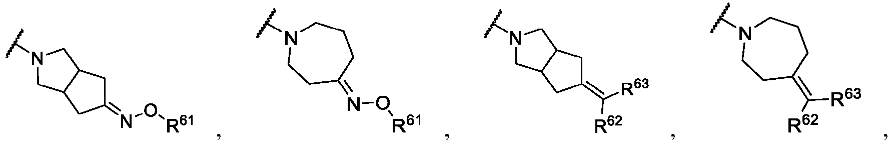 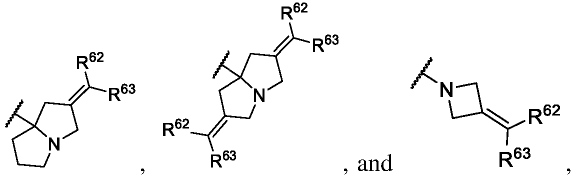 and R⁶¹, R⁶², R⁶³, and R⁶⁴ are as defined in general formula (IN); preferably, 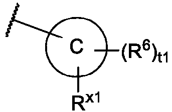 is selected from the group consisting of 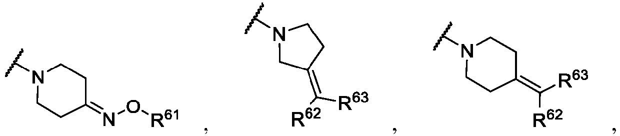 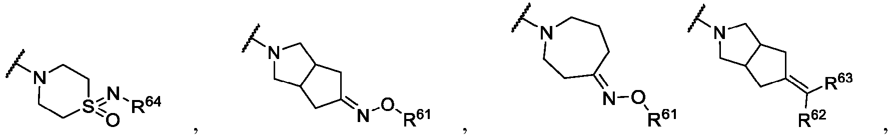 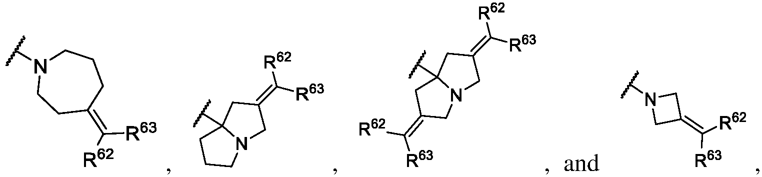 R⁶¹ is C₁₋₆ alkyl, R⁶² and R⁶³ are identical or different and are each independently a hydrogen atom or halogen, and R⁶⁴ is a hydrogen atom or C₁₋₆ alkyl; more preferably, 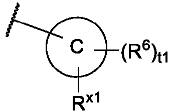 is selected from the group consisting of 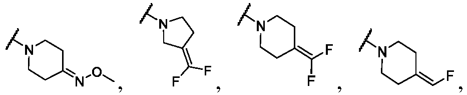 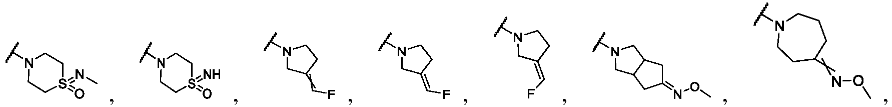 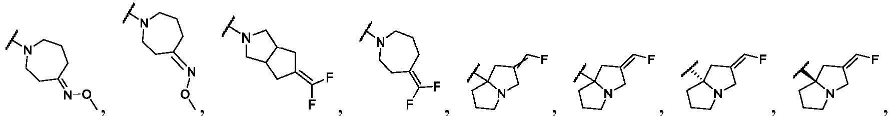 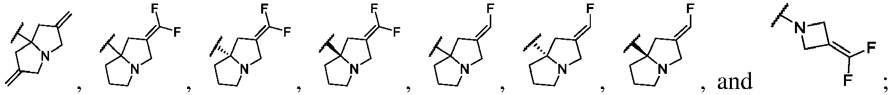 yet more preferably, 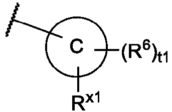 is selected from the group consisting of

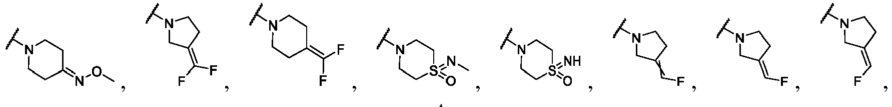

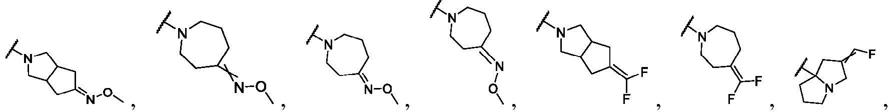

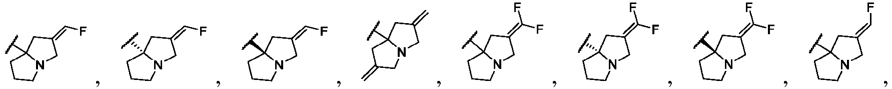

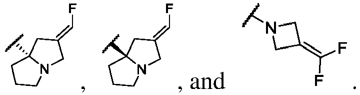

In some embodiments of the present disclosure, provided is the compound represented by general formula (IN), (IN'), or (IVV') or the pharmaceutically acceptable salt thereof, wherein 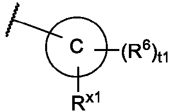 is selected from the group consisting of 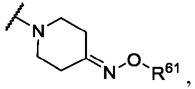 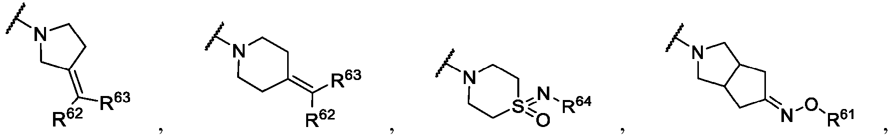 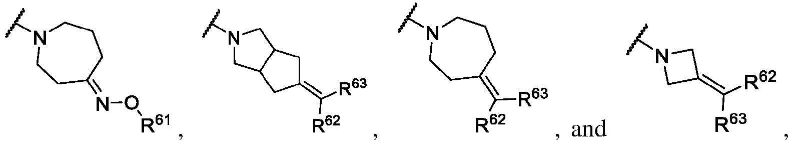 and R⁶¹, R⁶², R⁶³, and R⁶⁴ are as defined in general formula (IN); preferably, 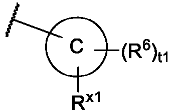 is selected from the group consisting of 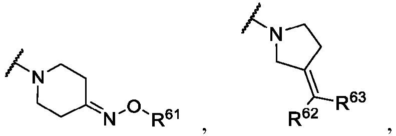 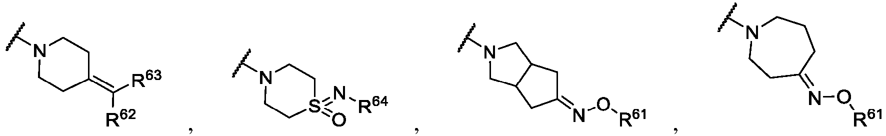 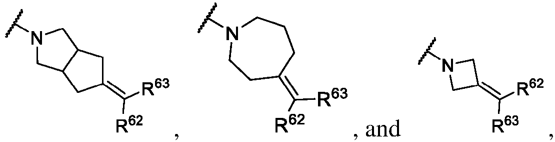 R⁶¹ is C₁₋₆ alkyl, R⁶² and R⁶³ are identical or different and are each independently a hydrogen atom or halogen, and R⁶⁴ is a hydrogen atom or C₁₋₆ alkyl; more preferably, 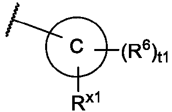 is selected from the group consisting of 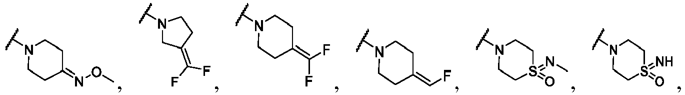 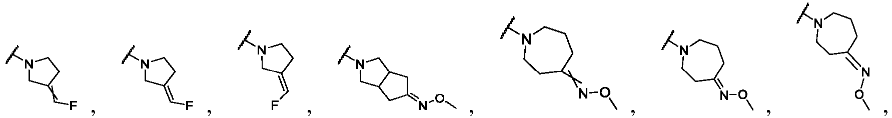 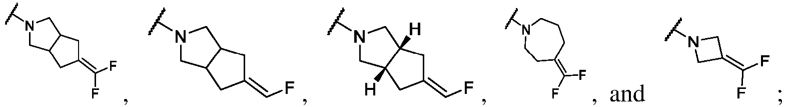 yet more preferably, 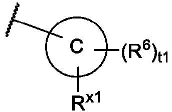 is selected from the group consisting of 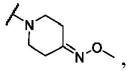 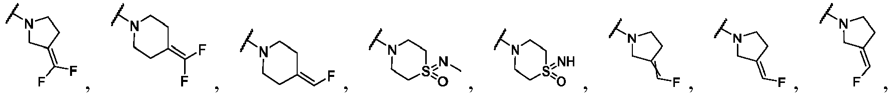 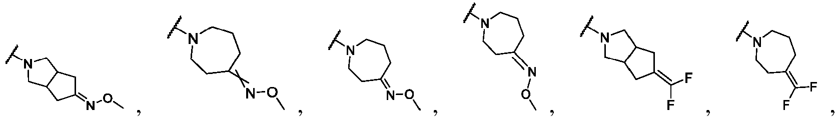 and 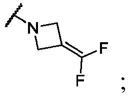 still more preferably, 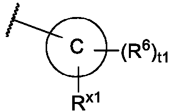 is selected from the group consisting of 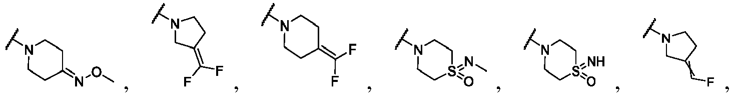 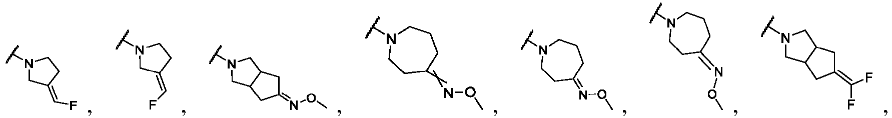 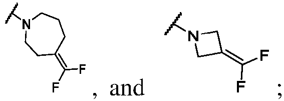 still more preferably, 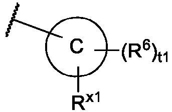 is 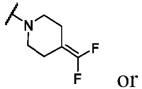 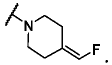 In some embodiments of the present disclosure, provided is the compound represented by general formula (IN), (IN'), or (IVV') or the pharmaceutically acceptable salt thereof, wherein 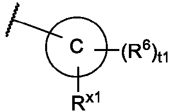 is 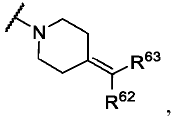 and R⁶² and R⁶³ are identical or different and are each independently a hydrogen atom or halogen; in some embodiments, 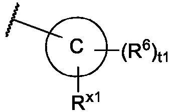 is 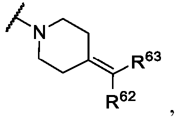 and both R⁶² and R⁶³ are halogens; in some embodiments, 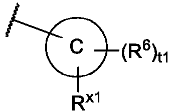 is 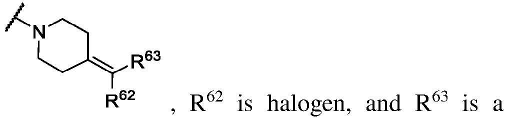 hydrogen atom.

In some embodiments of the present disclosure, provided is the compound represented by general formula (IN), (IN'), (VV'), or (VV'-1) or the pharmaceutically acceptable salt thereof, wherein 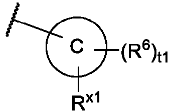 is 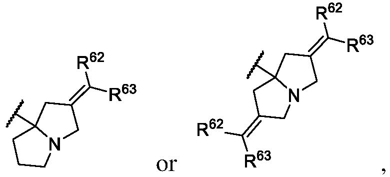 and R⁶² and R⁶³ are as defined in general formula (IN); preferably, 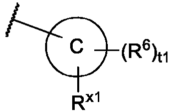 is 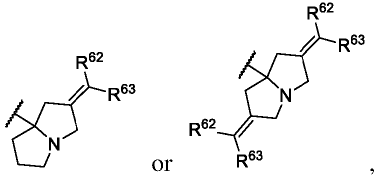 and R⁶² and R⁶³ are identical or different and are each independently a hydrogen atom or halogen; more preferably, 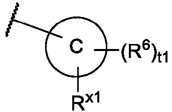 is selected from the group consisting of

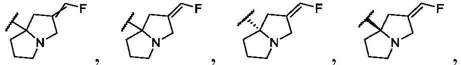

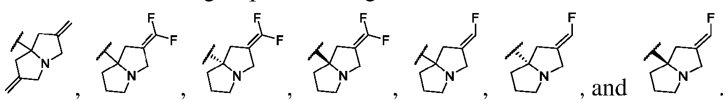

In some embodiments of the present disclosure, provided is the compound represented by general formula (IN), (IN'), (IVV'), (VV'), or (VV'-1) or the pharmaceutically acceptable salt thereof, wherein t1 is 0 or 1; preferably, t1 is 0.

In some embodiments of the present disclosure, provided is the compound represented by general formula (IN), (IN'), (IVV'), (VV'), or (VV'-1) or the pharmaceutically acceptable salt thereof, wherein each R⁶ is identical or different and is independently selected from the group consisting of =N-O-R⁶¹, =CR⁶²R⁶³, =N-R⁶⁴, and oxo, and R⁶¹, R⁶², R⁶³, and R⁶⁴ are as defined in general formula (IN); preferably, each R⁶ is identical or different and is independently selected from the group consisting of =N-O-R⁶¹, =CR⁶²R⁶³, =N-R⁶⁴, and oxo, R⁶¹ is C₁₋₆ alkyl, R⁶² and R⁶³ are identical or different and are each independently a hydrogen atom or halogen, and R⁶⁴ is a hydrogen atom or C₁₋₆ alkyl; more preferably, each R⁶ is identical or different and is independently =CR⁶²R⁶³ or oxo, and R⁶² and R⁶³ are identical or different and are each independently a hydrogen atom or halogen.

In some embodiments of the present disclosure, provided is the compound represented by general formula (IN), (IN'), (IM), (IM'), (IV'), (IV'-1), (V'), (V'-1), (IVV'), (VV'), or (VV'-1) or the pharmaceutically acceptable salt thereof, wherein R⁶¹ is a hydrogen atom or C₁₋₆ alkyl; preferably, R⁶¹ is C₁₋₆ alkyl; more preferably, R⁶¹ is methyl.

In some embodiments of the present disclosure, provided is the compound represented by general formula (IN), (IN'), (IM), (IM'), (IV'), (IV'-1), (V'), (V'-1), (IVV'), (VV'), or (VV'-1) or the pharmaceutically acceptable salt thereof, wherein R⁶² and R⁶³ are identical or different and are each independently a hydrogen atom or halogen; preferably, R⁶² and R⁶³ are identical or different and are each independently a hydrogen atom or F.

In some embodiments of the present disclosure, provided is the compound represented by general formula (IN), (IN'), (IM), (IM'), (IV'), (IV'-1), (V'), (V'-1), (IVV'), (VV'), or (VV'-1) or the pharmaceutically acceptable salt thereof, wherein R⁶⁴ is a hydrogen atom or C₁₋₆ alkyl; preferably, R⁶⁴ is a hydrogen atom or methyl.

In some embodiments of the present disclosure, provided is the compound represented by general formula (I), (I'), (II'), (II), (III'), (III'-1), or (III) or the pharmaceutically acceptable salt thereof, wherein 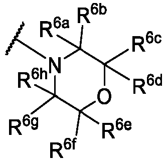 is selected from the group consisting of 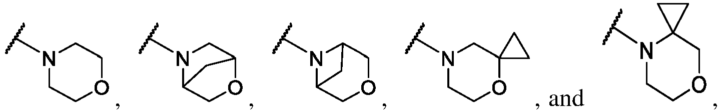 preferably from the group consisting of

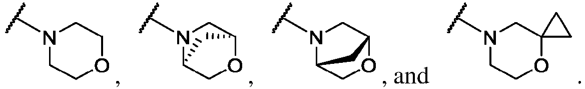

In some embodiments of the present disclosure, provided is the compound represented by general formula (I), (I'), (II'), (II), (III'), (III'-1), or (III) or the pharmaceutically acceptable salt thereof, wherein 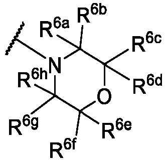 is selected from the group consisting of 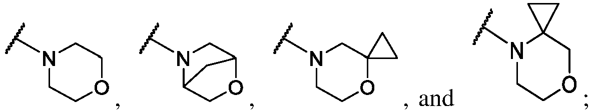 preferably, 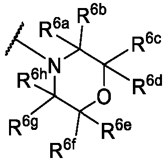 is selected from the group consisting of 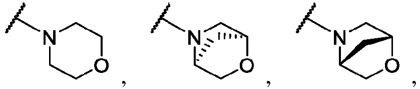 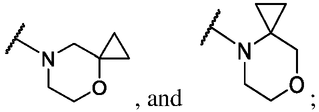 more preferably, 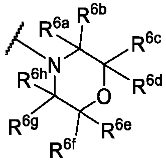 is

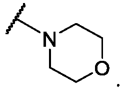

In some embodiments of the present disclosure, provided is the compound represented by general formula (IN), (IN'), (IM), (IM'), (I), (I'), (II'), (III'), (IV'), (V'), (IVV'), or (VV') or the pharmaceutically acceptable salt thereof, wherein R^{G1a} is selected from the group consisting of a hydrogen atom, halogen, C₁₋₆ alkyl, and C₁₋₆ haloalkyl; preferably, R^{G1a} is a hydrogen atom or C₁₋₆ alkyl; more preferably, R^{G1a} is C₁₋₆ alkyl; yet more preferably R^{G1a} is methyl.

In some embodiments of the present disclosure, provided is the compound represented by general formula (IN), (IN'), (IM), (IM'), (I), (I'), (II'), (III'), (III'-1), (IV'), (IV'-1), (V'), (V'-1), (IVV'), (VV'), or (VV'-1) or the pharmaceutically acceptable salt thereof, wherein R^{G1a} is selected from the group consisting of halogen, C₁₋₆ alkyl, and C₁₋₆ haloalkyl; preferably, R^{G1a} is C₁₋₆ alkyl; more preferably, R^{G1a} is methyl.

In some embodiments of the present disclosure, provided is the compound represented by general formula (I), (I'), (II'), (II), (III'), (III'-1), or (III) or the pharmaceutically acceptable salt thereof, wherein R^{6c} and R^{6g}, together with the carbon atoms to which they are attached, form a bridge, and the bridge also has 1, 2, 3, or 4 CH₂ in addition to the bridgehead carbon atoms; preferably, R^{6c} and R^{6g}, together with the carbon atoms to which they are attached, form a bridge, and the bridge also has 1 CH₂ in addition to the bridgehead carbon atoms.

In some embodiments of the present disclosure, provided is the compound represented by general formula (I), (I'), (II'), (II), (III'), (III'-1), or (III) or the pharmaceutically acceptable salt thereof, wherein both R^{6a} and R^{6b} are hydrogen atoms, or R^{6a} and R^{6b}, together with the carbon atoms to which they are attached, form 3- to 6-membered cycloalkyl; preferably, both R^{6a} and R^{6b} are hydrogen atoms, or R^{6a} and R^{6b}, together with the carbon atom to which they are attached, form cyclopropyl.

In some embodiments of the present disclosure, provided is the compound represented by general formula (I), (I'), (II'), (II), (III'), (III'-1), or (III) or the pharmaceutically acceptable salt thereof, wherein both R^{6c} and R^{6d} are hydrogen atoms, or R^{6c} and R^{6d}, together with the carbon atoms to which they are attached, form 3- to 6-membered cycloalkyl; preferably, both R^{6c} and R^{6d} are hydrogen atoms, or R^{6c} and R^{6d}, together with the carbon atom to which they are attached, form cyclopropyl.

In some embodiments of the present disclosure, provided is the compound represented by general formula (I), (I'), (II'), (II), (III'), (III'-1), or (III) or the pharmaceutically acceptable salt thereof, wherein both R^{6e} and R^{6f} are hydrogen atoms.

In some embodiments of the present disclosure, provided is the compound represented by general formula (I), (I'), (II'), (II), (III'), (III'-1), or (III) or the pharmaceutically acceptable salt thereof, wherein both R^{6g} and R^{6h} are hydrogen atoms.

In some embodiments of the present disclosure, provided is the compound represented by general formula (IN), (IN'), (IM), (IM'), (I), (I'), (II'), (II), (III'), (III'-1), (III), (IV'), (IV'-1), (V'), (V'-1), (IVV'), (VV'), or (VV'-1) or the pharmaceutically acceptable salt thereof, wherein Q is N or CH, preferably N.

In some embodiments of the present disclosure, provided is the compound represented by general formula (IN), (IN'), (IM), (IM'), (I), (I'), (II'), (II), (III'), (III'-1), (III), (IV'), (IV'-1), (V'), (V'-1), (IVV'), (VV'), or (VV'-1) or the pharmaceutically acceptable salt thereof, wherein R^{2a} is selected from the group consisting of a hydrogen atom, halogen, C₁₋₆ alkyl, and C₁₋₆ haloalkyl; preferably, R^{2a} is a hydrogen atom or C₁₋₆ alkyl; more preferably, R^{2a} is a hydrogen atom.

In some embodiments of the present disclosure, provided is the compound represented by general formula (IN), (IN'), (IM), (IM'), (I), (I'), (II'), or (II) or the pharmaceutically acceptable salt thereof, wherein ring A is 6- to 10-membered aryl or 5- to 10-membered heteroaryl; preferably, ring A is selected from the group consisting of naphthyl, phenyl, pyridinyl, benzothienyl, benzothiazolyl, and benzopyrazolyl; more preferably, ring A is selected from the group consisting of naphthyl, phenyl, and benzothienyl; yet more preferably, ring A is phenyl or naphthyl; still more preferably, ring A is naphthyl.

In some embodiments of the present disclosure, provided is the compound represented by general formula (IN), (IM), (I), (II'), or (II) or the pharmaceutically acceptable salt thereof, wherein 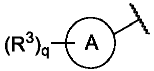 is selected from the group consisting of 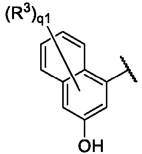 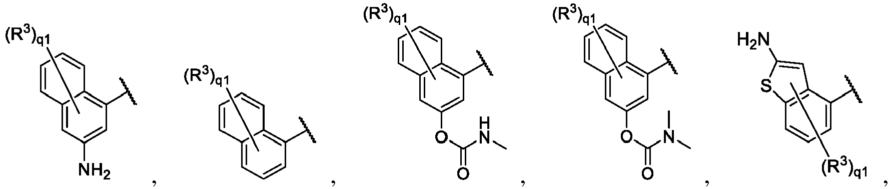 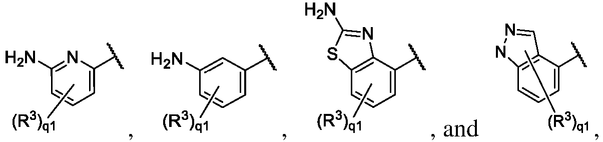 q1 is 0, 1, 2, 3, or 4, and R³ is as defined in general formula (IN); preferably, 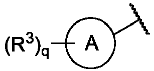 is selected from the group consisting of 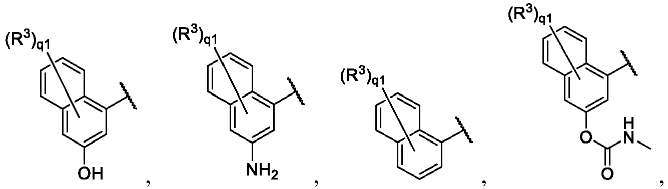 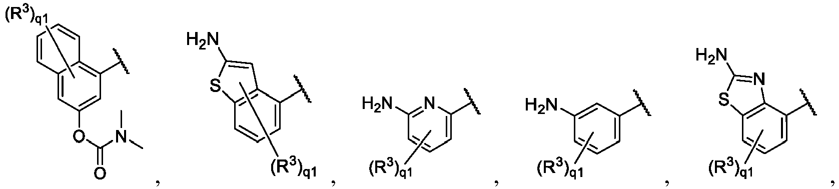 and 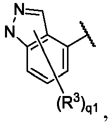 q1 is 2 or 3, and each R³ is identical or different and is independently selected from the group consisting of halogen, C₁₋₆ alkyl, C₁₋₆ haloalkyl, and cyano; more preferably, 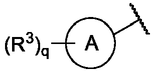 is selected from the group consisting of 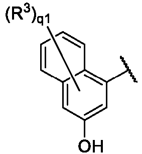 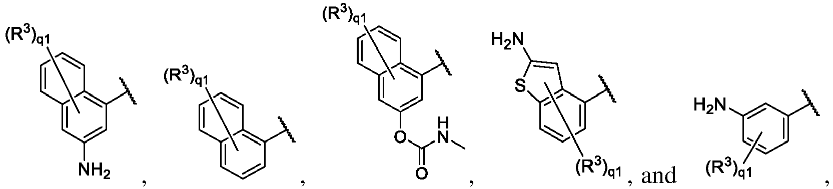 q1 is 2 or 3, and each R³ is identical or different and is independently selected from the group consisting of halogen, C₁₋₆ alkyl, C₁₋₆ haloalkyl, and cyano; in some embodiments, 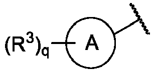 is selected from the group consisting of 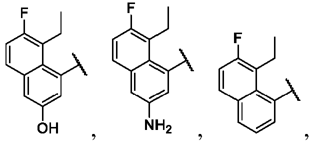 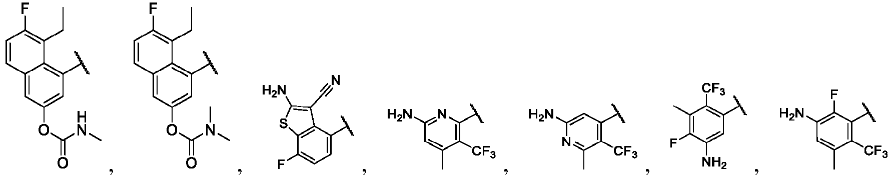 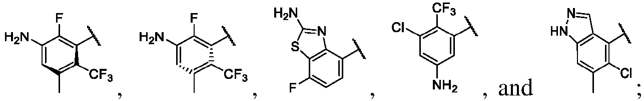 in some embodiments, 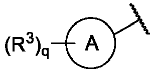 is selected from the group consisting of 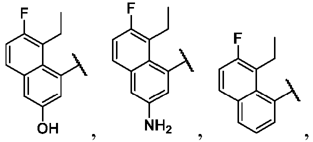 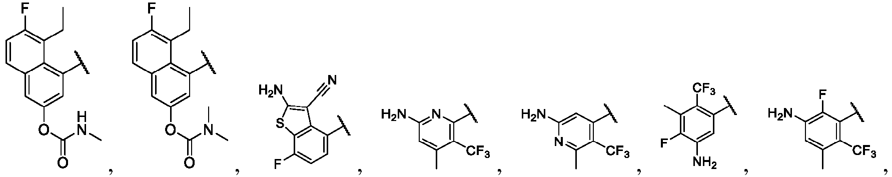 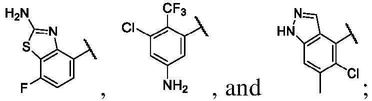 in some embodiments, 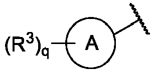 is selected from the group consisting of 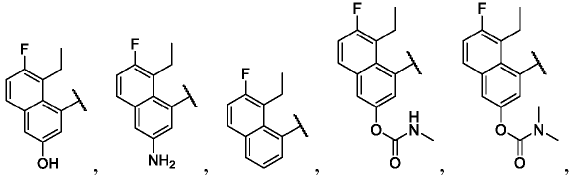 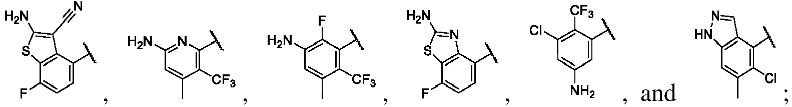 in some embodiments, 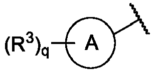 is selected from the group consisting of 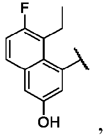 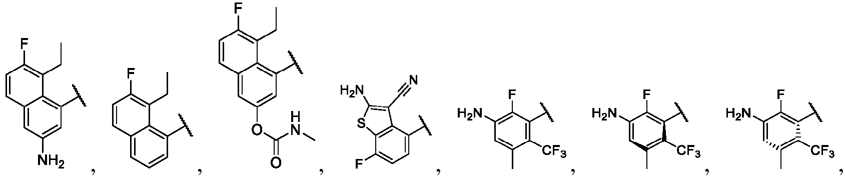 and 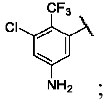 in some embodiments, 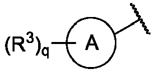 is selected from the group consisting of

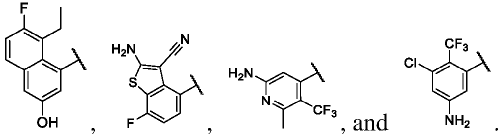

In some embodiments of the present disclosure, provided is the compound represented by general formula (IN), (IN'), (IM), (IM'), (I), (I'), (II'), (II), (III'), (III'-1), (III), (IV'), (IV'-1), (V'), (V'-1), (IVV'), (VV'), or (VV'-1) or the pharmaceutically acceptable salt thereof, wherein R^{4a} is selected from the group consisting of a hydrogen atom, halogen, C₁₋₆ alkyl, and C₁₋₆ haloalkyl; preferably, R^{4a} is a hydrogen atom or halogen; more preferably, R^{4a} is halogen; yet more preferably, R^{4a} is F.

In some embodiments of the present disclosure, provided is the compound represented by general formula (IN), (IN'), (IM), (IM'), (I), (I'), (II'), (II), (III'), (III'-1), (III), (IV'), (IV'-1), (V'), (V'-1), (IVV'), (VV'), or (VV'-1) or the pharmaceutically acceptable salt thereof, wherein R^{d} is a hydrogen atom or C₁₋₆ alkyl; preferably, R^{d} is a hydrogen atom.

In some embodiments of the present disclosure, provided is the compound represented by general formula (IN), (IN'), (IM), (IM'), (I), (I'), (II'), (II), (III'), (III'-1), (III), (IV'), (IV'-1), (V'), (V'-1), (IVV'), (VV'), or (VV'-1) or the pharmaceutically acceptable salt thereof, wherein G² is NH.

In some embodiments of the present disclosure, provided is the compound represented by general formula (IN), (IN'), (IM), (IM'), (I), (I'), (II'), (II), (III'), (III'-1), (III), (IV'), (IV'-1), (V'), (V'-1), (IVV'), (VV'), or (VV'-1) or the pharmaceutically acceptable salt thereof, wherein L is selected from the group consisting of CH₂, NH, and O; preferably, L is O. In some embodiments of the present disclosure, provided is the compound represented by general formula (IN), (IN'), (IM), (IM'), (I), (I'), (II'), (II), (III'), (III'-1), (III), (IV'), (IV'-1), (V'), (V'-1), (IVV'), (VV'), or (VV'-1) or the pharmaceutically acceptable salt thereof, wherein R^{e} is a hydrogen atom or C₁₋₆ alkyl; preferably, R^{e} is a hydrogen atom.

In some embodiments of the present disclosure, provided is the compound represented by general formula (IN), (IN'), (IM), (IM'), (I), (I'), (II'), (II), (III'), (III'-1), (III), (IV'), (IV'-1), (V'), (V'-1), (IVV'), (VV'), or (VV'-1) or the pharmaceutically acceptable salt thereof, wherein each R¹ is identical or different and is independently selected from the group consisting of halogen, C₁₋₆ alkyl, C₁₋₆ haloalkyl, cyano, amino, -(CH₂)ᵤ-NR^{f}R^{g}, hydroxy, and C₁₋₆ hydroxyalkyl, R^{f} and R^{g} are identical or different and are each independently a hydrogen atom or C₁₋₆ alkyl, and u is 0 or 1; preferably, each R¹ is identical or different and is independently selected from the group consisting of halogen, C₁₋₆ alkyl, and C₁₋₆ haloalkyl.

In some embodiments of the present disclosure, provided is the compound represented by general formula (IN), (IN'), (IM), (IM'), (I), (I'), (II'), (II), (III'), (III'-1), (III), (IV'), (IV'-1), (V'), (V'-1), (IVV'), (VV'), or (VV'-1) or the pharmaceutically acceptable salt thereof, wherein p is 0 or 1, preferably 0.

In some embodiments of the present disclosure, provided is the compound represented by general formula (IN), (IN'), (IM), (IM'), (I), (I'), (II'), or (II) or the pharmaceutically acceptable salt thereof, wherein each R³ is identical or different and is independently selected from the group consisting of halogen, C₁₋₆ alkyl, C₂₋₆ alkenyl, C₂₋₆ alkynyl, C₁₋₆ alkoxy, C₁₋₆ haloalkyl, C₁₋₆ haloalkoxy, cyano, amino, -(CH₂)_{w}-NR^{j}R^{k}, hydroxy, and C₁₋₆ hydroxyalkyl, R^{j} and R^{k} are identical or different and are each independently a hydrogen atom or C₁₋₆ alkyl, and w is 0 or 1; preferably, each R³ is identical or different and is independently selected from the group consisting of halogen, C₁₋₆ alkyl, C₂₋₆ alkynyl, C₁₋₆ haloalkyl, hydroxy, -O-C(O)NHC₁₋₆ alkyl, amino, cyano, C₁₋₆ hydroxyalkyl, and 3- to 8-membered cycloalkyl; more preferably, each R³ is identical or different and is independently selected from the group consisting of halogen, C₁₋₆ alkyl, C₂₋₆ alkynyl, C₁₋₆ haloalkyl, hydroxy, C₁₋₆ hydroxyalkyl, and 3- to 8-membered cycloalkyl; yet more preferably, each R³ is identical or different and is independently selected from the group consisting of halogen, C₁₋₆ alkyl, C₂₋₆ alkynyl, C₁₋₆ haloalkyl, hydroxy, and cyclopropyl; still more preferably, each R³ is identical or different and is independently selected from the group consisting of halogen, C₁₋₆ alkyl, and hydroxy; most preferably, each R³ is identical or different and is independently selected from the group consisting of F, ethyl, and hydroxy; in some embodiments, each R³ is identical or different and is independently selected from the group consisting of halogen, C₁₋₆ alkyl, C₁₋₆ haloalkyl, hydroxy, -O-C(O)NHC₁₋₆ alkyl, amino, and cyano; in some embodiments, each R³ is identical or different and is independently selected from the group consisting of halogen, C₁₋₆ alkyl, C₁₋₆ haloalkyl, and cyano.

In some embodiments of the present disclosure, provided is the compound represented by general formula (IN), (IM), (I), (II'), or (II) or the pharmaceutically acceptable salt thereof, wherein q is 2, 3, or 4; preferably, q is 2 or 3; more preferably, q is 3.

In some embodiments of the present disclosure, provided is the compound represented by general formula (IN), (IM), (I), (II'), or (II) or the pharmaceutically acceptable salt thereof, wherein q1 is 2 or 3, preferably 2.

In some embodiments of the present disclosure, provided is the compound represented by general formula (III'), (III'-1), (III), (IV'), (IV'-1), (V'), (V'-1), (IVV'), (VV'), or (VV'-1) or the pharmaceutically acceptable salt thereof, wherein R^{3a} is selected from the group consisting of a hydrogen atom, halogen, C₁₋₆ alkyl, C₂₋₆ alkynyl, C₁₋₆ haloalkyl, C₁₋₆ hydroxyalkyl, and 3- to 8-membered cycloalkyl; preferably, R^{3a} is selected from the group consisting of a hydrogen atom, halogen, and C₁₋₆ alkyl; more preferably, R^{3a} is halogen; yet more preferably, R^{3a} is F.

In some embodiments of the present disclosure, provided is the compound represented by general formula (III'), (III'-1), (III), (IV'), (IV'-1), (V'), (V'-1), (IVV'), (VV'), or (VV'-1) or the pharmaceutically acceptable salt thereof, wherein R^{3b} is selected from the group consisting of a hydrogen atom, halogen, C₁₋₆ alkyl, C₂₋₆ alkynyl, C₁₋₆ haloalkyl, C₁₋₆ hydroxyalkyl, and 3- to 8-membered cycloalkyl; preferably, R^{3b} is selected from the group consisting of a hydrogen atom, halogen, and C₁₋₆ alkyl; more preferably, R^{3b} is C₁₋₆ alkyl; yet more preferably, R^{3b} is ethyl.

In some embodiments of the present disclosure, provided is the compound represented by general formula (IM) or the pharmaceutically acceptable salt thereof, wherein G⁰ is O; G¹ is CR^{G1a}H, and R^{G1a} is C₁₋₆ alkyl; T is a single bond; Q is N; R^{4a} is halogen; G² is NH; L is O; p is 0; 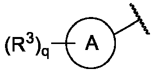 is selected from the group consisting of 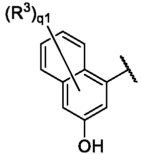 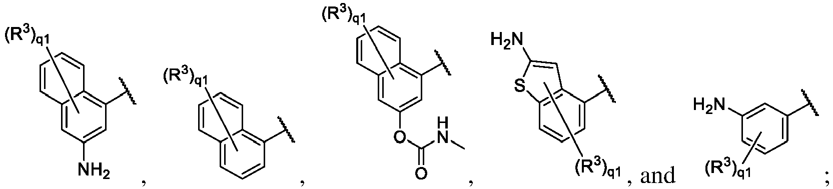 q1 is 2 or 3; each R³ is identical or different and is independently selected from the group consisting of halogen, C₁₋₆ alkyl, C₁₋₆ haloalkyl, and cyano; 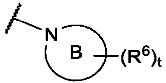 is selected from the group consisting of 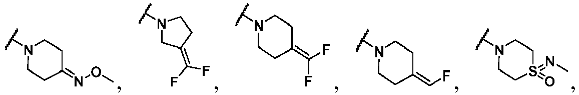 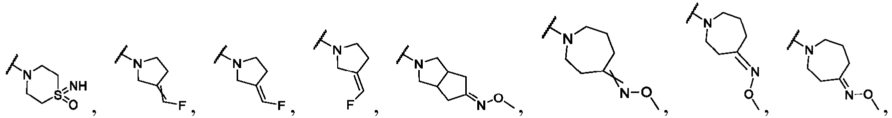 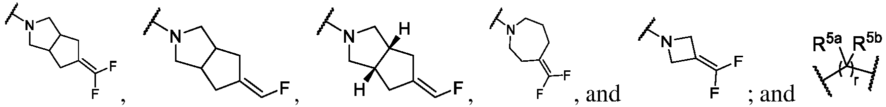 is

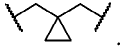

In some embodiments of the present disclosure, provided is the compound represented by general formula (IM) or the pharmaceutically acceptable salt thereof, wherein G⁰ is O; G¹ is CR^{G1a}H, and R^{G1a} is C₁₋₆ alkyl; T is a single bond; Q is N; R^{4a} is halogen; G² is NH; L is O; 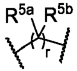 is 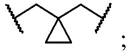 p is 0; 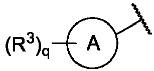 is selected from the group consisting of 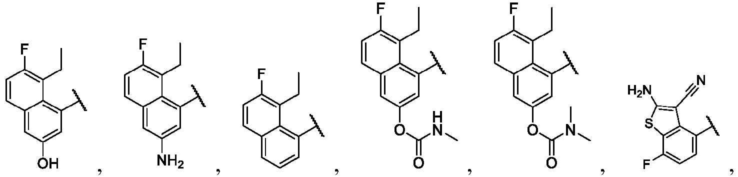 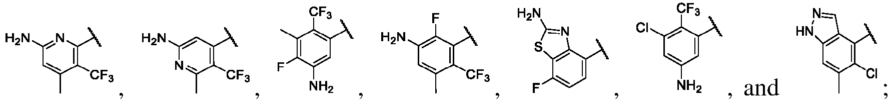 and 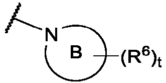 is selected from the group consisting of

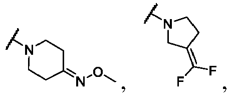

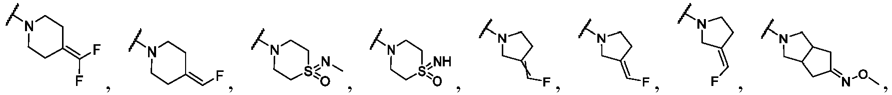

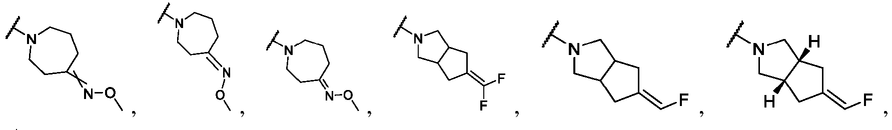

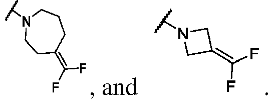

In some embodiments of the present disclosure, provided is the compound represented by general formula (IM) or the pharmaceutically acceptable salt thereof, wherein G⁰ is O; G¹ is CR^{G1a}H, and R^{G1a} is methyl; T is a single bond; Q is N; R^{4a} is halogen; G² is NH; L is O; 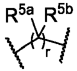 is 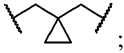 p is 0; 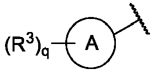 is selected from the group consisting 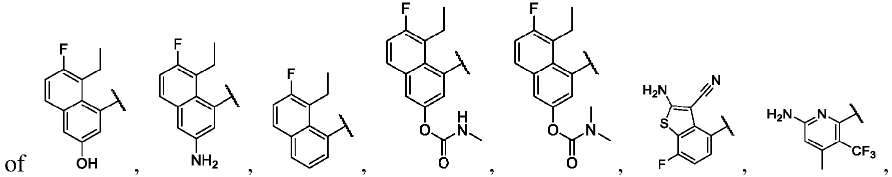 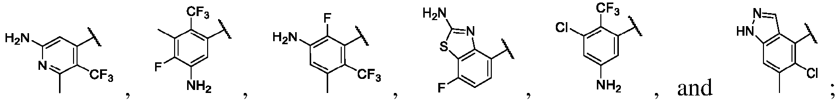 and 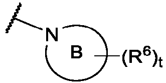 is

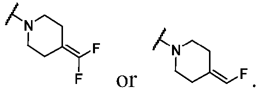

In some embodiments of the present disclosure, provided is the compound represented by general formula (IM) or the pharmaceutically acceptable salt thereof, wherein G⁰ is O; G¹ is CR^{G1a}H, and R^{G1a} is methyl; T is a single bond; Q is N; R^{4a} is halogen; G² is NH; L is O; 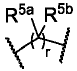 is 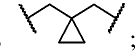 p is 0; 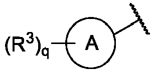 is selected from the group consisting of 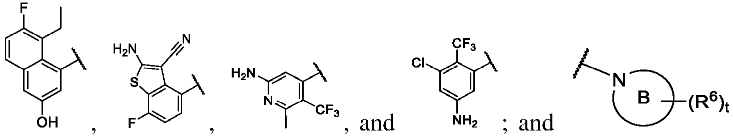 is selected from the group consisting of

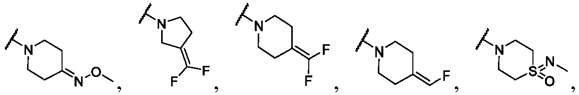

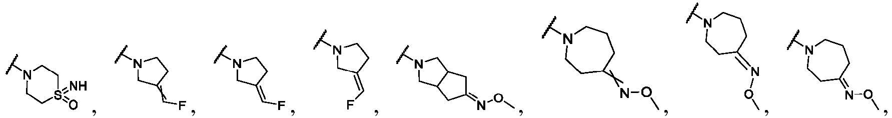

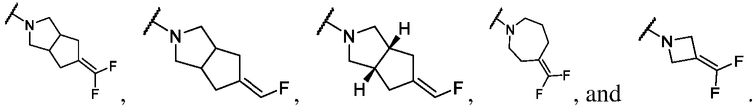

In some embodiments of the present disclosure, provided is the compound represented by general formula (IM) or the pharmaceutically acceptable salt thereof, wherein G⁰ is O; G¹ is CR^{G1a}H, and R^{G1a} is methyl; T is a single bond; Q is N; R^{4a} is halogen; G² is NH; L is O; 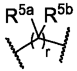 is 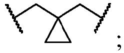 p is 0; 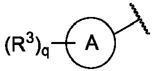 is selected from the group consisting of 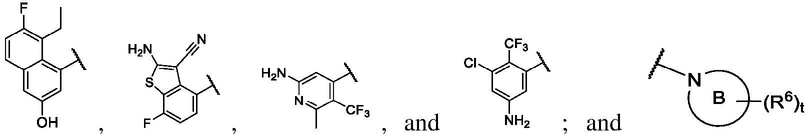 is

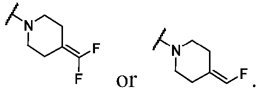

In some embodiments of the present disclosure, provided is the compound represented by general formula (IN) or the pharmaceutically acceptable salt thereof, wherein G⁰ is O; G¹ is CR^{G1a}H, and R^{G1a} is C₁₋₆ alkyl; T is a single bond; Q is N; R^{4a} is halogen; G² is NH; L is O; 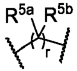 is 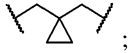 p is 0; 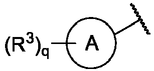 is selected from the group consisting of 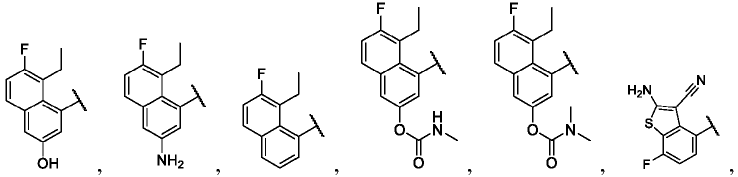 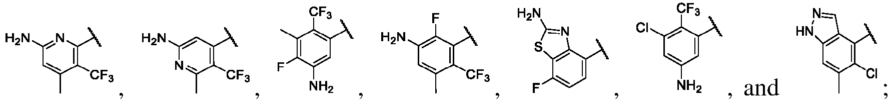 and 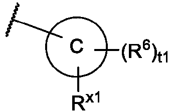 is selected from the group consisting of

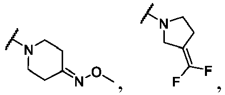

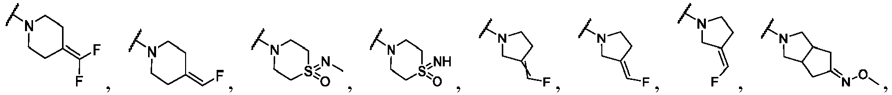

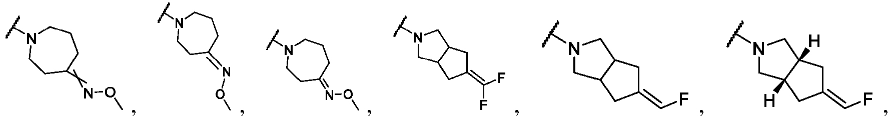

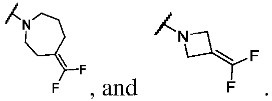

In some embodiments of the present disclosure, provided is the compound represented by general formula (IN) or the pharmaceutically acceptable salt thereof, wherein G⁰ is O; G¹ is CR^{G1a}H, and R^{G1a} is methyl; T is a single bond; Q is N; R^{4a} is halogen; G² is NH; L is O; 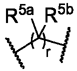 is 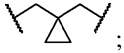 p is 0; 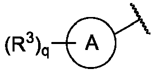 is selected from the group consisting 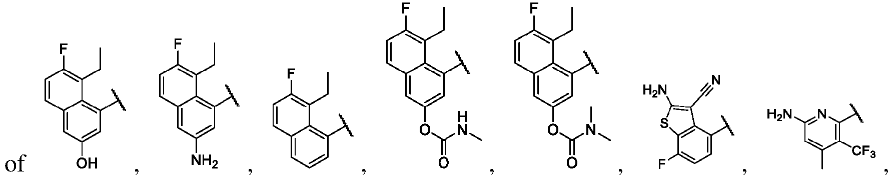 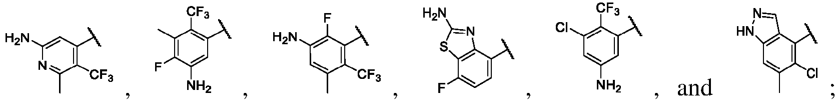 and 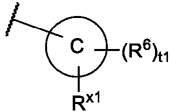 is

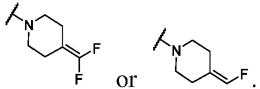

In some embodiments of the present disclosure, provided is the compound represented by general formula (IN) or the pharmaceutically acceptable salt thereof, wherein G⁰ is O; G¹ is CR^{G1a}H, and R^{G1a} is methyl; T is a single bond; Q is N; R^{4a} is halogen; G² is NH; L is O; 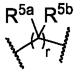 is 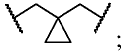 p is 0; 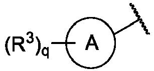 is selected from the group consisting of 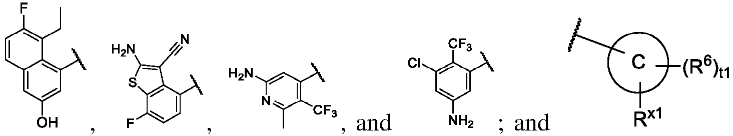 is selected from the group consisting of

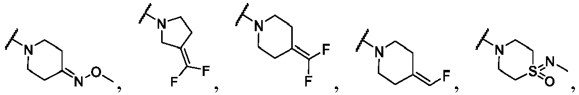

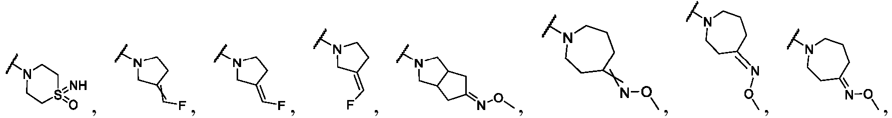

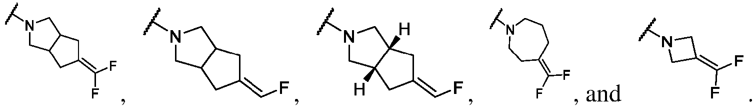

In some embodiments of the present disclosure, provided is the compound represented by general formula (IN) or the pharmaceutically acceptable salt thereof, wherein G⁰ is O; G¹ is CR^{G1a}H, and R^{G1a} is methyl; T is a single bond; Q is N; R^{4a} is halogen; G² is NH; L is O; 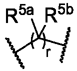 is 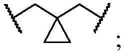 p is 0; 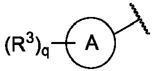 is selected from the group consisting of 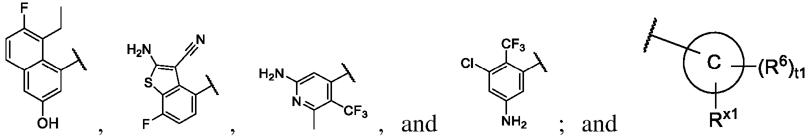 is

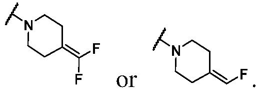

In some embodiments of the present disclosure, provided is the compound represented by general formula (II') or the pharmaceutically acceptable salt thereof, wherein R^{G1a} is C₁₋₆ alkyl; 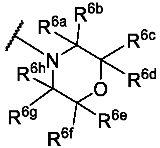 is selected from the group consisting of 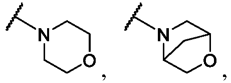 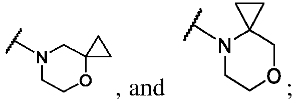 Q is N; ring A is phenyl or naphthyl; R^{4a} is halogen; G² is NH; L is O; p is 0; q is 2 or 3; and each R³ is identical or different and is independently selected from the group consisting of halogen, C₁₋₆ alkyl, and hydroxy.

In some embodiments of the present disclosure, provided is the compound represented by general formula (II) or the pharmaceutically acceptable salt thereof, wherein 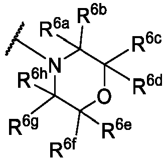 is selected from the group consisting of 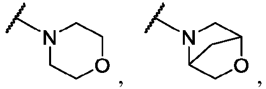 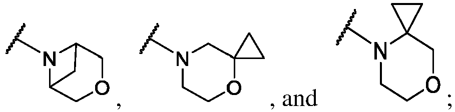 Q is N; ring A is phenyl or naphthyl; R^{4a} is halogen; G² is NH; L is O; p is 0; q is 2 or 3; and each R³ is identical or different and is independently selected from the group consisting of halogen, C₁₋₆ alkyl, and hydroxy.

In some embodiments of the present disclosure, provided is the compound represented by general formula (III') or the pharmaceutically acceptable salt thereof, wherein R^{G1a} is methyl; 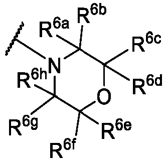 is selected from the group consisting of 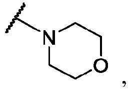 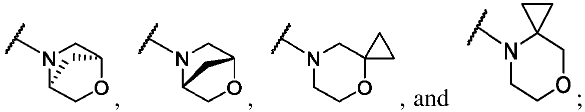 Q is N; R^{4a} is halogen; G² is NH; L is O; p is 0; R^{3a} is halogen; and R^{3b} is C₁₋₆ alkyl.

In some embodiments of the present disclosure, provided is the compound represented by general formula (III'-1) or the pharmaceutically acceptable salt thereof, wherein R^{G1a} is methyl; 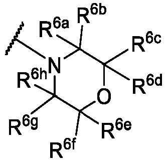 is selected from the group consisting of 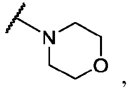 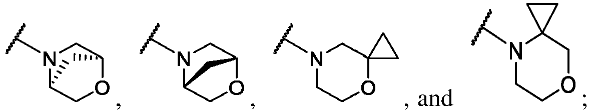 Q is N; R^{4a} is halogen; G² is NH; L is O; p is 0; R^{3a} is halogen; and R^{3b} is C₁₋₆ alkyl.

In some embodiments of the present disclosure, provided is the compound represented by general formula (III'-1) or the pharmaceutically acceptable salt thereof, wherein R^{G1a} is methyl; 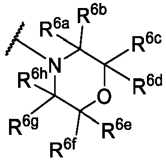 is 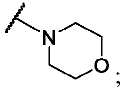 Q is N; R^{4a} is halogen; G² is NH; L is O; p is 0; R^{3a} is halogen; and R^{3b} is C₁₋₆ alkyl.

In some embodiments of the present disclosure, provided is the compound represented by general formula (III) or the pharmaceutically acceptable salt thereof, wherein 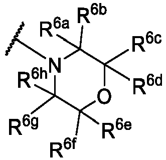 is selected from the group consisting of 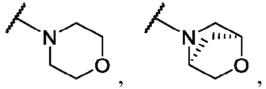 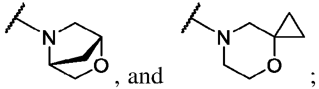 Q is N; R^{4a} is halogen; G² is NH; L is O; p is 0; R^{3a} is halogen; and R^{3b} is C₁₋₆ alkyl. In some embodiments of the present disclosure, provided is the compound represented by general formula (IV') or the pharmaceutically acceptable salt thereof, wherein R^{G1a} is methyl; 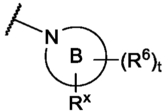 is selected from the group consisting of 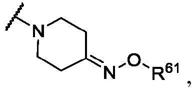 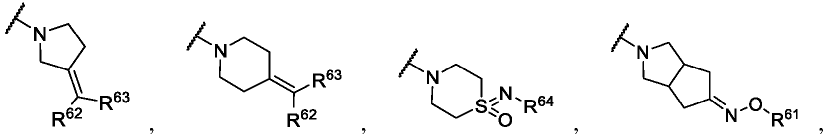 and 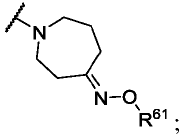 R⁶¹ is C₁₋₆ alkyl; R⁶² and R⁶³ are identical or different and are each independently a hydrogen atom or halogen; R⁶⁴ is a hydrogen atom or C₁₋₆ alkyl; Q is N; R^{4a} is halogen; G² is NH; L is O; p is 0; R^{3a} is halogen; and R^{3b} is C₁₋₆ alkyl.

In some embodiments of the present disclosure, provided is the compound represented by general formula (IV'-1) or the pharmaceutically acceptable salt thereof, wherein R^{G1a} is methyl; 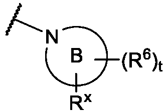 is selected from the group consisting of 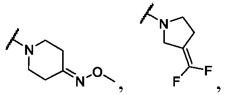 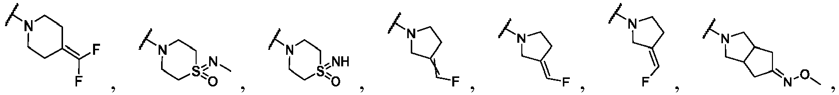 and 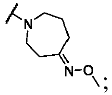 Q is N; R^{4a} is halogen; G² is NH; L is O; p is 0; R^{3a} is halogen; and R^{3b} is C₁₋₆ alkyl.

In some embodiments of the present disclosure, provided is the compound represented by general formula (IV'-1) or the pharmaceutically acceptable salt thereof, wherein R^{G1a} is methyl; 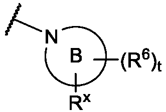 is selected from the group consisting of 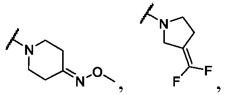 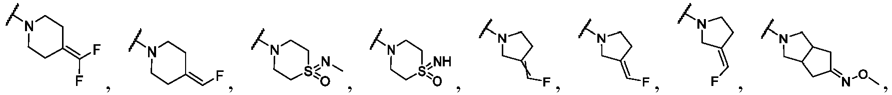 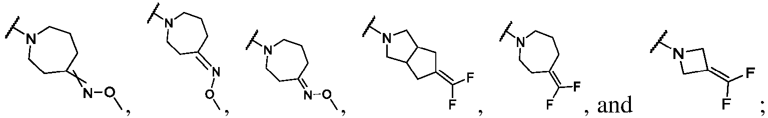 Q is N; R^{4a} is halogen; G² is NH; L is O; p is 0; R^{3a} is halogen; and R^{3b} is C₁₋₆ alkyl.

In some embodiments of the present disclosure, provided is the compound represented by general formula (IV') or (V') or the pharmaceutically acceptable salt thereof, wherein R^{G1a} is methyl; 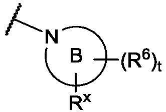 is selected from the group consisting of 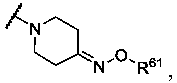 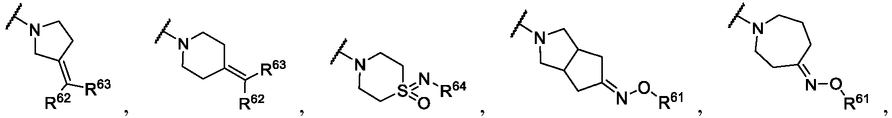 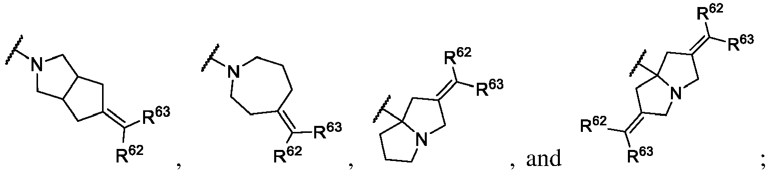 R⁶¹ is C₁₋₆ alkyl; R⁶² and R⁶³ are identical or different and are each independently a hydrogen atom or halogen; R⁶⁴ is a hydrogen atom or C₁₋₆ alkyl; Q is N; R^{4a} is halogen; G² is NH; L is O; p is 0; R^{3a} is halogen; and R^{3b} is C₁₋₆ alkyl.

In some embodiments of the present disclosure, provided is the compound represented by general formula (IV'-1) or (V'-1) or the pharmaceutically acceptable salt thereof, wherein R^{G1a} is methyl; 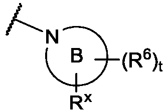 is selected from the group consisting of 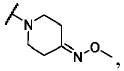 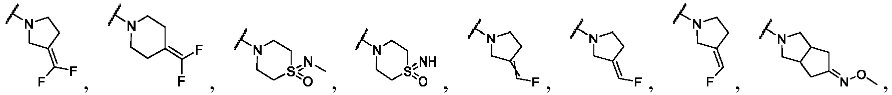 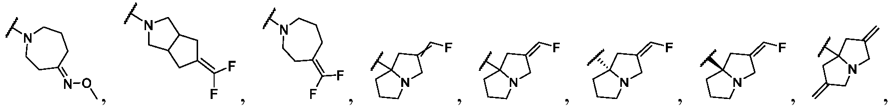 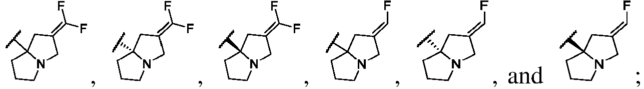 Q is N; R^{4a} is halogen; G² is NH; L is O; p is 0; R^{3a} is halogen; and R^{3b} is C₁₋₆ alkyl.

In some embodiments of the present disclosure, provided is the compound represented by general formula (IN) or the pharmaceutically acceptable salt thereof, wherein Q is N; G° is O; G¹ is CR^{G1a}H; R^{G1a} is a hydrogen atom or C₁₋₆ alkyl; G² is NH; T is a chemical bond; R^{4a} is halogen; L is O; ring A is naphthyl; each R³ is identical or different and is independently selected from the group consisting of halogen, C₁₋₆ alkyl, and hydroxy; q is 2 or 3; R^{5a} and R^{5b} are hydrogen atoms, or R^{5a} and R^{5b}, together with the carbon atom to which they are attached, form cyclopropyl; r is 1 or 3; ring C is 3- to 8-membered heterocyclyl; R⁶ is =CR⁶²R⁶³ or oxo; R^{x1} is selected from the group consisting of =N-OR⁶¹, =CR⁶²R⁶³, and =N-R⁶⁴; R⁶¹ is C₁₋₆ alkyl; R⁶² and R⁶³ are identical or different and are each independently a hydrogen atom or halogen; R⁶⁴ is a hydrogen atom or C₁₋₆ alkyl; t1 is 0 or 1; and p is 0.

In some embodiments of the present disclosure, provided is the compound represented by general formula (IVV') or (VV') or the pharmaceutically acceptable salt thereof, wherein R^{G1a} is a hydrogen atom or C₁₋₆ alkyl; 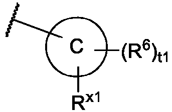 is selected from the group consisting of 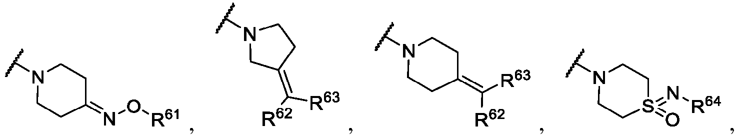 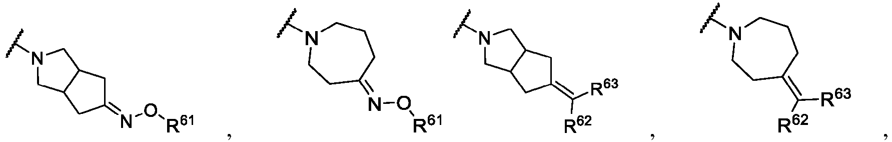 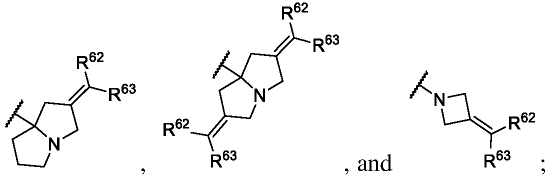 R⁶¹ is C₁₋₆ alkyl; R⁶² and R⁶³ are identical or different and are each independently a hydrogen atom or halogen; R⁶⁴ is a hydrogen atom or C₁₋₆ alkyl; Q is N; R^{4a} is halogen; G² is NH; L is O; p is 0; R^{3a} is halogen; and R^{3b} is C₁₋₆ alkyl.

In some embodiments of the present disclosure, provided is the compound represented by general formula (VV') or (VV'-1) or the pharmaceutically acceptable salt thereof, wherein R^{G1a} is C₁₋₆ alkyl; 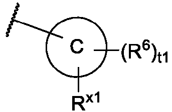 is selected from the group consisting of 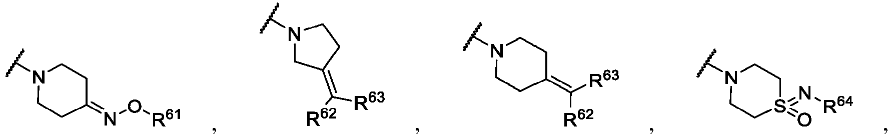 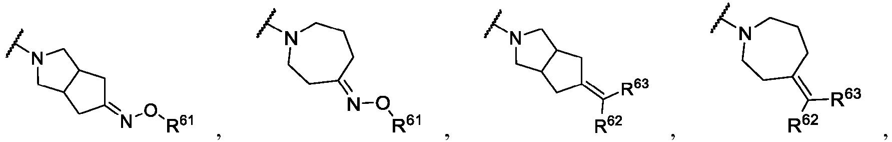 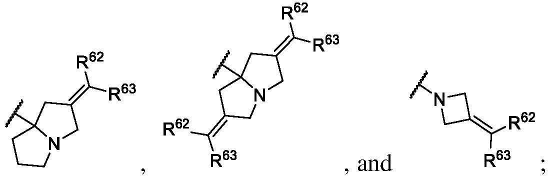 R⁶¹ is C₁₋₆ alkyl; R⁶² and R⁶³ are identical or different and are each independently a hydrogen atom or halogen; R⁶⁴ is a hydrogen atom or C₁₋₆ alkyl; Q is N; R^{4a} is halogen; G² is NH; L is O; p is 0; R^{3a} is halogen; and R^{3b} is C₁₋₆ alkyl.

**Table A. Typical compounds of the present disclosure include, but are not limited to:**

| Compound No. | Compound structure | Name |
|---|---|---|
| | 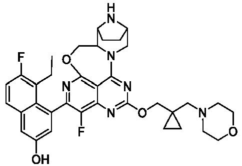 | 5-Ethyl-6-fluoro-4-(1-fluoro-12-((1-(morpholinomethyl)cyclopropyl)methoxy)-5*a*,6,7,8,9,10-hexahydro-5*H*-4-oxa-3,10*a*,11,13,14-pentaaza-6,9-methanonaphtho[1,8-*ab*]heptalen-2-yl)naphthalen-2-ol |
| 1 | 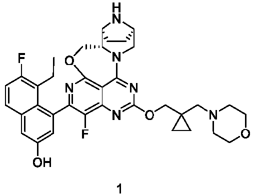 | 5-Ethyl-6-fluoro-4-((5*aS*,6*S*,9*R*)-1-fluoro-12-((1-(morpholinomethyl)cyclopropyl)methoxy)-5*a*,6,7,8,9,10-hexahydro-5*H*-4-oxa-3,10*a*,11,13,14-pentaaza-6,9-methanonaphtho[1,8-*ab*]heptalen-2-yl)naphthalen-2-ol **1** |
| | 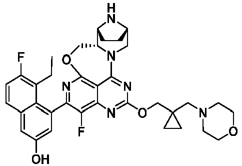 | 5-Ethyl-6-fluoro-4-((5*aS*,6*R*,9*S*)-1-fluoro-12-((1-(morpholinomethyl)cyclopropyl)methoxy)-5*a*,6,7,8,9,10-hexahydro-5*H*-4-oxa-3,10*a*,11,13,14-pentaaza-6,9-methanonaphtho[1,8-*ab*]heptalen-2-yl)naphthalen-2-ol |
| | 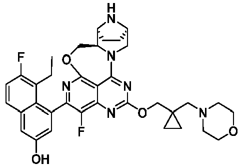 | 5-Ethyl-6-fluoro-4-((5*aR*,6*S*,9*R*)-1-fluoro-12-((1-(morpholinomethyl)cyclopropyl)methoxy)-5*a*,6,7,8,9,10-hexahydro-5*H*-4-oxa-3,10*a*,11,13,14-pentaaza-6,9-methanonaphtho[1,8-*ab*]heptalen-2-yl)naphthalen-2-ol |
| | 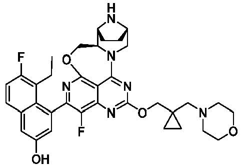 | 5-Ethyl-6-fluoro-4-((5*aR*,6*R*,9*S*)-1-fluoro-12-((1-(morpholinomethyl)cyclopropyl)methoxy)-5*a*,6,7,8,9,10-hexahydro-5*H*-4-oxa-3,10*a*,11,13,14-pentaaza-6,9-methanonaphtho[1,8-*ab*]heptalen-2-yl)naphthalen-2-ol |
| | 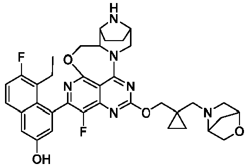 | 4-(12-((1-((2-Oxa-5-azabicyclo[2.2.1]heptan-5-yl)methyl)cyclopropyl)methoxy)-1-fluoro-5*a*,6,7,8,9,10-hexahydro-5*H*-4-oxa-3,10*a*,11,13,14-pentaaza-6,9-methanonaphtho[1,8-*ab*]heptalen-2-yl)-5-ethyl-6-fluoronaphthalen-2-ol |
| 2 | 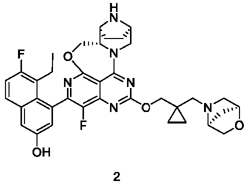 | 4-((5*aS,*6*S,*9*R*)-12-((1-(((1*S*,4*S*)-2-Oxa-5-*az*abicyclo[2.2.1]heptan-5-yl)methyl)cyclopropyl)methoxy)-1-fluoro-5*a*,6,7,8,9,10-hexahydro-5*H*-4-oxa-3,10*a*,11,13,14-pentaaza-6,9-methanonaphtho[1,8-*ab*]heptalen-2-yl)-5-ethyl-6-fluoronaphthalen-2-ol **2** |
| | 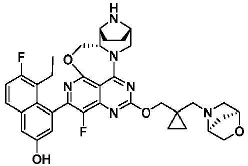 | 4-((5*aS*,6*R*,9*S*)-12-((1-(((1*S*,4*S*)-2-Oxa-5-azabicyclo[2.2.1]heptan-5-yl)methyl)cyclopropyl)methoxy)-1-fluoro-5*a*,6,7,8,9,10-hexahydro-5*H*-4-oxa-3,10*a*,11,13,14-pentaaza-6,9-methanonaphtho[1,8-*ab*]heptalen-2-yl)-5-ethyl-6-fluoronaphthalen-2-ol |
| | 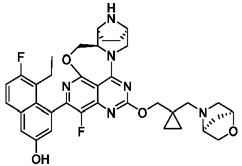 | 4-((5*aR*,6*S*,9*R*)-12-((1-(((1*S*,4*S*)-2-Oxa-5-azabicyclo[2.2.1]heptan-5-yl)methyl)cyclopropyl)methoxy)-1-fluoro-5*a*,6,7,8,9,10-hexahydro-5*H*-4-oxa-3,10*a*,11,13,14-pentaaza-6,9-methanonaphtho[1,8-*ab*]heptalen-2-yl)-5-ethyl-6-fluoronaphthalen-2-ol |
| | 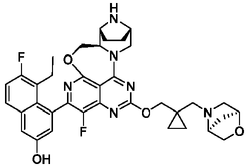 | 4-((5*aR*,6*R*,9*S*)-12-((1-(((1*S*,4*S*)-2-Oxa-5-azabicyclo[2.2.1]heptan-5-yl)methyl)cyclopropyl)methoxy)-1-fluoro-5*a*,6,7,8,9,10-hexahydro-5*H*-4-oxa-3,10*a*,11,13,14-pentaaza-6,9-methanonaphtho[1,8-*ab*]heptalen-2-yl)-5-ethyl-6-fluoronaphthalen-2-ol |
| 3 | 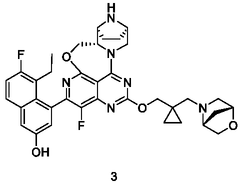 | 4-((5*aS*,6*S*,9*R*)-12-((1-(((1*R*,4*R*)-2-Oxa-5-azabicyclo[2.2.1]heptan-5-yl)methyl)cyclopropyl)methoxy)-1-fluoro-5a,6,7,8,9,10-hexahydro-5H-4-oxa-3,10*a*,11,13,14-pentaaza-6,9-methanonaphtho[1,8-*ab*]heptalen-2-yl)-5-ethyl-6-fluoronaphthalen-2-ol **3** |
| | 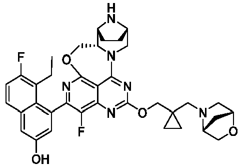 | 4-((5*aS*,6*R*,9*S*)-12-((1-(((1*R*,4*R*)-2-Oxa-5-*az*abicyclo[2.2.1]heptan-5-yl)methyl)cyclopropyl)methoxy)-1-fluoro-5a,6,7,8,9,10-hexahydro-5*H*-4-oxa-3,10*a*,11,13,14-pentaaza-6,9-methanonaphtho[1,8-*ab*]heptalen-2-yl)-5-ethyl-6-fluoronaphthalen-2-ol |
| | 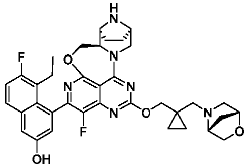 | 4-((5*aR*,6*S*,9*R*)-12-((1-(((1*R*,4*R*)-2-Oxa-5-azabicyclo[2.2.1]heptan-5-yl)methyl)cyclopropyl)methoxy)-1-fluoro-5a,6,7,8,9,10-hexahydro-5*H*-4-oxa-3,10*a*,11,13,14-pentaaza-6,9-methanonaphtho[1,8-*ab*]heptalen-2-yl)-5-ethyl-6-fluoronaphthalen-2-ol |
| | 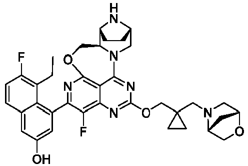 | 4-((5*aR*,6*R*,9*S*)-12-((1-(((1*R*,4*R*)-2-Oxa-5-azabicyclo[2.2.1]heptan-5-yl)methyl)cyclopropyl)methoxy)-1-fluoro-5a,6,7,8,9,10-hexahydro-5*H*-4-oxa-3,10*a*,11,13,14-pentaaza-6,9-methanonaphtho[1,8-*ab*]heptalen-2-yl)-5-ethyl-6-fluoronaphthalen-2-ol |
| | 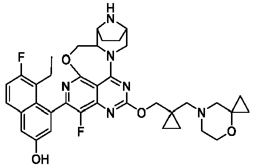 | 4-(12-((1-((4-Oxa-7-azaspiro[2.5]octan-7-yl)methyl)cyclopropyl)methoxy)-1-fluoro-5a,6,7,8,9,10-hexahydro-5*H*-4-oxa-3,10*a*,11,13,14-pentaaza[6,9]methanonaphtho[1,8-*ab*]heptalen-2-yl)-5-ethyl-6-fluoronaphthalen-2-ol |
| 4 | 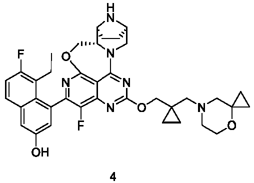 | 4-((5*aS*,6*S*,9*R*)-12-((1-((4-Oxa-7-azaspiro[2.5]octan-7-yl)methyl)cyclopropyl)methoxy)-1-fluoro-5a,6,7,8,9,10-hexahydro-5*H*-4-oxa-3,10*a*,11,13,14-pentaaza[6,9]methanonaphtho[1,8-*ab*]heptalen-2-yl)-5-ethyl-6-fluoronaphthalen-2-ol **4** |
| | 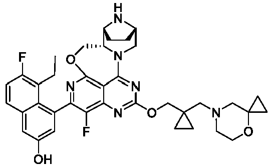 | 4-((5*aS,*6*R*,9*S*)-12-((1-((4-Oxa-7-azaspiro[2.5]octan-7-yl)methyl)cyclopropyl)methoxy)-1-fluoro-5a,6,7,8,9,10-hexahydro-5*H*-4-oxa-3,10*a*,11,13,14-pentaaza[6,9]methanonaphtho[1,8-*ab*]heptalen-2-yl)-5-ethyl-6-fluoronaphthalen-2-ol |
| | 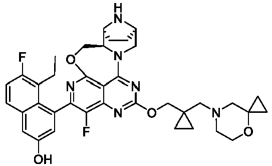 | 4-((5*aR*,6*S*,9*R*)-12-((1-((4-Oxa-7-azaspiro[2.5]octan-7-yl)methyl)cyclopropyl)methoxy)-1-fluoro-5a,6,7,8,9,10-hexahydro-5*H*-4-oxa-3,10*a*,11,13,14-pentaaza[6,9]methanonaphtho[1,8-*ab*]heptalen-2-yl)-5-ethyl-6-fluoronaphthalen-2-ol |
| | 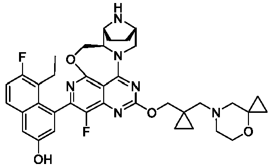 | 4-((5*aR*,6*R*,9*S*)-12-((1-((4-Oxa-7-azaspiro[2.5]octan-7-yl)methyl)cyclopropyl)methoxy)-1-fluoro-5a,6,7,8,9,10-hexahydro-5*H*-4-oxa-3,10*a*,11,13,14-pentaaza[6,9]methanonaphtho[1,8-*ab*]heptalen-2-yl)-5-ethyl-6-fluoronaphthalen-2-ol |
| | 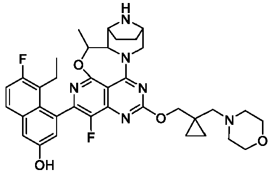 | 5-Ethyl-6-fluoro-4-(1-fluoro-5-methyl-12-((1-(morpholinomethyl)cyclopropyl)methoxy)-5*a*,6,7,8,9,10-hexahydro-5*H*-4-oxa-3,10*a*,11,13,14-pentaaza-6,9-methanonaphtho[1,8-*ab*]heptalen-2-yl)naphthalen-2-ol |
| 5 | 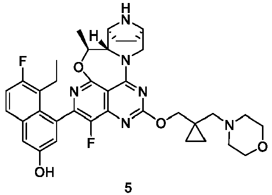 | 5-Ethyl-6-fluoro-4-((5*S*,5*aS*,6*S*,9*R*)-1-fluoro-5-methyl-12-((1-(morpholinomethyl)cyclopropyl)methoxy)-5*a*,6,7,8,9,10-hexahydro-5*H*-4-oxa-3,10*a*,11,13,14-pentaaza-6,9-methanonaphtho[1,8*-ab*]heptalen-2-yl)naphthalen-2-ol **5** |
| 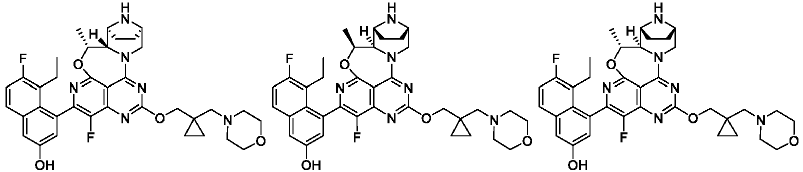 | | |
| 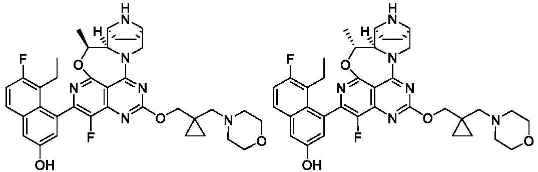 | | |
| 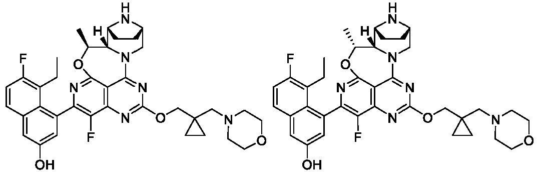 | | |
| 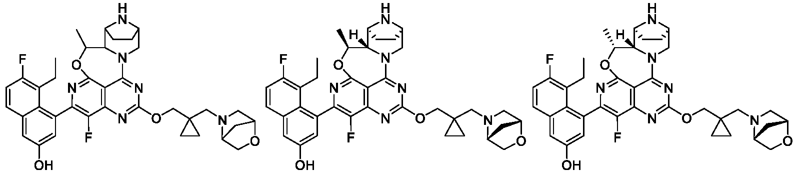 | | |
| 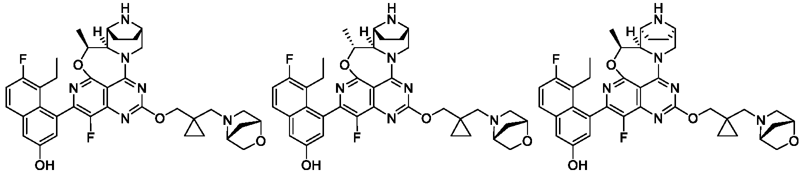 | | |
| 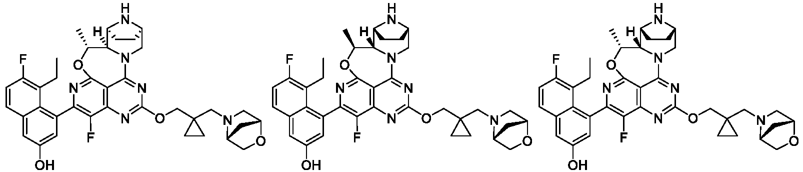 | | |
| 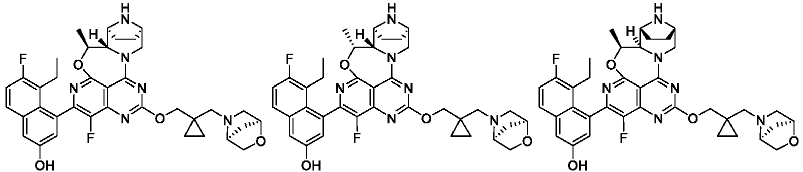 | | |
| 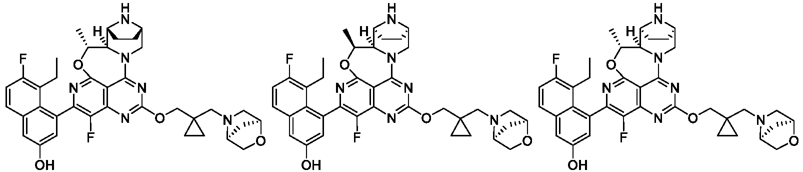 | | |
| 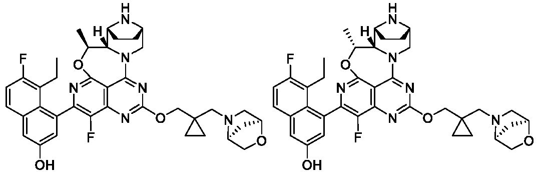 | | |
| 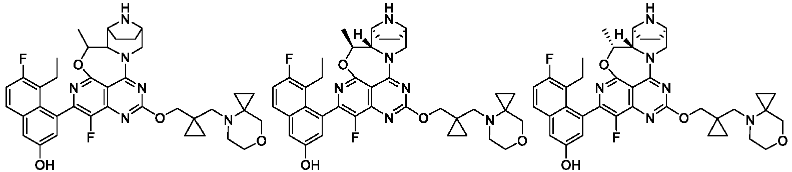 | | |
| 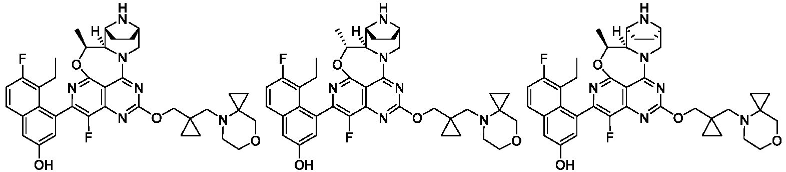 | | |
| 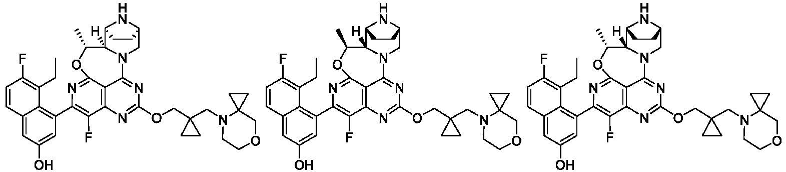 | | |
| | 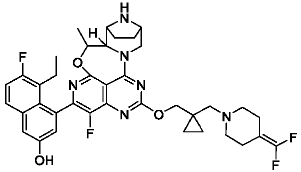 | 4-(12-((1-((4-(Difluoromethylene)piperidin-1-yl)methyl)cyclopropyl)methoxy)-1-fluoro-5-methyl-5*a*,6,7,8,9,10-hexahydro-5*H*-4-oxa-3,10*a*,11,13,14-pentaaza-6,9-methanonaphtho[1,8-*ab*]heptalen-2-yl)-5-ethyl-6-fluoronaphthalen-2-ol |
| 6 | 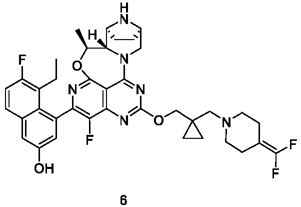 | 4-((5*S*,5*aS*,6*S*,9*R*)-12-((1-((4-(Difluoromethylene)piperidin-1-yl)methyl)cyclopropyl)methoxy)-1-fluoro-5-methyl-5*a*,6,7,8,9,10-hexahydro-5*H*-4-oxa-3,10*a*,11,13,14-pentaaza-6,9-methanonaphtho[1,8-*ab*]heptalen-2-yl)-5-ethyl-6-fluoronaphthalen-2-ol **6** |
| 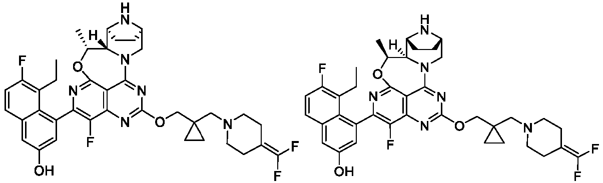 | | |
| 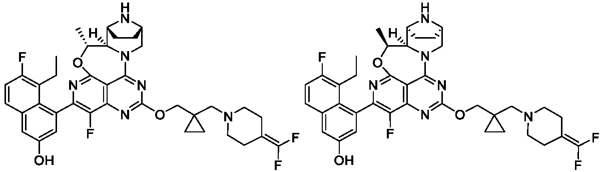 | | |
| 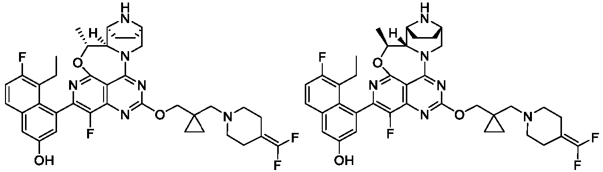 | | |
| 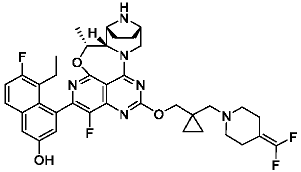 | | |
| | 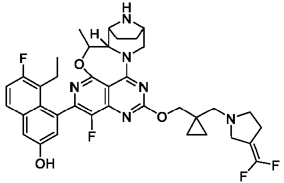 | 4-(12-((1-((3-(Difluoromethylene)pyrrolidin-1-yl)methyl)cyclopropyl)methoxy)-1-fluoro-5-methyl-5*a*,6,7,8,9,10-hexahydro-5*H*-4-oxa-3,10*a*,11,13,14-pentaaza-6,9-methanonaphtho[1,8*-ab*]heptalen-2-yl)-5-ethyl-6-fluoronaphthalen-2-ol |
| 7 | 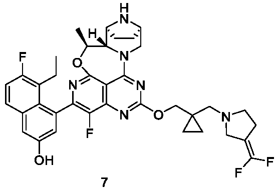 | 4-((5*S*,5*aS*,6*S*,9*R*)-12-((1-((3-(Difluoromethylene)pyrrolidin-1-yl)methyl)cyclopropyl)methoxy)-1-fluoro-5-methyl-5*a*,6,7,8,9,10-hexahydro-5*H*-4-oxa-3,10*a,*11,13,14-pentaaza-6,9-methanonaphtho[1,8-*ab*]heptalen-2-yl)-5-ethyl-6-fluoronaphthalen-2-ol **7** |
| 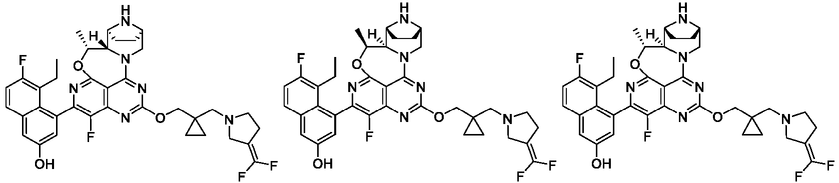 | | |
| 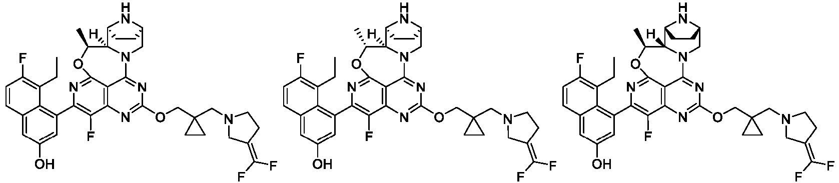 | | |
| 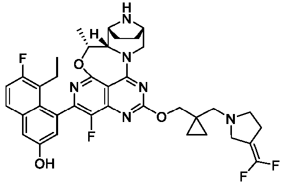 | | |
| | 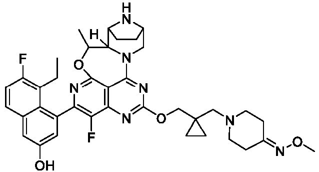 | 1-((1-(((2-(8-Ethyl-7-fluoro-3-hydroxynaphthalen-1-yl)-1-fluoro-5-methyl-5*a*,6,7,8,9,10-hexahydro-5*H*-4-oxa-3,10*a*,11,13,14-pentaaza-6,9-methanonaphtho[1,8-*ab*]heptalen-12-yl)oxy)methyl)cyclopropyl)methyl)piperidin -4-one *O*-methyl oxime |
| 8 | 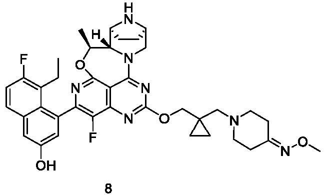 | 1-((1-((((5*S*,5*aS*,6*S*,9*R*)-2-(8-Ethyl-7-fluoro-3-hydroxynaphthalen-1-yl)-1-fluoro-5-methyl-5*a*,6,7,8,9,10-hexahydro-5*H*-4-oxa-3,10*a*,11,13,14-pentaaza-6,9-methanonaphtho[1,8-*ab*]heptalen-12-yl)oxy)methyl)cyclopropyl)methyl)piperidin -4-one *O*-methyl oxime **8** |
| 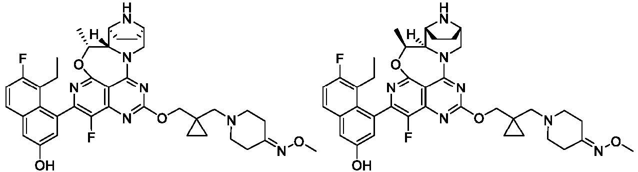 | | |
| 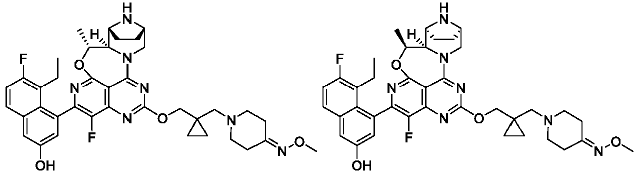 | | |
| 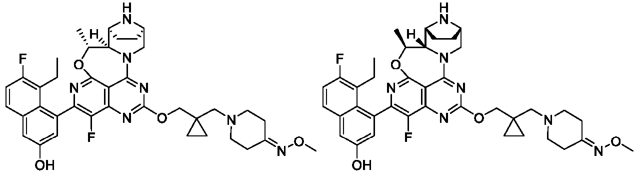 | | |
| 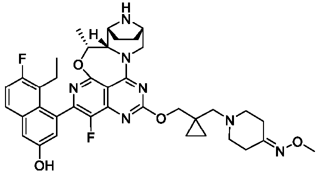 | | |
| | 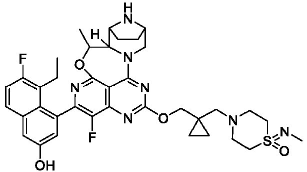 | 4-((1-(((2-(8-Ethyl-7-fluoro-3-hydroxynaphthalen-1-yl)-1-fluoro-5-methyl-5*a*,6,7,8,9,10-hexahydro-5*H*-4-oxa-3,10*a*,11,13,14-pentaaza-6,9-methanonaphtho[1,8-*ab*]heptalen-12-yl)oxy)methyl)cyclopropyl)methyl)-1-(methylimino)-1λ⁶-thiomorpholine 1-oxide |
| 9 | 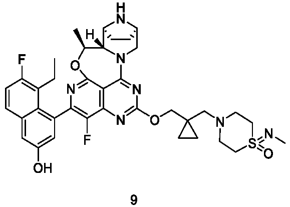 | 4-((1-((((5*S*,5a*S*,6*S*,9*R*)-2-(8-Ethyl-7-fluoro-3-hydroxynaphthalen-1-yl)-1-fluoro-5-methyl-5*a*,6,7,8,9,10-hexahydro-5*H*-4-oxa-3,10*a*,11,13,14-pentaaza-6,9-methanonaphtho[1,8-*ab*]heptalen-12-yl)oxy)methyl)cyclopropyl)methyl)-1-(methylimino)-1λ⁶-thiomorpholine 1-oxide **9** |
| 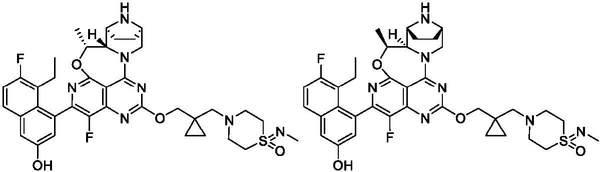 | | |
| 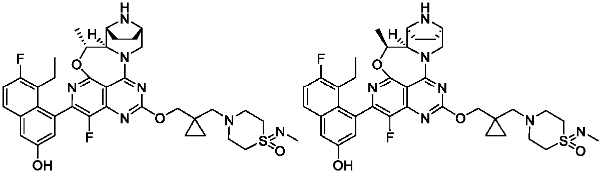 | | |
| 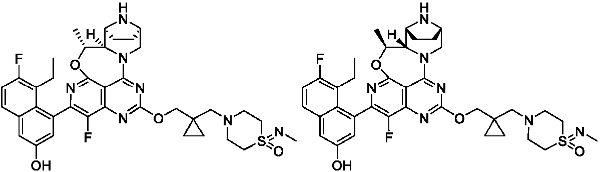 | | |
| 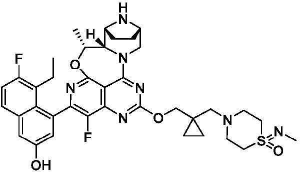 | | |
| | 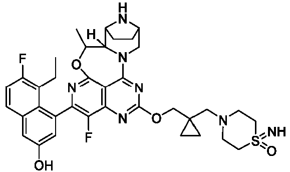 | 4-((1-(((2-(8-Ethyl-7-fluoro-3-hydroxynaphthalen-1-yl)-1-fluoro-5-methyl-5*a*,6,7,8,9,10-hexahydro-5*H*-4-oxa-3,10*a*,11,13,14-pentaaza-6,9-methanonaphtho[1,8-*ab*]heptalen-12-yl)oxy)methyl)cyclopropyl)methyl)-1-imino-1λ⁶-thiomorpholine 1-oxide |
| 10 | 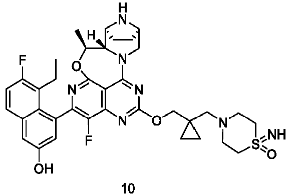 | 4-((1-((((5*S*,5*aS*,6*S*,9*R*)-2-(8-Ethyl-7-fluoro-3-hydroxynaphthalen-1-yl)-1-fluoro-5-methyl-5*a*,6,7,8,9,10-hexahydro-5*H*-4-oxa-3,10*a*,11,13,14-pentaaza-6,9-methanonaphtho[1,8-*ab*]heptalen-12-yl)oxy)methyl)cyclopropyl)methyl)-1-imino-1λ⁶-thiomorpholine 1-oxide **10** |
| 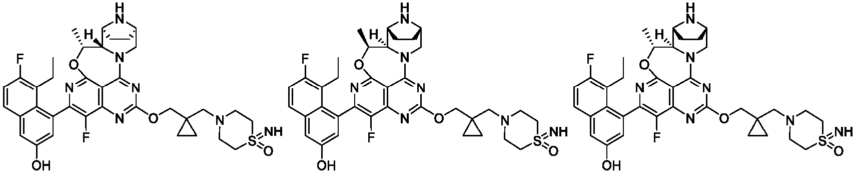 | | |
| 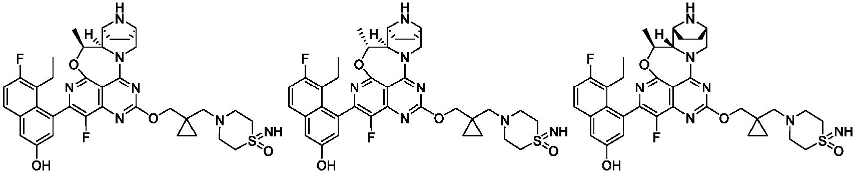 | | |
| 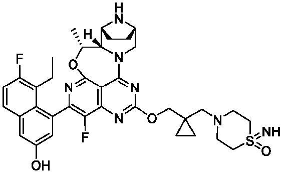 | | |
| | 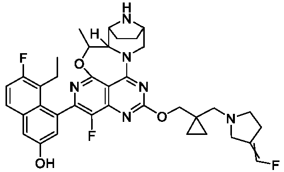 | 5-Ethyl-6-fluoro-4-(1-fluoro-12-((1-((3-(fluoromethylene)pyrrolidin-1-yl)methyl)cyclopropyl)methoxy)-5-methyl-5*a*,6,7,8,9,10-hexahydro-5*H*-4-oxa-3,10*a*,11,13,14-pentaaza-6,9-methanonaphtho[1,8-*ab*]heptalen-2-yl)naphthalen-2-ol |
| | 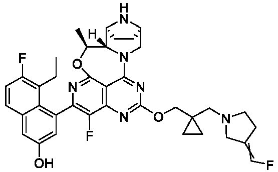 | 5-Ethyl-6-fluoro-4-((5*S*,5*aS*,6*S*,9*R*)-1-fluoro-12-((1-((3-(fluoromethylene)pyrrolidin-1-yl)methyl)cyclopropyl)methoxy)-5-methyl-5*a*,6,7,8,9,10-hexahydro-5*H*-4-oxa-3,10*a*,11,13,14-pentaaza-6,9-methanonaphtho[1,8*-ab*]heptalen-2-yl)naphthalen-2-ol |
| 11 | 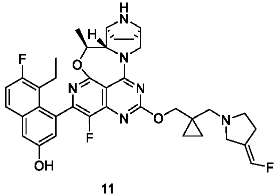 | 5-Ethyl-6-fluoro-4-((5*S*,5*aS*,6*S*,9*R*)-1-fluoro-12-((1-(((*E*)-3-(fluoromethylene)pyrrolidin-1-yl)methyl)cyclopropyl)methoxy)-5-methyl-5*a*,6,7,8,9,10-hexahydro-5*H*-4-oxa-3,10*a*,11,13,14-pentaaza-6,9-methanonaphtho[1,8-*ab*]heptalen-2-yl)naphthalen-2-ol **11** |
| 25 | 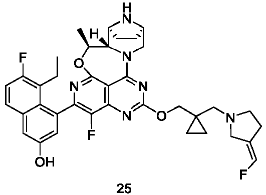 | 5-Ethyl-6-fluoro-4-((5*S*,5*aS*,6*S*,9*R*)-1-fluoro-12-((1-(((*Z*)-3-(fluoromethylene)pyrrolidin-1-yl)methyl)cyclopropyl)methoxy)-5-methyl-5*a*,6,7,8,9,10-hexahydro-5*H*-4-oxa-3,10*a*,11,13,14-pentaaza-6,9-methanonaphtho[1,8-*ab*]heptalen-2-yl)naphthalen-2-ol **25** |
| 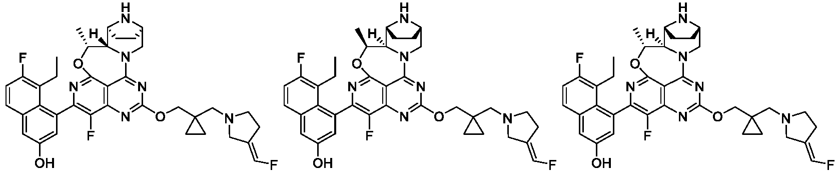 | | |
| 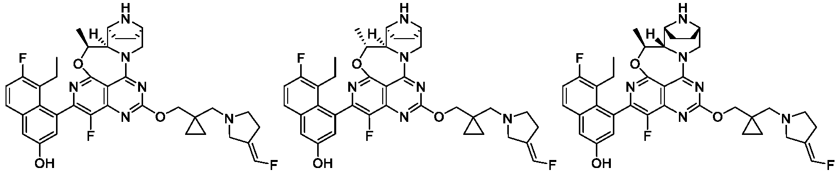 | | |
| 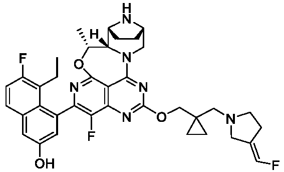 | | |
| 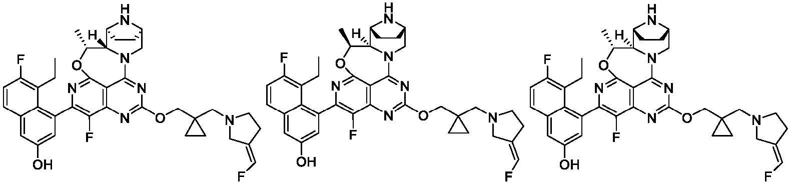 | | |
| 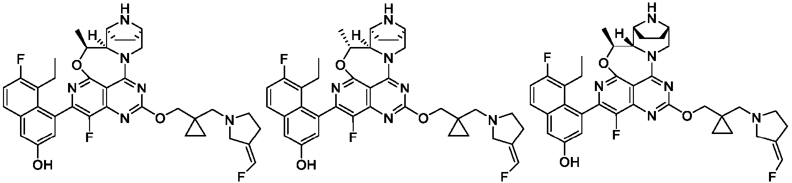 | | |
| 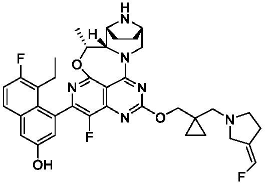 | | |
| | 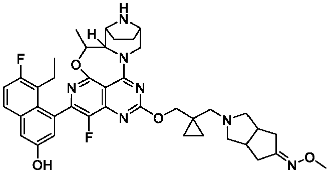 | 2-((1-(((2-(8-Ethyl-7-fluoro-3-hydroxynaphthalen-1-yl)-1-fluoro-5-methyl-5*a*,6,7,8,9,10-hexahydro-5*H*-4-oxa-3,10*a*,11,13,14-pentaaza-6,9-methanonaphtho[1,8-*ab*]heptalen-12-yl)oxy)methyl)cyclopropyl)methyl)hexahydr ocyclopenta[c]pyrrol-5(1*H*)-one *O*-methyl oxime |
| 12 | 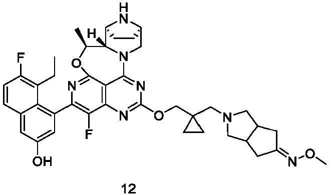 | 2-((1-((((5*S*,5*aS*,6*S*,9*R*)-2-(8-Ethyl-7-fluoro-3-hydroxynaphthalen-1-yl)-1-fluoro-5-methyl-5*a*,6,7,8,9,10-hexahydro-5*H*-4-oxa-3,10*a*,11,13,14-pentaaza-6,9-methanonaphtho[1,8-*ab*]heptalen-12-yl)oxy)methyl)cyclopropyl)methyl)hexahydr ocyclopenta[c]pyrrol-5(1*H*)-one *O*-methyl oxime **12** |
| 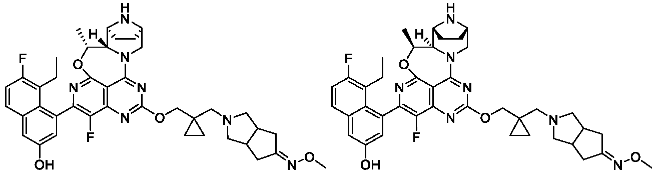 | | |
| 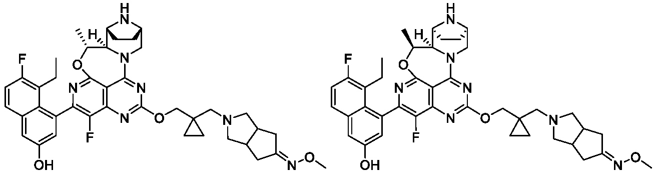 | | |
| 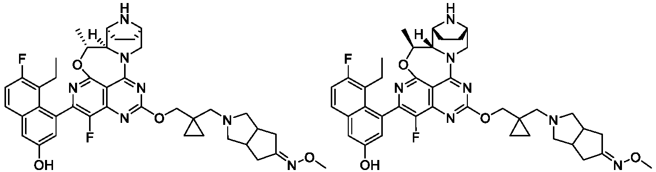 | | |
| 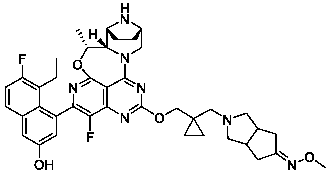 | | |
| | 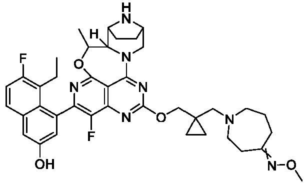 | 1-((1-(((2-(8-Ethyl-7-fluoro-3-hydroxynaphthalen-1-yl)-1-fluoro-5-methyl-5*a*,6,7,8,9,10-hexahydro-5*H*-4-oxa-3,10*a*,11,13,14-pentaaza-6,9-methanonaphtho[1,8-*ab*]heptalen-12-yl)oxy)methyl)cyclopropyl)methyl)azepan-4-one *O*-methyl oxime |
| 13 | 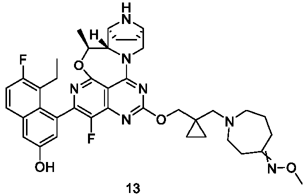 | 1-((1-((((5*S*,5*aS*,6*S*,9*R*)-2-(8-Ethyl-7-fluoro-3-hydroxynaphthalen-1-yl)-1-fluoro-5-methyl-5*a*,6,7,8,9,10-hexahydro-5*H*-4-oxa-3,10*a*,11,13,14-pentaaza-6,9-methanonaphtho[1,8-*ab*]heptalen-12-yl)oxy)methyl)cyclopropyl)methyl)azepan-4-one *O*-methyl oxime **13** |
| 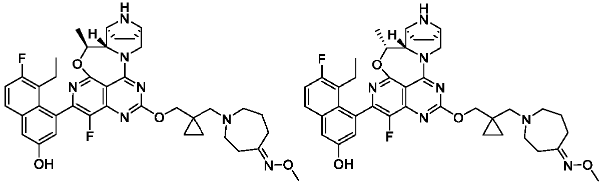 | | |
| 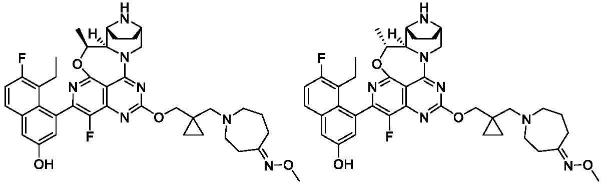 | | |
| 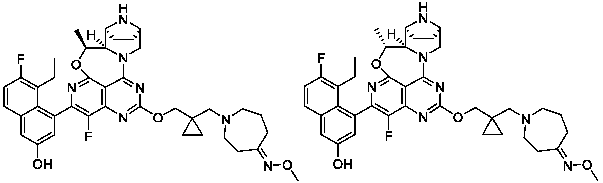 | | |
| 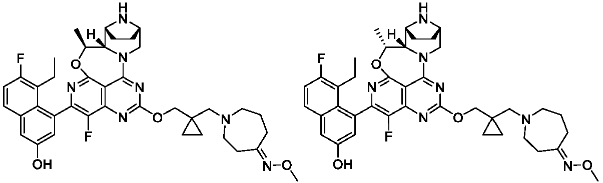 | | |
| 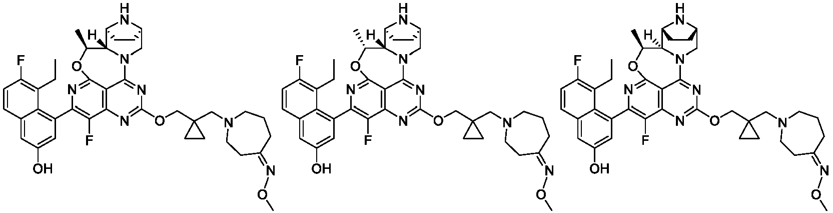 | | |
| 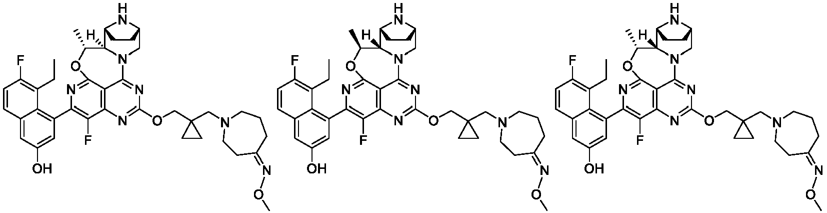 | | |
| 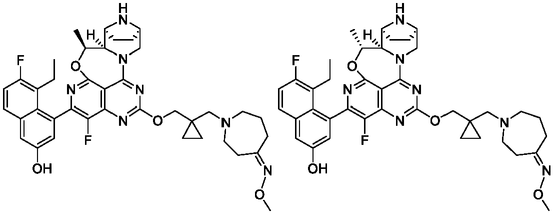 | | |
| | 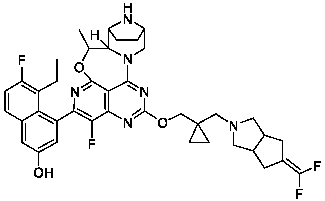 | 4-(12-(1-((5-(Difluoromethylene)hexahydrocyclopenta[c] pyrrol-2(1H)-yl)methyl)cyclopropyl)methoxy)-1-fluoro-5-methyl-5*a*,6,7,8,9,10-hexahydro-5*H*-4-oxa-3,10*a*,11,13,14-pentaaza-6,9-methanonaphtho[1,8-*ab*]heptalen-2-yl)-5-ethyl-6-fluoronaphthalen-2-ol |
| 14 | 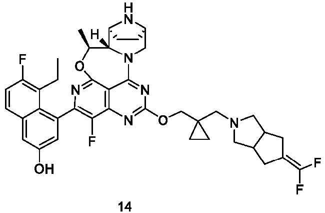 | 4*-*((5*S*,5*aS*,6*S,*9*R*)-12-(1-((5-(Difluoromethylene)hexahydrocyclopenta[c] pyrrol-2(1H)-yl)methyl)cyclopropyl)methoxy)-1-fluoro-5-methyl-5*a*,6,7,8,9,10-hexahydro-5*H*-4-oxa-3,10*a*,11,13,14-pentaaza-6,9-methanonaphtho[1,8-*ab*]heptalen-2-yl)-5-ethyl-6-fluoronaphthalen-2-ol **14** |
| 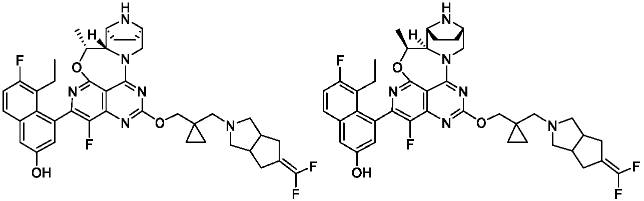 | | |
| 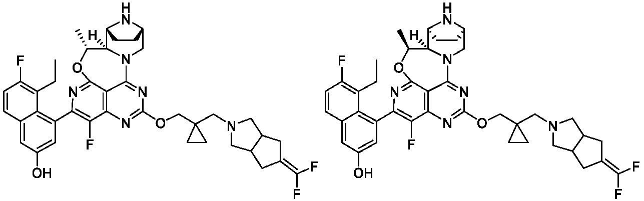 | | |
| 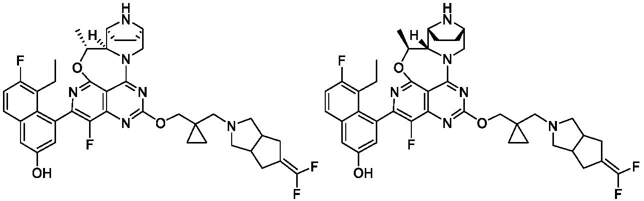 | | |
| 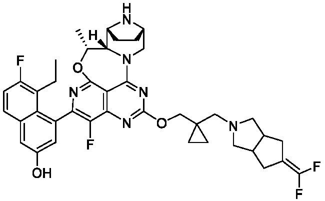 | | |
| | 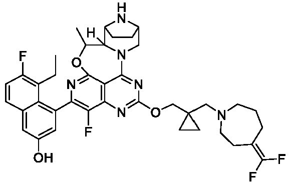 | 4-(12-((1-((4-(Difluoromethylene)azepan-1-yl)methyl)cyclopropyl)methoxy)-1-fluoro-5-methyl-5*a*,6,7,8,9,10-hexahydro-5*H*-4-oxa-3,10*a*,11,13,14-pentaaza-6,9-methanonaphtho[1,8-*ab*]heptalen-2-yl)-5-ethyl-6-fluoronaphthalen-2-ol |
| 15 | 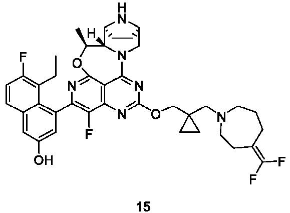 | 4-((5*S*,5*aS*,6*S*,9*R*)-12-((1-((4-(Difluoromethylene)azepan-1-yl)methyl)cyclopropyl)methoxy)-1-fluoro-5-methyl-5*a*,6,7,8,9,10-hexahydro-5*H*-4-oxa-3,10*a*,11,13,14-pentaaza-6,9-methanonaphtho[1,8-*ab*]heptalen-2-yl)-5-ethyl-6-fluoronaphthalen-2-ol **15** |
| 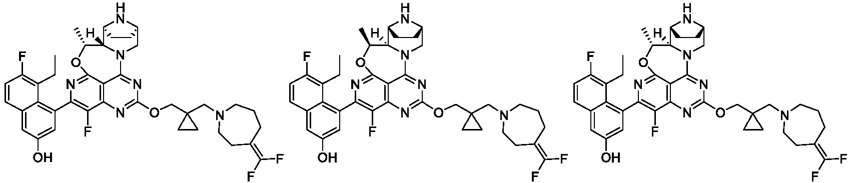 | | |
| 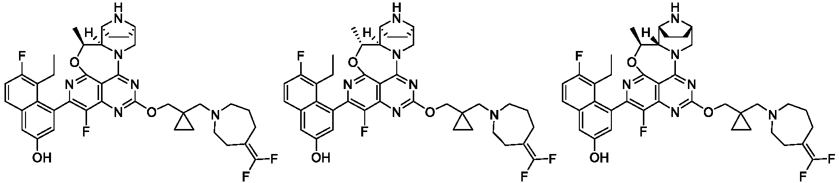 | | |
| 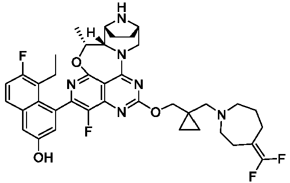 | | |
| | 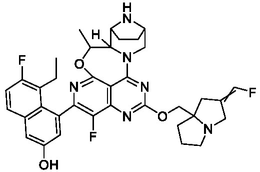 | 5-Ethyl-6-fluoro-4-(1-fluoro-12-((2-(fluoromethylene)tetrahydro-1*H*-pyrrolizin-7*a*(5*H*)-yl)methoxy)-5-methyl-5*a*,6,7,8,9,10-hexahydro-5*H*-4-oxa-3,10*a*,11,13,14-pentaaza-6,9-methanonaphtho[1,8-*ab*]heptalen-2-yl)naphthalen-2-ol |
| 16 | 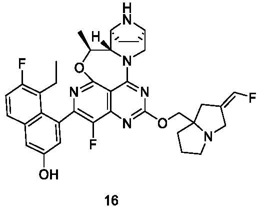 | 5-Ethyl-6- fluoro-4-((5*S*,5*aS*,6*S*,9*R*)-1-fluoro-12-(((Z)-2-(fluoromethylene)tetrahydro-1*H-*pyrrolizin-7*a*(5*H*)-yl)methoxy)-5-methyl-5*a*,6,7,8,9,10-hexahydro-5*H*-4-oxa-3,10*a*,11,13,14-pentaaza-6,9-methanonaphtho[1,8-*ab*]heptalen-2-yl)naphthalen-2-ol **16** |
| 16-p1 | 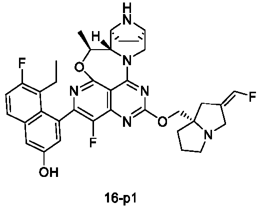 | 5-Ethyl-6-fluoro-4-((5*S*,5*aS*,6*S*,9*R*)-1-fluoro-12-(((*S*,Z)-2-(fluoromethylene)tetrahydro-1*H*-pyrrolizin-7*a*(5*H*)-yl)methoxy)-5-methyl-5*a*,6,7,8,9,10-hexahydro-5*H*-4-oxa-3,10*a*,11,13,14-pentaaza-6,9-methanonaphtho[1,8-*ab*]heptalen-2-yl)naphthalen-2-ol **16-p1** |
| 16-p2 | 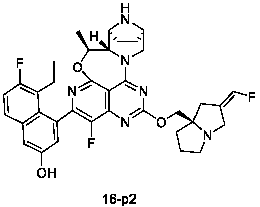 | 5-Ethyl-6-fluoro-4-((5*S*,5*aS*,6*S*,9*R*)-1-fluoro-12-(((R,Z)-2-(fluoromethylene)tetrahydro-1*H*-pyrrolizin-7a(5*H*)-yl)methoxy)-5-methyl-5*a*,6,7,8,9,10-hexahydro-5*H*-4-oxa-3,10*a*,11,13,14-pentaaza-6,9-methanonaphtho[1,8-*ab*]heptalen-2-yl)naphthalen-2-ol **16-p2** |
| 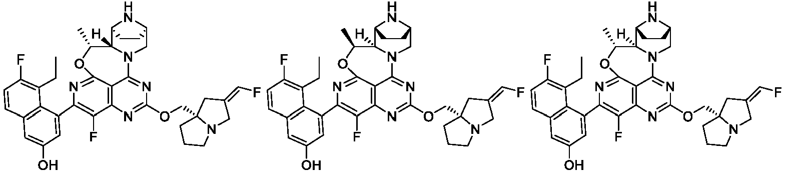 | | |
| 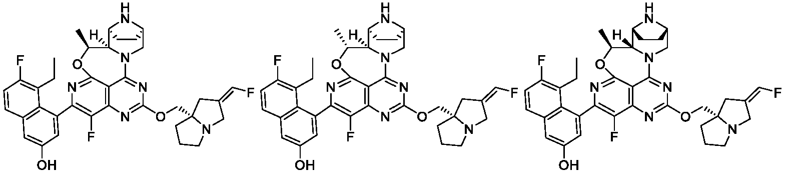 | | |
| 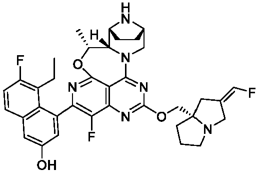 | | |
| 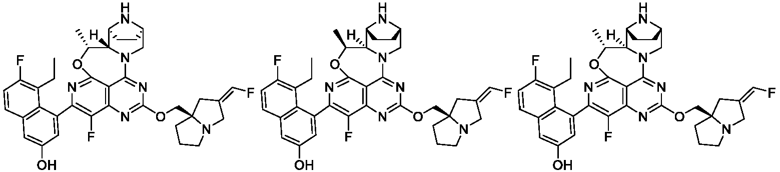 | | |
| 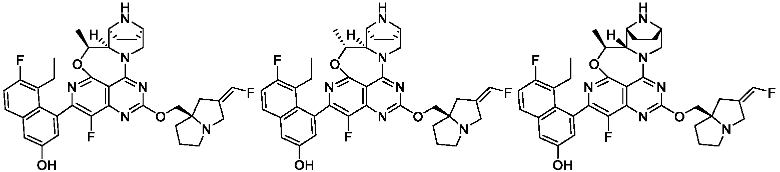 | | |
| 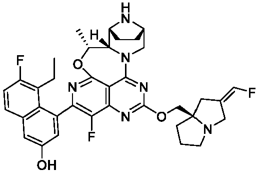 | | |
| | 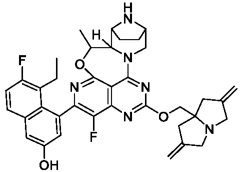 | 4-(12-((2,6-Dimethylenetetrahydro-1*H-*pyrrolizin-7*a*(5*H*)-yl)methoxy)-1-fluoro-5-methyl-5*a*,6,7,8,9,10-hexahydro-5*H*-4-oxa-3,10*a*,11,13,14-pentaaza-6,9-methanonaphtho[1,8-*ab*]heptalen-2-yl)-5-ethyl-6-fluoronaphthalen-2-ol |
| 17 | 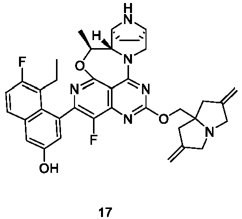 | 4-((5*S*,5*aS*,6*S*,9*R*)-12-((2,6-*D*imethylenetetrahydro-1*H*-pyrrolizin-7*a*(5*H*)-yl)methoxy)-1-fluoro-5-methyl-5*a*,6,7,8,9,10-hexahydro-5*H*-4-oxa-3,10*a*,11,13,14-pentaaza-6,9-methanonaphtho[1,8-*ab*]heptalen-2-yl)-5-ethyl-6-fluoronaphthalen-2-ol **17** |
| 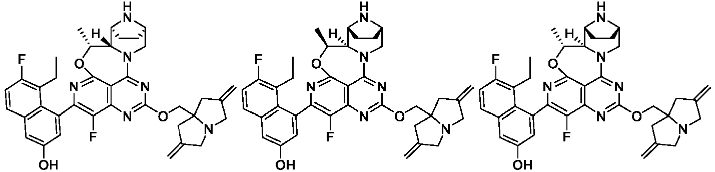 | | |
| 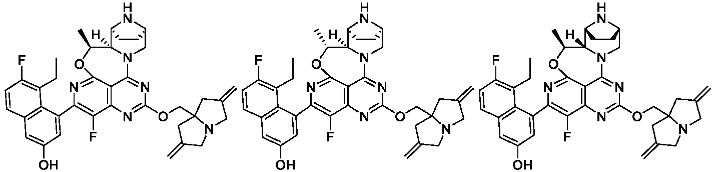 | | |
| 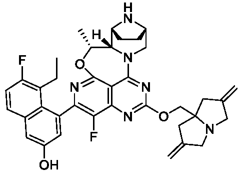 | | |
| | 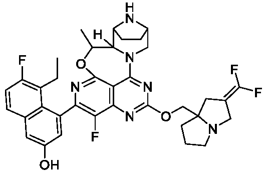 | 4-(12-((2-(Difluoromethylene)tetrahydro-1*H*-pyrrolizin-7*a*(5*H*)-yl)methoxy)-1-fluoro-5-methyl-5*a*,6,7,8,9,10-hexahydro-5*H*-4-oxa-3,10*a*,11,13,14-pentaaza-6,9-methanonaphtho[1,8-*ab*]heptalen-2-yl)-5-ethyl-6-fluoronaphthalen-2-ol |
| 18 | 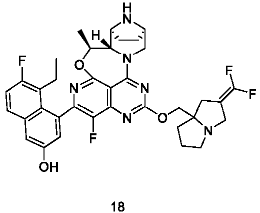 | 4-((5*S*,5*aS*,6*S*,9*R*)-12-((2-(Difluoromethylene)tetrahydro-1*H-*pyrrolizin-7*a*(5*H*)-yl)methoxy)-1-fluoro-5-methyl-5*a*,6,7,8,9,10-hexahydro-5*H*-4-oxa-3,10*a*,11,13,14-pentaaza-6,9-methanonaphtho[1,8-*ab*]heptalen-2-yl)-5-ethyl-6-fluoronaphthalen-2-ol **18** |
| 18-p1 | 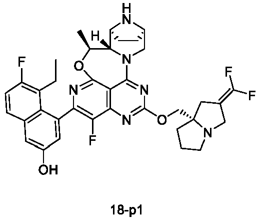 | 4-((5*S*,5*aS*,6*S*,9*R*)-12-(((*S*)-2-(Difluoromethylene)tetrahydro-1H-pyrrolizin-7*a*(5*H*)-yl)methoxy)-1-fluoro-5-methyl-5*a*,6,7,8,9,10-hexahydro-5*H*-4-oxa-3,10*a*,11,13,14-pentaaza-6,9-methanonaphtho[1,8-*ab*]heptalen-2-yl)-5-ethyl-6-fluoronaphthalen-2-ol **18-p1** |
| 18-p2 | 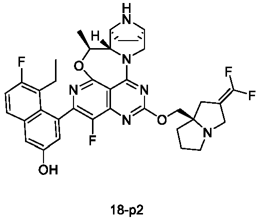 | 4-((5*S*,5*aS*,6*S*,9*R*)-12-(((*R*)-2-(Difluoromethylene)tetrahydro-1*H-*pyrrolizin-7*a*(5*H*)-yl)methoxy)-1-fluoro-5-methyl-5*a*,6,7,8,9,10-hexahydro-5*H*-4-oxa-3,10*a*,11,13,14-pentaaza-6,9-methanonaphtho[1,8-*ab*]heptalen-2-yl)-5-ethyl-6-fluoronaphthalen-2-ol **18-p2** |
| 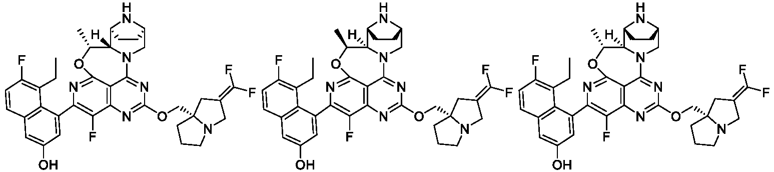 | | |
| 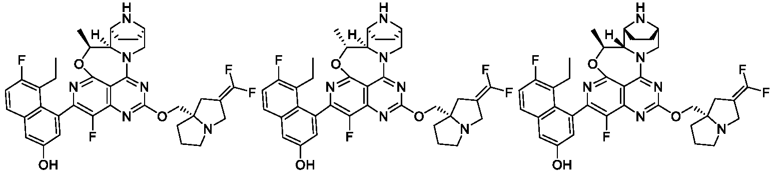 | | |
| 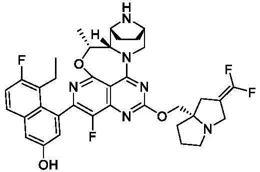 | | |
| 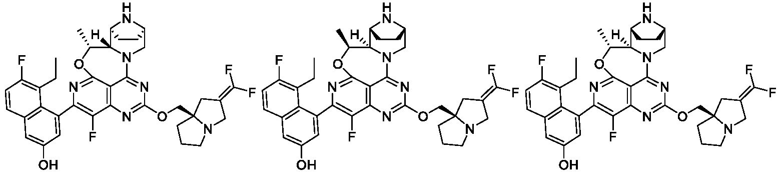 | | |
| 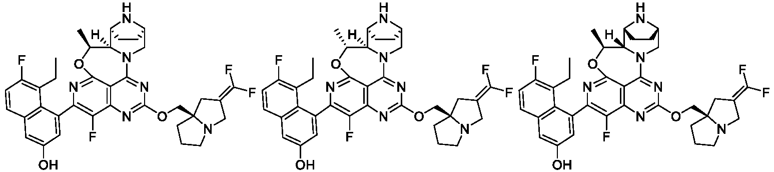 | | |
| 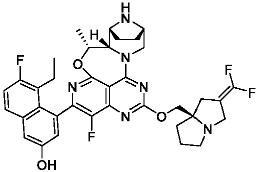 | | |
| 19 | 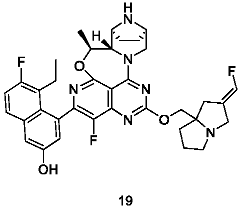 | 5-Ethyl-6-fluoro-4-((5*S*,5*aS*,6*S*,9*R*)-1-fluoro-12-(((*E*)-2-(fluoromethylene)tetrahydro-1*H-*pyrrolizin-7a(5*H*)-yl)methoxy)-5-methyl-5*a*,6,7,8,9,10-hexahydro-5*H*-4-oxa-3,10*a*,11,13,14-pentaaza-6,9-methanonaphtho[1,8-*ab*]heptalen-2-yl)naphthalen-2-ol **19** |
| 19-p1 | 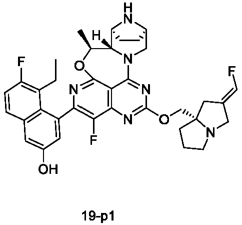 | 5-Ethyl-6-fluoro-4-((5*S*,5*aS*,6*S*,9*R*)-1-fluoro-12-(((*S,E*)-2-(fluoromethylene)tetrahydro-1*H*-pyrrolizin-7*a*(5*H*)-yl)methoxy)-5-methyl-5*a*,6,7,8,9,10-hexahydro-5*H*-4-oxa-3,10*a*,11,13,14-pentaaza-6,9-methanonaphtho[1,8-*ab*]heptalen-2-yl)naphthalen-2-ol **19-p1** |
| 19-p2 | 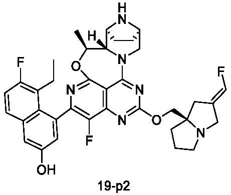 | 5-Ethyl-6-fluoro-4-((5*S*,5*aS*,6*S*,9*R*)-1-fluoro-12-(((*R,E*)-2-(fluoromethylene)tetrahydro-1*H-*pyrrolizin-7*a*(5*H*)-yl)methoxy)-5-methyl-5*a*,6,7,8,9,10-hexahydro-5*H*-4-oxa-3,10*a*,11,13,14-pentaaza-6,9-methanonaphtho[1,8-*ab*]heptalen-2-yl)naphthalen-2-ol **19-p2** |
| 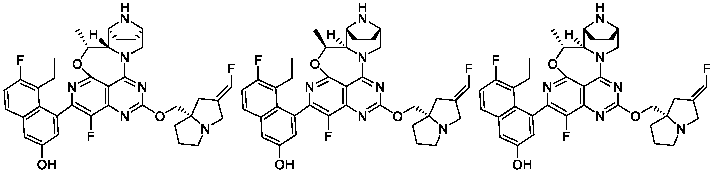 | | |
| 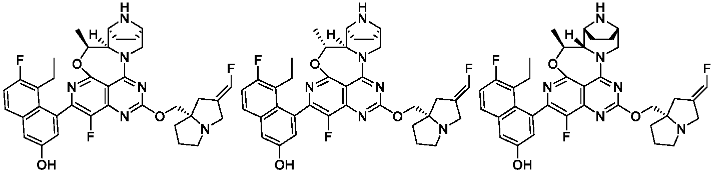 | | |
| 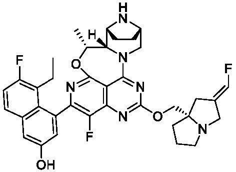 | | |
| 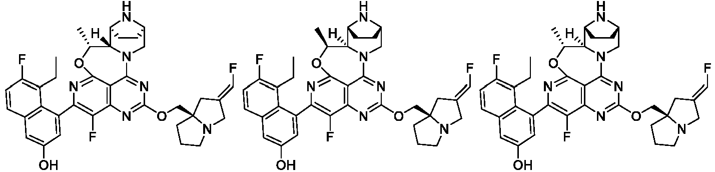 | | |
| 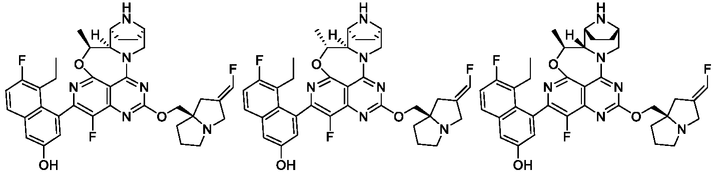 | | |
| 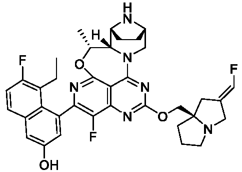 | | |
| | 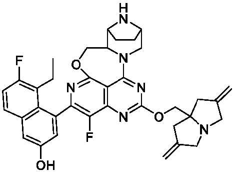 | 4-(12-((2,6-Dimethylenetetrahydro-1*H-*pyrrolizin-7*a*(5*H*)-yl)methoxy)-1-fluoro-5*a*,6,7,8,9,10-hexahydro-5*H*-4-oxa-3,10*a*,11,13,14-pentaaza-6,9-methanonaphtho[1,8-*ab*]heptalen-2-yl)-5-ethyl-6-fluoronaphthalen-2-ol |
| 20 | 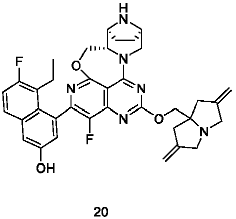 | 4-((5*aS,*6*S,*9*R*)-12-((2,6-Dimethylenetetrahydro-1*H*-pyrrolizin-7*a*(5*H*)-yl)methoxy)-1-fluoro-5*a*,6,7,8,9,10-hexahydro-5*H*-4-oxa-3,10*a*,11,13,14-pentaaza-6,9-methanonaphtho[1,8-*ab*]heptalen-2-yl)-5-ethyl-6-fluoronaphthalen-2-ol **20** |
| 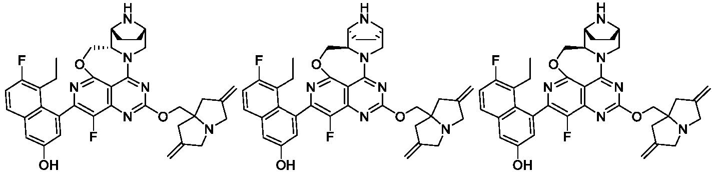 | | |
| | 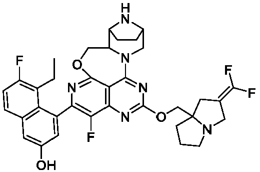 | 4-(12-((2-(Difluoromethylene)tetrahydro-1*H*-pyrrolizin-7*a*(5*H*)-yl)methoxy)-1-fluoro-5*a*,6,7,8,9,10-hexahydro-5*H*-4-oxa-3,10*a*,11,13,14-pentaaza-6,9-methanonaphtho[1,8-*ab*]heptalen-2-yl)-5-ethyl-6-fluoronaphthalen-2-ol |
| 21 | 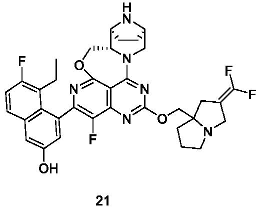 | 4-((5*aS*,6*S*,9*R*)-12-((2-(Difluoromethylene)tetrahydro-1*H-*pyrrolizin-7*a*(5*H*)-yl)methoxy)-1-fluoro-5*a,*6,7,8,9,10-hexahydro-5*H*-4-oxa-3,10*a*,11,13,14-pentaaza-6,9-methanonaphtho[1,8-*ab*]heptalen-2-yl)-5-ethyl-6-fluoronaphthalen-2-ol **21** |
| 21-p1 | 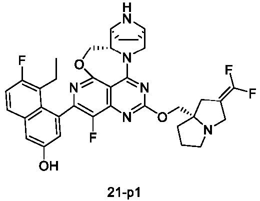 | 4-((5*aS*,6*S*,9*R*)-12-(((*S*)-2-(Difluoromethylene)tetrahydro-1H-pyrrolizin-7*a*(5*H*)-yl)methoxy)-1-fluoro-5*a*,6,7,8,9,10-hexahydro-5*H*-4-oxa-3,10*a*,11,13,14-pentaaza-6,9-methanonaphtho[1,8-*ab*]heptalen-2-yl)-5-ethyl-6-fluoronaphthalen-2-ol **21-p1** |
| 21-p2 | 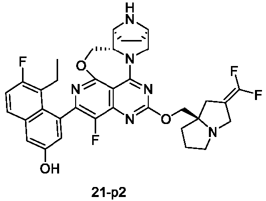 | 4-((5*aS*,6*S*,9*R*)-12-(((*R*)-2-(Difluoromethylene)tetrahydro-1*H-*pyrrolizin-7*a*(5*H*)-yl)methoxy)-1-fluoro-5*a*,6,7,8,9,10-hexahydro-5*H*-4-oxa-3,10*a*,11,13,14-pentaaza-6,9-methanonaphtho[1,8-*ab*]heptalen-2-yl)-5-ethyl-6-fluoronaphthalen-2-ol **21-p2** |
| 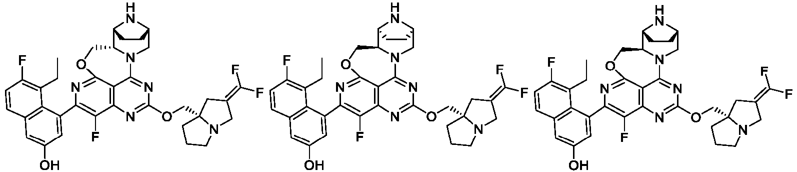 | | |
| 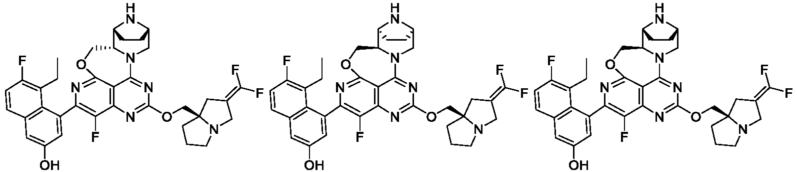 | | |
| | 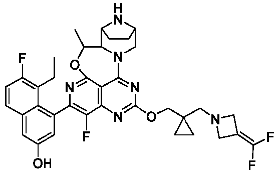 | 4-(12-((1-((3-(Difluoromethylene)azetidin-1-yl)methyl)cyclopropyl)methoxy)-1-fluoro-5-methyl-5*a*,6,7,8,9,10-hexahydro-5*H*-4-oxa-3,10*a*,11,13,14-pentaaza-6,9-methanonaphtho[1,8-*ab*]heptalen-2-yl)-5-ethyl-6-fluoronaphthalen-2-ol |
| 22 | 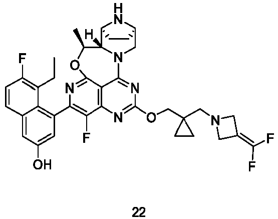 | 4-((5*S*,5*aS*,6*S*,9*R*)-12-((1-((3-(Difluoromethylene)azetidin-1-yl)methyl)cyclopropyl)methoxy)-1-fluoro-5-methyl-5*a*,6,7,8,9,10-hexahydro-5*H*-4-oxa-3,10*a*,11,13,14-pentaaza-6,9-methanonaphtho[1,8-*ab*]heptalen-2-yl)-5-ethyl-6-fluoronaphthalen-2-ol **22** |
| 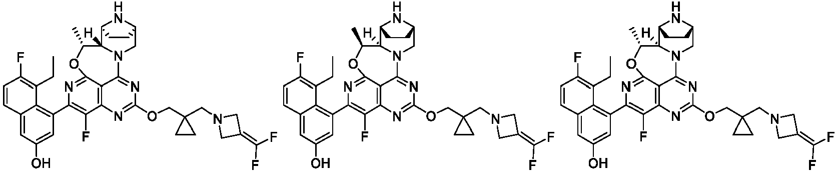 | | |
| 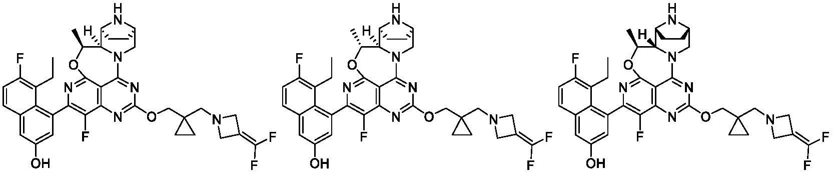 | | |
| 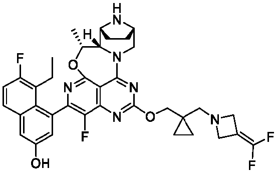 | | |
| | 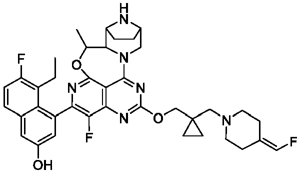 | |
| 23 | 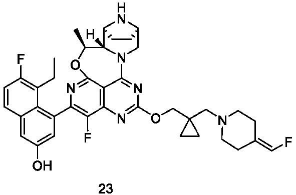 | 5-Ethyl-6-fluoro-4-((5*S*,5*aS*,6*S*,9*R*)-1-fluoro-12-((1-((4-(fluoromethylene)piperidin-1-yl)methyl)cyclopropyl)methoxy)-5-methyl-5*a*,6,7,8,9,10-hexahydro-5*H*-4-oxa-3,10*a*,11,13,14-pentaaza-6,9-methanonaphtho[1,8-*ab*]heptalen-2-yl)naphthalen-2-ol **23** |
| 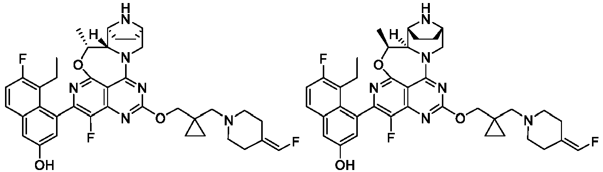 | | |
| 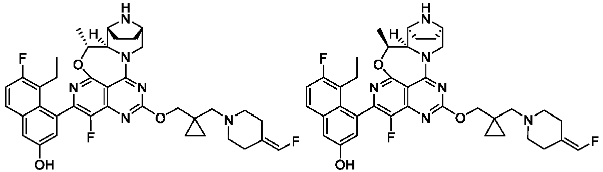 | | |
| 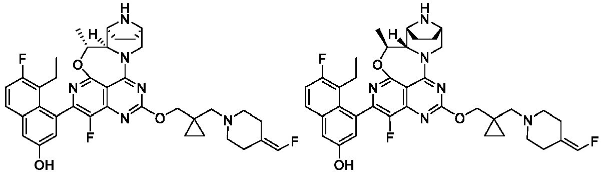 | | |
| 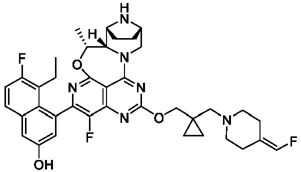 | | |
| 24 | 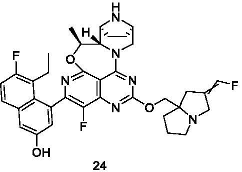 | 5-Ethyl-6-fluoro-4-((5*S*,5*aS*,6*S*,9*R*)-1-fluoro-12-((2-(fluoromethylene)tetrahydro-1*H-*pyrrolizin-7*a*(5*H*)-yl)methoxy)-5-methyl-5*a*,6,7,8,9,10-hexahydro-5*H*-4-oxa-3,10*a*,11,13,14-pentaaza-6,9-methanonaphtho[1,8-*ab*]heptalen-2-yl)naphthalen-2-ol **24** |
| | 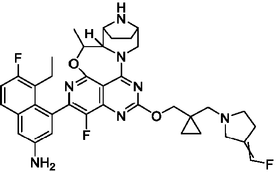 | 5-Ethyl-6-fluoro-4-(1-fluoro-12-((1-((3-(fluoromethylene)pyrrolidin-1-yl)methyl)cyclopropyl)methoxy)-5-methyl-5*a*,6,7,8,9,10-hexahydro-5*H*-4-oxa-3,10*a*,11,13,14-pentaaza-6,9-methanonaphtho[1,8-*ab*]heptalen-2-yl)naphthalen-2-amine |
| | 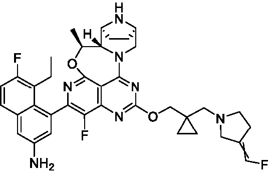 | 5-Ethyl-6-fluoro-4-((5*S*,5*aS*,6*S*,9*R*)-1-fluoro-12-((1-((3-(fluoromethylene)pyrrolidin-1-yl)methyl)cyclopropyl)methoxy)-5-methyl-5*a*,6,7,8,9,10-hexahydro-5*H*-4-oxa-3,10*a*,11,13,14-pentaaza-6,9-methanonaphtho[1,8-*ab*]heptalen-2-yl)naphthalen-2-amine |
| 26 | 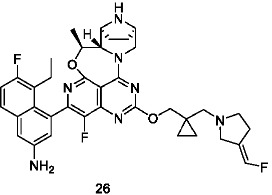 | 5-Ethyl-6-fluoro-4-((5*S*,5*aS*,6*S*,9*R*)-1-fluoro-12-((1-(((*E*)-3-(fluoromethylene)pyrrolidin-1-yl)methyl)cyclopropyl)methoxy)-5-methyl-5*a*,6,7,8,9,10-hexahydro-5*H*-4-oxa-3,10*a*,11,13,14-pentaaza-6,9-methanonaphtho[1,8-*ab*]heptalen-2-yl)naphthalen-2-amine **26** |
| 27 | 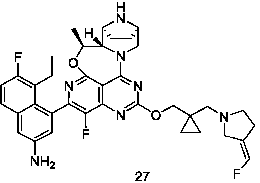 | 5-Ethyl-6-fluoro-4-((5*S*,5*aS*,6*S*,9*R*)-1-fluoro-12-((1-(((Z)-3-(fluoromethylene)pyrrolidin-1-yl)methyl)cyclopropyl)methoxy)-5-methyl-5*a*,6,7,8,9,10-hexahydro-5*H*-4-oxa-3,10*a*,11,13,14-pentaaza-6,9-methanonaphtho[1,8-*ab*]heptalen-2-yl)naphthalen-2-amine **27** |
| 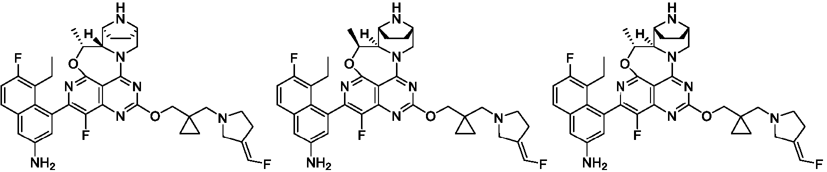 | | |
| 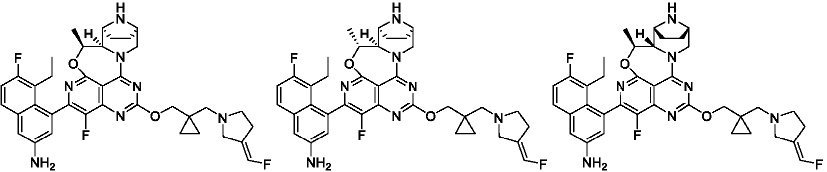 | | |
| 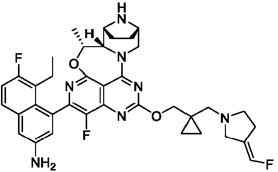 | | |
| 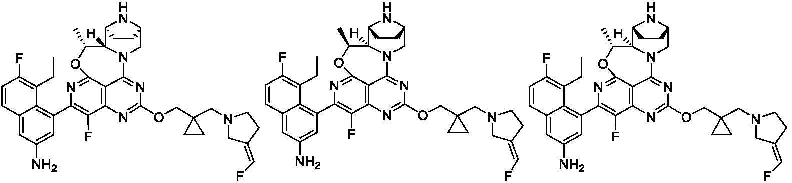 | | |
| 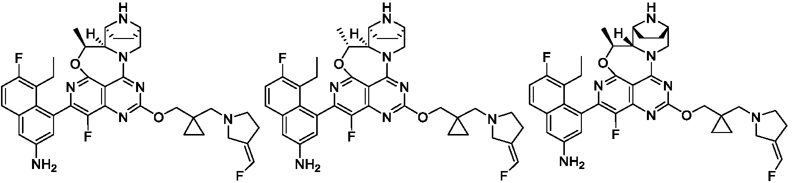 | | |
| 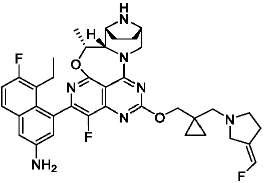 | | |
| | 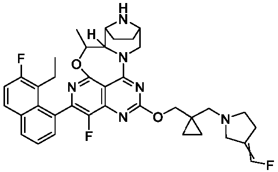 | 2-(8-Ethyl-7-fluoronaphthalen-1-yl)-1-fluoro-12-((1-((3-(fluoromethylene)pyrrolidin-1-yl)methyl)cyclopropyl)methoxy)-5-methyl-5*a*,6,7,8,9,10-hexahydro-5*H*-4-oxa-3,10*a*,11,13,14-pentaaza-6,9-methanonaphtho[1,8-*ab*]heptalene |
| | 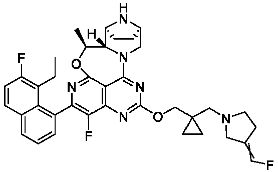 | (5*S*,5*aS*,6*S*,9*R*)-2-(8-Ethyl-7-fluoronaphthalen-1-yl)-1-fluoro-12-((1-((3-(fluoromethylene)pyrrolidin-1-yl)methyl)cyclopropyl)methoxy)-5-methyl-5*a*,6,7,8,9,10-hexahydro-5*H*-4-oxa-3,10*a*,11,13,14-pentaaza-6,9-methanonaphtho[1,8-*ab*]heptalene |
| 28 | 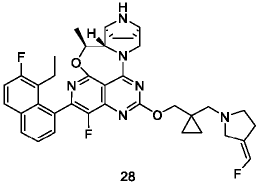 | (5*S*,5*aS*,6*S*,9*R*)-2-(8-Ethyl-7-fluoronaphthalen-1-yl)-1-fluoro-12-((1-(((Z)-3-(fluoromethylene)pyrrolidin-1-yl)methyl)cyclopropyl)methoxy)-5-methyl-5*a*,6,7,8,9,10-hexahydro-5*H*-4-oxa-3,10*a*,11,13,14-pentaaza-6,9-methanonaphtho[1,8-*ab*]heptalene **28** |
| 29 | 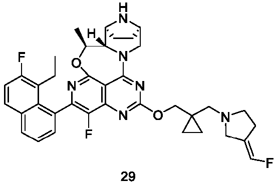 | (5*S*,5*aS*,6*S*,9*R*)-2-(8-Ethyl-7-fluoronaphthalen-1-yl)-1-fluoro-12-((1-(((*E*)-3-(fluoromethylene)pyrrolidin-1-yl)methyl)cyclopropyl)methoxy)-5-methyl-5*a*,6,7,8,9,10-hexahydro-5*H*-4-oxa-3,10*a*,11,13,14-pentaaza-6,9-methanonaphtho[1,8-*ab*]heptalene **29** |
| 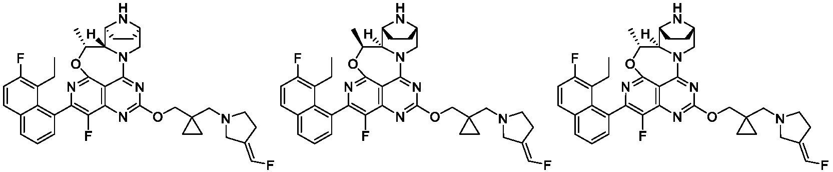 | | |
| 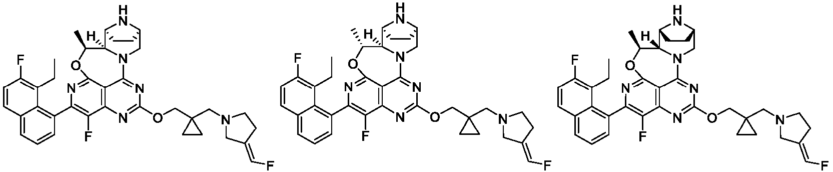 | | |
| 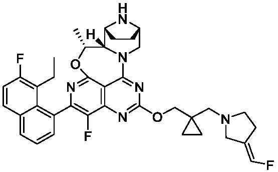 | | |
| 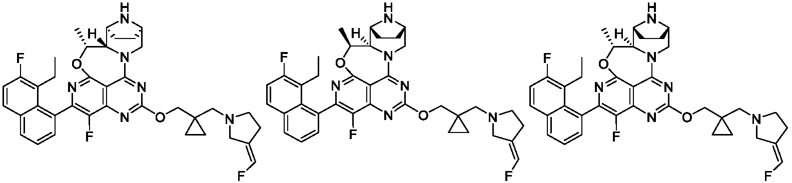 | | |
| 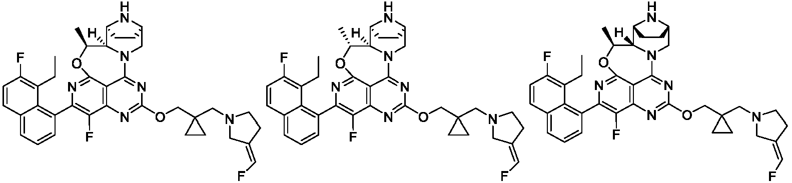 | | |
| 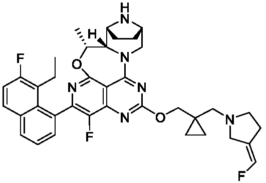 | | |
| | 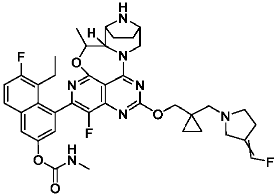 | 5-Ethyl-6-fluoro-4-(1-fluoro-12-((1-((3-(fluoromethylene)pyrrolidin-1-yl)methyl)cyclopropyl)methoxy)-5-methyl-5*a*,6,7,8,9,10-hexahydro-5*H*-4-oxa-3,10*a*,11,13,14-pentaaza-6,9-methanonaphtho[1,8-*ab*]heptalen-2-yl)naphthalen-2-yl methylcarbamate |
| | 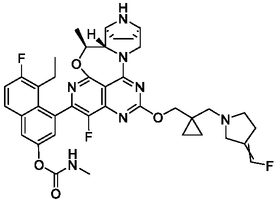 | 5-Ethyl-6-fluoro-4-((5*S*,5*aS*,6*S*,9*R*)-1-fluoro-12-((1-((3-(fluoromethylene)pyrrolidin-1-yl)methyl)cyclopropyl)methoxy)-5-methyl-5*a*,6,7,8,9,10-hexahydro-5*H*-4-oxa-3,10*a*,11,13,14-pentaaza-6,9-methanonaphtho[1,8-*ab*]heptalen-2-yl)naphthalen-2-yl methylcarbamate |
| 30 | 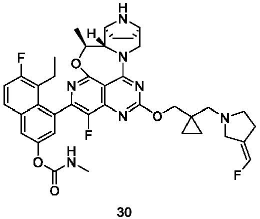 | 5-Ethyl-6-fluoro-4-((5*S*,5*aS*,6*S*,9*R*)-1-fluoro-12-((1-(((Z)-3-(fluoromethylene)pyrrolidin-1-yl)methyl)cyclopropyl)methoxy)-5-methyl-5*a*,6,7,8,9,10-hexahydro-5*H*-4-oxa-3,10*a*,11,13,14-pentaaza-6,9-methanonaphtho[1,8-*ab*]heptalen-2-yl)naphthalen-2-yl methylcarbamate **30** |
| 31 | 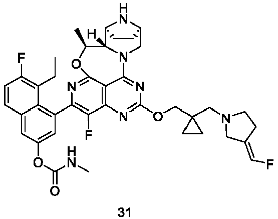 | 5-Ethyl-6-fluoro-4-((5*S*,5*aS*,6*S*,9*R*)-1-fluoro-12-((1-(((*E*)-3-(fluoromethylene)pyrrolidin-1-yl)methyl)cyclopropyl)methoxy)-5-methyl-5*a*,6,7,8,9,10-hexahydro-5*H*-4-oxa-3,10*a*,11,13,14-pentaaza-6,9-methanonaphtho[1,8-*ab*]heptalen-2-yl)naphthalen-2-yl methylcarbamate **31** |
| 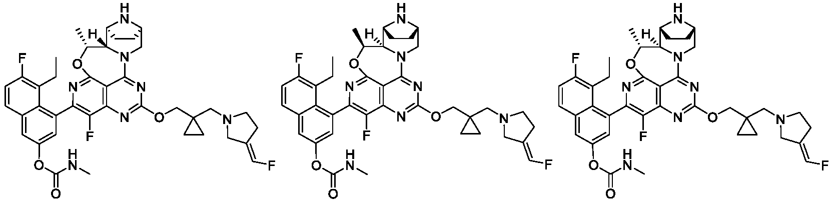 | | |
| 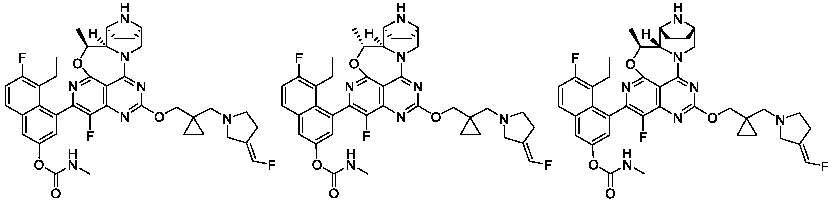 | | |
| 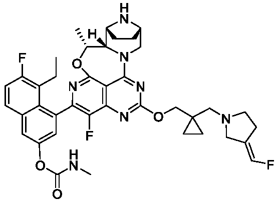 | | |
| 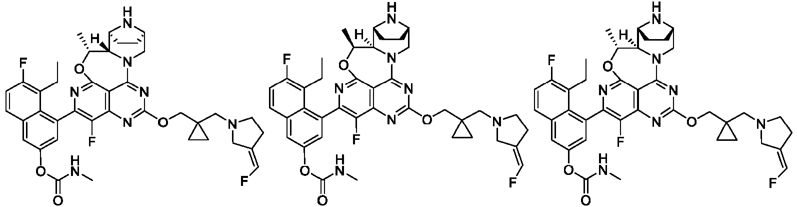 | | |
| 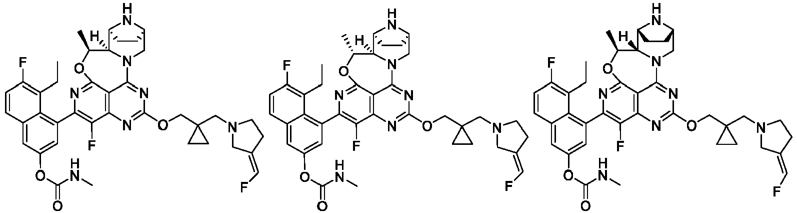 | | |
| 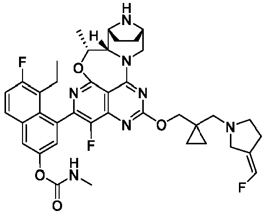 | | |
| | 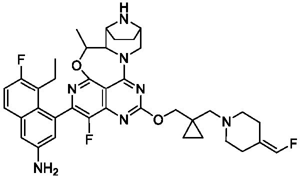 | 5-Ethyl-6-fluoro-4-(1-fluoro-12-((1-((4-(fluoromethylene)piperidin-1-yl)methyl)cyclopropyl)methoxy)-5-methyl-5*a*,6,7,8,9,10-hexahydro-5*H*-4-oxa-3,10*a*,11,13,14-pentaaza-6,9-methanonaphtho[1,8-*ab*]heptalen-2-yl)naphthalen-2-amine |
| 32 | 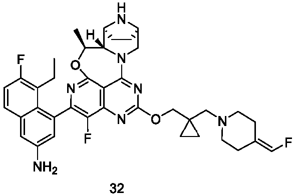 | 5-Ethyl-6-fluoro-4-((5*S*,5*aS*,6*S*,9*R*)-1-fluoro-12-((1-((4-(fluoromethylene)piperidin-1-yl)methyl)cyclopropyl)methoxy)-5-methyl-5*a*,6,7,8,9,10-hexahydro-5*H*-4-oxa-3,10*a*,11,13,14-pentaaza-6,9-methanonaphtho[1,8-*ab*]heptalen-2-yl)naphthalen-2-amine **32** |
| 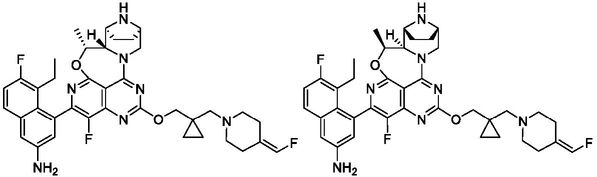 | | |
| 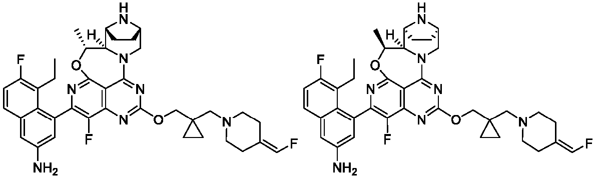 | | |
| 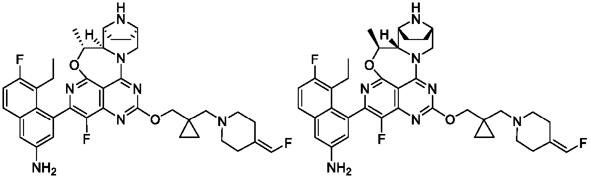 | | |
| 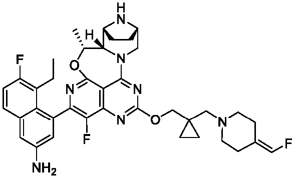 | | |
| | 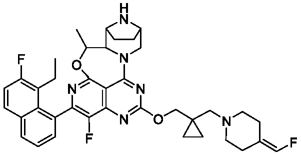 | 2-(8-Ethyl-7-fluoronaphthalen-1-yl)-1-fluoro-12-((1-((4-(fluoromethylene)piperidin-1-yl)methyl)cyclopropyl)methoxy)-5-methyl-5*a*,6,7,8,9,10-hexahydro-5*H*-4-oxa-3,10*a*,11,13,14-pentaaza-6,9-methanonaphtho[1,8-*ab*]heptalene |
| 33 | 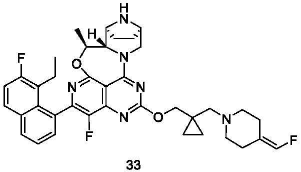 | (5*S*,5*aS*,6*S*,9*R*)-2-(8-Ethyl-7-fluoronaphthalen-1-yl)-1-fluoro-12-((1-((4-(fluoromethylene)piperidin-1-yl)methyl)cyclopropyl)methoxy)-5-methyl-5*a*,6,7,8,9,10-hexahydro-5*H*-4-oxa-3,10*a*,11,13,14-pentaaza-6,9-methanonaphtho[1,8-*ab*]heptalene **33** |
| 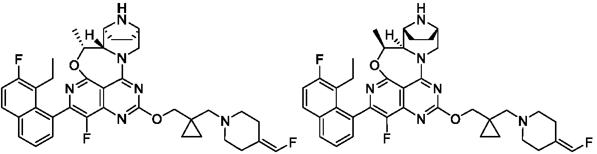 | | |
| 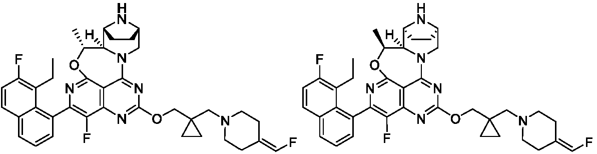 | | |
| 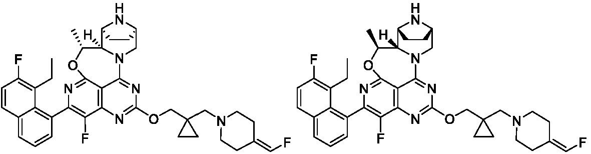 | | |
| 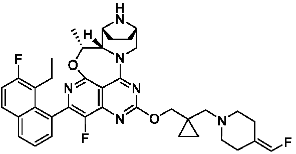 | | |
| | 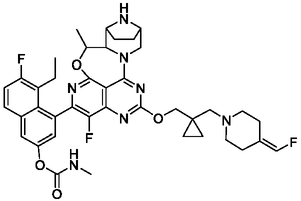 | 5-Ethyl-6-fluoro-4-(1-fluoro-12-((1-((4-(fluoromethylene)piperidin-1-yl)methyl)cyclopropyl)methoxy)-5-methyl-5*a*,6,7,8,9,10-hexahydro-5*H*-4-oxa-3,10*a*,11,13,14-pentaaza-6,9-methanonaphtho[1,8-*ab*]heptalen-2-yl)naphthalen-2-yl methylcarbamate |
| 34 | 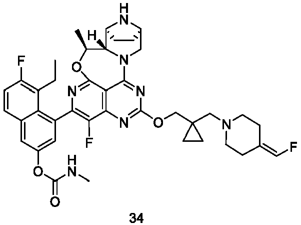 | 5-Ethyl-6-fluoro-4-((5*S*,5*aS*,6*S*,9*R*)-1-fluoro-12-((1-((4-(fluoromethylene)piperidin-1-yl)methyl)cyclopropyl)methoxy)-5-methyl-5*a*,6,7,8,9,10-hexahydro-5*H*-4-oxa-3,10*a*,11,13,14-pentaaza-6,9-methanonaphtho[1,8-*ab*]heptalen-2-yl)naphthalen-2-yl methylcarbamate **34** |
| 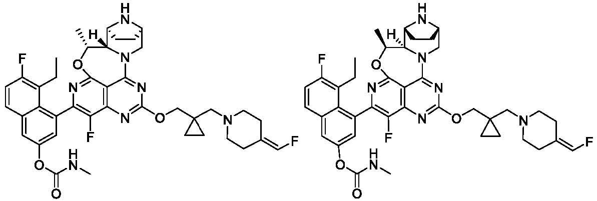 | | |
| 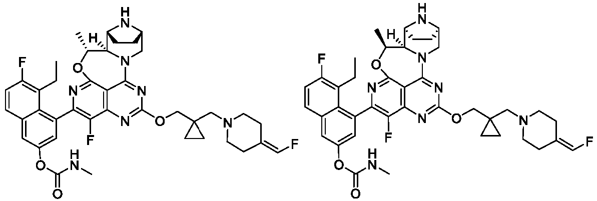 | | |
| 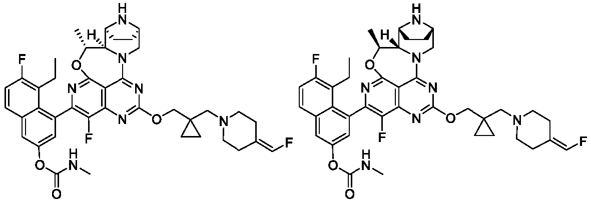 | | |
| 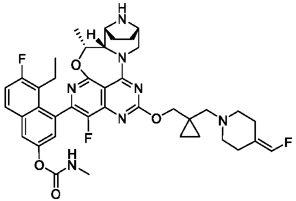 | | |
| | 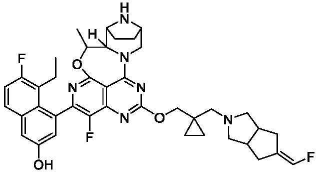 | |
| 35 | 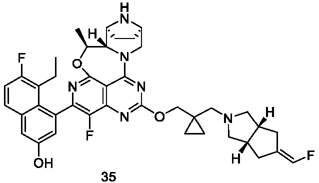 | 5-Ethyl-6-fluoro-4-((5*S*,5*aS*,6*R*,9*R*)-1-fluoro-12-((1-((5-(fluoromethylene)hexahydrocyclopenta[*c*]py rrol-2(1*H*)-yl)methyl)cyclopropyl)methoxy)-5-methyl-5*a*,6,7,8,9,10-hexahydro-5*H*-4-oxa-3,10*a*,11,13,14-pentaaza-6,9-methanonaphtho[1,8-*ab*]heptalen-2-yl)naphthalen-2-ol **35** |
| 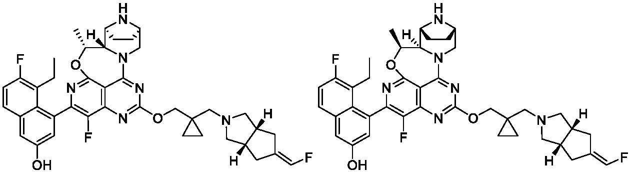 | | |
| 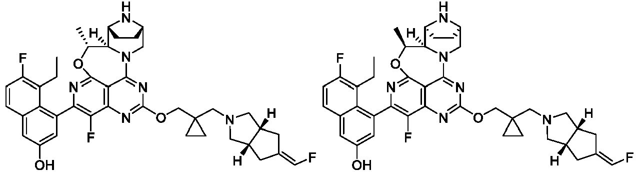 | | |
| 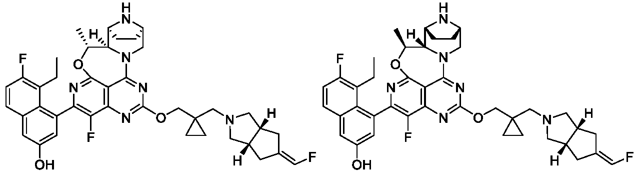 | | |
| 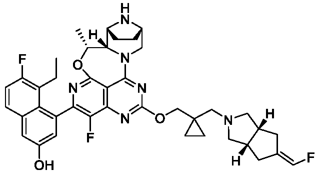 | | |
| 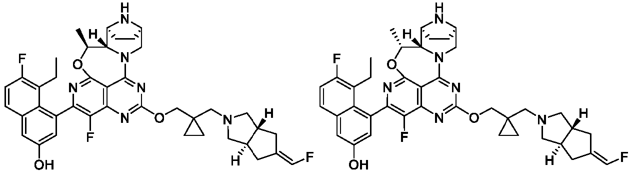 | | |
| 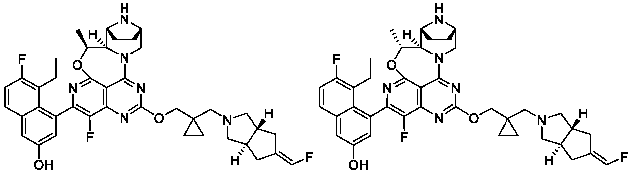 | | |
| 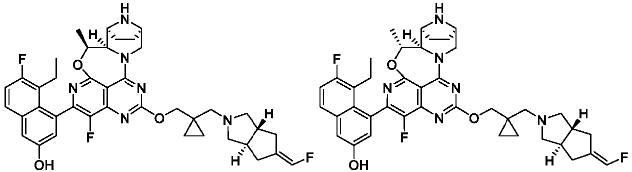 | | |
| 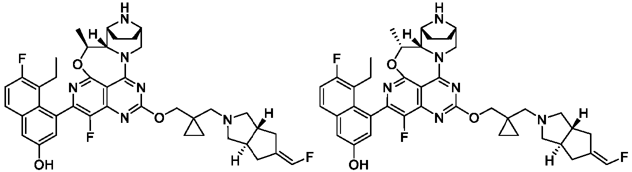 | | |
| | 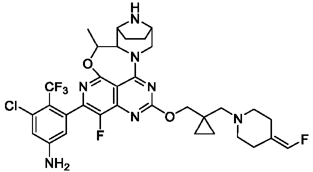 | |
| 36 | 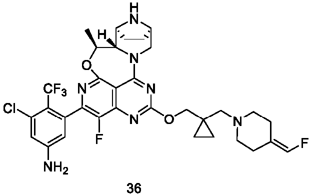 | 3-Chloro-5-((5*S*,5*aS*,6*S*,9*R*)-1-fluoro-12-((1-((4-(fluoromethylene)piperidin-1-yl)methyl)cyclopropyl)methoxy)-5-methyl-5*a*,6,7,8,9,10-hexahydro-5*H*-4-oxa-3,10*a*,11,13,14-pentaaza-6,9-methanonaphtho[1,8-*ab*]heptalen-2-yl)-4-(trifluoromethyl)aniline **36** |
| 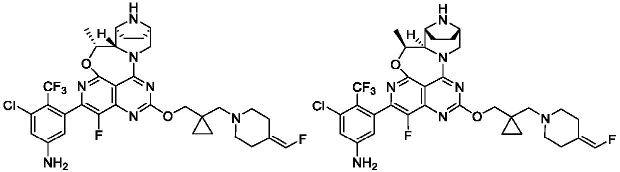 | | |
| 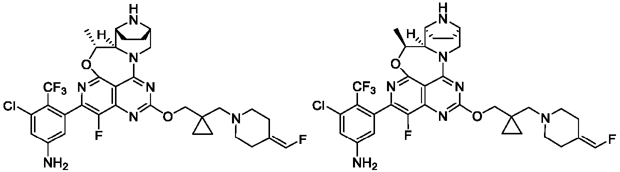 | | |
| 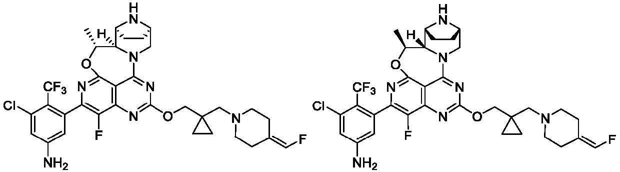 | | |
| 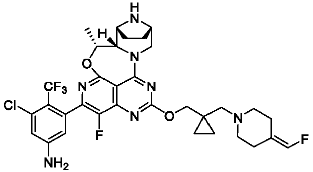 | | |
| | 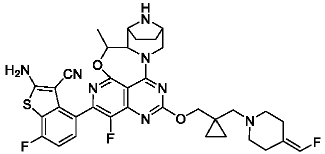 | |
| 37 | 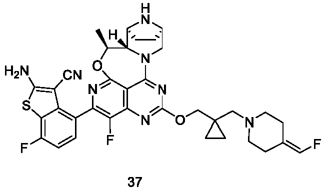 | 2-Amino-7-fluoro-4-((5*S*,5*aS*,6*S*,9*R*)-1-fluoro-12-((1-((4-(fluoromethylene)piperidin-1-yl)methyl)cyclopropyl)methoxy)-5-methyl-5*a*,6,7,8,9,10-hexahydro-5*H*-4-oxa-3,10*a*,11,13,14-pentaaza-6,9-methanonaphtho[1,8-*ab*]heptalen-2-yl)benzo[*b*]thiophene-3-carbonitrile **37** |
| 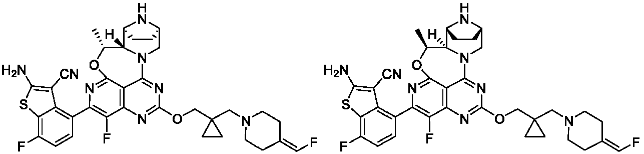 | | |
| 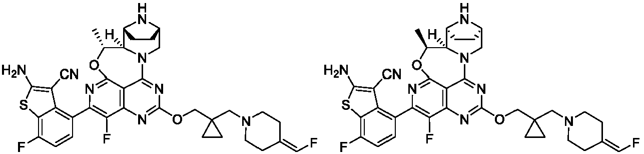 | | |
| 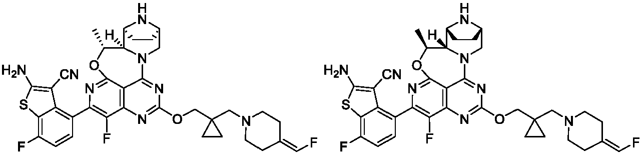 | | |
| 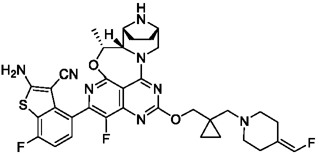 | | |
| | 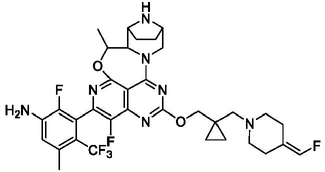 | |
| 38 | 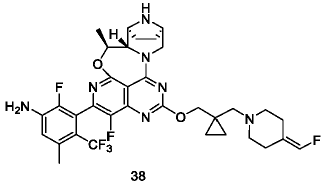 | 2-Fluoro-3-((5*S*,5*aS*,6*S*,9*R*)-1-fluoro-12-((1-((4-(fluoromethylene)piperidin-1-yl)methyl)cyclopropyl)methoxy)-5-methyl-5*a*,6,7,8,9,10-hexahydro-5*H*-4-oxa-3,10*a*,11,13,14-pentaaza-6,9-methanonaphtho[1,8-*ab*]heptalen-2-yl)-5-methyl-4-(trifluoromethyl)aniline **38** |
| 38-p1 | 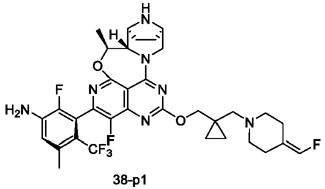 | 2-Fluoro-3-((2*S*,5*S*,5*aS*,6*S*,9*R*)-1-fluoro-12-((1-((4-(fluoromethylene)piperidin-1-yl)methyl)cyclopropyl)methoxy)-5-methyl-5*a*,6,7,8,9,10-hexahydro-5*H*-4-oxa-3,10*a*,11,13,14-pentaaza-6,9-methanonaphtho[1,8-*ab*]heptalen-2-yl)-5-methyl-4-(trifluoromethyl)aniline **38-p1** |
| 38-p2 | 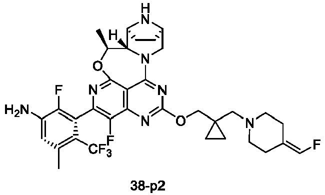 | 2-Fluoro-3-((2*R*,5*S*,5*aS*,6*S*,9*R*)-1-fluoro-12-((1-((4-(fluoromethylene)piperidin-1-yl)methyl)cyclopropyl)methoxy)-5-methyl-5*a*,6,7,8,9,10-hexahydro-5*H*-4-oxa-3,10*a*,11,13,14-pentaaza-6,9-methanonaphtho[1,8-*ab*]heptalen-2-yl)-5-methyl-4-(trifluoromethyl)aniline **38-p2** |
| 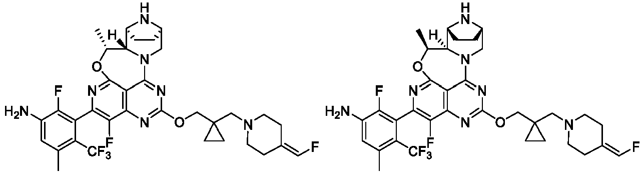 | | |
| 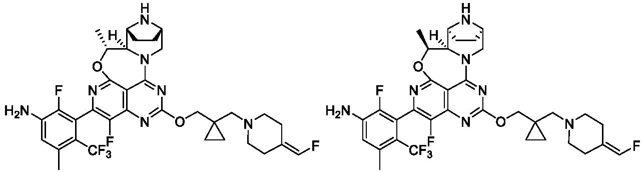 | | |
| 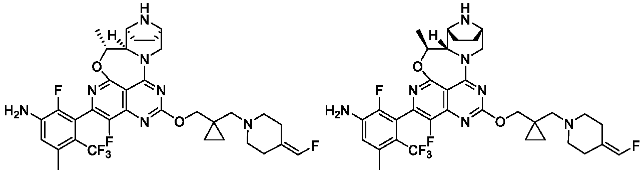 | | |
| 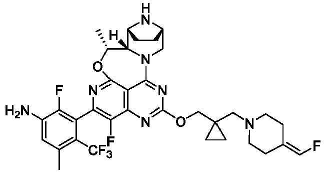 | | |
| 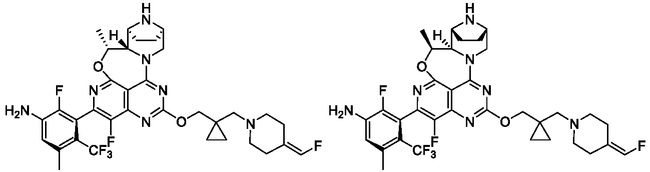 | | |
| 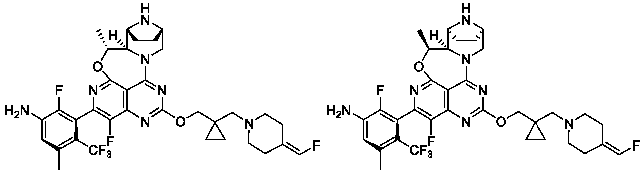 | | |
| 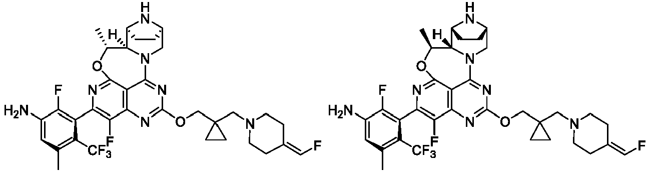 | | |
| 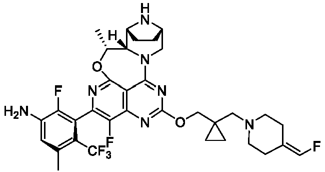 | | |
| 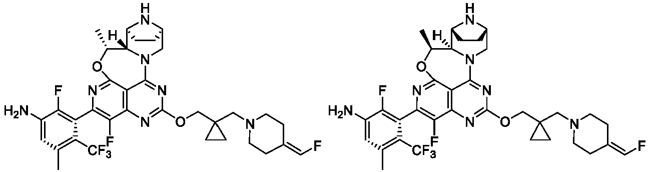 | | |
| 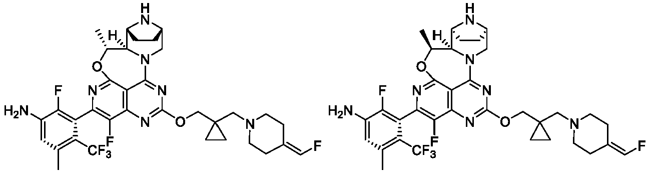 | | |
| 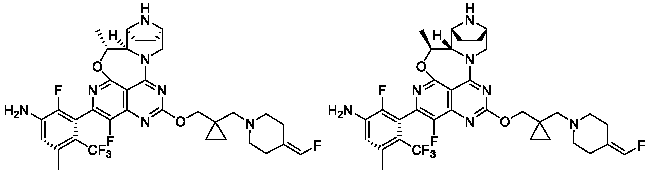 | | |
| 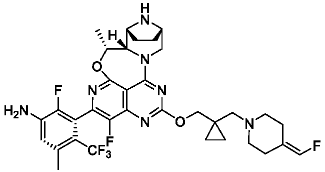 | | |
| | 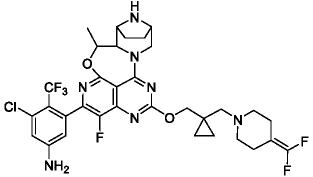 | |
| 39 | 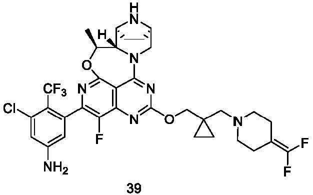 | 3-Chloro-5-((5*S*,5*aS*,6*S*,9*R*)-12-((1-((4-(difluoromethylene)piperidin-1-yl)methyl)cyclopropyl)methoxy)-1-fluoro-5-methyl-5*a*,6,7,8,9,10-hexahydro-5*H*-4-oxa-3,10*a*,11,13,14-pentaaza-6,9-methanonaphtho[1,8-*ab*]heptalen-2-yl)-4-(trifluoromethyl)aniline **39** |
| 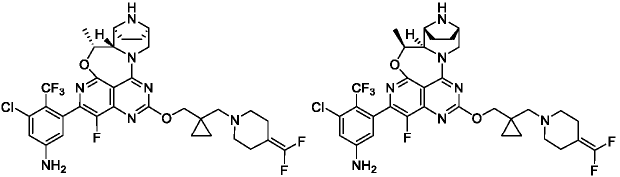 | | |
| 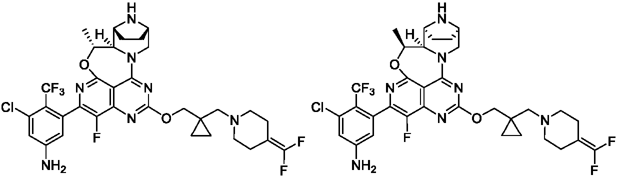 | | |
| 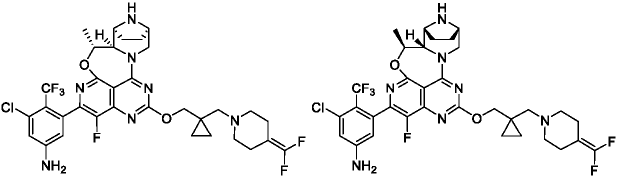 | | |
| 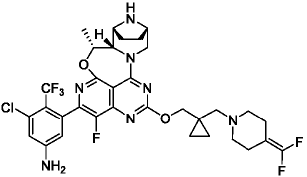 | | |
| | 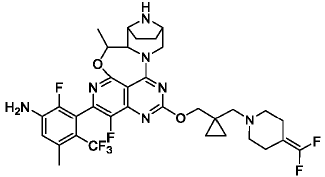 | |
| 40 | 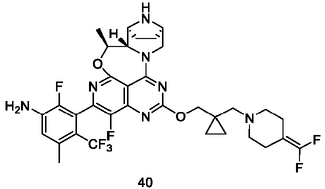 | 3-((5*S*,5*aS*,6*S*,9*R*)-12-((1-((4-(Difluoromethylene)piperidin-1-yl)methyl)cyclopropyl)methoxy)-1-fluoro-5-methyl-5*a*,6,7,8,9,10-hexahydro-5*H*-4-oxa-3,10*a*,11,13,14-pentaaza-6,9-methanonaphtho[1,8-*ab*]heptalen-2-yl)-2-fluoro-5-methyl-4-(trifluoromethyl)aniline **40** |
| 40-p1 | 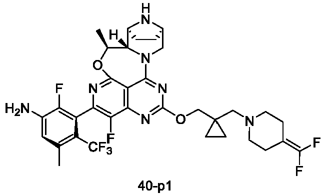 | 3-((2*S*,5*S*,5*aS*,6*S*,9*R*)-12-((1-((4-(Difluoromethylene)piperidin-1-yl)methyl)cyclopropyl)methoxy)-1-fluoro-5-methyl-5*a*,6,7,8,9,10-hexahydro-5*H*-4-oxa-3,10*a*,11,13,14-pentaaza-6,9-methanonaphtho[1,8-*ab*]heptalen-2-yl)-2-fluoro-5-methyl-4-(trifluoromethyl)aniline **40-p1** |
| 40-p2 | 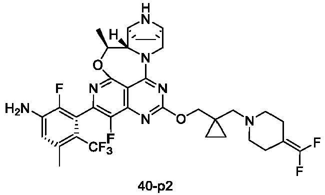 | 3-((2*R*,5*S*,5*aS*,6*S*,9*R*)-12-((1-((4-(Difluoromethylene)piperidin-1-yl)methyl)cyclopropyl)methoxy)-1-fluoro-5-methyl-5*a*,6,7,8,9,10-hexahydro-5*H*-4-oxa-3,10*a*,11,13,14-pentaaza-6,9-methanonaphtho[1,8-*ab*]heptalen-2-yl)-2-fluoro-5-methyl-4-(trifluoromethyl)aniline **40-p2** |
| 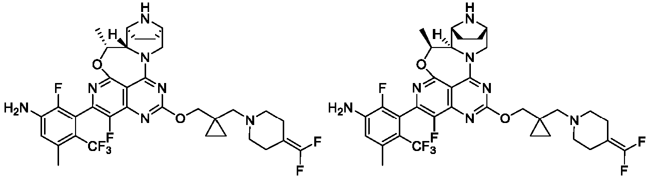 | | |
| 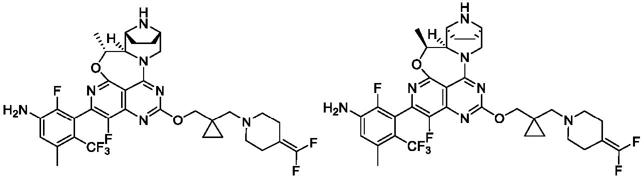 | | |
| 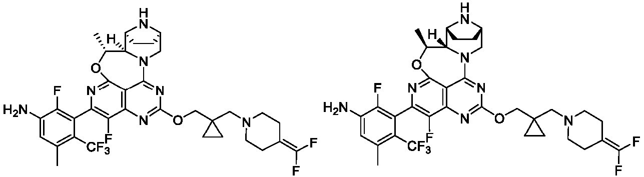 | | |
| 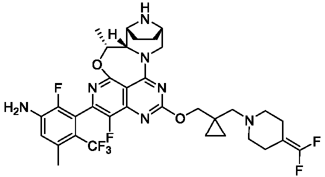 | | |
| 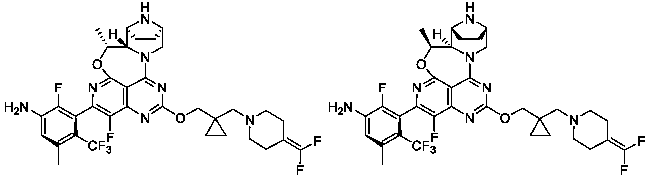 | | |
| 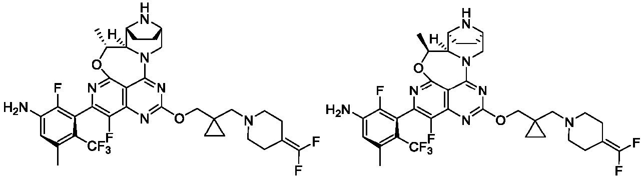 | | |
| 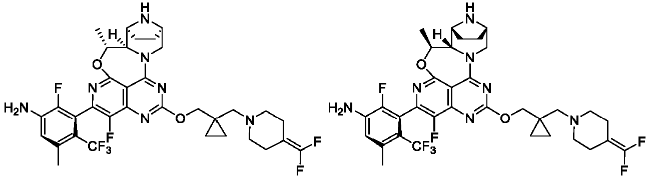 | | |
| 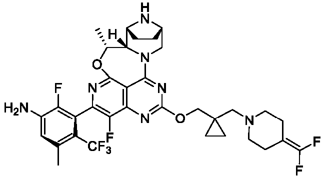 | | |
| 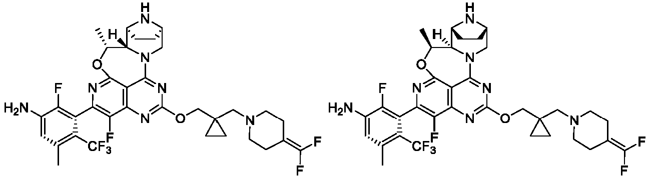 | | |
| 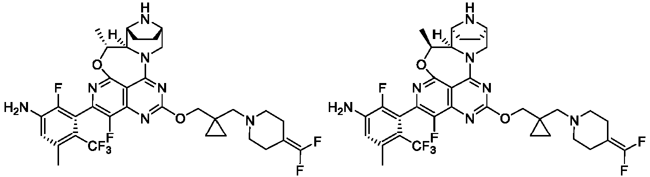 | | |
| 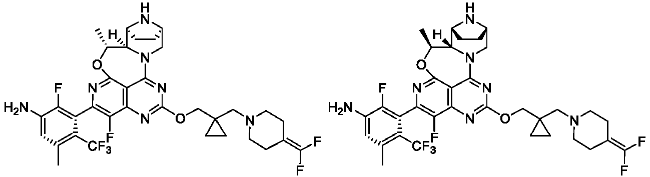 | | |
| 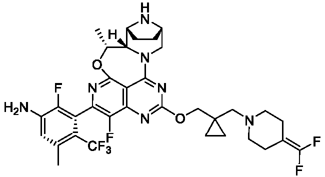 | | |
| | 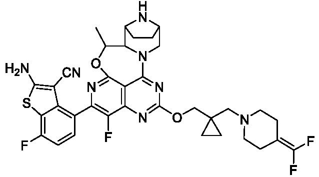 | |
| 41 | 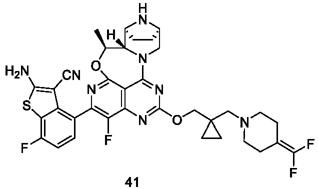 | 2-Amino-4-((5*S*,5*aS*,6*S*,9*R*)-12-((1-((4-(difluoromethylene)piperidin-1-yl)methyl)cyclopropyl)methoxy)-1-fluoro-5-methyl-5*a*,6,7,8,9,10-hexahydro-5*H*-4-oxa-3,10*a*,11,13,14-pentaaza-6,9-methanonaphtho[1,8-*ab*]heptalen-2-yl)-7-fluorobenzo[b]thiophene-3-carbonitrile **41** |
| 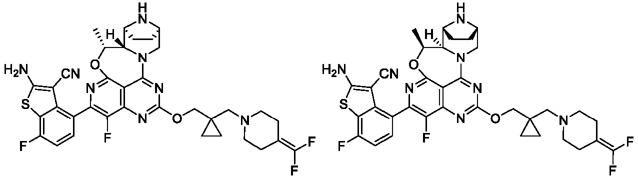 | | |
| 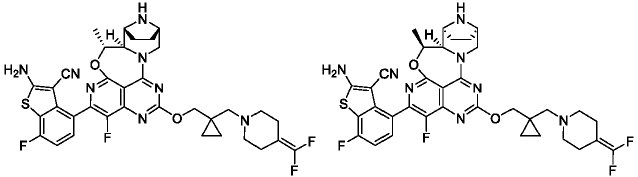 | | |
| 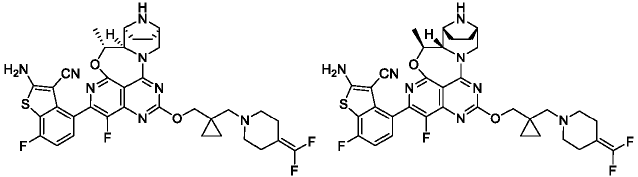 | | |
| 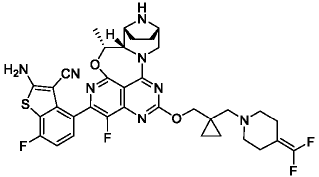 | | |
| | 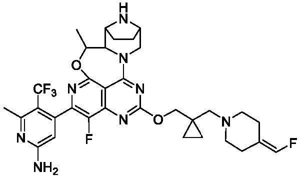 | 4-(1-Fluoro-12-((1-((4-(fluoromethylene)piperidin-1-yl)methyl)cyclopropyl)methoxy)-5-methyl-5*a*,6,7,8,9,10-hexahydro-5*H*-4-oxa-3,10*a*,11,13,14-pentaaza-6,9-methanonaphtho[1,8-*ab*]heptalen-2-yl)-6-methyl-5-(trifluoromethyl)pyridin-2-amine |
| 42 | 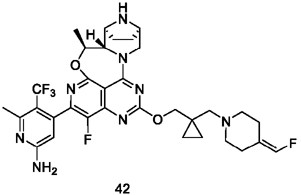 | 4-((5*S*,5*aS*,6*S*,9*R*)-1-Fluoro-12-((1-((4-(fluoromethylene)piperidin-1-yl)methyl)cyclopropyl)methoxy)-5-methyl-5*a*,6,7,8,9,10-hexahydro-5*H*-4-oxa-3,10*a*,11,13,14-pentaaza-6,9-methanonaphtho[1,8-*ab*]heptalen-2-yl)-6-methyl-5-(trifluoromethyl)pyridin-2-amine **42** |
| 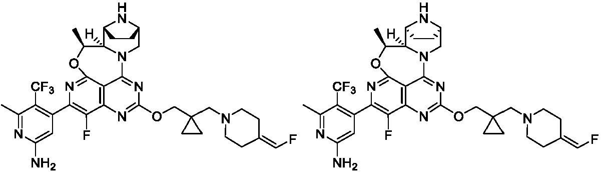 | | |
| 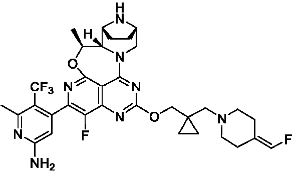 | | |
| 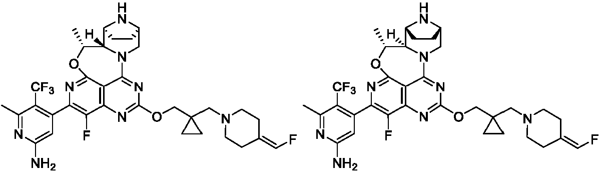 | | |
| 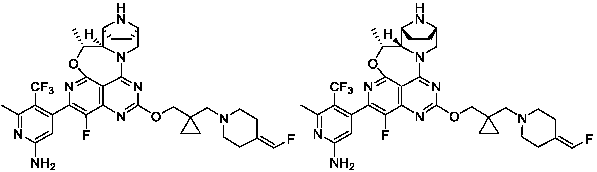 | | |
| | 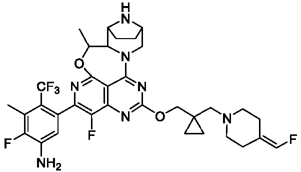 | 2-Fluoro-5-(1-fluoro-12-((1-((4-(fluoromethylene)piperidin-1-yl)methyl)cyclopropyl)methoxy)-5-methyl-5*a*,6,7,8,9,10-hexahydro-5*H*-4-oxa-3,10*a*,11,13,14-pentaaza-6,9-methanonaphtho[1,8-*ab*]heptalen-2-yl)-3-methyl-4-(trifluoromethyl)aniline |
| 43 | 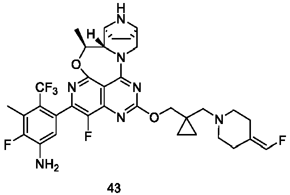 | 2-Fluoro-5-((5*S*,5*aS*,6*S*,9*R*)-1-fluoro-12-((1-((4-(fluoromethylene)piperidin-1-yl)methyl)cyclopropyl)methoxy)-5-methyl-5*a*,6,7,8,9,10-hexahydro-5*H*-4-oxa-3,10*a*,11,13,14-pentaaza-6,9-methanonaphtho[1,8-*ab*]heptalen-2-yl)-3-methyl-4-(trifluoromethyl)aniline **43** |
| 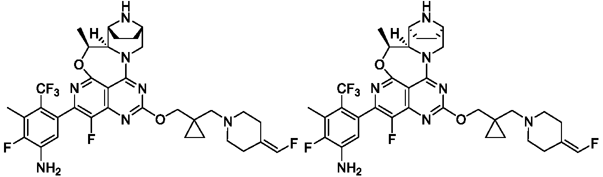 | | |
| 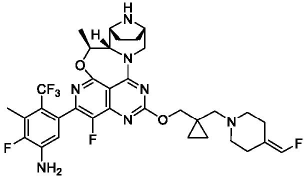 | | |
| 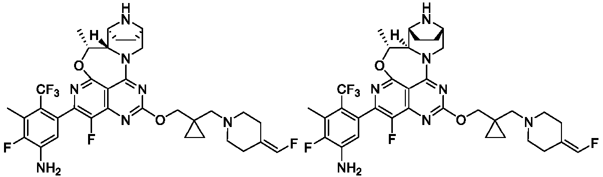 | | |
| 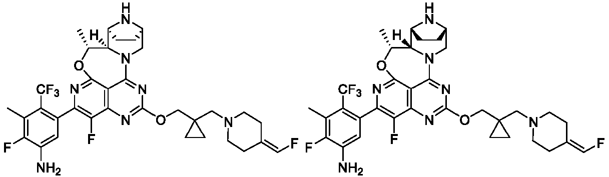 | | |
| | 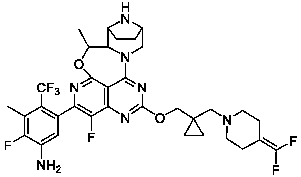 | 5-(12-((1-((4-(Difluoromethylene)piperidin-1-yl)methyl)cyclopropyl)methoxy)-1-fluoro-5-methyl-5*a*,6,7,8,9,10-hexahydro-5*H*-4-oxa-3,10*a*,11,13,14-pentaaza-6,9-methanonaphtho[1,8-*ab*]heptalen-2-yl)-2-fluoro-3-methyl-4-(trifluoromethyl)aniline |
| 44 | 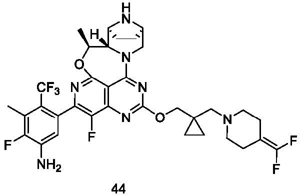 | 5-((5*S*,5*aS*,6*S*,9*R*)-12-((1-((4-(Difluoromethylene)piperidin-1-yl)methyl)cyclopropyl)methoxy)-1-fluoro-5-methyl-5*a*,6,7,8,9,10-hexahydro-5*H*-4-oxa-3,10*a*,11,13,14-pentaaza-6,9-methanonaphtho[1,8-*ab*]heptalen-2-yl)-2-fluoro-3-methyl-4-(trifluoromethyl)aniline 44 |
| 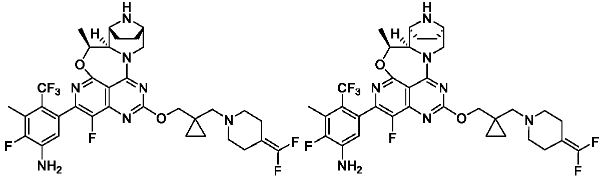 | | |
| 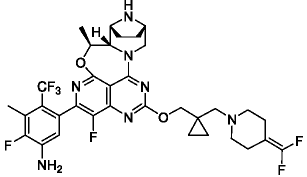 | | |
| 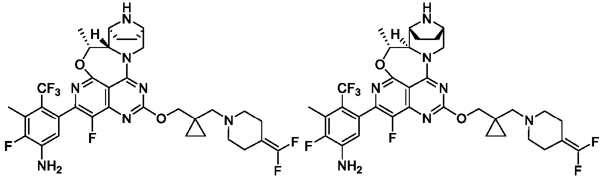 | | |
| 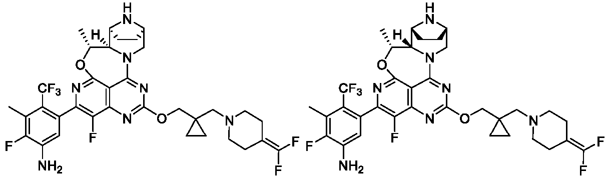 | | |
| | 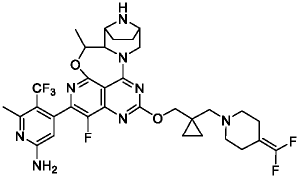 | 4-(12-((1-((4-(Difluoromethylene)piperidin-1-yl)methyl)cyclopropyl)methoxy)-1-fluoro-5-methyl-5*a*,6,7,8,9,10-hexahydro-5*H*-4-oxa-3,10*a*,11,13,14-pentaaza-6,9-methanonaphtho[1,8-*ab*]heptalen-2-yl)-6-methyl-5-(trifluoromethyl)pyridin-2-amine |
| 45 | 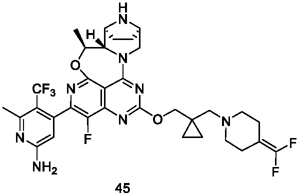 | 4-((5*S*,5*aS*,6*S*,9*R*)-12-((1-((4-(Difluoromethylene)piperidin-1-yl)methyl)cyclopropyl)methoxy)-1-fluoro-5-methyl-5*a*,6,7,8,9,10-hexahydro-5*H*-4-oxa-3,10*a*,11,13,14-pentaaza-6,9-methanonaphtho[1,8-*ab*]heptalen-2-yl)-6-methyl-5-(trifluoromethyl)pyridin-2-amine 45 |
| 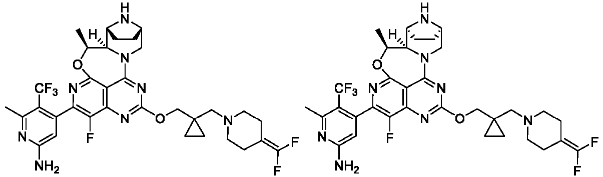 | | |
| 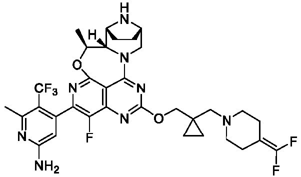 | | |
| 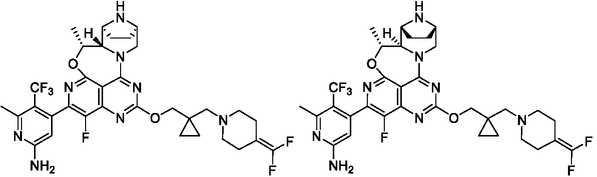 | | |
| 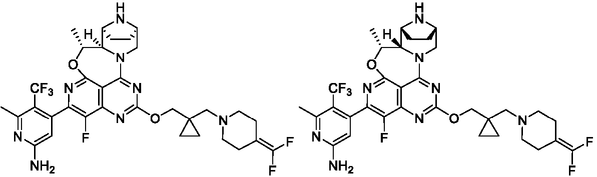 | | |

Another aspect of the present disclosure relates to a compound represented by general formula (INA) or a salt thereof, 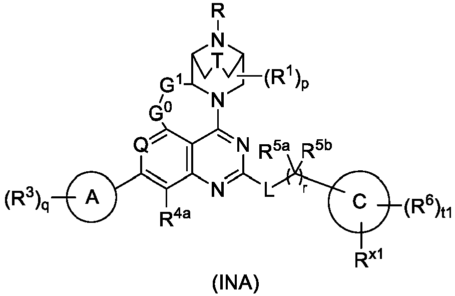 wherein:
R is an amino protecting group, preferably Boc;
G⁰, G¹, T, ring A, ring C, Q, L, R¹, R³, R^{4a}, R^{5a}, R^{5b}, R⁶, R^{x1}, p, q, t1, and r are as defined in general formula (IN).

Another aspect of the present disclosure relates to a compound represented by general formula (IN'A) or a salt thereof, 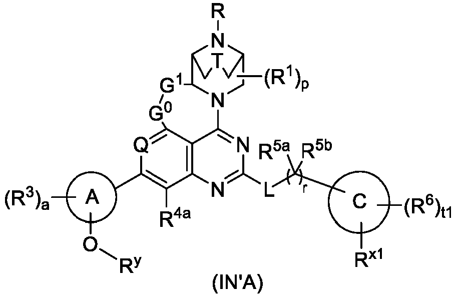 wherein:
R is an amino protecting group, preferably Boc;
R^{y} is a hydroxy protecting group, preferably MOM;
a is 0, 1, 2, 3, or 4;
G⁰, G¹, T, ring A, ring C, Q, L, R¹, R³, R^{4a}, R^{5a}, R^{5b}, R⁶, R^{x1}, p, t1, and r are as defined in general formula (IN').

Another aspect of the present disclosure relates to a compound represented by general formula (IMA) or a salt thereof, 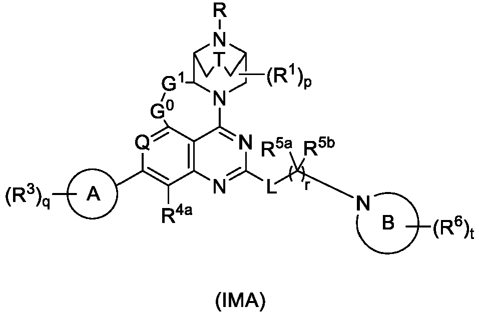 wherein:
R is an amino protecting group, preferably Boc;
G⁰, G¹, T, ring A, ring B, Q, L, R¹, R³, R^{4a}, R^{5a}, R^{5b}, R⁶, p, q, t, and r are as defined in general formula (IM).

Another aspect of the present disclosure relates to a compound represented by general formula (IM'A) or a salt thereof, 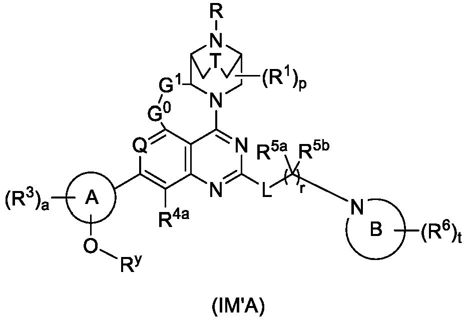 wherein:
R is an amino protecting group, preferably Boc;
R^{y} is a hydroxy protecting group, preferably MOM;
a is 0, 1, 2, 3, or 4;
G⁰, G¹, T, ring A, ring B, Q, L, R¹, R³, R^{4a}, R^{5a}, R^{5b}, R⁶, p, t, and r are as defined in general formula (IM').

Another aspect of the present disclosure relates to a compound represented by general formula (IA) or a salt thereof, 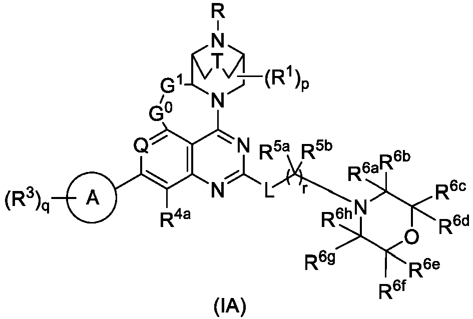 wherein:
R is an amino protecting group, preferably Boc;
G⁰, G¹, T, ring A, Q, L, R¹, R³, R^{4a}, R^{5a}, R^{5b}, R^{6a}, R^{6b}, R^{6c}, R^{6d}, R^{6e}, R^{6f}, R^{6g}, R^{6h}, p, q, and r are as defined in general formula (I).

Another aspect of the present disclosure relates to a compound represented by general formula (I'A) or a salt thereof, 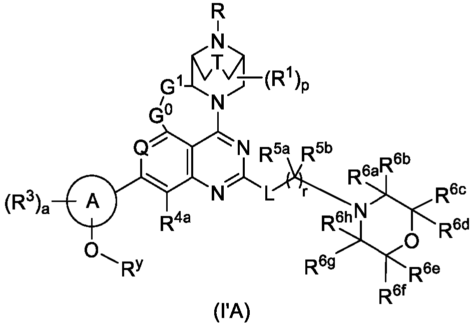 wherein:
R is an amino protecting group, preferably Boc;
R^{y} is a hydroxy protecting group, preferably MOM;
a is 0, 1, 2, 3, or 4;
G⁰, G¹, T, ring A, Q, L, R¹, R³, R^{4a}, R^{5a}, R^{5b}, R^{6a}, R^{6b}, R^{6c}, R^{6d} R^{6e}, R^{6f}, R^{6g}, R^{6h}, p, and r are as defined in general formula (I').

Another aspect of the present disclosure relates to a compound represented by general formula (II'A) or a salt thereof, 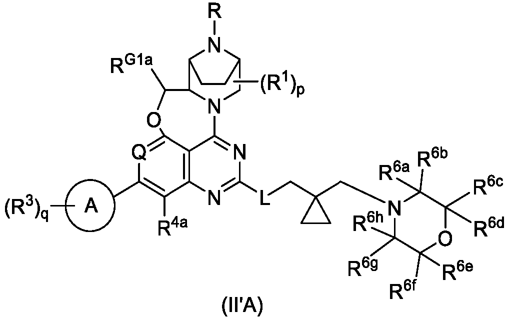 wherein:
R is an amino protecting group, preferably Boc;
ring A, Q, L, R^{G1a}, R¹, R³, R^{4a}, R^{6a}, R^{6b}, R^{6c}, R^{6d}, R^{6e}, R^{6f}, R^{6g}, R^{6h}, p, and q are as defined in general formula (II').

Another aspect of the present disclosure relates to a compound represented by general formula (IIA) or a salt thereof, 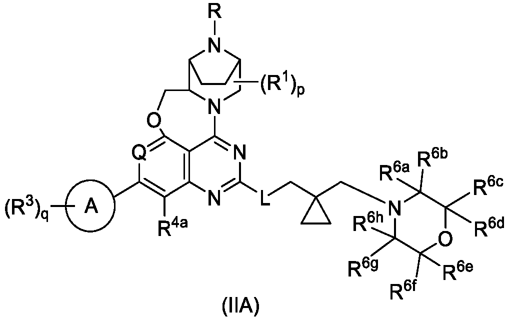 wherein:
R is an amino protecting group, preferably Boc;
ring A, Q, L, R¹, R³, R^{4a}, R^{6a}, R^{6b}, R^{6c}, R^{6d}, R^{6e}, R^{6f}, R^{6g}, R^{6h}, p, and q are as defined in general formula (II).

Another aspect of the present disclosure relates to a compound represented by general formula (III'A) or a salt thereof, 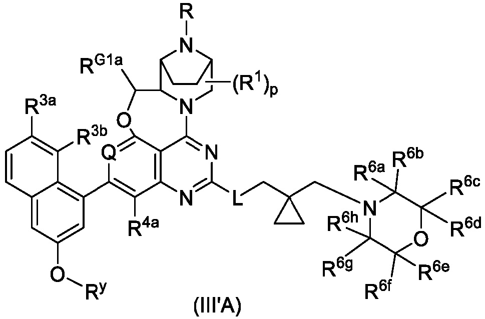 wherein:
R is an amino protecting group, preferably Boc;
R^{y} is a hydroxy protecting group, preferably MOM;
Q, L, R^{G1a}, R¹, R^{3a}, R^{3b}, R^{4a}, R^{6a}, R^{6b}, R^{6c}, R^{6d}, R^{6e}, R^{6f}, R^{6g}, R^{6h}, and p are as defined in general formula (III').

Another aspect of the present disclosure relates to a compound represented by general formula (III'-1A) or a salt thereof, 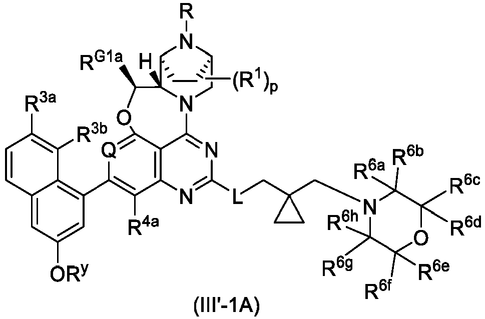 wherein:
R is an amino protecting group, preferably Boc;
R^{y} is a hydroxy protecting group, preferably MOM;
Q, L, R^{G1a}, R¹, R^{3a}, R^{3b}, R^{4a}, R^{6a}, R^{6b}, R^{6c}, R^{6d}, R^{6e}, R^{6f}, R^{6g}, R^{6h}, and p are as defined in general formula (III'-1).

Another aspect of the present disclosure relates to a compound represented by general formula (IIIA) or a salt thereof, 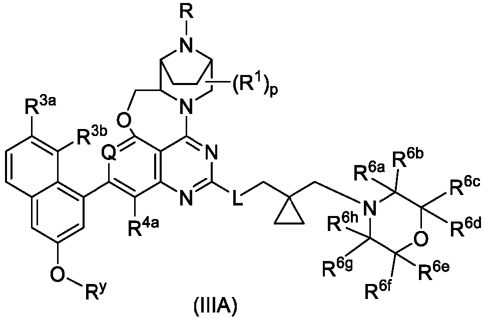 wherein:
R is an amino protecting group, preferably Boc;
R^{y} is a hydroxy protecting group, preferably MOM;
Q, L, R¹, R^{3a}, R^{3b}, R^{4a}, R^{6a}, R^{6b}, R^{6c}, R^{6d}, R^{6e}, R^{6f}, R^{6g}, R^{6h}, and p are as defined in general formula (III).

Another aspect of the present disclosure relates to a compound represented by general formula (IVV'A) or a salt thereof, 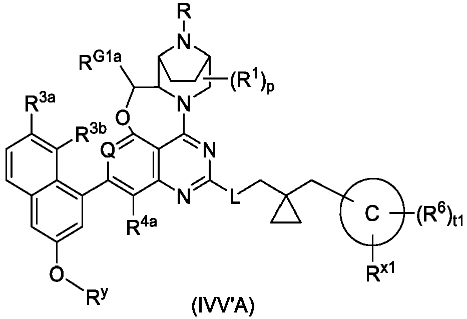 wherein:
R is an amino protecting group, preferably Boc;
R^{y} is a hydroxy protecting group, preferably MOM;
ring C, Q, L, R^{G1a}, R¹, R^{3a}, R^{3b}, R^{4a}, R⁶, R^{x1}, t1, and p are as defined in general formula (IVV').

Another aspect of the present disclosure relates to a compound represented by general formula (IV'A) or a salt thereof, 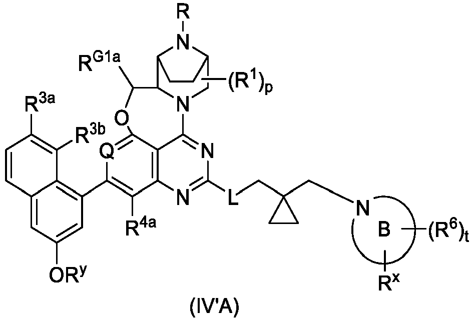 wherein:
R is an amino protecting group, preferably Boc;
R^{y} is a hydroxy protecting group, preferably MOM;
ring B, Q, L, R^{G1a}, R¹, R^{3a}, R^{3b}, R^{4a}, R⁶, R^{x}, t, and p are as defined in general formula (IV').

Another aspect of the present disclosure relates to a compound represented by general formula (IV'-1A) or a salt thereof, 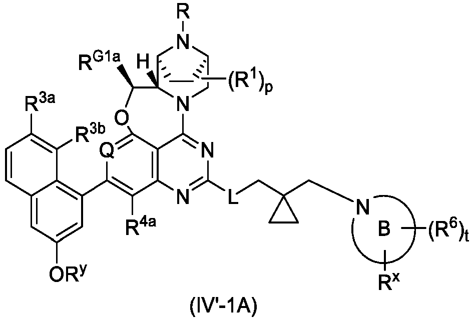 wherein:
R is an amino protecting group, preferably Boc;
R^{y} is a hydroxy protecting group, preferably MOM;
ring B, Q, L, R^{G1a}, R¹, R^{3a}, R^{3b}, R^{4a}, R⁶, R^{x}, t, and p are as defined in general formula (IV'-1).

Another aspect of the present disclosure relates to a compound represented by general formula (VV'A) or a salt thereof, 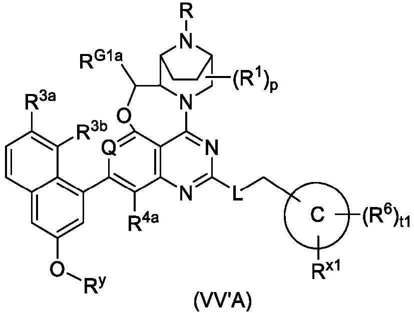 wherein:
R is an amino protecting group, preferably Boc;
R^{y} is a hydroxy protecting group, preferably MOM;
ring C, Q, L, R^{G1a}, R¹, R^{3a}, R^{3b}, R^{4a}, R⁶, R^{x1}, t1, and p are as defined in general formula (VV').

Another aspect of the present disclosure relates to a compound represented by general formula (VV'-1A) or a salt thereof, 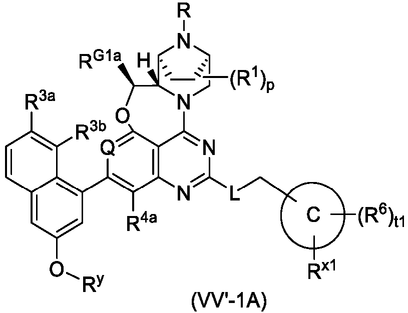 wherein:
R is an amino protecting group, preferably Boc;
R^{y} is a hydroxy protecting group, preferably MOM;
ring C, Q, L, R^{G1a}, R¹, R^{3a}, R^{3b}, R^{4a}, R⁶, R^{x1}, t1, and p are as defined in general formula (VV'-1).

Another aspect of the present disclosure relates to a compound represented by general formula (V'A) or a salt thereof, 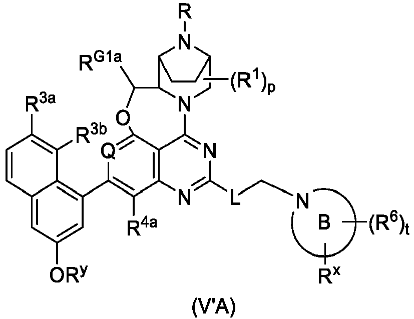 wherein:
R is an amino protecting group, preferably Boc;
R^{y} is a hydroxy protecting group, preferably MOM;
ring B, Q, L, R^{G1a}, R¹, R^{3a}, R^{3b}, R^{4a}, R⁶, R^{x}, t, and p are as defined in general formula (V').

Another aspect of the present disclosure relates to a compound represented by general formula (V'-1A) or a salt thereof, 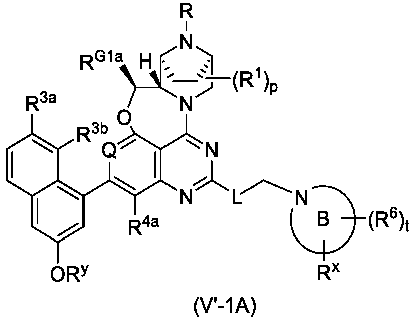 wherein:
R is an amino protecting group, preferably Boc;
R^{y} is a hydroxy protecting group, preferably MOM;
ring B, Q, L, R^{G1a}, R¹, R^{3a}, R^{3b}, R^{4a}, R⁶, R^{x}, t, and p are as defined in general formula (V'-1).

In some embodiments of the present disclosure, provided is the compound represented by general formula (IN), (INA), (IN'), (IN'A), (IVV'), (IVV'A), (VV'), (VV'A), (VV'-1), or (VV'-1A) or the pharmaceutically acceptable salt thereof, wherein R^{x1} is R^{x}, and R^{x} is as defined in general formula (IN); preferably, R^{x1} is selected from the group consisting of =N-O-R⁶¹, =CR⁶²R⁶³, and =N-R⁶⁴, R⁶¹ is C₁₋₆ alkyl, R⁶² and R⁶³ are identical or different and are each independently a hydrogen atom or halogen, and R⁶⁴ is a hydrogen atom or C₁₋₆ alkyl.

**Table B. Typical intermediate compounds of the present disclosure include, but are not limited to:**

| Compound No. | Compound structure | Name |
|---|---|---|
| | 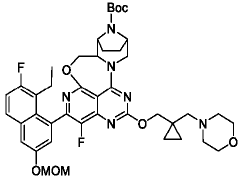 | tert-Butyl 2-(8-ethyl-7-fluoro-3-(methoxymethoxy)naphthalen-1-yl)-1-fluoro-12-((1-(morpholinomethyl)cyclopropyl)methoxy)-5*a*,6,7,8,9,10-hexahydro-5*H*-4-oxa-3,10*a,*11,13,14-pentaaza-6,9-methanonaphtho[1,8-*ab*]heptalene-14-carboxylate |
| 1s | 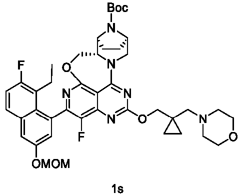 | tert-Butyl (5*aS*,6*S*,9*R*)-2-(8-ethyl-7-fluoro-3-(methoxymethoxy)naphthalen-1-yl)-1-fluoro-12-((1-(morpholinomethyl)cyclopropyl)methoxy)-5*a*,6,7,8,9,10-hexahydro-5*H*-4-oxa-3,10*a*,11,13,14-pentaaza-6,9-methanonaphtho[1,8-*ab*]heptalene-14-carboxylate **1s** |
| | 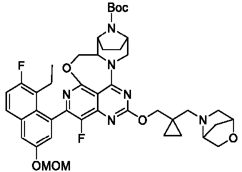 | tert-Butyl 12-((1-((2-oxa-5-azabicyclo[2.2.1]heptan-5-yl)methyl)cyclopropyl)methoxy)-2-(8-ethyl-7-fluoro-3-(methoxymethoxy)naphthalen-1-yl)-1-fluoro-5*a*,6,7,8,9,10-hexahydro-5*H*-4-oxa-3,10*a*,11,13,14-pentaaza-6,9-methanonaphtho[1,8-*ab*]heptalene-14-carboxylate |
| 2a | 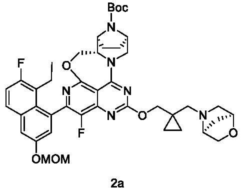 | tert-Butyl (5*aS*,6*S*,9*R*)-12-((1-(((1*S*,4*S*)-2-oxa-5-azabicyclo[2.2.1]heptan-5-yl)methyl)cyclopropyl)methoxy)-2-(8-ethyl-7-fluoro-3-(methoxymethoxy)naphthalen-1-yl)-1-fluoro-5*a*,6,7,8,9,10-hexahydro-5*H*-4-oxa-3,10*a*,11,13,14-pentaaza-6,9-methanonaphtho[1,8-*ab*]heptalene-14-carboxylate **2a** |
| 3a | 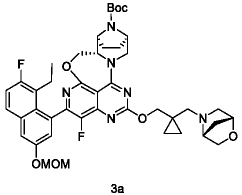 | tert-Butyl (5*aS*,6*S*,9*R*)-12-((1-(((1*R*,4*R*)-2-oxa-5-azabicyclo[2.2.1]heptan-5-yl)methyl)cyclopropyl)methoxy)-2-(8-ethyl-7-fluoro-3-(methoxymethoxy)naphthalen-1-yl)-1-fluoro-5*a*,6,7,8,9,10-hexahydro-5*H*-4-oxa-3,10*a*,11,13,14-pentaaza-6,9-methanonaphtho[1,8-*ab*]heptalene-14-carboxylate **3a** |
| | 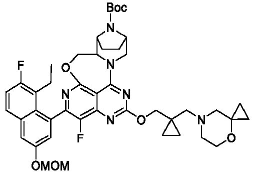 | tert-Butyl 12-((1-((4-oxa-7-azaspiro[2.5]octan-7-yl)methyl)cyclopropyl)methoxy)-2-(8-ethyl-7-fluoro-3-(methoxymethoxy)naphthalen-1-yl)-1-fluoro-5*a*,6,7,8,9,10-hexahydro-5*H*-4-oxa-3*,*10*a,*11,13,14-pentaaza-[6,9]methanonaphtho[1,8-*ab*]heptalene-14-carboxylate |
| 4a | 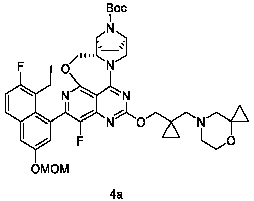 | tert-Butyl (5*aS*,6*S*,9*R*)-12-((1-((4-oxa-7-azaspiro[2.5]octan-7-yl)methyl)cyclopropyl)methoxy)-2-(8-ethyl-7-fluoro-3-(methoxymethoxy)naphthalen-1-yl)-1-fluoro-5*a*,6,7,8,9,10-hexahydro-5*H*-4-oxa-3,10*a*,11,13,14-pentaaza-[6,9]methanonaphtho[1,8-*ab*]heptalene-14-carboxylate **4a** |
| | 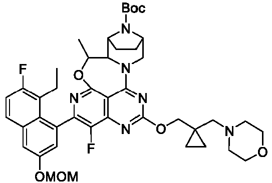 | tert-Butyl 2-(8-ethyl-7-fluoro-3-(methoxymethoxy)naphthalen-1-yl)-1-fluoro-5-methyl-12-((1-(morpholinomethyl)cyclopropyl)methoxy)-5*a*,6,7,8,9,10-hexahydro-5*H*-4-oxa-3,10*a*,11,13,14-pentaaza-6,9-methanonaphtho[1,8-*ab*]heptalene-14-formate |
| 5h | 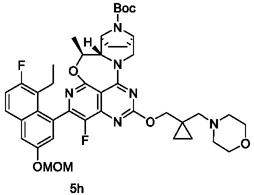 | tert-Butyl (5*S*,5*aS*,6*S*,9*R*)-2-(8-ethyl-7-fluoro-3-(methoxymethoxy)naphthalen-1-yl)-1-fluoro-5-methyl-12-((1-(morpholinomethyl)cyclopropyl)methoxy)-5*a*,6,7,8,9,10-hexahydro-5*H*-4-oxa-3,10*a*,11,13,14-pentaaza-6,9-methanonaphtho[1,8-*ab*]heptalene-14-formate **5h** |
| | 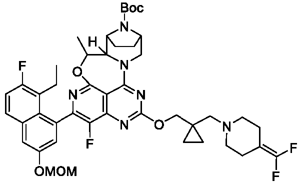 | tert-Butyl 12-((1-((4-(difluoromethylene)piperidin-1-yl)methyl)cyclopropyl)methoxy)-2-(8-ethyl-7-fluoro-3-(methoxymethoxy)naphthalen-1-yl)-1-fluoro-5-methyl-5*a*,6,7,8,9,10-hexahydro-5*H*-4-oxa-3,10*a*,11,13,14-pentaaza-6,9-methanonaphtho[1,8-*ab*]heptalene-14-formate |
| 6f | 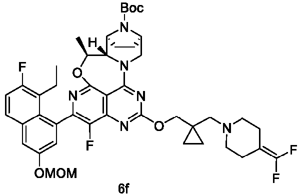 | tert-Butyl (5*S*,5*aS*,6*S*,9*R*)-12-((1-((4-(difluoromethylene)piperidin-1-yl)methyl)cyclopropyl)methoxy)-2-(8-ethyl-7-fluoro-3-(methoxymethoxy)naphthalen-1-yl)-1-fluoro-5-methyl-5a,6,7,8,9,10-hexahydro-5H-4-oxa-3,10*a*,11,13,14-pentaaza-6,9-methanonaphtho[1,8-*ab*]heptalene-14-formate 6f |
| | 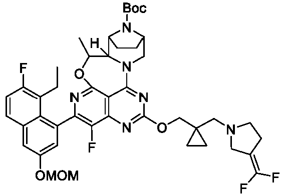 | |
| | 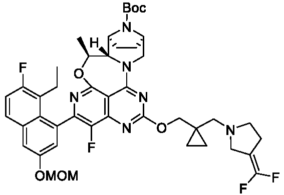 | |
| | 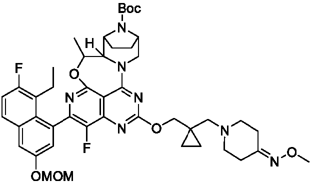 | |
| | 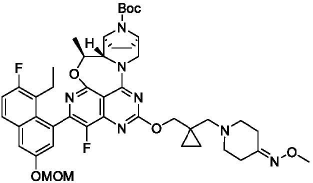 | |
| | 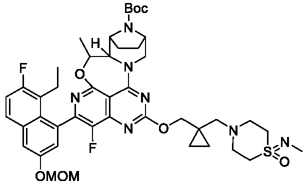 | |
| | 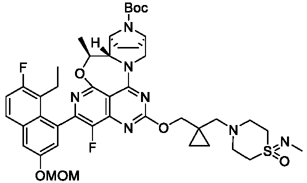 | |
| | 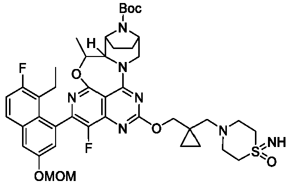 | |
| | 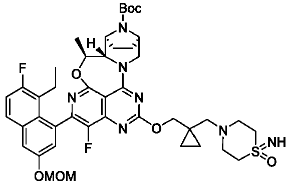 | |
| | 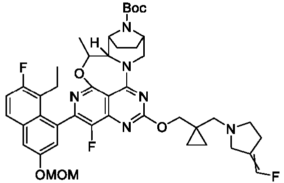 | |
| | 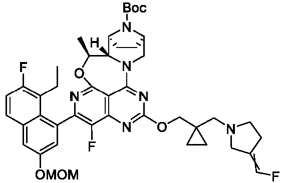 | |
| | 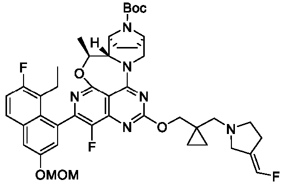 | |
| | 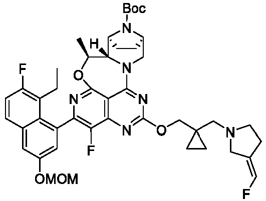 | |
| | 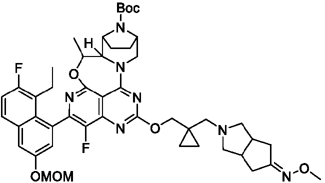 | |
| | 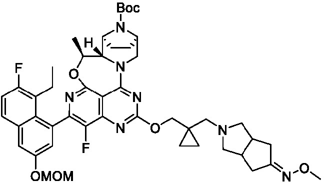 | |
| | 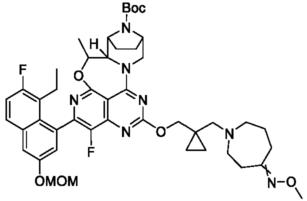 | |
| | 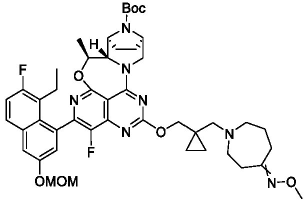 | |
| | 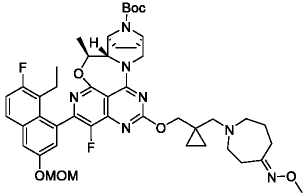 | |
| | 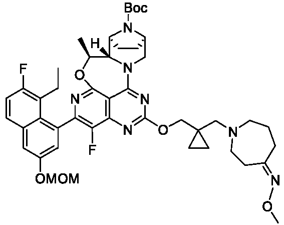 | |
| | 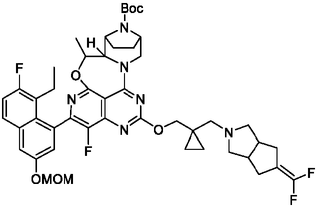 | |
| | 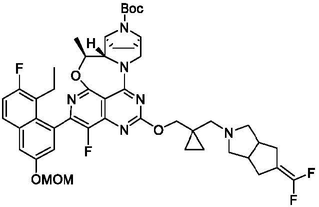 | |
| | 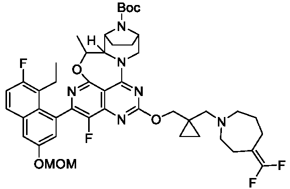 | |
| | 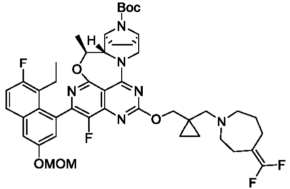 | |
| | 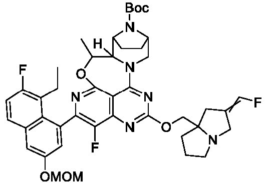 | |
| | 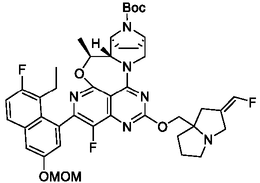 | |
| | 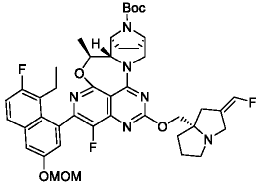 | |
| | 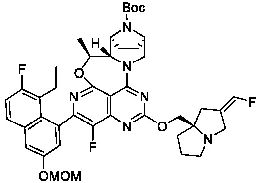 | |
| | 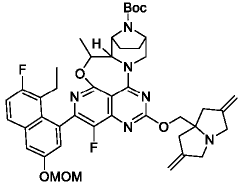 | |
| | 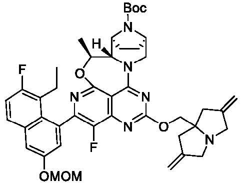 | |
| | 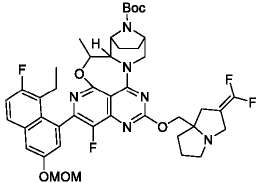 | |
| | 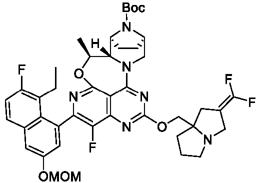 | |
| | 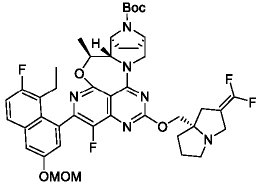 | |
| | 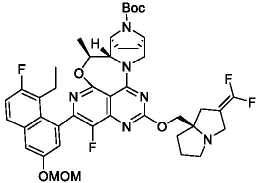 | |
| | 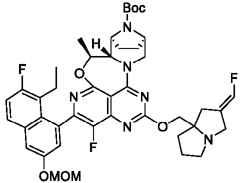 | |
| | 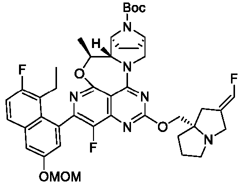 | |
| | 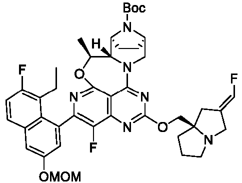 | |
| | 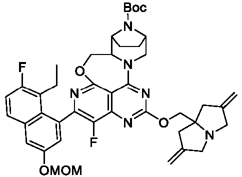 | |
| | 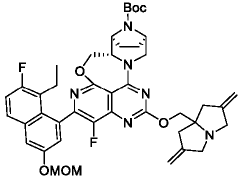 | |
| | 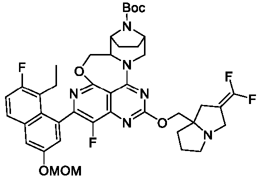 | |
| | 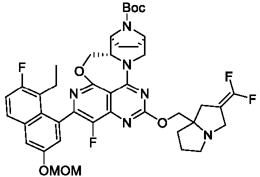 | |
| | 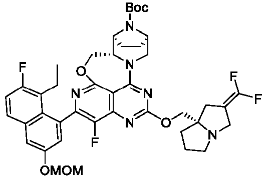 | |
| | 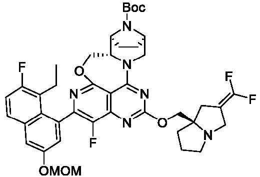 | |
| | 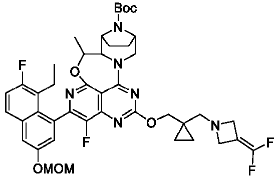 | |
| | 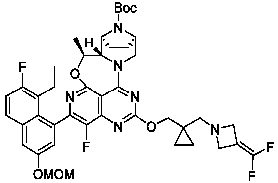 | |
| | 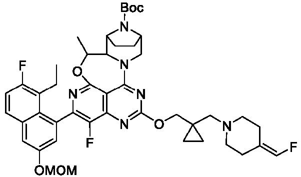 | |
| | 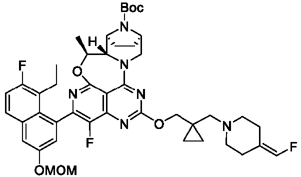 | |
| | 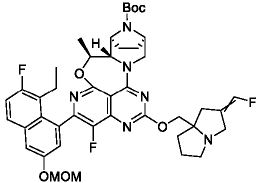 | |
| 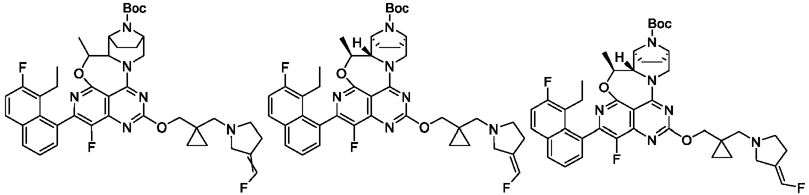 | | |
| 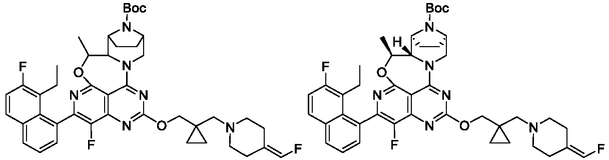 | | |
| | 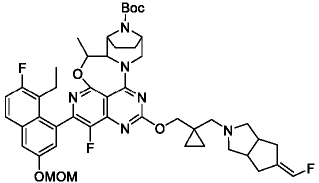 | |
| | 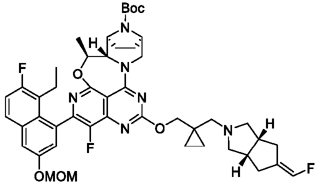 | |
| | 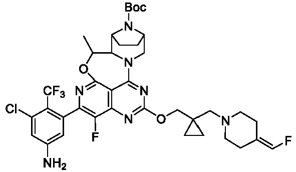 | tert-Butyl 2-(5-amino-3-chloro-2-(trifluoromethyl)phenyl)-1-fluoro-12-((1-(4-(fluoromethylene)piperidin-1-yl)methyl)cyclopropyl)methoxy)-5-methyl-5*a*,6,7,8,9,10-hexahydro-5*H*-4-oxa-3,10*a*,11,13,14-pentaaza-6,9-methanonaphtho[1,8-*ab*]heptalene-14-carboxylate |
| 36e | 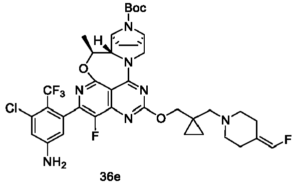 | tert-Butyl (5*S*,5*aS*,6*S*,9*R*)-2-(5-amino-3-chloro-2-(trifluoromethyl)phenyl)-1-fluoro-12-((1-(4-(fluoromethylene)piperidin-1-yl)methyl)cyclopropyl)methoxy)-5-methyl-5*a*,6,7,8,9,10-hexahydro-5*H*-4-oxa-3,10*a*,11,13,14-pentaaza-6,9-methanonaphtho[1,8-*ab*]heptalene-14-carboxylate 3**6e** |
| | 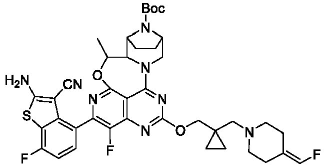 | |
| | 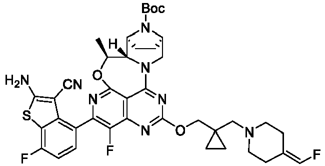 | |
| | 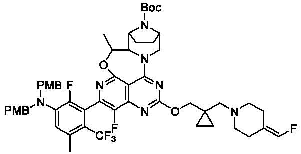 | tert-Butyl 2-(3-(bis(4-methoxybenzyl)amino)-2-fluoro-5-methyl-6-(trifluoromethyl)phenyl)-1-fluoro-12-(1-((4-(fluoromethylene)piperidin-1-yl)methyl)cyclopropyl)methoxy)-5-methyl-5*a*,6,7,8,9,10-hexahydro-5*H*-4-oxa-3,10*a*,11,13,14-pentaaza-6,9-methanonaphtho[1,8-*ab*]heptalene-14-carboxylate |
| 38e | 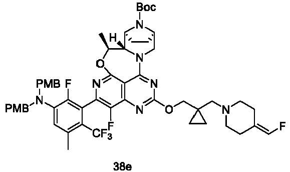 | tert-Butyl (5*S*,5*aS*,6*S*,9*R*)-2-(3-(bis(4-methoxybenzyl)amino)-2-fluoro-5-methyl-6-(trifluoromethyl)phenyl)-1-fluoro-12-(1-((4-(fluoromethylene)piperidin-1-yl)methyl)cyclopropyl)methoxy)-5-methyl-5*a*,6,7,8,9,10-hexahydro-5*H*-4-oxa-3,10*a*,11,13,14-pentaaza-6,9-methanonaphtho[1,8-*ab*]heptalene-14-carboxylate **38e** |
| | 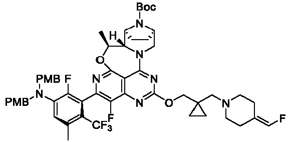 | |
| | 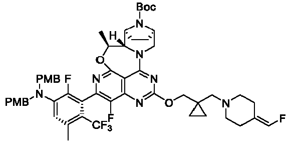 | |
| | 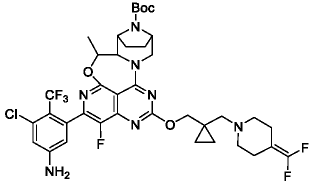 | tert-Butyl 2-(5-amino-3-chloro-2-(trifluoromethyl)phenyl)-12-((1-(4-(difluoromethylene)piperidin-1-yl)methyl)cyclopropyl)methoxy)-1-fluoro-5-methyl-5a,6,7,8,9,10-hexahydro-5*H*-4-oxa-3,10*a*,11,13,14-pentaaza-6,9-methanonaphtho[1,8-*ab*]heptalene-14-carboxylate |
| 39b | 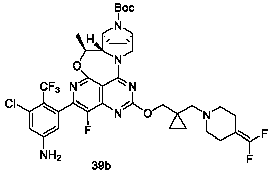 | tert-Butyl (5*S*,5*aS*,6*S*,9*R*)-2-(5-amino-3-chloro-2-(trifluoromethyl)phenyl)-12-((1-(4-(difluoromethylene)piperidin-1-yl)methyl)cyclopropyl)methoxy)-1-fluoro-5-methyl-5*a*,6,7,8,9,10-hexahydro-5*H*-4-oxa-3,10*a*,11,13,14-pentaaza-6,9-methanonaphtho[1,8-*ab*]heptalene-14-carboxylate **39b** |
| | 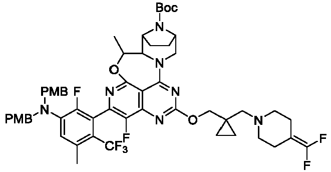 | tert-Butyl 2-(3-(bis(4-methoxybenzyl)amino)-2-fluoro-5-methyl-6-(trifluoromethyl)phenyl)-12-((1-((4-(difluoromethylene)piperidin-1-yl)methyl)cyclopropyl)methoxy)-1-fluoro-5-methyl-5a,6,7,8,9,10-hexahydro-5*H*-4-oxa-3,10*a*,11,13,14-pentaaza-6,9-methanonaphtho[1,8-*ab*]heptalene-14-carboxylate |
| 40a | 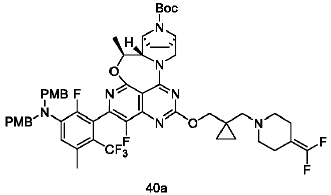 | tert-Butyl (5*S*,5*aS*,6*S*,9*R*)-2-(3-(bis(4-methoxybenzyl)amino)-2-fluoro-5-methyl-6-(trifluoromethyl)phenyl)-12-((1-((4-(difluoromethylene)piperidin-1-yl)methyl)cyclopropyl)methoxy)-1-fluoro-5-methyl-5*a*,6,7,8,9,10-hexahydro-5H-4-oxa-3,10*a*,11,13,14-pentaaza-6,9-methanonaphtho[1,8-ab]heptalene-14-carboxylate **40a** |
| | 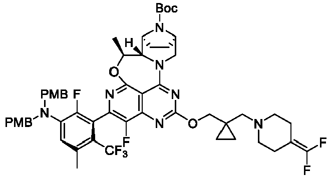 | |
| | 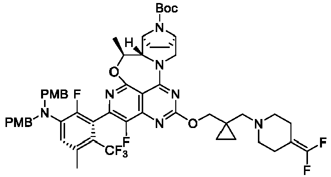 | |
| | 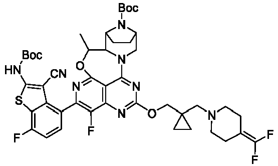 | tert-Butyl 2-(2-((tert-butoxycarbonyl)amino)-3-cyano-7-fluorobenzo[*b*]thiophen-4-yl)-12-(1-((4-(difluoromethylene)piperidin-1-yl)methyl)cyclopropyl)methoxy)-1-fluoro-5-methyl-5*a*,6,7,8,9,10-hexahydro-5*H*-4-oxa-3,10a,11,13,14-pentaaza-6,9-methanonaphtho[1,8-*ab*]heptalene-14-formate |
| 41b | 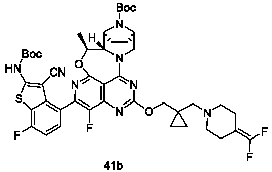 | tert-Butyl (5*S*,5*aS*,6*S*,9*R*)-2-(2-((tert-butoxycarbonyl)amino)-3-cyano-7-fluorobenzo[*b*]thiophen-4-yl)-12-(1-((4-(difluoromethylene)piperidin-1-yl)methyl)cyclopropyl)methoxy)-1-fluoro-5-methyl-5a,6,7,8,9,10-hexahydro-5*H*-4-oxa-3,10*a*,11,13,14-pentaaza-6,9-methanonaphtho[1,8-*ab*]heptalene-14-formate **41b** |
| | 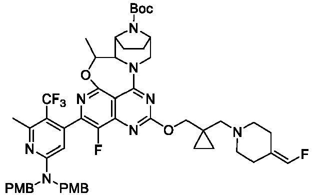 | tert-Butyl 2-(6-(bis(4-methoxybenzyl)amino)-2-methyl-3-(trifluoromethyl)pyridin-4-yl)-1-fluoro-12-(1-((4-(fluoromethylene)piperidin-1-yl)methyl)cyclopropyl)methoxy)-5-methyl-5*a*,6,7,8,9,10-hexahydro-5*H*-4-oxa-3,10*a*,11,13,14-pentaaza-6,9-methanonaphtho[1,8-*ab*]heptalene-4-carboxylate |
| 42b | 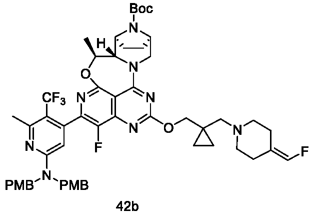 | tert-Butyl (5*S*,5*aS*,6*S*,9*R*)-2-(6-(bis(4-methoxybenzyl)amino)-2-methyl-3-(trifluoromethyl)pyridin-4-yl)-1-fluoro-12-(1-((4-(fluoromethylene)piperidin-1-yl)methyl)cyclopropyl)methoxy)-5-methyl-5a,6,7,8,9,10-hexahydro-5*H*-4-oxa-3,10*a*,11,13,14-pentaaza-6,9-methanonaphtho[1,8-*ab*]heptalene-4-carboxylate **42b** |
| | 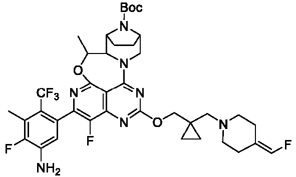 | tert-Butyl 2-(5-amino-4-fluoro-3-methyl-2-(trifluoromethyl)phenyl)-1-fluoro-12-((1-((4-(fluoromethylene)piperidin-1-yl)methyl)cyclopropyl)methoxy)-5-methyl-5*a*,6,7,8,9,10-hexahydro-5*H*-4-oxa-3,10*a*,11,13,14-pentaaza-6,9-methanonaphtho[1,8-*ab*]heptalene-14-carboxylate |
| 43h | 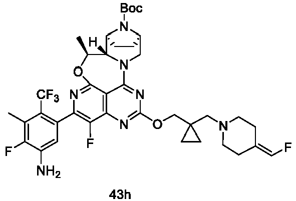 | tert-Butyl (5*S*,5*aS*,6*S*,9*R*)-2-(5-amino-4-fluoro-3-methyl-2-(trifluoromethyl)phenyl)-1-fluoro-12-((1-((4-(fluoromethylene)piperidin-1-yl)methyl)cyclopropyl)methoxy)-5-methyl-5*a*,6,7,8,9,10-hexahydro-5*H*-4-oxa-3,10*a*,11,13,14-pentaaza-6,9-methanonaphtho[1,8-*ab*]heptalene-14-carboxylate **43h** |
| 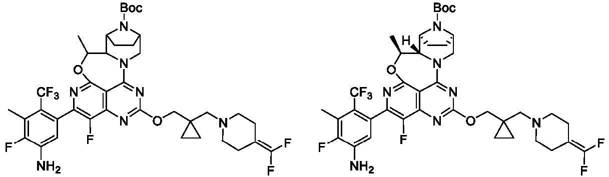 | | |
| 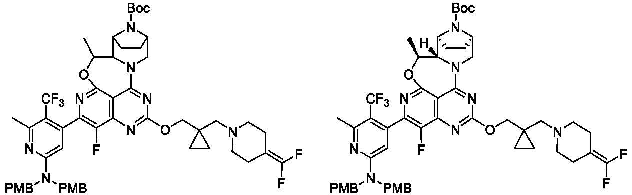 | | |

Another aspect of the present disclosure relates to a method for preparing a compound represented by general formula (IN) or a pharmaceutically acceptable salt thereof, the method comprising: 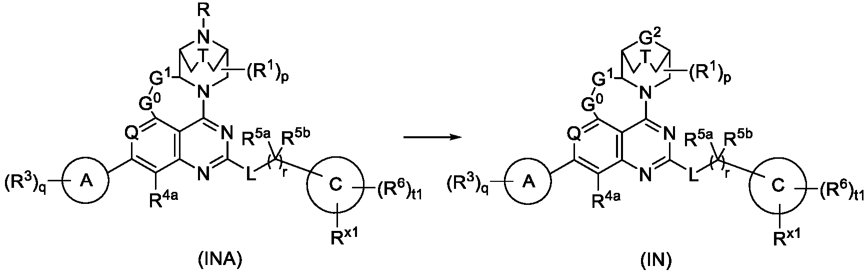
subjecting a compound of general formula (INA) or a salt thereof to a deprotection reaction to give the compound of general formula (IN) or the pharmaceutically acceptable salt thereof and, when group R³ has a protecting group thereon, optionally a step of removing the protecting group from group R³ before, concurrent with, or after the deprotection reaction,
wherein:
   R is an amino protecting group, preferably Boc;
   G² is NH;
   G⁰, G¹, T, ring A, ring C, Q, L, R¹, R³, R^{4a}, R^{5a}, R^{5b}, R⁶, R^{x1}, p, q, t1, and r are as defined in general formula (IN).

Another aspect of the present disclosure relates to a method for preparing a compound represented by general formula (IN') or a pharmaceutically acceptable salt thereof, the method comprising: 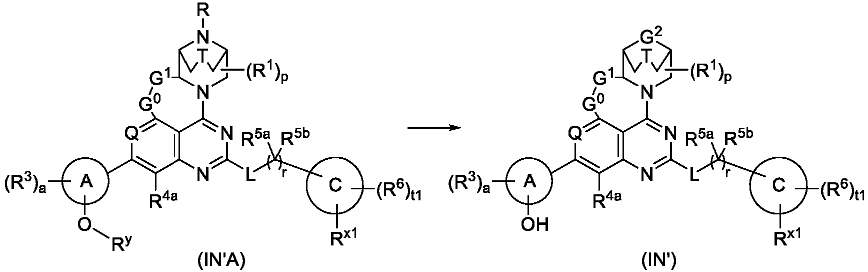
subjecting a compound of general formula (IN'A) or a salt thereof to a deprotection reaction to give the compound of general formula (IN') or the pharmaceutically acceptable salt thereof and, when group R³ has a protecting group thereon, optionally a step of removing the protecting group from group R³ before, concurrent with, or after the deprotection reaction,
wherein:
   R is an amino protecting group, preferably Boc;
   R^{y} is a hydroxy protecting group, preferably MOM;
   a is 0, 1, 2, 3, or 4;
   G² is NH;
   G⁰, G¹, T, ring A, ring C, Q, L, R¹, R³, R^{4a}, R^{5a}, R^{5b}, R⁶, R^{x1}, p, t1, and r are as defined in general formula (IN').

Another aspect of the present disclosure relates to a method for preparing a compound represented by general formula (IM) or a pharmaceutically acceptable salt thereof, the method comprising: 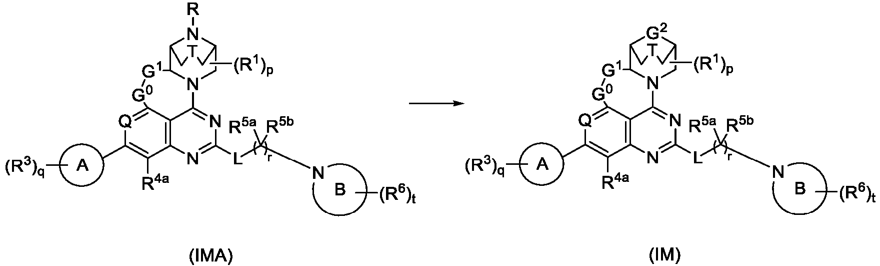
subjecting a compound of general formula (IMA) or a salt thereof to a deprotection reaction to give the compound of general formula (IM) or the pharmaceutically acceptable salt thereof and, when group R³ has a protecting group thereon, optionally a step of removing the protecting group from group R³ before, concurrent with, or after the deprotection reaction,
wherein:
   R is an amino protecting group, preferably Boc;
   G² is NH;
   G⁰, G¹, T, ring A, ring B, Q, L, R¹, R³, R^{4a}, R^{5a}, R^{5b}, R⁶, p, q, t, and r are as defined in general formula (IM).

Another aspect of the present disclosure relates to a method for preparing a compound represented by general formula (IM') or a pharmaceutically acceptable salt thereof, the method comprising: 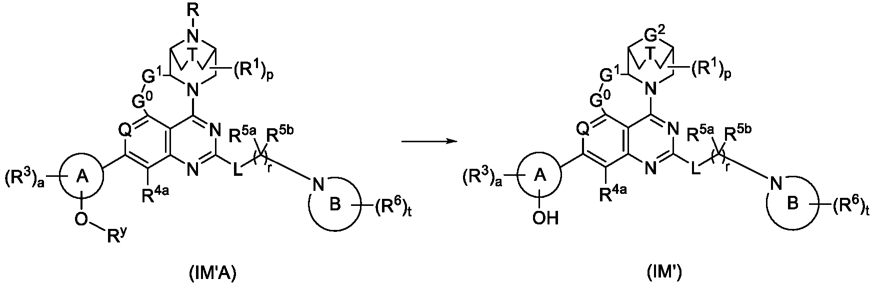
subjecting a compound of general formula (IM'A) or a salt thereof to a deprotection reaction to give the compound of general formula (IM') or the pharmaceutically acceptable salt thereof and, when group R³ has a protecting group thereon, optionally a step of removing the protecting group from group R³ before, concurrent with, or after the deprotection reaction,
wherein:
   R is an amino protecting group, preferably Boc;
   R^{y} is a hydroxy protecting group, preferably MOM;
   a is 0, 1, 2, 3, or 4;
   G² is NH;
   G⁰, G¹, T, ring A, ring B, Q, L, R¹, R³, R^{4a}, R^{5a}, R^{5b}, R⁶, p, t, and r are as defined in general formula (IM').

Another aspect of the present disclosure relates to a method for preparing a compound represented by general formula (I) or a pharmaceutically acceptable salt thereof, the method comprising: 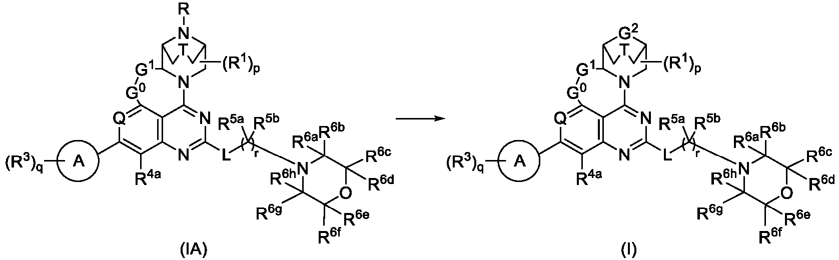
subjecting a compound of general formula (IA) or a salt thereof to a deprotection reaction to give the compound of general formula (I) or the pharmaceutically acceptable salt thereof and, when group R³ has a protecting group thereon, optionally a step of removing the protecting group from group R³ before, concurrent with, or after the deprotection reaction,
wherein:
   R is an amino protecting group, preferably Boc;
   G² is NH;
   G⁰, G¹, T, ring A, Q, L, R¹, R³, R^{4a}, R^{5a}, R^{5b}, R^{6a}, R^{6b}, R^{6c}, R^{6d}, R^{6e}, R^{6f}, R^{6g}, R^{6h}, p, q, and r are as defined in general formula (I).

Another aspect of the present disclosure relates to a method for preparing a compound represented by general formula (I') or a pharmaceutically acceptable salt thereof, the method comprising: 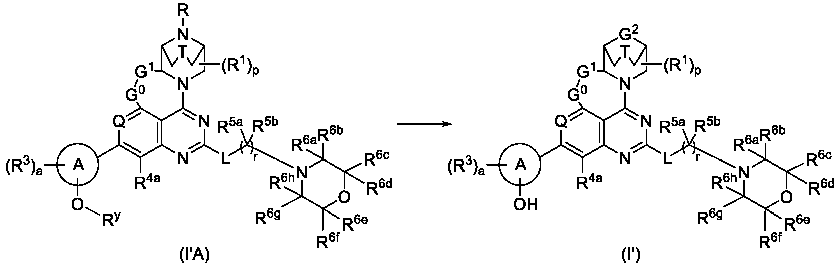
subjecting a compound of general formula (I'A) or a salt thereof to a deprotection reaction to give the compound of general formula (I') or the pharmaceutically acceptable salt thereof and, when group R³ has a protecting group thereon, optionally a step of removing the protecting group from group R³ before, concurrent with, or after the deprotection reaction,
wherein:
   R is an amino protecting group, preferably Boc;
   R^{y} is a hydroxy protecting group, preferably MOM;
   a is 0, 1, 2, 3, or 4;
   G² is NH;
   G⁰, G¹, T, ring A, Q, L, R¹, R³, R^{4a}, R^{5a}, R^{5b}, R^{6a}, R^{6b}, R^{6c}, R^{6d} R^{6e}, R^{6f}, R^{6g}, R^{6h}, p, and r are as defined in general formula (I').

Another aspect of the present disclosure relates to a method for preparing a compound represented by general formula (II') or a pharmaceutically acceptable salt thereof, the method comprising: 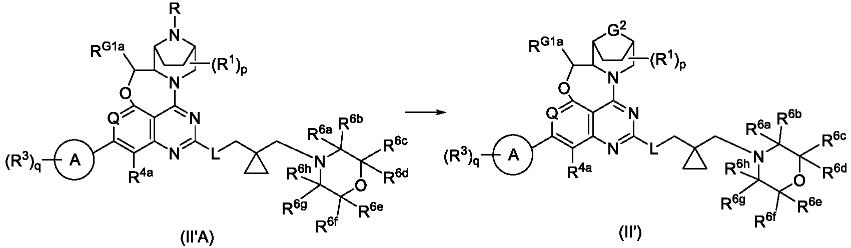
subjecting a compound of general formula (II'A) or a salt thereof to a deprotection reaction to give the compound of general formula (II') or the pharmaceutically acceptable salt thereof and, when group R³ has a protecting group thereon, optionally a step of removing the protecting group from group R³ before, concurrent with, or after the deprotection reaction,
wherein R is an amino protecting group, preferably Boc;
G² is NH;
ring A, Q, L, R^{G1a}, R¹, R³, R^{4a}, R^{6a}, R^{6b}, R^{6c}, R^{6d}, R^{6e}, R^{6f}, R^{6g}, R^{6h}, p, and q are as defined in general formula (II').

Another aspect of the present disclosure relates to a method for preparing a compound represented by general formula (II) or a pharmaceutically acceptable salt thereof, the method comprising: 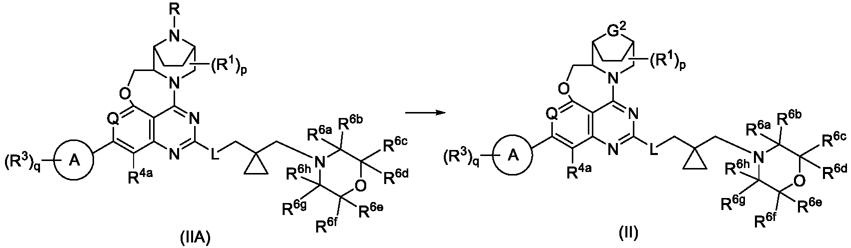
subjecting a compound of general formula (IIA) or a salt thereof to a deprotection reaction to give the compound of general formula (II) or the pharmaceutically acceptable salt thereof and, when group R³ has a protecting group thereon, optionally a step of removing the protecting group from group R³ before, concurrent with, or after the deprotection reaction,
wherein R is an amino protecting group, preferably Boc;
G² is NH;
ring A, Q, L, R¹, R³, R^{4a}, R^{6a}, R^{6b}, R^{6c}, R^{6d}, R^{6e}, R^{6f}, R^{6g}, R^{6h}, p, and q are as defined in general formula (II).

Another aspect of the present disclosure relates to a method for preparing a compound represented by general formula (III') or a pharmaceutically acceptable salt thereof, the method comprising: 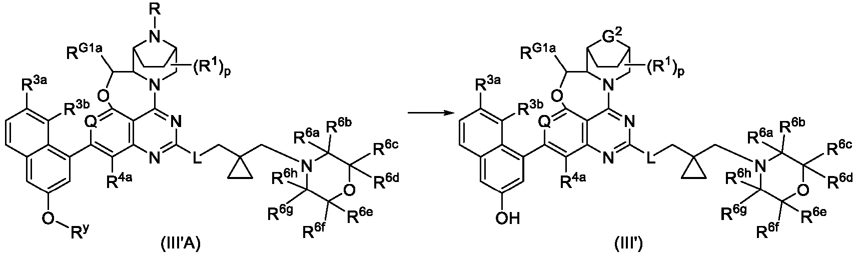
subjecting a compound of general formula (III'A) or a salt thereof to a deprotection reaction to give the compound of general formula (III') or the pharmaceutically acceptable salt thereof;
wherein:
   R is an amino protecting group, preferably Boc;
   R^{y} is a hydroxy protecting group, preferably MOM;
   G² is NH;
   Q, L, R^{G1a}, R¹, R^{3a}, R^{3b}, R^{4a}, R^{6a}, R^{6b}, R^{6c}, R^{6d}, R^{6e}, R^{6f}, R^{6g}, R^{6h}, and p are as defined in general formula (III').

Another aspect of the present disclosure relates to a method for preparing a compound represented by general formula (III'-1) or a pharmaceutically acceptable salt thereof, the method comprising: 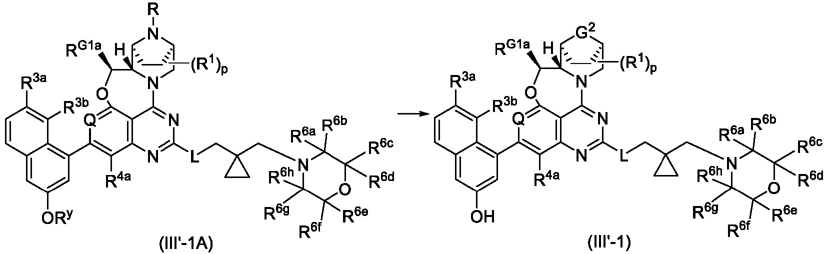
subjecting a compound of general formula (III'-1A) or a salt thereof to a deprotection reaction to give the compound of general formula (III'-1) or the pharmaceutically acceptable salt thereof;
wherein:
   R is an amino protecting group, preferably Boc;
   R^{y} is a hydroxy protecting group, preferably MOM;
   G² is NH;
   Q, L, R^{G1a}, R¹, R^{3a}, R^{3b}, R^{4a}, R^{6a}, R^{6b}, R^{6c}, R^{6d}, R^{6e}, R^{6f}, R^{6g}, R^{6h}, and p are as defined in general formula (III'-1).

Another aspect of the present disclosure relates to a method for preparing a compound represented by general formula (III) or a pharmaceutically acceptable salt thereof, the method comprising: 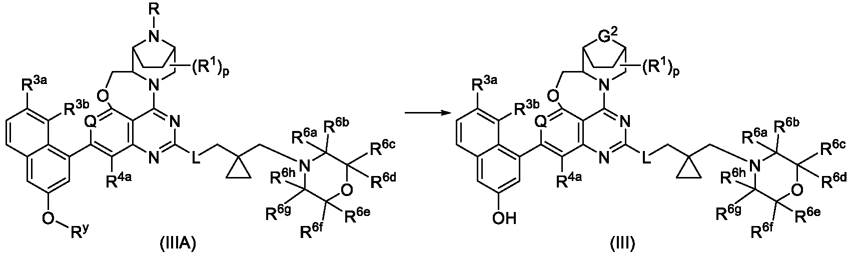
subjecting a compound of general formula (IIIA) or a salt thereof to a deprotection reaction to give the compound of general formula (III) or the pharmaceutically acceptable salt thereof;
wherein:
   R is an amino protecting group, preferably Boc;
   R^{y} is a hydroxy protecting group, preferably MOM;
   G² is NH;
   Q, L, R¹, R^{3a}, R^{3b}, R^{4a}, R^{6a}, R^{6b}, R^{6c}, R^{6d}, R^{6e}, R^{6f}, R^{6g}, R^{6h}, and p are as defined in general formula (III).

Another aspect of the present disclosure relates to a method for preparing a compound represented by general formula (IVV') or a pharmaceutically acceptable salt thereof, the method comprising: 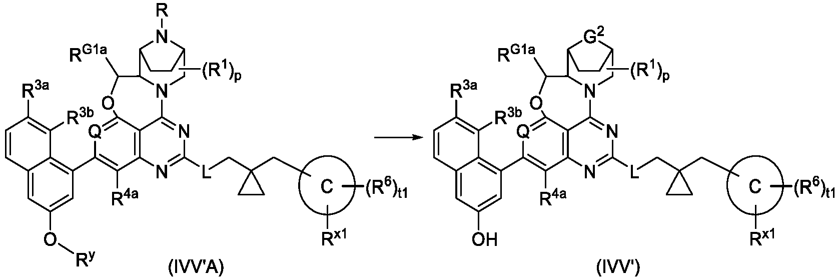
subjecting a compound of general formula (IVV'A) or a salt thereof to a deprotection reaction to give the compound of general formula (IVV') or the pharmaceutically acceptable salt thereof;
wherein:
   R is an amino protecting group, preferably Boc;
   R^{y} is a hydroxy protecting group, preferably MOM;
   G² is NH;
   ring C, Q, L, R^{G1a}, R¹, R^{3a}, R^{3b}, R^{4a}, R⁶, R^{x1}, t1, and p are as defined in general formula
   (IVV').

Another aspect of the present disclosure relates to a method for preparing a compound represented by general formula (IV') or a pharmaceutically acceptable salt thereof, the method comprising: 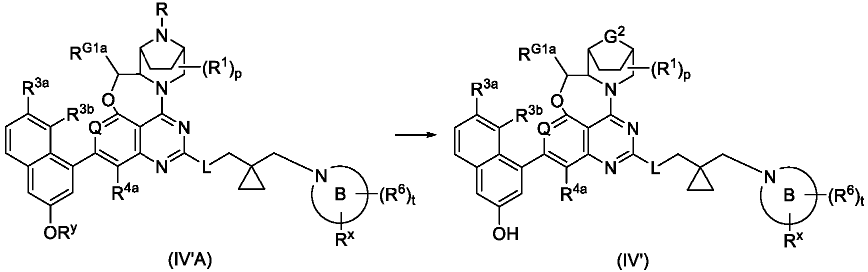
subjecting a compound of general formula (IV'A) or a salt thereof to a deprotection reaction to give the compound of general formula (IV') or the pharmaceutically acceptable salt thereof;
wherein:
   R is an amino protecting group, preferably Boc;
   R^{y} is a hydroxy protecting group, preferably MOM;
   G² is NH;
   ring B, Q, L, R^{G1a}, R¹, R^{3a}, R^{3b}, R^{4a}, R⁶, R^{x}, t, and p are as defined in general formula (IV').

Another aspect of the present disclosure relates to a method for preparing a compound represented by general formula (IV'-1) or a pharmaceutically acceptable salt thereof, the method comprising: 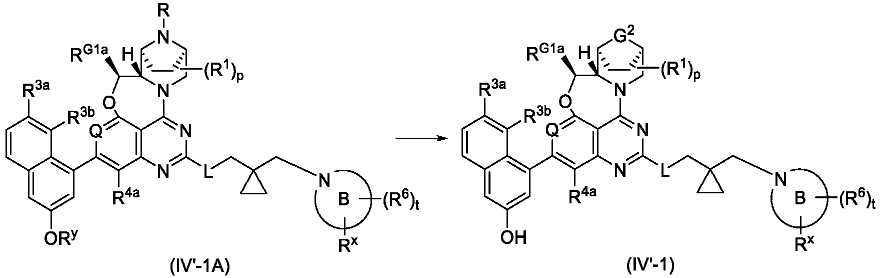
subjecting a compound of general formula (IV'-1A) or a salt thereof to a deprotection reaction to give the compound of general formula (IV'-1) or the pharmaceutically acceptable salt thereof;
wherein:
   R is an amino protecting group, preferably Boc;
   R^{y} is a hydroxy protecting group, preferably MOM;
   G² is NH;
   ring B, Q, L, R^{G1a}, R¹, R^{3a}, R^{3b}, R^{4a}, R⁶, R^{x}, t, and p are as defined in general formula (IV'-1).

Another aspect of the present disclosure relates to a method for preparing a compound represented by general formula (VV') or a pharmaceutically acceptable salt thereof, the method comprising: 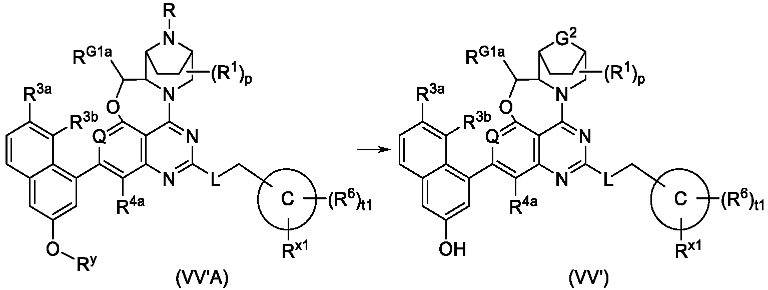
subjecting a compound of general formula (VV'A) or a salt thereof to a deprotection reaction to give the compound of general formula (VV') or the pharmaceutically acceptable salt thereof;
wherein:
   R is an amino protecting group, preferably Boc;
   R^{y} is a hydroxy protecting group, preferably MOM;
   G² is NH;
   ring C, Q, L, R^{G1a}, R¹, R^{3a}, R^{3b}, R^{4a}, R⁶, R^{x1}, t1, and p are as defined in general formula (VV').

Another aspect of the present disclosure relates to a method for preparing a compound represented by general formula (VV'-1) or a pharmaceutically acceptable salt thereof, the method comprising: 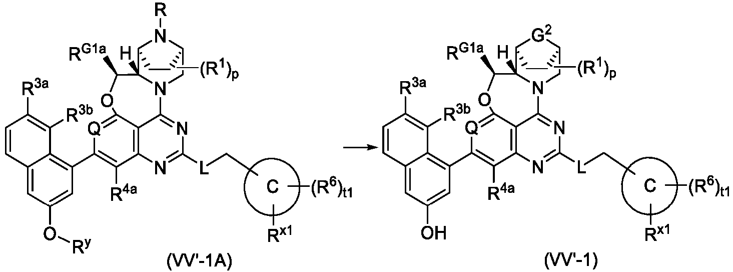
subjecting a compound of general formula (VV'-1A) or a salt thereof to a deprotection reaction to give the compound of general formula (VV'-1) or the pharmaceutically acceptable salt thereof;
wherein:
   R is an amino protecting group, preferably Boc;
   R^{y} is a hydroxy protecting group, preferably MOM;
   G² is NH;
   ring C, Q, L, R^{G1a}, R¹, R^{3a}, R^{3b}, R^{4a}, R⁶, R^{x1}, t1, and p are as defined in general formula (VV'-1).

Another aspect of the present disclosure relates to a method for preparing a compound represented by general formula (V') or a pharmaceutically acceptable salt thereof, the method comprising: 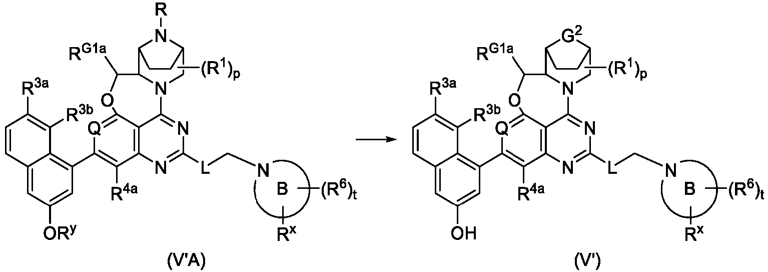
subjecting a compound of general formula (V'A) or a salt thereof to a deprotection reaction to give the compound of general formula (V') or the pharmaceutically acceptable salt thereof;
wherein:
   R is an amino protecting group, preferably Boc;
   R^{y} is a hydroxy protecting group, preferably MOM;
   G² is NH;
   ring B, Q, L, R^{G1a}, R¹, R^{3a}, R^{3b}, R^{4a}, R⁶, R^{x}, t, and p are as defined in general formula (V').

Another aspect of the present disclosure relates to a method for preparing a compound represented by general formula (V'-1) or a pharmaceutically acceptable salt thereof, the method comprising: 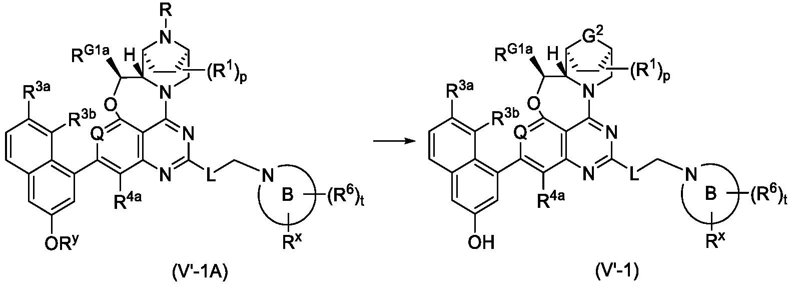
subjecting a compound of general formula (V'-1A) or a salt thereof to a deprotection reaction to give the compound of general formula (V'-1) or the pharmaceutically acceptable salt thereof;
wherein:
   R is an amino protecting group, preferably Boc;
   R^{y} is a hydroxy protecting group, preferably MOM;
   G² is NH;
   ring B, Q, L, R^{G1a}, R¹, R^{3a}, R^{3b}, R^{4a}, R⁶, R^{x}, t, and p are as defined in general formula (V'-1).

Another aspect of the present disclosure relates to a pharmaceutical composition comprising the compound represented by general formula (IN), (IN'), (IM), (IM'), (I), (I'), (II'), (II), (III'), (III'-1), (III), (IV'), (IV'-1), (V'), (V'-1), (IVV'), (VV'), or (VV'-1) of the present disclosure or a compound shown in Table A or a pharmaceutically acceptable salt thereof, and one or more pharmaceutically acceptable carriers, diluents, or excipients. The present disclosure further relates to use of a compound represented by general formula (IN), (IN'), (IM), (IM'), (I), (I'), (II'), (II), (III'), (III'-1), (III), (IV'), (IV'-1), (V'), (V'-1), (IVV'), (VV'), or (VV'-1) or a compound shown in Table A or a pharmaceutically acceptable salt thereof, or a pharmaceutical composition comprising same in the preparation of a medicament for inhibiting KRAS G12D.

The present disclosure further relates to use of a compound represented by general formula (IN), (IN'), (IM), (IM'), (I), (I'), (II'), (II), (III'), (III'-1), (III), (IV'), (IV'-1), (V'), (V'-1), (IVV'), (VV'), or (VV'-1) or a compound shown in Table A or a pharmaceutically acceptable salt thereof, or a pharmaceutical composition comprising same in the preparation of a medicament for treating and/or preventing a KRAS G12D-mediated disease or disorder.

The present disclosure further relates to use of a compound represented by general formula (IN), (IN'), (IM), (IM'), (I), (I'), (II'), (II), (III'), (III'-1), (III), (IV'), (IV'-1), (V'), (V'-1), (IVV'), (VV'), or (VV'-1) or a compound shown in Table A or a pharmaceutically acceptable salt thereof, or a pharmaceutical composition comprising same in the preparation of a medicament for treating and/or preventing a tumor; preferably, the tumor is selected from the group consisting of brain cancer, thyroid cancer, head and neck cancer, nasopharyngeal cancer, laryngeal cancer, oral cancer, salivary gland cancer, esophageal cancer, gastric cancer, lung cancer, liver cancer, renal cancer, pleural cancer, peritoneal cancer, pancreatic cancer, gallbladder cancer, bile duct cancer, colorectal cancer, small intestine cancer, gastrointestinal stromal tumor, urothelial cancer, urinary tract cancer, bladder cancer, anal cancer, joint cancer, breast cancer, vaginal cancer, ovarian cancer, endometrial cancer, cervical cancer, fallopian tube cancer, testicular cancer, prostate cancer, hemangioma, leukemia, lymphoma, myeloma, skin cancer, melanoma, lipoma, bone cancer, soft tissue sarcoma, neurofibroma, neuroglioma, neuroblastoma, and glioblastoma; more preferably, the tumor is selected from the group consisting of pancreatic cancer, colorectal cancer, and non-small cell lung cancer.

The present disclosure further relates to a method for inhibiting KRAS G12D, the method comprising administering to a patient in need thereof a therapeutically effective amount of a compound represented by general formula (IN), (IN'), (IM), (IM'), (I), (I'), (II'), (II), (III'), (III'-1), (III), (IV'), (IV'-1), (V'), (V'-1), (IVV'), (VV'), or (VV'-1) or a compound shown in Table A or a pharmaceutically acceptable salt thereof, or a pharmaceutical composition comprising same.

The present disclosure further relates to a method for treating and/or preventing a KRAS G12D-mediated disease or disorder, the method comprising administering to a patient in need thereof a therapeutically effective amount of a compound represented by general formula (IN), (IN'), (IM), (IM'), (I), (I'), (II'), (II), (III'), (III'-1), (III), (IV'), (IV'-1), (V'), (V'-1), (IVV'), (VV'), or (VV'-1) or a compound shown in Table A or a pharmaceutically acceptable salt thereof, or a pharmaceutical composition comprising same.

The present disclosure further relates to a method for treating and/or preventing a tumor, the method comprising administering to a patient in need thereof a therapeutically effective amount of a compound represented by general formula (IN), (IN'), (IM), (IM'), (I), (I'), (II'), (II), (III'), (III'-1), (III), (IV'), (IV'-1), (V'), (V'-1), (IVV'), (VV'), or (VV'-1) or a compound shown in Table A or a pharmaceutically acceptable salt thereof, or a pharmaceutical composition comprising same; preferably, the tumor is selected from the group consisting of brain cancer, thyroid cancer, head and neck cancer, nasopharyngeal cancer, laryngeal cancer, oral cancer, salivary gland cancer, esophageal cancer, gastric cancer, lung cancer, liver cancer, renal cancer, pleural cancer, peritoneal cancer, pancreatic cancer, gallbladder cancer, bile duct cancer, colorectal cancer, small intestine cancer, gastrointestinal stromal tumor, urothelial cancer, urinary tract cancer, bladder cancer, anal cancer, joint cancer, breast cancer, vaginal cancer, ovarian cancer, endometrial cancer, cervical cancer, fallopian tube cancer, testicular cancer, prostate cancer, hemangioma, leukemia, lymphoma, myeloma, skin cancer, melanoma, lipoma, bone cancer, soft tissue sarcoma, neurofibroma, neuroglioma, neuroblastoma, and glioblastoma; more preferably, the tumor is selected from the group consisting of pancreatic cancer, colorectal cancer, and non-small cell lung cancer.

The present disclosure further relates to a compound represented by general formula (IN), (IN'), (IM), (IM'), (I), (I'), (II'), (II), (III'), (III'-1), (III), (IV'), (IV'-1), (V'), (V'-1), (IVV'), (VV'), or (VV'-1) or a compound shown in Table A or a pharmaceutically acceptable salt thereof, or a pharmaceutical composition comprising same for use as a medicament.

The present disclosure further relates to a compound represented by general formula (IN), (IN'), (IM), (IM'), (I), (I'), (II'), (II), (III'), (III'-1), (III), (IV'), (IV'-1), (V'), (V'-1), (IVV'), (VV'), or (VV'-1) or a compound shown in Table A or a pharmaceutically acceptable salt thereof, or a pharmaceutical composition comprising same for inhibiting KRAS G12D.

The present disclosure further relates to a compound represented by general formula (IN), (IN'), (IM), (IM'), (I), (I'), (II'), (II), (III'), (III'-1), (III), (IV'), (IV'-1), (V'), (V'-1), (IVV'), (VV'), or (VV'-1) or a compound shown in Table A or a pharmaceutically acceptable salt thereof, or a pharmaceutical composition comprising same for treating and/or preventing a KRAS G12D-mediated disease or disorder.

The present disclosure further relates to a compound represented by general formula (IN), (IN'), (IM), (IM'), (I), (I'), (II'), (II), (III'), (III'-1), (III), (IV'), (IV'-1), (V'), (V'-1), (IVV'), (VV'), or (VV'-1) or a compound shown in Table A or a pharmaceutically acceptable salt thereof, or a pharmaceutical composition comprising same for treating and/or preventing a tumor; preferably, the tumor is selected from the group consisting of brain cancer, thyroid cancer, head and neck cancer, nasopharyngeal cancer, laryngeal cancer, oral cancer, salivary gland cancer, esophageal cancer, gastric cancer, lung cancer, liver cancer, renal cancer, pleural cancer, peritoneal cancer, pancreatic cancer, gallbladder cancer, bile duct cancer, colorectal cancer, small intestine cancer, gastrointestinal stromal tumor, urothelial cancer, urinary tract cancer, bladder cancer, anal cancer, joint cancer, breast cancer, vaginal cancer, ovarian cancer, endometrial cancer, cervical cancer, fallopian tube cancer, testicular cancer, prostate cancer, hemangioma, leukemia, lymphoma, myeloma, skin cancer, melanoma, lipoma, bone cancer, soft tissue sarcoma, neurofibroma, neuroglioma, neuroblastoma, and glioblastoma; more preferably, the tumor is selected from the group consisting of pancreatic cancer, colorectal cancer, and non-small cell lung cancer.

The disease or disorder described in the present disclosure is one that is treated and/or prevented by inhibiting KRAS G12D.

Preferably, the KRAS G12D-mediated disease or disorder described in the present disclosure is a tumor; preferably, the tumor is selected from the group consisting of brain cancer, thyroid cancer, head and neck cancer, nasopharyngeal cancer, laryngeal cancer, oral cancer, salivary gland cancer, esophageal cancer, gastric cancer, lung cancer, liver cancer, renal cancer, pleural cancer, peritoneal cancer, pancreatic cancer, gallbladder cancer, bile duct cancer, colorectal cancer, small intestine cancer, gastrointestinal stromal tumor, urothelial cancer, urinary tract cancer, bladder cancer, anal cancer, joint cancer, breast cancer, vaginal cancer, ovarian cancer, endometrial cancer, cervical cancer, fallopian tube cancer, testicular cancer, prostate cancer, hemangioma, leukemia, lymphoma, myeloma, skin cancer, melanoma, lipoma, bone cancer, soft tissue sarcoma, neurofibroma, neuroglioma, neuroblastoma, and glioblastoma; more preferably, the tumor is selected from the group consisting of pancreatic cancer, colorectal cancer, and non-small cell lung cancer.

The colorectal cancer described in the present disclosure is preferably colon cancer or rectal cancer.

Preferably, the brain cancer described in the present disclosure is selected from the group consisting of glioblastoma multiforme and neuroblastoma; the soft tissue cancer is selected from the group consisting of fibrosarcoma, gastrointestinal sarcoma, rhabdomyoma, leiomyosarcoma, dedifferentiated liposarcoma, polymorphic liposarcoma, malignant fibrous histiocytoma, round cell sarcoma, and synovial sarcoma; the lymphoma is selected from the group consisting of Hodgkin's disease and non-Hodgkin's lymphoma (e.g., mantle cell lymphoma, diffuse large B cell lymphoma, follicular center lymphoma, marginal zone B cell lymphoma, lymphoplasmacytic lymphoma, and peripheral T cell lymphoma); the liver cancer is preferably hepatocellular carcinoma; the lung cancer (also known as bronchogenic lung cancer) is selected from the group consisting of non-small cell lung cancer (NSCLC), small cell lung cancer (SCLC), and squamous cell carcinoma; the renal cancer is selected from the group consisting of renal cell carcinoma, clear cell carcinoma, and renal oncocytoma; the leukemia is selected from the group consisting of chronic lymphocytic leukemia (CLL), chronic myelogenous leukemia, acute lymphoblastic leukemia (ALL), T-cell acute lymphoblastic leukemia (T-ALL), chronic myelogenous leukemia (CML), and acute myelogenous leukemia (AML); the skin cancer is selected from the group consisting of malignant melanoma, squamous cell carcinoma, basal cell carcinoma, and angiosarcoma; the myeloma is preferably multiple myeloma.

The active compound may be formulated into a form suitable for administration by any suitable route, and one or more pharmaceutically acceptable carriers are used to formulate the composition of the present disclosure by conventional methods. Thus, the active compound of the present disclosure may be formulated into a variety of dosage forms for oral administration, administration by injection (e.g., intravenous, intramuscular, or subcutaneous), or administration by inhalation or insufflation. The compounds of the present disclosure may also be formulated into a dosage form, such as tablets, hard or soft capsules, aqueous or oily suspensions, emulsions, injections, dispersible powders or granules, suppositories, lozenges or syrups.

As a general guide, the active compound of the present disclosure is preferably in a form of a unit dose, or in a form of a single dose that can be self-administered by a patient. The unit dose of the compound or composition of the present disclosure may be expressed in the form of a tablet, capsule, cachet, vial, powder, granule, lozenge, suppository, regenerating powder, or liquid formulation. A suitable unit dose may be 0.1-1000 mg. The pharmaceutical composition of the present disclosure may comprise, in addition to the active compound, one or more auxiliary materials selected from the group consisting of a filler (diluent), a binder, a wetting agent, a disintegrant, or an excipient or the like. Depending on the method of administration, the composition may comprise 0.1 to 99 wt.% of the active compound.

The tablet comprises the active ingredient, and non-toxic pharmaceutically acceptable excipients that are used for mixing and are suitable for the preparation of the tablet. These excipients may be an inert excipient, a granulating agent, a disintegrant, a binder, and a lubricant. These tablets may be uncoated or coated using known techniques that mask the taste of the drug or delay disintegration and absorption in the gastrointestinal tract, thereby providing a sustained-release effect over an extended period of time.

An oral formulation may also be provided in the form of a soft gelatin capsule in which the active ingredient is mixed with an inert solid diluent or with a water-soluble carrier or oil vehicle.

An aqueous suspension comprises the active substance and an excipient that is used for mixing and is suitable for the preparation of the aqueous suspension. Such an excipient is a suspending agent, a dispersant, or a wetting agent. The aqueous suspension may also comprise one or more preservatives, one or more colorants, one or more corrigents, and one or more sweeteners.

An oil suspension may be formulated by suspending the active ingredient in a vegetable oil, or in a mineral oil. The oil suspension may comprise a thickening agent. The sweeteners and corrigents described above may be added to provide a palatable formulation. Antioxidants may also be added to preserve the compositions.

The pharmaceutical composition of the present disclosure may also be in the form of an oil-in-water emulsion. The oil phase may be a vegetable oil or a mineral oil, or a mixture thereof. Suitable emulsifiers may be naturally occurring phospholipids, and the emulsion may also comprise a sweetener, a corrigent, a preservative, and an antioxidant. Such a formulation may also comprise a palliative, a preservative, a colorant, and an antioxidant. The pharmaceutical composition of the present disclosure may be in the form of a sterile injectable aqueous solution. Acceptable vehicles or solvents that can be used include water, Ringer's solution, and isotonic sodium chloride solution. A sterile injectable formulation may be a sterile injectable oil-in-water microemulsion in which an active ingredient is dissolved in an oil phase. The injection or microemulsion can be locally injected into the bloodstream of a patient in large quantities. Alternatively, it may be desirable to administer the solution and microemulsion in such a way as to maintain a constant circulating concentration of the compound of the present disclosure. To maintain such a constant concentration, a continuous intravenous delivery device may be used. An example of such a device is a Deltec CADD-PLUS. TM. 5400 intravenous injection pump.

The pharmaceutical composition of the present disclosure may be in the form of a sterile injectable aqueous or oil suspension for intramuscular and subcutaneous administration. The suspension can be prepared according to the prior art using those suitable dispersants or wetting agents and suspending agents as described above. The sterile injectable formulation may also be a sterile injection or suspension prepared in a parenterally acceptable non-toxic diluent or solvent. In addition, a sterile fixed oil may be conventionally used as a solvent or a suspending medium. For this purpose, any blend fixed oil may be used. In addition, fatty acids may also be used to prepare injections.

The compound of the present disclosure may be administered in the form of a suppository for rectal administration. Such a pharmaceutical composition can be prepared by mixing a drug with a suitable non-irritating excipient which is a solid at ambient temperature but a liquid in the rectum and therefore will melt in the rectum to release the drug.

The compound of the present disclosure can be administered in the form of dispersible powders and granules that are formulated into aqueous suspensions by adding water. Such a pharmaceutical composition can be prepared by mixing the active ingredient with a dispersant or a wetting agent, a suspending agent, or one or more preservatives.

As is well known to those skilled in the art, the dose of the drug administered depends on a variety of factors, including but not limited to, the activity of the particular compound employed, the severity of the disease, the age of the patient, the weight of the patient, the health condition of the patient, the behavior of the patient, the diet of the patient, the time of administration, the route of administration, the rate of excretion, the combination of drugs, and the like. In addition, the optimal treatment regimen, such as the mode of administration, the daily dose of the compound or the type of pharmaceutically acceptable salts, can be verified according to conventional treatment regimens.

### Terminology

Unless otherwise stated, the terms used in the specification and claims have the following meanings.

The term "alkyl" refers to a saturated aliphatic hydrocarbon group which is a linear or branched group containing 1 to 20 carbon atoms, preferably to alkyl having 1 to 12 (e.g., 1, 2, 3, 4, 5, 6, 7, 8, 9, 10, 11, and 12) carbon atoms (i.e., C₁₋₁₂ alkyl), and more preferably to alkyl having 1 to 6 carbon atoms (i.e., C₁₋₆ alkyl). Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof, and the like. Alkyl may be substituted or unsubstituted, and when it is substituted, it may be substituted at any accessible point of attachment, and the substituent is preferably selected from the group consisting of one or more of a D atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkenyl" refers to alkyl containing at least one carbon-carbon double bond in the molecule, wherein alkyl is as defined above, preferably to alkenyl having 2 to 12 (e.g., 2, 3, 4, 5, 6, 7, 8, 9, 10, 11, and 12) carbon atoms (i.e., C₂₋₁₂ alkenyl), and more preferably to alkenyl having 2 to 6 carbon atoms (i.e., C₂₋₆ alkenyl). Non-limiting examples include: ethenyl, propenyl, isopropenyl, butenyl, and the like. Alkenyl may be substituted or unsubstituted, and when it is substituted, the substituent is preferably selected from the group consisting of one or more of alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkynyl" refers to alkyl containing at least one carbon-carbon triple bond in the molecule, wherein the alkyl is as defined above, preferably to alkynyl having 2 to 12 (e.g., 2, 3, 4, 5, 6, 7, 8, 9, 10, 11, and 12) carbon atoms (i.e., C₂₋₁₂ alkynyl), and more preferably to alkynyl having 2 to 6 carbon atoms (i.e., C₂₋₆ alkynyl). Non-limiting examples include: ethynyl, propynyl, butynyl, pentynyl, hexynyl, and the like. Alkynyl may be substituted or unsubstituted, and when it is substituted, the substituent is preferably selected from the group consisting of one or more of alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "cycloalkyl" refers to a saturated or partially unsaturated monocyclic or polycyclic hydrocarbon substituent. The cycloalkyl ring contains 3 to 20 carbon atoms, preferably 3 to 14 (e.g., 3, 4, 5, 6, 7, 8, 9, 10, 11, 12, 13, and 14) carbon atoms (i.e., 3-14 membered cycloalkyl), preferably 3 to 8 (e.g., 3, 4, 5, 6, 7, and 8) carbon atoms (i.e., 3-8 membered cycloalkyl), and more preferably 3 to 6 carbon atoms (i.e., 3-6 membered cycloalkyl). Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like. Polycyclic cycloalkyl includes spirocycloalkyl, fused cycloalkyl, and bridged cycloalkyl.

The term "spirocycloalkyl" refers to a 5- to 20-membered polycyclic group in which monocyclic rings share one carbon atom (referred to as the spiro atom), and the group may contain one or more double bonds. It is preferably 6- to 14-membered, and is more preferably 7- to 10-membered (e.g., 7-, 8-, 9-, or 10-membered). According to the number of the spiro atoms shared among the rings, spirocycloalkyl may be monospirocycloalkyl or polyspirocycloalkyl (e.g., bispirocycloalkyl), preferably monospirocycloalkyl and bispirocycloalkyl, and more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered monospirocycloalkyl. Non-limiting examples of spirocycloalkyl include:

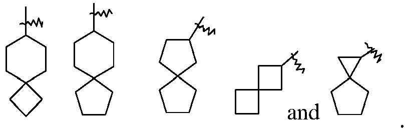

The term "fused cycloalkyl" refers to a 5- to 20-membered all-carbon polycyclic group in which each ring shares a pair of adjacent carbon atoms with the other rings in the system, wherein one or more rings may contain one or more double bonds. It is preferably 6- to 14-membered, and is more preferably 7- to 10-membered (e.g., 7-, 8-, 9-, or 10-membered). According to the number of constituent rings, it may be bicyclic or polycyclic fused cycloalkyl (e.g., tricyclic or tetracyclic fused cycloalkyl), preferably bicyclic or tricyclic fused cycloalkyl, and more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered bicyclic alkyl. Non-limiting examples of fused cycloalkyl include:

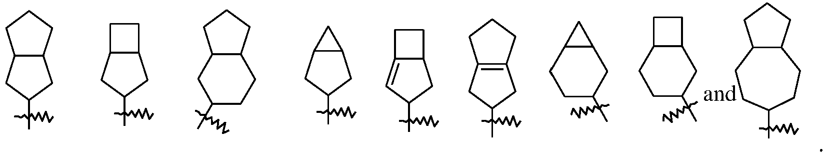

The term "bridged cycloalkyl" refers to a 5- to 20-membered all-carbon polycyclic group in which any two rings share two carbon atoms that are not directly connected, and the group may contain one or more double bonds. It is preferably 6- to 14-membered, and is more preferably 7- to 10-membered (e.g., 7-, 8-, 9-, or 10-membered). According to the number of constituent rings, it may be bicyclic or polycyclic (e.g., tricyclic or tetracyclic) bridged cycloalkyl, preferably bicyclic, tricyclic, or tetracyclic bridged cycloalkyl, and more preferably bicyclic or tricyclic bridged cycloalkyl. Non-limiting examples of bridged cycloalkyl include:

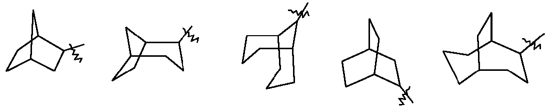

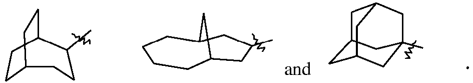

The cycloalkyl ring includes those in which the cycloalkyl described above (including monocyclic, spiro, fused, and bridged rings) is fused to an aryl, heteroaryl, or heterocycloalkyl ring, wherein the ring attached to the parent structure is cycloalkyl. Non-limiting examples include 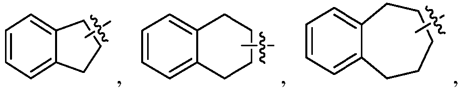 and the like, preferably

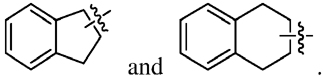

Cycloalkyl may be substituted or unsubstituted, and when it is substituted, it may be substituted at any accessible point of attachment, and the substituent is preferably selected from the group consisting of one or more of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkoxy" refers to -O-(alkyl), wherein the alkyl is as defined above. Non-limiting examples of alkoxy include: methoxy, ethoxy, propoxy, and butoxy. Alkoxy may be optionally substituted or unsubstituted, and when it is substituted, the substituent is preferably selected from the group consisting of a D atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "heterocyclyl" refers to a saturated or partially unsaturated monocyclic or polycyclic substituent containing 3 to 20 (e.g., 3, 4, 5, 6, 7, 8, 9, 10, 11, 12, 13, 14, 15, 16, 17, 18, 19, or 20) ring atoms (i.e., 3- to 20-membered heterocyclyl), wherein one or more ring atoms are heteroatoms selected from the group consisting of nitrogen, oxygen, and sulfur, and the sulfur may be optionally substituted with oxo (i.e., to form sulfoxide or sulfone), but a cyclic portion of -O-O-, -O-S-, or -S-S- is not included; the other ring atoms are carbons. The heterocyclyl preferably contains 3 to 14 (e.g., 3, 4, 5, 6, 7, 8, 9, 10, 11, 12, 13, and 14) ring atoms (i.e., 3-14 membered heterocyclyl), of which 1 to 4 (e.g., 1, 2, 3, and 4) are heteroatoms; more preferably 3 to 8 ring atoms (e.g., 3, 4, 5, 6, 7, and 8) (i.e., 3-8 membered heterocyclyl) or 6 to 14 ring atoms (e.g., 6, 7, 8, 9, 10, 11, 12, 13, and 14), of which 1 to 3 (e.g., 1, 2, and 3) are heteroatoms; more preferably 3 to 8 ring atoms, of which 1 to 3 (e.g. 1, 2, and 3) are heteroatoms; most preferably 5 or 6 ring atoms (i.e., 5- or 6-membered heterocyclyl), of which 1 to 3 are heteroatoms. Non-limiting examples of monocyclic heterocyclyl include pyrrolidinyl, tetrahydropyranyl, 1,2,3,6-tetrahydropyridinyl, piperidinyl, piperazinyl, morpholinyl, thiomorpholinyl, homopiperazinyl, and the like. Polycyclic heterocyclyl includes spiroheterocyclyl, fused heterocyclyl, and bridged heterocyclyl.

The term "spiroheterocyclyl" refers to a 5-20 membered polycyclic heterocyclyl group in which monocyclic rings share one atom (referred to as the spiro atom), wherein one or more ring atoms are heteroatoms selected from the group consisting of nitrogen, oxygen, and sulfur, and the sulfur may be optionally substituted with oxo (i.e., to form sulfoxide or sulfone); the other ring atoms are carbons. It may contain one or more double bonds. It is preferably 6- to 14-membered (e.g., 6-, 7-, 8-, 9-, 10-, 11-, 12-, 13-, and 14-membered) (i.e., 6- to 14-membered spiroheterocyclyl), and is more preferably 7- to 10-membered (e.g., 7-, 8-, 9-, or 10-membered) (i.e., 7- to 10-membered spiroheterocyclyl). According to the number of spiro atoms shared among the rings, spiroheterocyclyl may be monospiroheterocyclyl or polyspiroheterocyclyl (e.g., bispiroheterocyclyl), preferably monospiroheterocyclyl and bispiroheterocyclyl, and more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered monospiroheterocyclyl. Non-limiting examples of spiroheterocyclyl include:

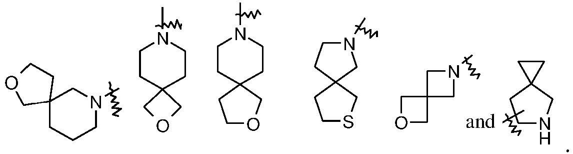

The term "fused heterocyclyl" refers to a 5- to 20-membered polycyclic heterocyclyl group in which each ring shares a pair of adjacent atoms with the other rings in the system, wherein one or more rings may contain one or more double bonds; one or more ring atoms are heteroatoms selected from the group consisting of nitrogen, oxygen, and sulfur, and the sulfur is optionally substituted with oxo (i.e., to form sulfoxide or sulfone); the other ring atoms are carbons. It is preferably 6- to 14-membered (e.g., 6-, 7-, 8-, 9-, 10-, 11-, 12-, 13-, and 14-membered) (i.e., 6- to 14-membered fused heterocyclyl), and is more preferably 7- to 10-membered (e.g., 7-, 8-, 9-, or 10-membered) (i.e., 7- to 10-membered fused heterocyclyl). According to the number of constituent rings, it may be bicyclic or polycyclic (e.g., tricyclic or tetracyclic) fused heterocyclyl, preferably bicyclic or tricyclic fused heterocyclyl, and more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered bicyclic fused heterocyclyl. Non-limiting examples of fused heterocyclyl include: 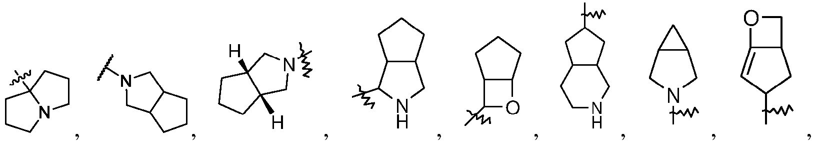 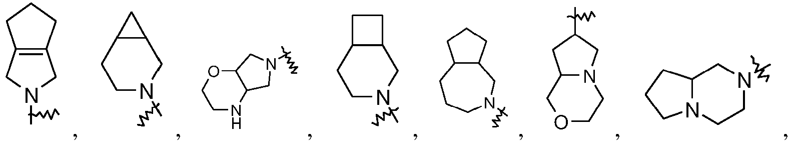 and

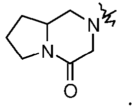

The term "bridged heterocyclyl" refers to a 5- to 20-membered polycyclic heterocyclyl group in which any two rings share two atoms that are not directly connected, and the group may contain one or more double bonds, wherein one or more ring atoms are heteroatoms selected from the group consisting of nitrogen, oxygen, and sulfur, and the sulfur may be optionally substituted with oxo (i.e., to form sulfoxide or sulfone); the other ring atoms are carbons. It is preferably 6- to 14-membered (e.g., 6-, 7-, 8-, 9-, 10-, 11-, 12-, 13-, and 14-membered) (i.e., 6- to 14-membered bridged heterocyclyl), and is more preferably 7- to 10-membered (e.g., 7-, 8-, 9-, or 10-membered) (i.e., 7- to 10-membered bridged heterocyclyl). According to the number of constituent rings, it may be bicyclic or polycyclic (e.g., tricyclic or tetracyclic) bridged heterocyclyl, preferably bicyclic, tricyclic, or tetracyclic bridged heterocyclyl, and more preferably bicyclic or tricyclic bridged heterocyclyl. Non-limiting examples of bridged heterocyclyl include:

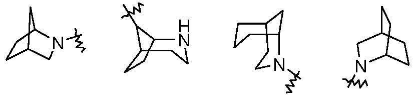

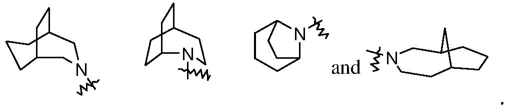

The heterocyclyl ring includes those in which the heterocyclyl described above (including monocyclic, spiroheterocyclic, fused heterocyclic, and bridged heterocyclic rings) is fused to an aryl, heteroaryl, or cycloalkyl ring, wherein the ring attached to the parent structure is heterocyclyl; its non-limiting examples include: 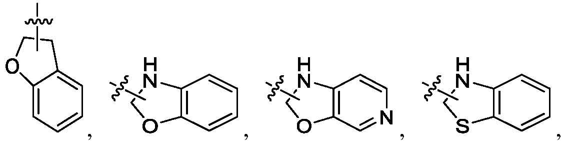 and the like.

Heterocyclyl may be substituted or unsubstituted, and when it is substituted, it may be substituted at any accessible point of attachment, and the substituent is preferably selected from the group consisting of one or more of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "aryl" refers to a 6- to 14-membered, preferably 6- to 10-membered, all-carbon monocyclic or fused polycyclic (which refers to rings sharing a pair of adjacent carbon atoms) group having a conjugated π-electron system, such as phenyl and naphthyl. The aryl ring includes those in which the aryl ring described above is fused to a heteroaryl, heterocyclyl, or cycloalkyl ring, wherein the ring attached to the parent structure is an aryl ring; its non-limiting examples include:

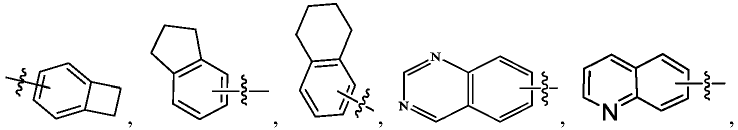

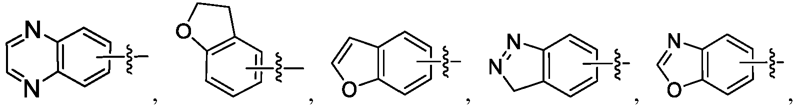

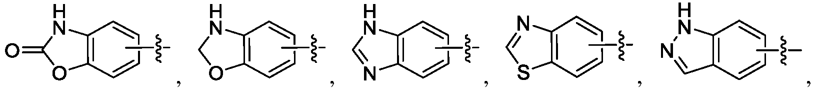

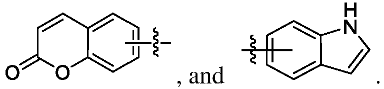

Aryl may be substituted or unsubstituted, and when it is substituted, it may be substituted at any accessible point of attachment, and the substituent is preferably selected from the group consisting of one or more of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "heteroaryl" refers to a heteroaromatic system containing 1 to 4 (e.g., 1, 2, 3, and 4) heteroatoms and 5 to 14 ring atoms, wherein the heteroatoms are selected from the group consisting of oxygen, sulfur, and nitrogen. Heteroaryl is preferably 5- to 10-membered (e.g., 5-, 6-, 7-, 8-, 9-, or 10-membered) (i.e., 5- to 10-membered heteroaryl), is more preferably 8- to 10-membered (e.g., 8-, 9-, or 10-membered), and is yet more preferably 5- or 6-membered (i.e., 5- or 6-membered heteroaryl), such as furyl, thienyl, pyridinyl, pyrrolyl, N-alkylpyrrolyl, pyrimidinyl, pyrazinyl, pyridazinyl, imidazolyl, pyrazolyl, triazolyl, and tetrazolyl. The heteroaryl ring includes those in which the heteroaryl described above is fused to an aryl, heterocyclyl, or cycloalkyl ring, wherein the ring attached to the parent structure is a heteroaryl ring; its non-limiting examples include:

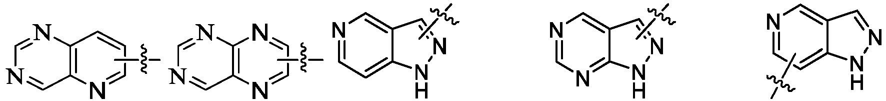

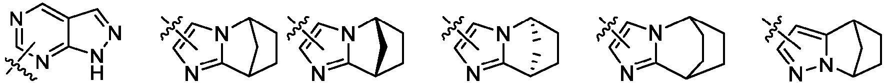

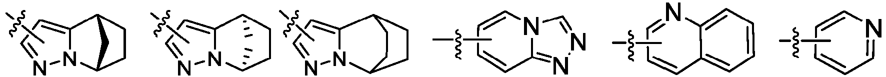

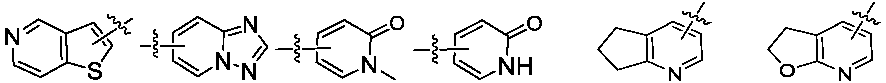

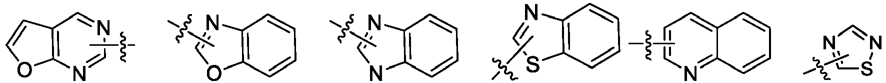

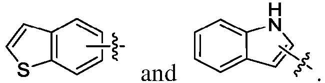

Heteroaryl may be substituted or unsubstituted, and when it is substituted, it may be substituted at any accessible point of attachment, and the substituent is preferably selected from the group consisting of one or more of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The cycloalkyl, heterocyclyl, aryl, and heteroaryl described above include residues derived by removing one hydrogen atom from a ring atom of the parent structure, or residues derived by removing two hydrogen atoms from the same ring atom or two different ring atoms of the parent structure, i.e., "divalent cycloalkyl", "divalent heterocyclyl", "arylene", and "heteroarylene".

The term "amino protecting group" refers to an easily removable group for protecting an amino group from being changed when a reaction is taking place elsewhere in the molecule. Non-limiting examples include (trimethylsilyl)ethoxymethyl (SEM), tetrahydropyranyl, tert-butyloxycarbonyl (Boc), acetyl, benzyl, allyl, p-methylbenzenesulfonyl (Ts), p-methoxybenzyl, and the like. These groups may be optionally substituted with 1 to 3 substituents selected from the group consisting of halogen, alkoxy, and nitro; the amino protecting group is preferably Boc.

The term "hydroxy protecting group" refers to a hydroxy derivative commonly used to block or protect hydroxy when a reaction is taking place on another functional group of the compound. As an example, preferably, the hydroxy protecting group includes, for example: triethylsilyl, triisopropylsilyl, tert-butyldimethylsilyl (TBS), tert-butyldiphenylsilyl, methyl, tert-butyl, allyl, benzyl, methoxymethyl (MOM), ethoxyethyl, 2-tetrahydropyranyl (THP), formyl, acetyl, benzoyl, p-nitrobenzoyl, and the like; the hydroxy protecting group is preferably MOM.

The term "alkynyl protecting group" refers to an easily removable group introduced onto alkynyl to keep the active hydrogen in the acetylene or terminal alkyne unchanged when a reaction is taking place elsewhere in the molecule. Non-limiting examples include: trimethylsilyl (TMS), triethylsilyl (TES), tert-butyldimethylsilyl (TBS), triisopropylsilyl (TIPS), tert-butyldimethylsilyl (TBDMS), tert-butyldiphenylsilyl (TBDPS), methyl, tert-butyl, allyl, benzyl, methoxymethyl (MOM), ethoxyethyl, 2-tetrahydropyranyl (THP), formyl, acetyl, benzoyl, p-nitrobenzoyl, and the like; the alkynyl protecting group is preferably TIPS.

The term "cycloalkyloxy" refers to cycloalkyl-O-, wherein the cycloalkyl is as defined above.

The term "heterocyclyloxy" refers to heterocyclyl-O-, wherein the heterocyclyl is as defined above.

The term "aryloxy" refers to aryl-O-, wherein the aryl is as defined above.

The term "heteroaryloxy" refers to heteroaryl-O-, wherein the heteroaryl is as defined above.

The term "alkylthio" refers to alkyl-S-, wherein the alkyl is as defined above.

The term "haloalkyl" refers to alkyl substituted with one or more halogens, wherein the alkyl is as defined above.

The term "haloalkoxy" refers to alkoxy substituted with one or more halogens, wherein the alkoxy is as defined above.

The term "deuterated alkyl" refers to alkyl substituted with one or more deuterium atoms, wherein the alkyl is as defined above.

The term "hydroxyalkyl" refers to alkyl substituted with one or more hydroxy groups, wherein the alkyl is as defined above.

The term "halogen" refers to fluorine, chlorine, bromine, or iodine.

The term "hydroxy" refers to -OH.

The term "sulfhydryl" refers to -SH.

The term "amino" refers to -NH₂.

The term "cyano" refers to -CN.

The term "nitro" refers to -NO₂.

The term "oxo" refers to "=O".

The term "carbonyl" refers to C=O.

The term "carboxyl" refers to -C(O)OH.

The term "carboxylate group" refers to -C(O)O(alkyl), -C(O)O(cycloalkyl), (alkyl)C(O)O-, or (cycloalkyl)C(O)O-, wherein the alkyl and cycloalkyl are as defined above.

MOM refers to methoxymethyl.

Boc refers to tert-butyloxycarbonyl.

TIPS refers to triisopropylsilyl.

TBS refers to tert-butyldimethylsilyl.

The compounds of the present disclosure may include all forms of rotamers and conformationally restricted states thereof. Also included are atropisomers. The term "atropisomer" refers to conformational stereoisomers that arise due to hindered or greatly slowed rotation about a single bond in a molecule (as a result of the steric interactions with other parts of the molecule and the asymmetry of the substituents at both ends of the single bond), which interconvert sufficiently slowly to allow separation and isolation under predetermined conditions. For example, certain compounds of the present disclosure may exist in the form of a mixture of atropisomers (e.g., an equal ratio mixture, a mixture enriched in one atropisomer) or a purified atropisomer. The compounds and intermediates of the present disclosure may also exist in different tautomeric forms, and all such forms are included within the scope of the present disclosure. The term "tautomer" or "tautomeric form" refers to structural isomers of different energies that can interconvert via a low-energy barrier. For example, proton tautomers (also known as proton transfer tautomers) include interconversion via proton migration, such as keto-enol and imine-enamine, lactam-lactim isomerization. Examples of keto-enol equilibria are shown below:

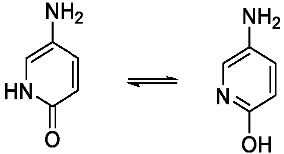

All tautomeric forms are within the scope of the present disclosure. The names of the compounds do not exclude any tautomers.

The compounds of the present disclosure may exist in specific stereoisomeric forms. The term "stereoisomer" refers to isomers that are structurally identical but differ in the arrangement of the atoms in space. It includes cis and trans (or *Z* and *E*) isomers, (-)- and (+)-isomers, (*R*)- and (*S*)-enantiomers, diastereomers, (*D*)- and (*L*)-isomers, tautomers, atropisomers, conformers, and mixtures thereof (e.g., mixtures of racemates and diastereomers). Additional asymmetric atoms may be present in the substituents in the compounds of the present disclosure. All such stereoisomers and mixtures thereof are included within the scope of the present disclosure. For all carbon-carbon double bonds, both *Z*- and *E*-forms are included, even if only one configuration is named. Optically active (-)- and (+)-isomers, (*R*)- and (*S*)-enantiomers, and (*D*)- and (*L*)-isomers can be prepared by chiral synthesis, chiral reagents, or other conventional techniques. One isomer of a certain compound of the present disclosure may be prepared by asymmetric synthesis or derivatization with a chiral auxiliary, or, when the molecule contains a basic functional group (e.g., amino) or an acidic functional group (e.g., carboxyl), a diastereomeric salt is formed with an appropriate optically active acid or base, followed by diastereomeric resolution by conventional methods known in the art to give the pure isomer. In addition, the separation of enantiomers and diastereomers is generally accomplished by chromatography.

In the chemical structures of the compounds of the present disclosure, a bond " 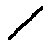 " represents an unspecified configuration; that is, if chiral isomers exist in the chemical structures, the bond " 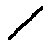 " may be " 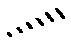 " " or " 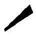 ", or both the configurations of " 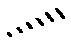 " and " 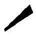 " are included simultaneously.

The compounds of the present disclosure include all suitable isotopic derivatives of the compounds thereof. The term "isotopic derivative" refers to a compound in which at least one atom is replaced with an atom having the same atomic number but a different atomic mass. Examples of isotopes that can be incorporated into the compounds of the present disclosure include stable and radioactive isotopes of hydrogen, carbon, nitrogen, oxygen, phosphorus, sulfur, fluorine, chlorine, bromine, iodine, etc., such as ²H (deuterium, D), ³H (tritium, T), ¹¹C, ¹³C, ¹⁴C, ¹⁵N, ¹⁷O, ¹⁸O, 32ₚ, ³³ₚ, ³³S, ³⁴S, ³⁵S, ³⁶S, ¹⁸F, ³⁶Cl, ⁸²Br, ¹²³I, ¹²⁴I, ¹²⁵I, ¹²⁹I, and ¹³¹I; deuterium is preferred.

Compared to non-deuterated drugs, deuterated drugs have the advantages of reduced toxic and side effects, increased drug stability, enhanced efficacy, prolonged biological half-lives, and the like. All isotopic variations of the compounds of the present disclosure, whether radioactive or not, are intended to be included within the scope of the present disclosure. Each available hydrogen atom connected to a carbon atom may be independently replaced with a deuterium atom, wherein the replacement with deuterium may be partial or complete, and the partial replacement with deuterium refers to the replacement of at least one hydrogen atom with at least one deuterium atom.

"Optionally" or "optional" means that the event or circumstance subsequently described may, but does not necessarily, occur, and this description includes instances where the event or circumstance occurs or does not occur. For example, "C₁₋₆ alkyl optionally substituted with halogen or cyano" means that the halogen or cyano may, but does not necessarily, exist, and this description includes an instance where the alkyl is substituted with halogen or cyano and an instance where the alkyl is not substituted with halogen or cyano.

"Substituted" means that one or more, preferably 1-6, more preferably 1-3 hydrogen atoms in the group are independently substituted with a corresponding number of substituents. Those skilled in the art are able to determine (experimentally or theoretically) possible or impossible substitution without undue effort. For example, it may be unstable when an amino or hydroxy group having free hydrogen is bound to a carbon atom having an unsaturated (e.g., olefinic) bond.

"Pharmaceutical composition" refers to a mixture containing one or more of the compounds described herein or a pharmaceutically acceptable salt or pro-drug thereof, and other chemical components, as well as other components, for example, pharmaceutically acceptable carriers and excipients. The pharmaceutical composition is intended to promote administration to an organism and facilitate the absorption of the active ingredient so that it can exert its bioactivity.

"Pharmaceutically acceptable salt" refers to a salt of the compound of the present disclosure, which may be selected from the group consisting of inorganic or organic salts. Such salts are safe and effective when used in the body of a mammal and possess the requisite biological activity. The salts may be prepared separately during the final separation and purification of the compound, or by reacting an appropriate group with an appropriate base or acid. Bases commonly used to form pharmaceutically acceptable salts include inorganic bases such as sodium hydroxide and potassium hydroxide, and organic bases such as ammonia. Acids commonly used to form pharmaceutically acceptable salts include inorganic acids and organic acids.

For drugs or pharmacologically active agents, the term "therapeutically effective amount" refers to an amount of the drug or agent sufficient to achieve, or at least partially achieve, the desired effect. The determination of the therapeutically effective amount varies from person to person. It depends on the age and general condition of a subject, as well as the specific active substance used. The appropriate therapeutically effective amount in a case may be determined by those skilled in the art in the light of routine tests.

The term "pharmaceutically acceptable" as used herein means that those compounds, materials, compositions, and/or dosage forms that are, within the scope of reasonable medical judgment, suitable for use in contact with the tissues of patients without excessive toxicity, irritation, allergic reaction, or other problems or complications, and are commensurate with a reasonable benefit/risk ratio and effective for the intended use. As used herein, the singular forms "a", "an" and "the" include plural references and vice versa, unless otherwise clearly defined in the context.

When the term "about" is applied to parameters such as pH, concentration, and temperature, it means that the parameter may vary by ±10%, and sometimes more preferably within ±5%. As will be understood by those skilled in the art, when the parameters are not critical, the numbers are generally given for illustrative purposes only and are not intended to be limiting.

### Synthesis methods for the compounds of the present disclosure

To achieve the purposes of the present disclosure, the following technical solutions are adopted in the present disclosure:

### Scheme 1-1

The present disclosure provides a preparation method for a compound represented by general formula (IN) or a pharmaceutically acceptable salt thereof, the method comprising: 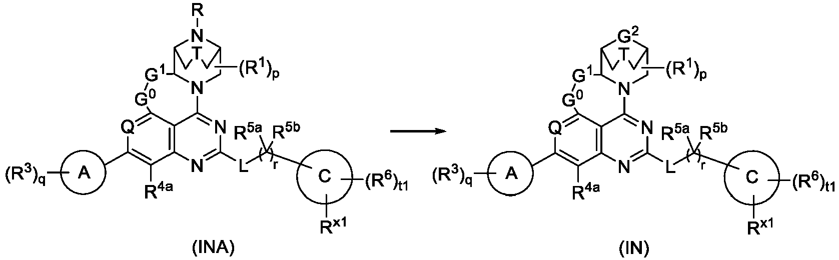
subjecting a compound of general formula (INA) or a salt thereof to a deprotection reaction under acidic conditions to give the compound of general formula (IN) or the pharmaceutically acceptable salt thereof and, when group R³ has a protecting group thereon, optionally a step of removing the protecting group from group R³ under acidic or alkaline conditions before, concurrent with, or after the deprotection reaction, wherein R is an amino protecting group, preferably Boc;
G² is NH;
G⁰, G¹, T, ring A, ring C, Q, L, R¹, R³, R^{4a}, R^{5a}, R^{5b}, R⁶, R^{x1}, p, q, t1, and r are as defined in general formula (IN).

### Scheme 1-2

The present disclosure provides a preparation method for a compound represented by general formula (IN') or a pharmaceutically acceptable salt thereof, the method comprising: 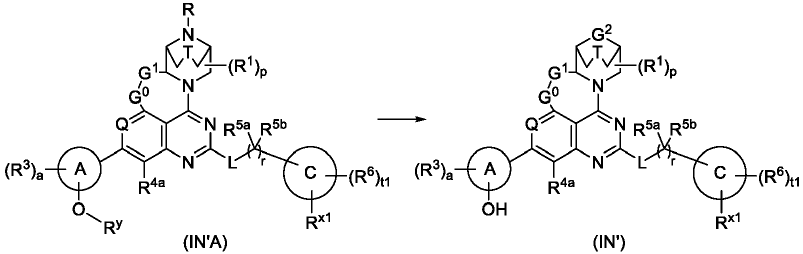
subjecting a compound of general formula (IN'A) or a salt thereof to a deprotection reaction under acidic conditions to give the compound of general formula (IN') or the pharmaceutically acceptable salt thereof and, when group R³ has a protecting group thereon, optionally a step of removing the protecting group from group R³ before, concurrent with, or after the deprotection reaction,
wherein:
   R is an amino protecting group, preferably Boc;
   R^{y} is a hydroxy protecting group, preferably MOM;
   a is 0, 1, 2, 3, or 4;
   G² is NH;
   G⁰, G¹, T, ring A, ring C, Q, L, R¹, R³, R^{4a}, R^{5a}, R^{5b}, R⁶, R^{x1}, p, t1, and r are as defined in general formula (IN').

### Scheme 1-3

The present disclosure provides a preparation method for a compound represented by general formula (IM) or a pharmaceutically acceptable salt thereof, the method comprising: 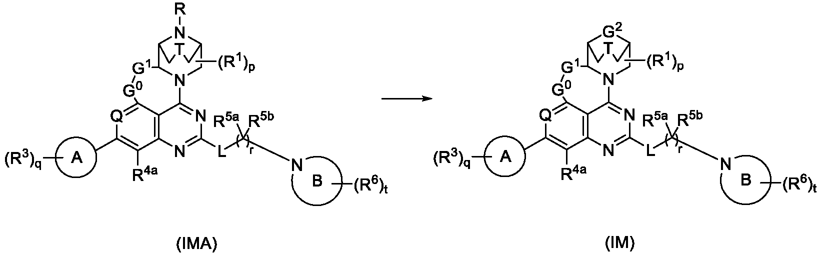
subjecting a compound of general formula (IMA) or a salt thereof to a deprotection reaction under acidic conditions to give the compound of general formula (IM) or the pharmaceutically acceptable salt thereof and, when group R³ has a protecting group thereon, optionally a step of removing the protecting group from group R³ under acidic or alkaline conditions before, concurrent with, or after the deprotection reaction,
wherein R is an amino protecting group, preferably Boc;
G² is NH;
G⁰, G¹, T, ring A, ring B, Q, L, R¹, R³, R^{4a}, R^{5a}, R^{5b}, R⁶, p, q, t, and r are as defined in general formula (IM).

### Scheme 1-4

The present disclosure provides a preparation method for a compound represented by general formula (IM') or a pharmaceutically acceptable salt thereof, the method comprising: 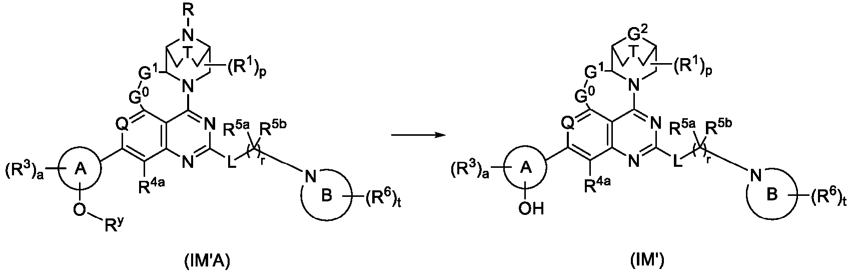
subjecting a compound of general formula (IM'A) or a salt thereof to a deprotection reaction under acidic conditions to give the compound of general formula (IM') or the pharmaceutically acceptable salt thereof and, when group R³ has a protecting group thereon, optionally a step of removing the protecting group from group R³ under acidic or alkaline conditions before, concurrent with, or after the deprotection reaction,
wherein:
   R is an amino protecting group, preferably Boc;
   R^{y} is a hydroxy protecting group, preferably MOM;
   a is 0, 1, 2, 3, or 4;
   G² is NH;
   G⁰, G¹, T, ring A, ring B, Q, L, R¹, R³, R^{4a}, R^{5a}, R^{5b}, R⁶, p, t, and r are as defined in general formula (IM').

### Scheme 2-1

The present disclosure provides a preparation method for a compound represented by general formula (I) or a pharmaceutically acceptable salt thereof, the method comprising: 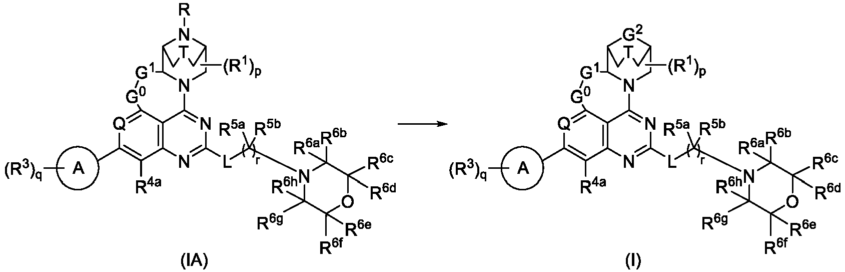
subjecting a compound of general formula (IA) or a salt thereof to a deprotection reaction under acidic conditions to give the compound of general formula (I) or the pharmaceutically acceptable salt thereof and, when group R³ has a protecting group thereon, optionally a step of removing the protecting group from group R³ under acidic or alkaline conditions before, concurrent with, or after the deprotection reaction,
wherein R is an amino protecting group, preferably Boc;
G² is NH;
G⁰, G¹, T, ring A, Q, L, R¹, R³, R^{4a}, R^{5a}, R^{5b}, R^{6a}, R^{6b}, R^{6c}, R^{6d} R^{6e}, R^{6f}, R^{6g}, R^{6h}, p, q, and r are as defined in general formula (I).

### Scheme 2-2

The present disclosure provides a preparation method for a compound represented by general formula (I') or a pharmaceutically acceptable salt thereof, the method comprising: 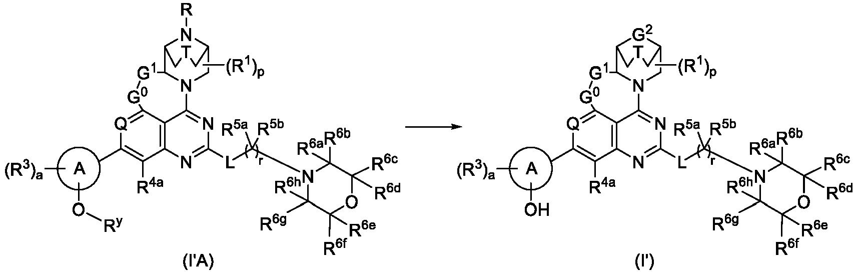
subjecting a compound of general formula (I'A) or a salt thereof to a deprotection reaction under acidic conditions to give the compound of general formula (I') or the pharmaceutically acceptable salt thereof and, when group R³ has a protecting group thereon, optionally a step of removing the protecting group from group R³ under acidic or alkaline conditions before, concurrent with, or after the deprotection reaction,
wherein:
   R is an amino protecting group, preferably Boc;
   R^{y} is a hydroxy protecting group, preferably MOM;
   a is 0, 1, 2, 3, or 4;
   G² is NH;
   G⁰, G¹, T, ring A, Q, L, R¹, R³, R^{4a}, R^{5a}, R^{5b}, R^{6a}, R^{6b}, R^{6c}, R^{6d} R^{6e}, R^{6f}, R^{6g}, R^{6h}, p, and r are as defined in general formula (I').

### Scheme 3-1

The present disclosure provides a preparation method for a compound represented by general formula (II') or a pharmaceutically acceptable salt thereof, the method comprising: 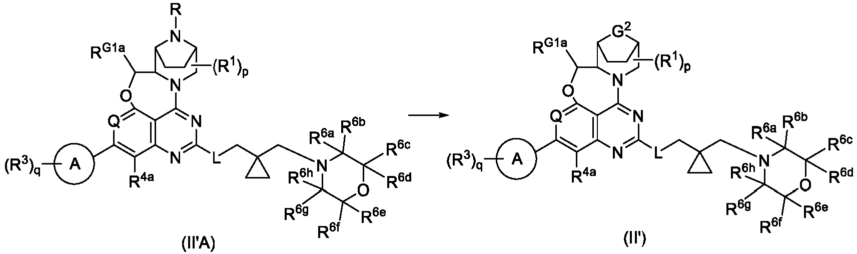
subjecting a compound of general formula (II'A) or a salt thereof to a deprotection reaction under acidic conditions to give the compound of general formula (II') or the pharmaceutically acceptable salt thereof and, when group R³ has a protecting group thereon, optionally a step of removing the protecting group from group R³ under acidic or alkaline conditions before, concurrent with, or after the deprotection reaction,
wherein R is an amino protecting group, preferably Boc;
G² is NH;
ring A, Q, L, R^{G1a}, R¹, R³, R^{4a}, R^{6a}, R^{6b}, R^{6c}, R^{6d}, R^{6e}, R^{6f}, R^{6g}, R^{6h}, p, and q are as defined in general formula (II').

### Scheme 3-2

The present disclosure provides a preparation method for a compound represented by general formula (II) or a pharmaceutically acceptable salt thereof, the method comprising: 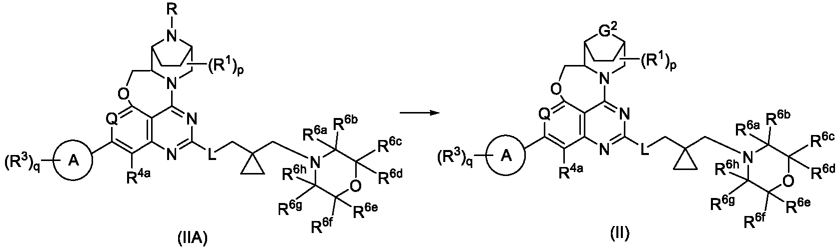
subjecting a compound of general formula (IIA) or a salt thereof to a deprotection reaction under acidic conditions to give the compound of general formula (II) or the pharmaceutically acceptable salt thereof and, when group R³ has a protecting group thereon, optionally a step of removing the protecting group from group R³ under acidic or alkaline conditions before, concurrent with, or after the deprotection reaction,
wherein R is an amino protecting group, preferably Boc;
G² is NH;
ring A, Q, L, R¹, R³, R^{4a}, R^{6a}, R^{6b}, R^{6c}, R^{6d}, R^{6e}, R^{6f}, R^{6g}, R^{6h}, p, and q are as defined in general formula (II).

### Scheme 4-1

The present disclosure provides a preparation method for a compound represented by general formula (III') or a pharmaceutically acceptable salt thereof, the method comprising: 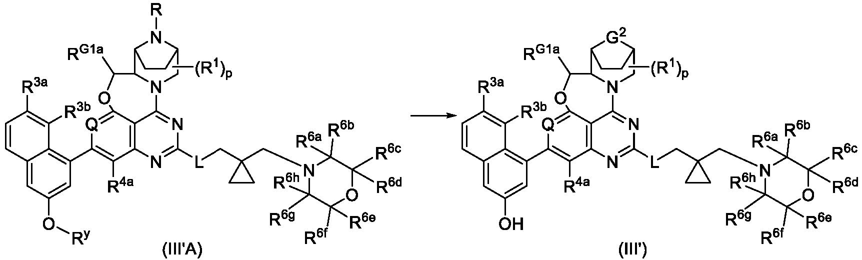
subjecting a compound of general formula (III'A) or a salt thereof to a deprotection reaction under acidic conditions to give the compound of general formula (III') or the pharmaceutically acceptable salt thereof,
wherein:
   R is an amino protecting group, preferably Boc;
   R^{y} is a hydroxy protecting group, preferably MOM;
   G² is NH;
   Q, L, R^{G1a}, R¹, R^{3a}, R^{3b}, R^{4a}, R^{6a}, R^{6b}, R^{6c}, R^{6d}, R^{6e}, R^{6f}, R^{6g}, R^{6h}, and p are as defined in general formula (III').

### Scheme 4-2

The present disclosure provides a preparation method for a compound represented by general formula (III'-1) or a pharmaceutically acceptable salt thereof, the method comprising: 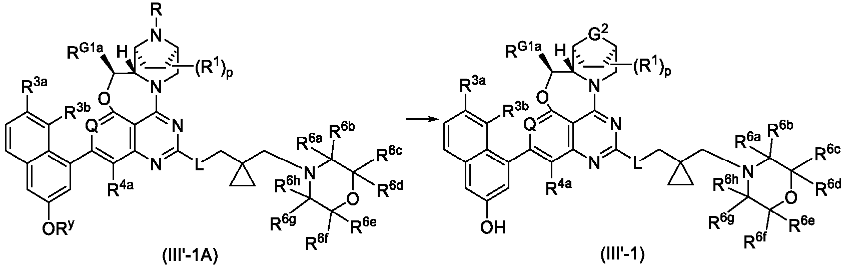
subjecting a compound of general formula (III'-1A) or a salt thereof to a deprotection reaction under acidic conditions to give the compound of general formula (III'-1) or the pharmaceutically acceptable salt thereof,
wherein:
   R is an amino protecting group, preferably Boc;
   R^{y} is a hydroxy protecting group, preferably MOM;
   G² is NH;
   Q, L, R^{G1a}, R¹, R^{3a}, R^{3b}, R^{4a}, R^{6a}, R^{6b}, R^{6c}, R^{6d}, R^{6e}, R^{6f}, R^{6g}, R^{6h}, and p are as defined in general formula (III'-1).

### Scheme 4-3

The present disclosure provides a preparation method for a compound represented by general formula (III) or a pharmaceutically acceptable salt thereof, the method comprising: 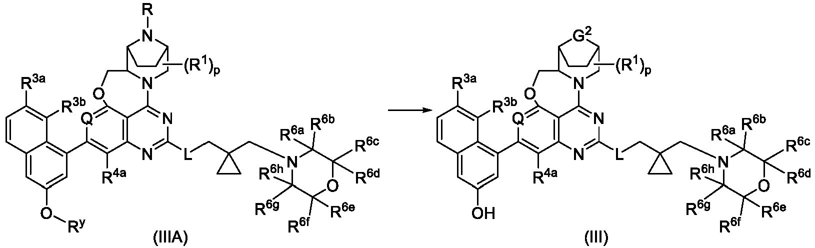
subjecting a compound of general formula (IIIA) or a salt thereof to a deprotection reaction under acidic conditions to give the compound of general formula (III) or the pharmaceutically acceptable salt thereof,
wherein:
   R is an amino protecting group, preferably Boc;
   R^{y} is a hydroxy protecting group, preferably MOM;
   G² is NH;
   Q, L, R¹, R^{3a}, R^{3b}, R^{4a}, R^{6a}, R^{6b}, R^{6c}, R^{6d}, R^{6e}, R^{6f}, R^{6g}, R^{6h}, and p are as defined in general formula (III).

### Scheme 5-1

The present disclosure provides a preparation method for a compound represented by general formula (IVV') or a pharmaceutically acceptable salt thereof, the method comprising: 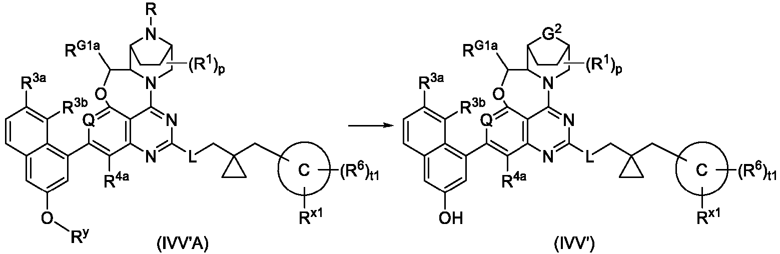
subjecting a compound of general formula (IVV'A) or a salt thereof to a deprotection reaction under acidic conditions to give the compound of general formula (IVV') or the pharmaceutically acceptable salt thereof,
wherein:
   R is an amino protecting group, preferably Boc;
   R^{y} is a hydroxy protecting group, preferably MOM;
   G² is NH;
   ring C, Q, L, R^{G1a}, R¹, R^{3a}, R^{3b}, R^{4a}, R⁶, R^{x1}, t1, and p are as defined in general formula (IVV').

### Scheme 5-2

The present disclosure provides a preparation method for a compound represented by general formula (IV') or a pharmaceutically acceptable salt thereof, the method comprising: 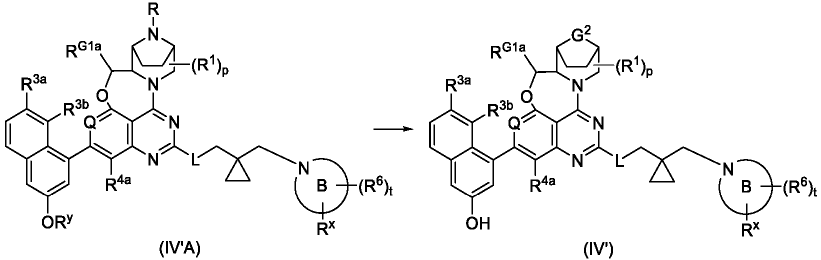
subjecting a compound of general formula (IV'A) or a salt thereof to a deprotection reaction under acidic conditions to give the compound of general formula (IV') or the pharmaceutically acceptable salt thereof,
wherein:
   R is an amino protecting group, preferably Boc;
   R^{y} is a hydroxy protecting group, preferably MOM;
   G² is NH;
   ring B, Q, L, R^{G1a}, R¹, R^{3a}, R^{3b}, R^{4a}, R⁶, R^{x}, t, and p are as defined in general formula (IV').

### Scheme 5-3

The present disclosure provides a preparation method for a compound represented by general formula (IV'-1) or a pharmaceutically acceptable salt thereof, the method comprising: 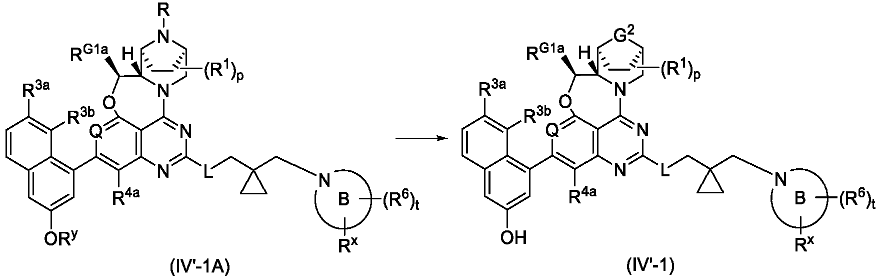
subjecting a compound of general formula (IV'-1A) or a salt thereof to a deprotection reaction under acidic conditions to give the compound of general formula (IV'-1) or the pharmaceutically acceptable salt thereof,
wherein:
   R is an amino protecting group, preferably Boc;
   R^{y} is a hydroxy protecting group, preferably MOM;
   G² is NH;
   ring B, Q, L, R^{G1a}, R¹, R^{3a}, R^{3b}, R^{4a}, R⁶, R^{x}, t, and p are as defined in general formula (IV'-1).

### Scheme 6-1

The present disclosure provides a preparation method for a compound represented by general formula (VV') or a pharmaceutically acceptable salt thereof, the method comprising: 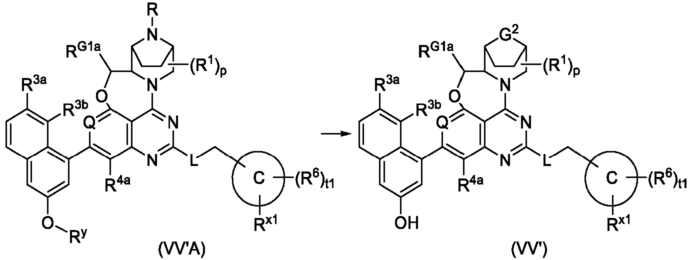
subjecting a compound of general formula (VV'A) or a salt thereof to a deprotection reaction under acidic conditions to give the compound of general formula (VV') or the pharmaceutically acceptable salt thereof,
wherein:
   R is an amino protecting group, preferably Boc;
   R^{y} is a hydroxy protecting group, preferably MOM;
   G² is NH;
   ring C, Q, L, R^{G1a}, R¹, R^{3a}, R^{3b}, R^{4a}, R⁶, R^{x1}, t1, and p are as defined in general formula (VV').

### Scheme 6-2

The present disclosure provides a preparation method for a compound represented by general formula (VV'-1) or a pharmaceutically acceptable salt thereof, the method comprising: 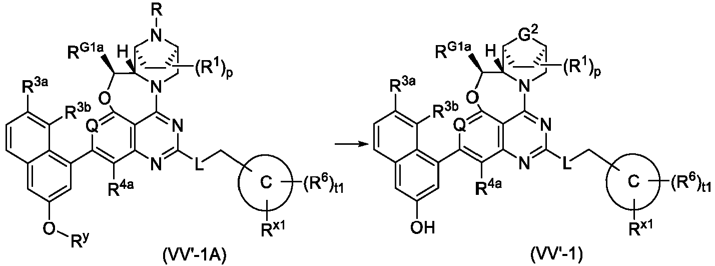
subjecting a compound of general formula (VV'-1A) or a salt thereof to a deprotection reaction under acidic conditions to give the compound of general formula (VV'-1) or the pharmaceutically acceptable salt thereof,
wherein:
   R is an amino protecting group, preferably Boc;
   R^{y} is a hydroxy protecting group, preferably MOM;
   G² is NH;
   ring C, Q, L, R^{G1a}, R¹, R^{3a}, R^{3b}, R^{4a}, R⁶, R^{x1}, t1, and p are as defined in general formula (VV'-1).

### Scheme 6-3

The present disclosure provides a preparation method for a compound represented by general formula (V') or a pharmaceutically acceptable salt thereof, the method comprising: 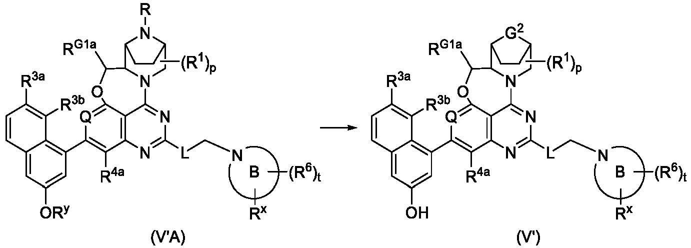
subjecting a compound of general formula (V'A) or a salt thereof to a deprotection reaction under acidic conditions to give the compound of general formula (V') or the pharmaceutically acceptable salt thereof,
wherein:
   R is an amino protecting group, preferably Boc;
   R^{y} is a hydroxy protecting group, preferably MOM;
   G² is NH;
   ring B, Q, L, R^{G1a}, R¹, R^{3a}, R^{3b}, R^{4a}, R⁶, R^{x}, t, and p are as defined in general formula (V').

### Scheme 6-4

The present disclosure provides a preparation method for a compound represented by general formula (V'-1) or a pharmaceutically acceptable salt thereof, the method comprising: 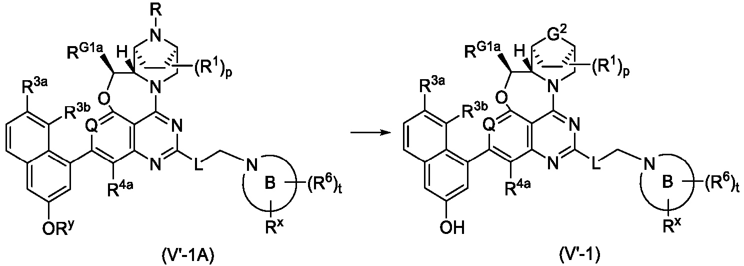
subjecting a compound of general formula (V'-1A) or a salt thereof to a deprotection reaction under acidic conditions to give the compound of general formula (V'-1) or the pharmaceutically acceptable salt thereof,
wherein:
   R is an amino protecting group, preferably Boc;
   R^{y} is a hydroxy protecting group, preferably MOM;
   G² is NH;
   ring B, Q, L, R^{G1a}, R¹, R^{3a}, R^{3b}, R^{4a}, R⁶, R^{x}, t, and p are as defined in general formula (V'-1).

Reagents for providing acidic conditions in the above synthesis schemes include organic acids and inorganic acids; the organic acids include, but are not limited to, trifluoroacetic acid, formic acid, acetic acid, methanesulfonic acid, p-toluenesulfonic acid, Me₃SiCl, and TMSOTf, and the inorganic acids include, but are not limited to, hydrogen chloride, hydrochloric acid in dioxane, hydrochloric acid, sulfuric acid, nitric acid, and phosphoric acid; hydrochloric acid in dioxane is preferred.

Reagents for providing alkaline conditions in the above synthesis schemes include organic bases and inorganic bases; the organic bases include, but are not limited to, triethylamine, N,N-diisopropylethylamine, n-butyllithium, lithium diisopropylamide, potassium acetate, sodium tert-butoxide, potassium tert-butoxide, tetrabutylammonium fluoride, tetrabutylammonium fluoride in tetrahydrofuran, or 1,8-diazabicycloundec-7-ene, and the inorganic bases include, but are not limited to, sodium hydride, potassium phosphate, sodium carbonate, sodium acetate, potassium acetate, potassium carbonate, cesium carbonate, sodium hydroxide, lithium hydroxide, cesium fluoride, and potassium hydroxide.

In the above synthetic schemes, when R³ contains a terminal alkynyl group, the terminal alkynyl group may be protected by TIPS, and the reagent for removing TIPS is preferably tetrabutylammonium fluoride in tetrahydrofuran or cesium fluoride.

The reactions of the above steps are preferably performed in a solvent, including, but not limited to: pyridine, ethylene glycol dimethyl ether, acetic acid, methanol, ethanol, acetonitrile, n-butanol, toluene, tetrahydrofuran, dichloromethane, petroleum ether, ethyl acetate, n-hexane, dimethyl sulfoxide, 1,4-dioxane, water, N,N-dimethylformamide, N,N-dimethylacetamide, 1,2-dibromoethane, and mixtures thereof.

### DETAILED DESCRIPTION

The present disclosure is further described below with reference to examples, but these examples are not intended to limit the scope of the present disclosure.

### Examples

The structures of the compounds were determined by nuclear magnetic resonance (NMR) spectroscopy or/and mass spectrometry (MS). The NMR shifts (δ) are given in 10⁻⁶ (ppm). The NMR analyses were performed using a Bruker AVANCE-400 nuclear magnetic resonance instrument or Bruker AVANCE NEO 500M, with dimethyl sulfoxide-D6 (DMSO-*d₆*), chloroform-D (CDCl₃), and methanol-D4 (CD₃OD) as solvents and tetramethylsilane (TMS) as an internal standard.

The MS analyses were performed using an Agilent 1200/1290 DAD-6110/6120 Quadrupole MS liquid chromatography-mass spectrometry system (manufacturer: Agilent; MS model: 6110/6120 Quadrupole MS), waters ACQuity UPLC-QD/SQD (manufacturer: waters; MS model: waters ACQuity Qda Detector/waters SQ Detector), and THERMO Ultimate 3000-Q Exactive (manufacturer: THERMO; MS model: THERMO Q Exactive).

The high performance liquid chromatography (HPLC) analyses were performed using Agilent HPLC 1200DAD, Agilent HPLC 1200VWD, and Waters HPLC e2695-2489 high performance liquid chromatographs.

The chiral HPLC analyses were performed using an Agilent 1260 DAD high performance liquid chromatograph.

The preparative high performance liquid chromatography steps were performed using Waters 2545-2767, Waters 2767-SQ Detecor2, Shimadzu LC-20AP, and Gilson GX-281 preparative chromatographs.

The preparative chiral chromatography steps were performed using a Shimadzu LC-20AP preparative chromatograph.

The CombiFlash preparative flash chromatograph used was Combiflash Rf200 (TELEDYNE ISCO).

The thin-layer chromatography silica gel plates used were Yantai Huanghai HSGF254 or Qingdao GF254 silica gel plates. The silica gel plates used in the thin-layer chromatography (TLC) had a layer thickness of 0.15 mm-0.2 mm, and those used in the thin-layer chromatography separation and purification had a layer thickness of 0.4 mm-0.5 mm.

In the silica gel column chromatography, a 200-300 mesh silica gel (Huanghai, Yantai) was generally used as the carrier.

The kinase mean inhibition rates and IC₅₀ values were measured using a NovoStar microplate reader (BMG, Germany).

Known starting materials described herein may be synthesized by using or according to methods known in the art, or may be purchased from ABCR GmbH & Co. KG, Acros Organics, Aldrich Chemical Company, Accela ChemBio Inc., Chembee Chemicals, and other companies.

In the examples, the reactions can all be performed under an argon atmosphere or a nitrogen atmosphere unless otherwise specified.

The argon atmosphere or nitrogen atmosphere means that the reaction flask was connected to a balloon containing about 1 L of argon or nitrogen gas.

The hydrogen atmosphere means that the reaction flask was connected to a balloon containing about 1 L of hydrogen gas.

The pressurized hydrogenation reactions were performed using a Parr 3916EKX hydrogenator and a Qinglan QL-500 hydrogenator, or an HC2-SS hydrogenator.

The hydrogenation reactions generally involved 3 cycles of vacuumization and hydrogen filling.

The microwave reactions were performed using a CEM Discover-S 908860 microwave reactor.

In the examples, the solutions were aqueous solutions unless otherwise specified.

In the examples, the reaction temperature was room temperature, i.e., 20 °C-30 °C, unless otherwise specified.

The monitoring of the reaction progress in the examples was conducted by thin-layer chromatography (TLC). The developing solvent used in the reactions, the eluent systems used in the column chromatography purification, and the developing solvent systems used in the thin-layer chromatography analyses include: A: a dichloromethane/methanol system, and B: n-hexane/ethyl acetate. The volume ratio of the solvents was adjusted depending on the polarity of the compound, or by adding a small amount of basic or acidic reagents such as triethylamine and acetic acid.

### Example 1

### 5-Ethyl-6-fluoro-4-((5aS,6S,9R)-1-fluoro-12-((1-(morpholinomethyl)cyclopropyl)methoxy)-5a,6,7,8,9,10-hexahydro-5H-4-oxa-3,10a,11,13,14-pentaaza-6,9-methanonaphtho[1,8-ab]heptalen-2-yl)naphthalen-2-ol 1

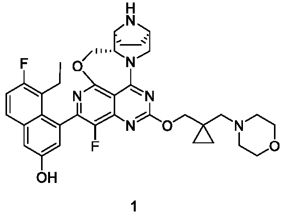

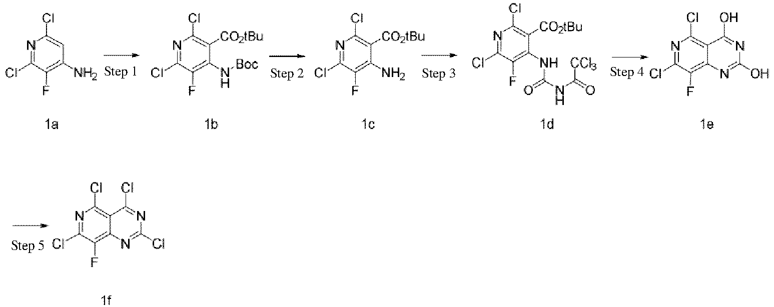

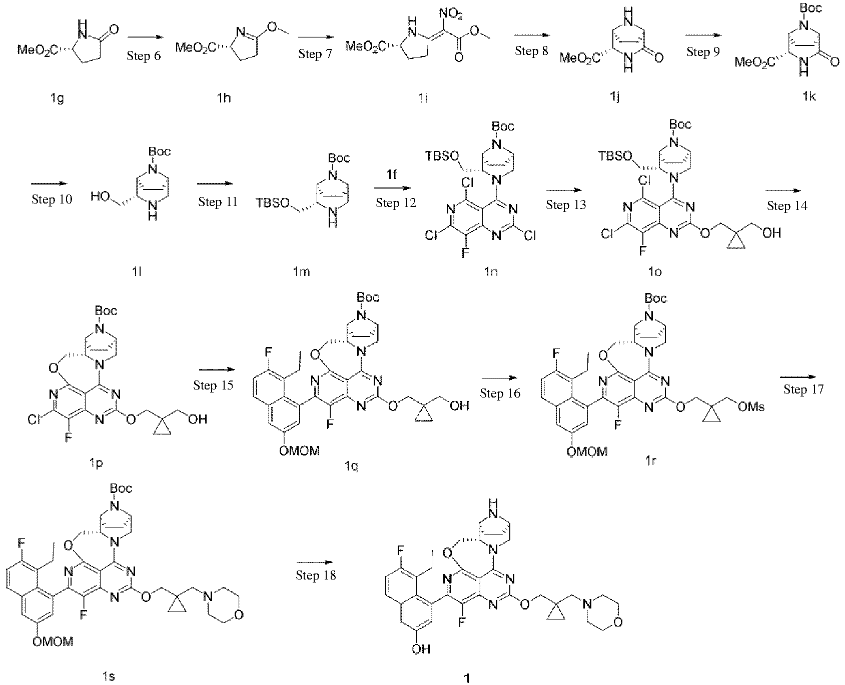

### Step 1

### tert-Butyl 4-((tert-butoxycarbonyl)amino)-2,6-dichloro-5-fluoronicotinate 1b

Crude 2,6-dichloro-3-fluoropyridin-4-amine **1a** (1.8 g, 9.94 mmol, prepared using the method disclosed in Preparation 14 (a) on page 46 of the specification in the patent application "WO2016191524A1") was dissolved in tetrahydrofuran (50 mL), and a 2 M solution of sodium bis(trimethylsilyl)amide in tetrahydrofuran (20 mL) was added under an ice bath. After 0.5 h of stirring, di-tert-butyl dicarbonate (6.5 g, 29.7 mmol) was added. After 14 h of stirring, the reaction mixture was quenched with a saturated aqueous ammonium chloride solution and extracted with ethyl acetate (50 mL × 3). The organic phases were combined and dried over anhydrous sodium sulfate. After the drying agent was removed by filtration, the filtrate was concentrated under reduced pressure, and the residue was purified using eluent system B to give title compound **1b** (1 g, yield: 26.3%). MS m/z (ESI): 381.1[M+1].

### Step 2

### tert-Butyl 4-amino-2,6-dichloro-5-fluoronicotinate 1c

Compound **1b** (1 g, 2.62 mmol) was dissolved in ethyl acetate (8 mL), and a 4 M solution of hydrochloric acid in dioxane (3 mL) was added. The mixture was stirred for 2 h, made neutral with a 4 M aqueous sodium hydroxide solution under an ice bath, and extracted with ethyl acetate (15 mL × 3). The organic phases were combined and dried over anhydrous sodium sulfate. After the drying agent was removed by filtration, the filtrate was concentrated under reduced pressure, and the residue was purified using eluent system A to give title compound **1c** (500 mg, yield: 67.8%).

MS m/z (ESI): 281.1[M+1].

### Step 3

### tert-Butyl 2,6-dichloro-5-fluoro-4-(3-(2,2,2-trichloroacetyl)ureido)nicotinate 1d

Compound **1c** (500 mg, 1.77 mmol) was dissolved in tetrahydrofuran (10 mL), and trichloroacetyl isocyanate (670 mg, 3.55 mmol, Shanghai Hanhong) was added. After 30 min of stirring, the reaction mixture was concentrated under reduced pressure to give crude title compound **1d** (835 mg). The product was directly used in the next step without purification.

MS m/z (ESI): 467.9[M+1].

### Step 4

### 5,7-dichloro-8-fluoro-pyrido[4,3-d]pyrimidine-2,4-diol 1e

Crude compound **1d** (835 mg, 1.77 mmol) was dissolved in a 7 M solution of ammonia in methanol (10 mL). After 1 h of stirring, the reaction mixture was concentrated under reduced pressure, and methyl tert-butyl ether (10 mL) was added to the residue. After 0.5 h of stirring, the mixture was filtered, and the filter cake was dried to give crude title compound **1e** (400 mg, yield: 89.9%). The product was directly used in the next step without purification.

MS m/z (ESI): 249.9[M+1].

### Step 5

### 2,4,5,7-tetrachloro-8-fluoro-pyrido[4,3-d]pyrimidine 1f

Crude compound **1e** (875 mg, 3.5 mmol) was dissolved in phosphorus oxychloride (25 mL), and *N*,*N*-diisopropylethylamine (2.3 g, 17.5 mmol) was added. After 1 h of stirring at 110 °C, the reaction mixture was cooled to room temperature and concentrated under reduced pressure to give crude title compound **1f** (1.14 g). The product was directly used in the next step without purification.

MS m/z (ESI): 285.8[M+1].

### Step 6

### Methyl (R)-5-methoxy-3,4-dihydro-2H-pyrrole-2-carboxylate 1h

Methyl (*R*)-2-pyrrolidone-5-formate **1g** (20 g, 139.7 mmol, Shanghai Bide) and dimethyl sulfate (22.1 g, 175.2 mmol) were mixed and left to react at 60 °C for 22 h. The reaction mixture was cooled to room temperature and poured into a solution of triethylamine (20 g) and water (100 mL) under an ice bath. After extraction with methyl tert-butyl ether (60 mL × 6), concentration was performed under reduced pressure to give crude title compound **1h** (16.3 g, yield: 74.2%). The crude product was directly used in the next step without purification.

MS m/z (ESI): 158.1[M+1].

### Step 7

### Methyl (R)-5-(2-methoxy-1-nitro-2-oxoethylidene)pyrrolidine-2-carboxylate 1i

Crude compound **1h** (16.3 g, 103.7 mmol) and methyl nitroacetate (13.6 g, 114.2 mmol, Shanghai Accela) were mixed, heated to 60 °C, and stirred for 24 h. The reaction mixture was cooled to room temperature and then concentrated under reduced pressure, and the residue was purified by silica gel column chromatography using eluent system B to give title compound **1i** (8.37 g, yield: 33%).

MS m/z (ESI): 245.1[M+1].

### Step 8

### Methyl (1S,2S,5R)-4-oxo-3,8-diazabicyclo[3.2.1]octane-2-carboxylate 1j

Compound **1i** (7.5 g, 30.7 mmol) was dissolved in 150 mL of methanol, and a 10% palladium on carbon catalyst (wet) (1.5 g) was added. The system was purged with hydrogen three times, heated to 50 °C, and stirred for 24 h. The reaction mixture was cooled to room temperature and filtered through diatomaceous earth. The filter cake was washed with methanol, and the filtrate was concentrated under reduced pressure to give crude title compound **1j** (5.6 g). The product was directly used in the next step without purification.

MS m/z (ESI): 185.2[M+1].

### Step 9

### 8-(tert-Butyl) 2-methyl (1S,2S,5R)-4-oxo-3,8-diazabicyclo[3.2.1]octane-2,8-dicarboxylate 1k

Crude compound **1j** (5.65 g, 30.4 mmol) was dissolved in 60 mL of dichloromethane, and triethylamine (6.2 g, 61.27 mmol) and di-tert-butyl dicarbonate (6.6 g, 30.24 mmol) were added under an ice bath. After 14 h of stirring, the reaction mixture was concentrated under reduced pressure, and the residue was purified by silica gel column chromatography using eluent system B to give title compound **1k** (3 g, yield: 34.7%).

MS m/z (ESI): 285.2[M+1].

### Step 10

### tert-Butyl (1S,2S,5R)-2-(hydroxymethyl)-3,8-diazabicyclo[3.2.1]octane-8-carboxylate 1l

Compound **1k** (3 g, 10.55 mmol) was dissolved in 30 mL of tetrahydrofuran, and 32 mL of a 1 M solution of lithium aluminum hydride in tetrahydrofuran was added dropwise under an ice bath. The mixture was warmed to room temperature and stirred for 4 h, and 1.05 mL of water, 1.05 mL of a 15% sodium hydroxide solution, and 3.15 mL of water were sequentially added under an ice bath. The mixture was warmed to room temperature and stirred for 15 min, and anhydrous magnesium sulfate (1 g) was added. The mixture was stirred for 15 min and filtered, and the filtrate was concentrated under reduced pressure to give crude title compound **1l** (2.4 g). The product was directly used in the next step without purification.

MS m/z (ESI): 243.2[M+1].

### Step 11

### tert-Butyl (1S,2S,5R)-2-(((tert-butyldimethylsilyl)oxy)methyl)-3,8-diazabicyclo[3.2.1]octane-8-carboxylate 1m

Crude compound **1l** (2.4 g, 9.9 mmol) was dissolved in 25 mL of dichloromethane, and tert-butyldimethylsilyl chloride (4.48 g, 29.7 mmol) and 4-dimethylaminopyridine (122 mg, 990 µmol) were added. Triethylamine (4 g, 39.5 mmol) was added dropwise, and the mixture was stirred for 16 h. 20 mL of water was added to the reaction mixture, and extraction was performed with dichloromethane (20 mL × 2). The organic phases were combined, washed with a saturated sodium chloride solution, and then concentrated under reduced pressure, and the residue was purified by silica gel column chromatography using eluent system B to give title compound **1m** (1.95 g, yield: 55.2%).

MS m/z (ESI): 357.1[M+1].

### Step 12

### tert-Butyl (1S,2S,5R)-2-(((tert-butyldimethylsilyl)oxy)methyl)-3-(2,5,7-trichloro-8-fluoropyrido[4,3-d]pyrimidin-4-yl)-3,8-diazabicyclo[3.2.1]octane-8-carboxylate 1n

Compound **1f** (50 g, 174 mmol) and *N*,*N*-diisopropylethylamine (65 g, 503 mmol) were dissolved in 75 mL of dichloromethane, and a solution of compound **1m** (60 g, 168.3 mmol) in dichloromethane (250 mL) was added dropwise at -78 °C. The mixture was naturally warmed to room temperature and left to react for 4 h. The reaction mixture was concentrated under reduced pressure, and the residue was purified using eluent system B to give title compound **1n** (86 g, yield: 84%).

MS m/z (ESI): 606.2[M+1].

### Step 13

### tert-Butyl (1S,2S,5R)-2-(((tert-butyldimethylsilyl)oxy)methyl)-3-(5,7-dichloro-8-fluoro-2-((1-(hydroxymethyl)cyclopropyl)methoxy)pyrido[4,3-d]pyrimidin-4-yl)-3,8-diazabicyclo[3.2.1]octane-8-carboxylate 1o

Compound **1n** (5 g, 8.23 mmol) was dissolved in 1,4-dioxane (80 mL), and 1,1-bis(hydroxymethyl)cyclopropane (1.3 g, 12.7 mmol, Shanghai Accela), cesium carbonate (5.4 g, 16.6 mmol), and a 4Å molecular sieve (5 g) were added. After 14 h of stirring at 110 °C, the reaction mixture was cooled to room temperature, then filtered, and concentrated under reduced pressure to give crude title compound **1o** (5.5 g). The product was directly used in the next step without purification.

MS m/z (ESI): 672.2[M+1].

### Step 14

### tert-Butyl (5aS,6S,9R)-2-chloro-1-fluoro-12-((1-(hydroxymethyl)cyclopropyl)methoxy)-5a,6,7,8,9,10-hexahydro-5H-4-oxa-3,10a,11,13,14-pentaaza-6,9-methanonaphtho[1,8-ab]heptalene-14-carboxylate 1p

Crude compound **1o** (5.5 g, 8.2 mmol) was dissolved in tetrahydrofuran (80 mL), and tetrabutylammonium fluoride (5.57 g, 24.7 mmol) and N,N-diisopropylethylamine (5.33 g, 41.2 mmol) were added. The mixture was stirred for 1 h, then heated to 60 °C, and stirred for 2 h. The reaction mixture was cooled to room temperature, diluted with water (80 mL), and extracted with ethyl acetate (50 mL × 2). The organic phases were combined and concentrated under reduced pressure, and the residue was purified by silica gel column chromatography using eluent system A to give title compound **1p** (3 g, yield: 69.7%).

MS m/z (ESI): 522.2[M+1].

### Step 15

### tert-Butyl (5aS,6S,9R)-2-(8-ethyl-7-fluoro-3-(methoxymethoxy)naphthalen-1-yl)-1-fluoro-12-((1-(hydroxymethyl)cyclopropyl)methoxy)-5a,6,7,8,9,10-hexahydro-5H-4-oxa-3,10a,11,13,14-pentaaza-6,9-methanonaphtho[1,8-ab]heptalene-14-carboxylate 1q

Compound **1p** (2 g, 3.83 mmol), 2-(8-ethyl-7-fluoro-3-(methoxymethoxy)naphthalen-1-yl)-4,4,5,5-tetramethyl-1,3,2-dioxaborolane (2.1 g, 5.8 mmol), tetrakis(triphenylphosphine)palladium(0) (889 mg, 769 µmol), and cesium carbonate (3.8 g, 11.66 mmol) were dissolved in 36 mL of a mixed solution of 1,4-dioxane and water (V:V = 10:1). The mixture was left to react at 100 °C for 14 h in a nitrogen atmosphere. The reaction mixture was concentrated under reduced pressure, and the residue was purified by silica gel column chromatography using eluent system A to give title compound **1q** (1 g, yield: 36.2%).

MS m/z (ESI): 720.2[M+1].

### Step 16

### tert-Butyl (5aS,6S,9R)-2-(8-ethyl-7-fluoro-3-(methoxymethoxy)naphthalen-1-yl)-1-fluoro-12-((1-(((methylsulfonyl)oxy)methyl)cyclopropyl)methoxy)-5a,6,7,8,9,10-hexahydro-5H-4-oxa-3,10a,11,13,14-pentaaza-6,9-methanonaphtho[1,8-ab]heptalene-14-carboxylate 1r

Compound **1q** (150 mg, 145.8 µmol) and N,N-diisopropylethylamine (55.6 mg, 437.6 µmol) were dissolved in dichloromethane (5 mL), and methylsulfonyl chloride (25 mg, 218.8 µmol) was added under an ice bath. After 1 h of stirring at that temperature, the reaction mixture was quenched with a saturated ammonium chloride solution and extracted with dichloromethane (5 mL × 2). The organic phases were combined and dried over anhydrous sodium sulfate. After the drying agent was removed by filtration, the filtrate was concentrated under reduced pressure to give crude title compound **1r** (130 mg). The product was directly used in the next step without purification.

MS m/z (ESI): 798.2[M+1].

### Step 17

### tert-Butyl (5aS,6S,9R)-2-(8-ethyl-7-fluoro-3-(methoxymethoxy)naphthalen-1-yl)-1-fluoro-12-((1-(morpholinomethyl)cyclopropyl)methoxy)-5a,6,7,8,9,10-hexahydro-5H-4-oxa-3,10a,11,13,14-pentaaza-6,9-methanonaphtho[1,8-ab]heptalene-14-carboxylate 1s

Crude compound **1r** (130 mg, 162.9 µmol) was dissolved in acetonitrile (3 mL), and morpholine (28.4 mg, 325.8 µmol), anhydrous potassium carbonate (67.6 mg, 488.8 µmol), sodium iodide (24.4 mg, 162.9 µmol) were added. The mixture was heated to 80 °C and stirred for 2 h. The reaction mixture was cooled to room temperature, diluted with water (20 mL), and extracted with ethyl acetate (10 mL × 2). The organic phases were combined and concentrated under reduced pressure to give crude title compound **1s** (128 mg). The product was directly used in the next step without purification.

MS m/z (ESI): 789.2[M+1].

### Step 18

### 5-Ethyl-6-fluoro-4-((5aS,6S,9R)-1-fluoro-12-((1-(morpholinomethyl)cyclopropyl)methoxy)-5a,6,7,8,9,10-hexahydro-5H-4-oxa-3,10a,11,13,14-pentaaza-6,9-methanonaphtho[1,8-ab]heptalen-2-yl)naphthalen-2-ol 1

Crude compound **1s** (128 mg, 162.2 µmol) was dissolved in ethyl acetate (3 mL), and 2 mL of a 4 M solution of hydrochloric acid in dioxane was added under an ice bath. The mixture was left to react at that temperature for 4 h. The reaction mixture was concentrated under reduced pressure, and the residue was purified by high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give title compound **1** (35 mg, yield: 33.4%).

MS m/z (ESI): 645.2[M+1].

¹H NMR (500 MHz, CD₃OD): δ 7.67 (t, 1H), 7.31-7.21 (m, 2H), 7.06 (d, 1H), 5.07 (dd, 2H), 4.62 (dd, 1H), 4.45 (tt, 3H), 4.15 (dd, 1H), 3.77 (s, 1H), 3.66 (d, 4H), 3.25 (s, 1H), 2.57-2.43 (m, 6H), 2.22 (d, 1H), 1.90 (dt, 4H), 0.93 (t, 2H), 0.84 (t, 2H), 0.73 (s, 2H), 0.52 (s, 2H).

### Example 2

### 4-((5aS,6S,9R)-12-((1-(((1S,4S)-2-Oxa-5-azabicyclo[2.2.1]heptan-5-yl)methyl)cyclopropyl)methoxy)-1-fluoro-5a,6,7,8,9,10-hexahydro-5H-4-oxa-3,10a,11,13,14-pentaaza-6,9-methanonaphtho[1,8-ab]heptalen-2-yl)-5-ethyl-6-fluoronaphthalen-2-ol 2

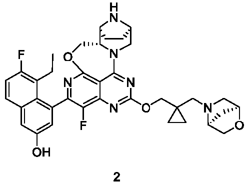

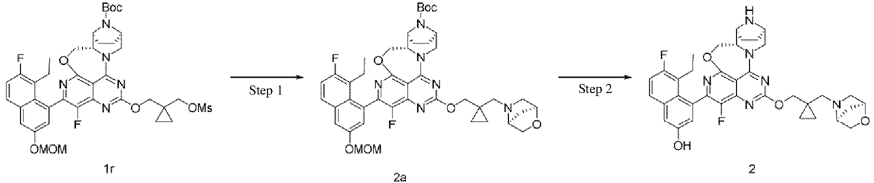

### Step 1

### tert-Butyl (5aS,6S,9R)-12-(1-(((1S,4S)-2-oxa-5-azabicyclo[2.2.1]heptan-5-yl)methyl)cyclopropyl)methoxy)-2-(8-ethyl-7-fluoro-3-(methoxymethoxy)naphthalenyl-1-fluoro-5a,6,7,8,9,10-hexahydro-5H-4-oxa-3,10a,11,13,14-pentaaza-6,9-methanonaphtho[1,8-ab]heptalene-14-carboxylate 2a

Crude compound **1r** (150 mg, 131.6 µmol) was dissolved in acetonitrile (3 mL), and (1*S*,4*S*)-2-oxa-5-azabicyclo[2.2.1]heptane hydrochloride (35.7 mg, 263.2 µmol, Shanghai Leyan), anhydrous potassium carbonate (72.7 mg, 526.4 µmol), sodium iodide (19.7 mg, 263.2 µmol) were added. The mixture was heated to 80 °C and stirred for 2 h. The reaction mixture was cooled to room temperature, diluted with water (20 mL), and extracted with ethyl acetate (10 mL × 2). The organic phases were combined and concentrated under reduced pressure to give crude title compound **2a** (105 mg). The product was directly used in the next step without purification.

MS m/z (ESI): 801.2[M+1].

### Step 2

### 4-((5aS,6S,9R)-12-((1-(((1S,4S)-2-Oxa-5-azabicyclo[2.2.1]heptan-5-yl)methyl)cyclopropyl)methoxy)-1-fluoro-5a,6,7,8,9,10-hexahydro-5H-4-oxa-3,10a,11,13,14-pentaaza-6,9-methanonaphtho[1,8-ab]heptalen-2-yl)-5-ethyl-6-fluoronaphthalen-2-ol 2

Crude compound **2a** (100 mg, 188 µmol) was dissolved in ethyl acetate (3 mL), and 2 mL of a 4 M solution of hydrochloric acid in dioxane was added under an ice bath. The mixture was left to react at that temperature for 4 h. The reaction mixture was concentrated under reduced pressure, and the residue was purified by high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give title compound **2** (35 mg, yield: 33.4%).

MS m/z (ESI): 657.2[M+1].

¹H NMR (500 MHz, CD₃OD): δ 7.67 (s, 1H), 7.27 (d, 2H), 7.06 (d, 1H), 5.36 (s, 1H), 5.06 (dd, 1H), 4.61 (s, 1H), 4.49 (d, 2H), 4.40 (s, 1H), 4.15 (d, 1H), 3.99 (s, 1H), 3.75 (s, 1H), 3.65 (s, 1H), 3.22 (d, 2H), 2.98 (s, 1H), 2.83 (s, 1H), 2.66 (d, 2H), 2.48 (s, 1H), 2.22 (s, 1H), 2.05 (s, 1H), 1.89 (d, 4H), 1.72 (s, 1H), 1.62 (s, 1H), 0.92 (s, 2H), 0.84 (d, 1H), 0.71 (s, 2H), 0.57 (d, 2H).

### Example 3

### 4-((5aS,6S,9R)-12-((1-(((1R,4R)-2-Oxa-5-azabicyclo[2.2.1]heptan-5-yl)methyl)cyclopropyl)methoxy)-1-fluoro-5a,6,7,8,9,10-hexahydro-5H-4-oxa-3,10a,11,13,14-pentaaza-6,9-methanonaphtho[1,8-ab]heptalen-2-yl)-5-ethyl-6-fluoronaphthalen-2-ol 3

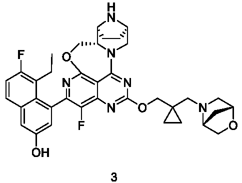

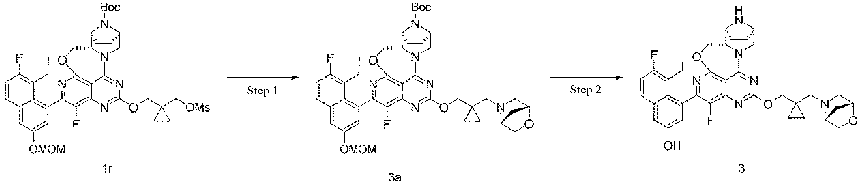

### Step 1

### tert-Butyl (5aS,6S,9R)-12-((1-(((1R,4R)-2-oxa-5-azabicyclo[2.2.1]heptan-5-yl)methyl)cyclopropyl)methoxy)-2-(8-ethyl-7-fluoro-3-(methoxymethoxy)naphthalen-1-yl)-1-fluoro-5a,6,7,8,9,10-hexahydro-5H-4-oxa-3,10a,11,13,14-pentaaza-6,9-methanonaphtho[1,8-ab]heptalene-14-carboxylate 3a

Crude compound **1r** (150 mg, 188 µmol) was dissolved in acetonitrile (3 mL), and (1R,4R)-2-oxa-5-azabicyclo[2.2.1]heptane hydrochloride (51 mg, 376.1 µmol, Shanghai Leyan), anhydrous potassium carbonate (100 mg, 723.5 µmol), sodium iodide (28 mg, 187 µmol) were added. The mixture was heated to 80 °C and stirred for 2 h. The reaction mixture was cooled to room temperature, diluted with water (20 mL), and extracted with ethyl acetate (10 mL × 2). The organic phases were combined and concentrated under reduced pressure to give crude title compound **3a** (150 mg). The product was directly used in the next step without purification.

MS m/z (ESI): 801.2[M+1].

### Step 2

### 4-((5aS,6S,9R)-12-((1-((1R,4R)-2-Oxa-5-azabicyclo[2.2.1]heptan-5-yl)methyl)cyclopropyl)methoxy)-1-fluoro-5a,6,7,8,9,10-hexahydro-5H-4-oxa-3,10a,11,13,14-pentaaza-6,9-methanonaphtho[1,8-ab]heptalen-2-yl)-5-ethyl-6-fluoronaphthalen-2-ol 3

Crude compound **3a** (100 mg, 124.8 µmol) was dissolved in ethyl acetate (3 mL), and 2 mL of a 4 M solution of hydrochloric acid in dioxane was added under an ice bath. The mixture was left to react at that temperature for 4 h. The reaction mixture was concentrated under reduced pressure, and the residue was purified by high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give title compound **3** (10 mg, yield: 12.2%).

MS m/z (ESI): 657.2[M+1].

¹H NMR (500 MHz, CD₃OD): δ 7.67 (d, 1H), 7.29 (d, 1H), 7.28-7.23 (m, 1H), 7.11 (s, 1H), 5.12-5.00 (m, 2H), 4.61 (d, 1H), 4.47 (dd, 3H), 4.15 (d, 1H), 4.04 (s, 1H), 3.73 (d, 2H), 3.25 (s, 1H), 3.02 (d, 1H), 2.80 (s, 3H), 2.58 (d, 1H), 2.47 (s, 1H), 2.25-2.17 (m, 1H), 2.05 (s, 1H), 2.00-1.71 (m, 6H), 0.88 (dq, 3H), 0.79-0.52 (m, 4H).

### Example 4

### 4-((5aS,6S,9R)- 12-((1-((4-Oxa-7-azaspiro[2.5]octan-7-yl)methyl)cyclopropyl)methoxy)-1-fluoro-5a,6,7,8,9,10-hexahydro-5H-4-oxa-3,10a,11,13,14-pentaaza[6,9]methanonaphtho[1,8-ab]heptalen-2-yl)-5-ethyl-6-fluoronaphthalen-2-ol 4

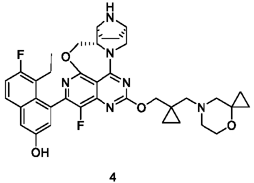

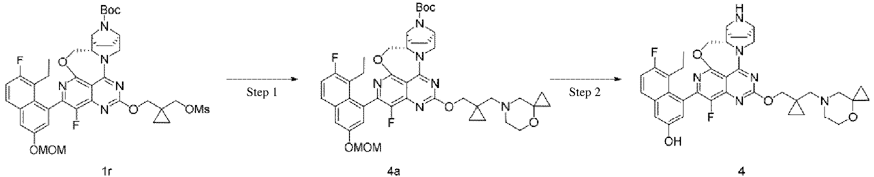

### Step 1

### tert-Butyl (5aS,6S,9R)-12-((1-((4-oxa-7-azaspiro[2.5]octan-7-yl)methyl)cyclopropyl)methoxy)-2-(8-ethyl-7-fluoro-3-(methoxymethoxy)naphthalen-1-yl)-1-fluoro-5a,6,7,8,9,10-hexahydro-5H-4-oxa-3,10a,11,13,14-pentaaza-[6,9]methanonaphtho[1,8-ab]heptalene-14-carboxylate 4a

Crude compound 1r (100 mg, 125 µmol) was dissolved in acetonitrile (3 mL), and 4-oxa-7-azaspiro[2.5]octane hydrochloride (40 mg, 267 µmol, Shanghai Bide), anhydrous potassium carbonate (70 mg, 506 µmol), sodium iodide (20 mg, 133 µmol) were added. The mixture was heated to 80 °C and stirred for 2 h. The reaction mixture was cooled to room temperature, diluted with water (20 mL), and extracted with ethyl acetate (10 mL × 2). The organic phases were combined and concentrated under reduced pressure to give crude title compound **4a** (102 mg). The product was directly used in the next step without purification.

MS m/z (ESI): 815.2[M+1].

### Step 2

### 4-((5aS,6S,9R)-12-((1-((4-Oxa-7-azaspiro[2.5]octan-7-yl)methyl)cyclopropyl)methoxy)-1-fluoro-5a,6,7,8,9,10-hexahydro-5H-4-oxa-3,10a,11,13,14-pentaaza[6,9]methanonaphtho[1,8-ab]heptalen-2-yl)-5-ethyl-6-fluoronaphthalen-2-ol 4

Crude compound **4a** (100 mg, 122 µmol) was dissolved in ethyl acetate (3 mL), and 2 mL of a 4 M solution of hydrochloric acid in dioxane was added under an ice bath. The mixture was left to react at that temperature for 4 h. The reaction mixture was concentrated under reduced pressure, and the residue was purified by high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give title compound **4** (15 mg, yield: 18.2%).

MS m/z (ESI): 671.2[M+1].

¹H NMR (500 MHz, CD₃OD): δ 7.67 (ddd, 1H), 7.31-7.22 (m, 2H), 7.06 (dd, 1H), 5.08 (ddd, 2H), 4.68-4.56 (m, 2H), 4.53-4.40 (m, 3H), 4.16 (dd, 1H), 3.82-3.72 (m, 3H), 3.67 (d, 1H), 3.27 (d, 1H), 2.70-2.43 (m, 6H), 2.29-2.19 (m, 1H), 2.05 (d, 1H), 2.01-1.79 (m, 4H), 0.92 (td, 2H), 0.84 (t, 1H), 0.78-0.67 (m, 3H), 0.56 (d, 3H).

### Example 5

### 5-Ethyl-6-fluoro-4-((5S,5aS,6S,9R)-1-fluoro-5-methyl-12-((1-(morpholinomethyl)cyclopropyl)methoxy)-5a,6,7,8,9,10-hexahydro-5H-4-oxa-3,10a,11,13,14-pentaaza-6,9-methanonaphtho[1,8-ab]heptalen-2-yl)naphthalen-2-ol 5

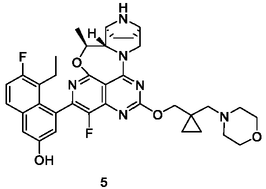

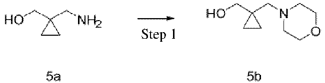

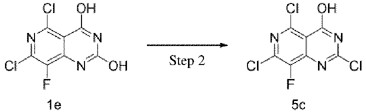

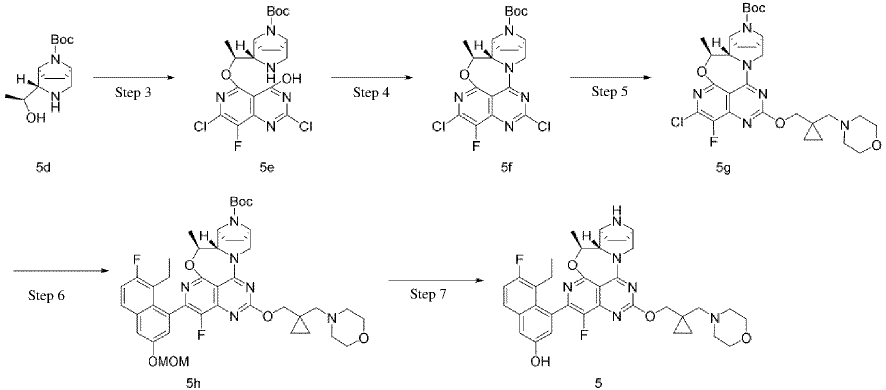

### Step 1

### (1-(Morpholinomethyl)cyclopropyl)methanol 5b

1-Aminomethylcyclopropylmethanol **5a** (1 g, 9.89 mmol, Shanghai Accela) and bis(2-bromoethyl)ether (2.3 g, 9.91 mmol, Shanghai Bide) were dissolved in acetonitrile (60 mL), and anhydrous sodium carbonate (3.1 g, 29.52 mmol) was added. The reaction mixture was refluxed for 14 h and concentrated under reduced pressure, and the residue was purified using eluent system A to give title compound **5b** (1.3 g, yield: 76.7%).

MS m/z (ESI): 172.2[M+1].

### Step 2

### 2,5,7-Trichloro-8-fluoropyrido[4,3-d]pyrimidin-4-ol 5c

Crude compound **1e** (2 g, 8 mmol) was dissolved in phosphorus oxychloride (25 mL), and *N*,*N*-diisopropylethylamine (5.16 g, 40 mmol) was added. After 14 h of stirring at 110 °C, the reaction mixture was cooled to room temperature and concentrated under reduced pressure, and the residue was dissolved in 1,4-dioxane. A 20% potassium carbonate solution was added dropwise to adjust the pH to 2-3. The mixture was stirred for 2 h and then filtered, and the filter cake was washed with water and dried to give crude title compound **5c** (1.5 g). The product was directly used in the next step without purification.

MS m/z (ESI): 267.8[M+1].

### Step 3

### tert-Butyl (1S,2S,5R)-2-((S)-1-((2,7-dichloro-8-fluoro-4-hydroxypyrido[4,3-d]pyrimidin-5-yl)oxy)ethyl)-3,8-diazabicyclo[3.2.1]octane-8-carboxylate 5e

tert-Butyl (1*S*,2*S*,5*R*)-2-((*S*)-1-hydroxyethyl)-3,8-diazabicyclo[3.2.1]octane-8-carboxylate **5d** (370 mg, 1.44 mmol, prepared using the method for Intermediate 29 disclosed on page 164 of the specification in the patent application "WO2022173678A1") was dissolved in tetrahydrofuran (10 mL), and sodium hydride (201 mg, 5.2 mmol, 60% purity) was added under an ice bath. The mixture was left to react for 30 min, and compound **5c** (353 mg, 1.31 mmol) was then added. After 2 h of stirring, the reaction mixture was quenched with water and concentrated under reduced pressure to give crude title compound **5e** (600 mg). The product was directly used in the next step without purification.

MS m/z (ESI): 488.2[M+1].

### Step 4

### tert-butyl (5S,5aS,6S,9R)-2,12-dichloro-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate 5f

Compound **5e** (78 mg, 159.7 µmol) was dissolved in dichloromethane (2 mL), and *N,N-*diisopropylethylamine (61.9 mg, 478.9 µmol) and phosphorus oxychloride (122.4 mg, 798.2 µmol) were added under an ice bath. After 2 h of stirring, the reaction mixture was quenched with a saturated sodium bicarbonate solution and extracted with dichloromethane (10 mL × 2). The organic phases were combined and dried over anhydrous sodium sulfate. After the drying agent was removed by filtration, the filtrate was concentrated under reduced pressure to give crude title compound **5f** (75 mg). The product was directly used in the next step without purification.

MS m/z (ESI): 470.2[M+1].

### Step 5

### tert-Butyl (5S,5aS,6S,9R)-2-chloro-1-fluoro-5-methyl-12-((1-(morpholinomethyl)cyclopropyl)methoxy)-5a,6,7,8,9,10-hexahydro-5H-4-oxa-3,10a,11,13,14-pentaaza-6,9-methanonaphtho[1,8-ab]heptalene-14-carboxylate 5g

Compound **5b** (123.8 mg, 722.9 µmol) and crude compound **5f** (170 mg, 361 µmol) were dissolved in dimethyl sulfoxide (4 mL), and potassium fluoride (105 mg, 1.8 mmol) was added. The mixture was left to react at 100 °C for 14 h. The reaction mixture was cooled to room temperature, and water was then added. Extraction was performed with ethyl acetate (30 mL × 2). The organic phases were combined and dried over anhydrous sodium sulfate. After the drying agent was removed by filtration, the filtrate was concentrated under reduced pressure, and the residue was purified by silica gel column chromatography using eluent system A to give title compound **5g** (200 mg, yield: 91.4%).

MS m/z (ESI): 605.2[M+1].

### Step 6

### tert-Butyl (5S,5aS,6S,9R)-2-(8-ethyl-7-fluoro-3-(methoxymethoxy)naphthalen-1-yl)-1-fluoro-5-methyl-12-((1-(morpholinomethyl)cyclopropyl)methoxy)-5a,6,7,8,9,10-hexahydro-5H-4-oxa-3,10a,11,13,14-pentaaza-6,9-methanonaphtho[1,8-ab]heptalene-14-formate 5h

Compound **5g** (218 mg, 360 µmol) and 2-(8-ethyl-7-fluoro-3-(methoxymethoxy)naphthalen-1-yl)-4,4,5,5-tetramethyl-1,3,2-dioxaborolane (195 mg, 540 µmol, prepared using the method for intermediate 18 disclosed on page 104 of the specification in the patent application "WO2021/041671") were dissolved in 1,4-dioxane (4 mL) and water (0.5 mL), and cesium carbonate (352 mg, 1.08 mmol) and tetrakis(triphenylphosphine)palladium(0) (83.26 mg, 72 µmol) were added. The mixture was left to react at 110 °C for 14 h in a nitrogen atmosphere. The reaction mixture was cooled to room temperature and concentrated under reduced pressure. The residue was dissolved in ethyl acetate, and the mixture was filtered. The filtrate was concentrated under reduced pressure, and the residue was purified by silica gel column chromatography using eluent system A to give title compound **5h** (200 mg, yield: 69%).

MS m/z (ESI): 803.2[M+1].

### Step 7

### 5-Ethyl-6-fluoro-4-((5S,5aS,6S,9R)-1-fluoro-5-methyl-12-((1-(morpholinomethyl)cyclopropyl)methoxy)-5a,6,7,8,9,10-hexahydro-5H-4-oxa-3,10a,11,13,14-pentaaza-6,9-methanonaphtho[1,8-ab]heptalen-2-yl)naphthalen-2-ol 5

Compound **5h** (200 mg, 249 µmol) was dissolved in ethyl acetate (5 mL), and a 4 M solution of hydrogen chloride in 1,4-dioxane (2 mL) was added under an ice bath. The mixture was left to react at that temperature for 0.5 h and then concentrated under reduced pressure, and the residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give title compound **5** (90 mg, yield: 54.8%).

MS m/z (ESI): 659.2[M+1].

¹H NMR (500 MHz, CD₃OD): δ 7.67 (dt, 1H), 7.29 (t, 1H), 7.24 (t, 1H), 7.05 (dd, 1H), 5.40 (ddd, 1H), 4.57 (dt, 1H), 4.51- 4.36 (m, 2H), 4.11 (t, 1H), 3.74 (d, 1H), 3.70-3.60 (m, 5H), 3.21 (t, 1H), 2.60-2.39 (m, 7H), 2.21 (ddt, 1H), 2.10 (dq, 1H), 1.94-1.75 (m, 3H), 1.58 (dd, 3H), 0.99 (d, 1H), 0.81 (t, 2H), 0.74 (d, 2H), 0.59-0.48 (m, 2H).

### Example 6

### 4-((5S,5aS,6S,9R)-12-((1-((4-(Difluoromethylene)piperidin-1-yl)methyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]heptalen-2-yl)-5-ethyl-6-fluoronaphthalen-2-ol 6

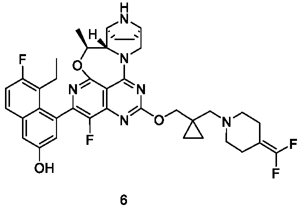

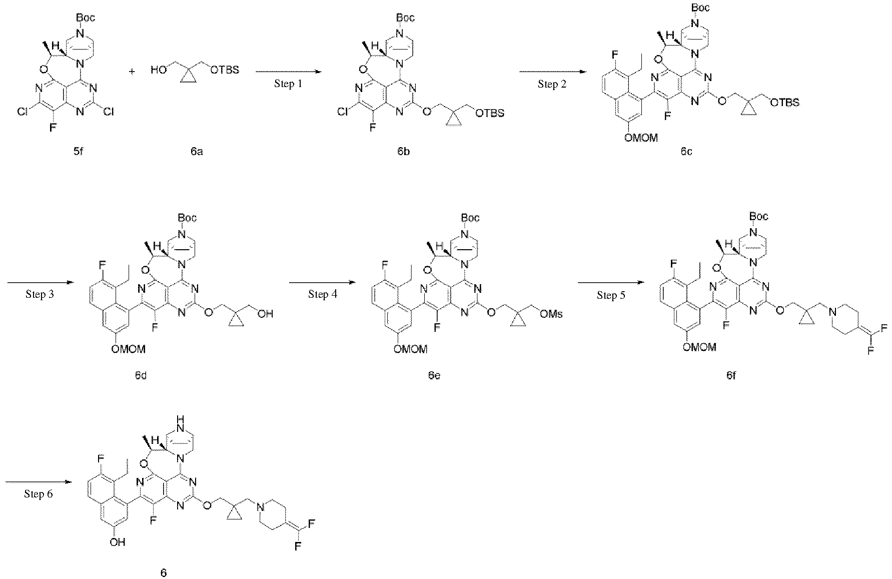

### Step 1

### tert-Butyl (5S,5aS,6S,9R)-12-((1-(((tert-butyldimethylsilyl)oxy)methyl)cyclopropyl)methoxy)-2-chloro-1-fluoro-5-methyl-5a,6,7,8,9,10-hexahydro-5H-4-oxa-3,10a,11,13,14-pentaaza-6,9-methanonaphtho[1,8-ab]heptalene-14-formate 6b

(1-(((tert-Butyldimethylsilyl)oxy)methyl)cyclopropyl)methanol **6a** (1.4 g, 6.4 mmol) was dissolved in tetrahydrofuran (15 mL), and a 2 M solution of sodium bis(trimethylsilyl)amide in tetrahydrofuran was added under an ice bath. After 30 min of stirring at that temperature, a solution of crude compound **5f** (2.3 g, 4.9 mmol) in tetrahydrofuran (20 mL) was added under an ice bath. After 1 h of stirring at that temperature, the reaction mixture was quenched with a saturated ammonium chloride solution and extracted with ethyl acetate (30 mL × 2). The organic phases were combined and dried over anhydrous sodium sulfate. After the drying agent was removed by filtration, the filtrate was concentrated under reduced pressure, and the residue was purified by silica gel column chromatography using eluent system B to give title compound **6b** (2 g, yield: 62.8%).

MS m/z (ESI): 650.2[M+1].

### Step 2

### tert-Butyl (5S,5aS,6S,9R)-12-((1-(((tertbutyldimethylsilyl)oxy)methyl)cyclopropyl)methoxy)-2-(8-ethyl-7-fluoro-3-(methoxymethoxy)naphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptalene-14-formate 6c

Compound **6b** (2 g, 3.07 mmol) and 2-(8-ethyl-7-fluoro-3-(methoxymethoxy)naphthalen-1-yl)-4,4,5,5-tetramethyl-1,3,2-dioxaborolane (1.66 g, 4.6 mmol) were dissolved in 1,4-dioxane (40 mL) and water (8 mL), and cesium carbonate (3 g, 9.2 mmol) and tetrakis(triphenylphosphine)palladium(0) (710 mg, 615 µmol) were added. The mixture was left to react at 105 °C for 14 h in a nitrogen atmosphere. The reaction mixture was cooled to room temperature and concentrated under reduced pressure. The residue was dissolved in ethyl acetate, and the mixture was filtered. The filtrate was concentrated under reduced pressure, and the residue was purified by silica gel column chromatography using eluent system B to give title compound **6c** (2.5 g, yield: 95.8%).

MS m/z (ESI): 848.2[M+1].

### Step 3

### tert-Butyl (5S,5aS,6S,9R)-2-(8-ethyl-7-fluoro-3-(methoxymethoxy)naphthalen-1-yl)-1-fluoro-12-((1-(hydroxymethyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalene-14-formate 6d

Compound **6c** (500 mg, 589.58 µmol) was dissolved in tetrahydrofuran (6 mL), and tetrabutylammonium fluoride (160 mg, 710.5 µmol) was added. After 1 h of stirring, the reaction mixture was diluted with ethyl acetate (20 mL) and washed with water and a saturated sodium chloride solution in sequence. The organic phase was dried with anhydrous sodium sulfate. After the drying agent was removed by filtration, the filtrate was concentrated under reduced pressure to give crude title compound **6d** (435 mg). The product was directly used in the next step without purification.

MS m/z (ESI): 734.2[M+1].

### Step 4

### tert-Butyl (5S,5aS,6S,9R)-2-(8-ethyl-7-fluoro-3-(methoxymethoxy)naphthalen-1-yl)-1-fluoro-5-methyl-12-((1-((methylsulfonyl)oxy)methyl)cyclopropyl)methoxy)-5a,6,7,8,9,10-hexahydro-5H-4-oxa-3,10a,11,13,14-pentaaza-6,9-methanonaphtho[1,8-ab]heptalene-14-formate 6e

Crude compound **6d** (435 mg, 592.8 µmol) was dissolved in dichloromethane (10 mL), and N,N-diisopropylethylamine (229 mg, 1.77 mmol) and methanesulfonyl chloride (95 mg, 829.3 µmol) were added under an ice bath. After 30 min of stirring at that temperature, the reaction mixture was quenched with a saturated ammonium chloride solution and extracted with dichloromethane (10 mL × 2). The organic phases were combined, washed with a saturated sodium chloride solution, and dried over anhydrous sodium sulfate. After the drying agent was removed by filtration, the filtrate was concentrated under reduced pressure to give crude title compound **6e** (480 mg). The product was directly used in the next step without purification.

MS m/z (ESI): 812.2[M+1].

### Step 5

### tert-Butyl (5S,5aS,6S,9R)-12-((1-((4-(difluoromethylene)piperidin-1-yl)methyl)cyclopropyl)methoxy)-2-(8-ethyl-7-fluoro-3-(methoxymethoxy)naphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptalene-14-formate 6f

Crude compound **6e** (235 mg, 289.4 µmol) and 4-(difluoromethylene)piperidine hydrochloride (74 mg, 436.32 µmol, prepared using the method disclosed in "Bioorganic and Medicinal Chemistry, 2004, vol. 12, # 7, p. 1713-1730") were dissolved in acetonitrile (5 mL), and anhydrous potassium carbonate (120 mg, 868.27 µmol) and sodium iodide (44 mg, 293.54 µmol) were added. After 1 h of stirring at 80 °C, the reaction mixture was cooled to room temperature and then filtered. Water was added to the filtrate, and extraction was performed with ethyl acetate (10 mL × 2). The organic phases were combined, washed with a saturated sodium chloride solution, and dried over anhydrous sodium sulfate. After the dying agent was removed by filtration, the filtrate was concentrated under reduced pressure to give crude title compound **6f** (245 mg). The product was directly used in the next step without purification.

MS m/z (ESI): 849.2[M+1].

### Step 6

### 4-((5S,5aS,6S,9R)-12-((1-((4-(Difluoromethylene)piperidin-1-yl)methyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]heptalen-2-yl)-5-ethyl-6-fluoronaphthalen-2-ol 6

Crude compound **6f** (250 mg, 294.5 µmol) was dissolved in ethyl acetate (5 mL), and a 4 M solution of hydrogen chloride in 1,4-dioxane (5 mL) was added under an ice bath. The mixture was left to react at that temperature for 0.5 h and then concentrated under reduced pressure, and the residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give title compound **6** (140 mg, yield: 67.4%).

MS m/z (ESI): 705.3[M+1].

¹H NMR (500 MHz, CD₃OD): δ 7.67 (dt, 1H), 7.32-7.22 (m, 2H), 7.12-6.98 (m, 1H), 5.40 (ddt, 1H), 4.57 (td, 1H), 4.53-4.36 (m, 2H), 4.10 (t, 1H), 3.72 (d, 1H), 3.62 (s, 1H), 3.20 (t, 1H), 2.63-2.39 (m, 7H), 2.29-2.01 (m, 6H), 1.94-1.86 (m, 1H), 1.81 (dt, 2H), 1.58 (dd, 3H), 0.89 (dt, 3H), 0.75 (d, 2H), 0.52 (s, 2H).

### Example 7

### 4-((5S,5aS,6S,9R)-12-((1-((3-(Difluoromethylene)pyrrolidin-1-yl)methyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]heptalen-2-yl)-5-ethyl-6-fluoronaphthalen-2-ol 7

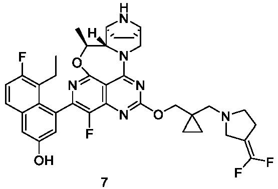

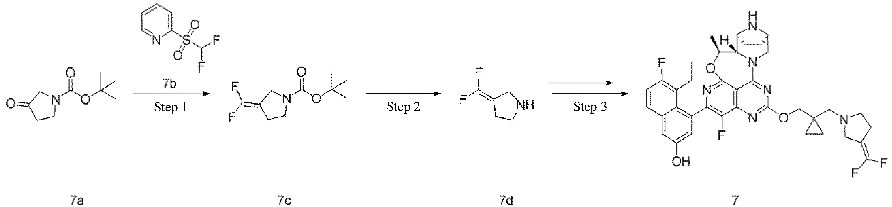

### Step 1

### tert-Butyl 3-(difluoromethylene)pyrrolidine-1-formate 7c

Potassium tert-butoxide (912 mg, 8.1 mmol) was dissolved in *N*,*N*-dimethylacetamide (3 mL), and 1-tert-butoxycarbonyl-3-pyrrolidone **7a** (1 g, 5.4 mmol) and 2-((difluoromethyl)sulfonyl)pyridine **7b** (872 mg, 4.5 mmol, Shanghai Bide) were added at -40 °C. The mixture was left to react at that temperature for 15 min. The reaction mixture was quenched with a saturated ammonium chloride solution. 3 N hydrochloric acid (10 mL) was added, and extraction was performed with ethyl acetate (30 mL × 3). The organic phases were combined, washed with a saturated sodium chloride solution, and dried over anhydrous sodium sulfate. After the drying agent was removed by filtration, the filtrate was concentrated under reduced pressure, and the residue was purified by silica gel column chromatography using eluent system B to give title compound **7c** (167 mg, yield: 16.8%).

MS m/z (ESI): 164[M-55].

### Step 2

### 3-(Difluoromethylene)pyrrolidine hydrochloride 7d

Compound 7c (167 mg, 761.7 µmol) was dissolved in a 4 M solution of hydrogen chloride in 1,4-dioxane (2 mL). After 1 h of stirring, the reaction mixture was concentrated under reduced pressure to give crude title compound **7d** (118 mg). The product was directly used in the next step without purification.

MS m/z (ESI): 120.2[M+1].

### Step 3

### 4-((5S,5aS,6S,9R)-12-((1-((3-(Difluoromethylene)pyrrolidin-1-yl)methyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]heptalen-2-yl)-5-ethyl-6-fluoronaphthalen-2-ol 7

Title compound 7 (3 mg, yield: 12%) was obtained by using the synthesis scheme of Example 6 and replacing the starting material of step 5, 4-(difluoromethylene)piperidine hydrochloride, with compound **7d.**

MS m/z (ESI): 691.3[M+1].

¹H NMR (500 MHz, CD₃OD): δ 7.68-7.63 (m, 1H), 7.29-7.23 (m, 2H), 7.12-7.01 (m, 1H), 5.41-5.39 (m, 1H), 4.59-4.42 (m, 3H), 4.11-4.08 (m, 1H), 3.70-3.61 (m,3H), 3.17-2.70 (m,3H), 2.70-2.44 (m, 7H), 2.21-1.79 (m,7H), 1.59-1.57 (m, 3H), 0.99-0.92 (m, 2H), 0.75-0.73 (m, 2H).

### Example 8

### 1-((1-((((5S,5aS,6S,9R)-2-(8-Ethyl-7-fluoro-3-hydroxynaphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptalen-12-yl)oxy)methyl)cyclopropyl)methyl)piperidin-4-one O-methyl oxime 8

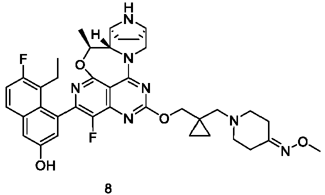

Title compound **8** (5 mg, yield: 20%) was obtained by using the synthesis scheme of Example 6 and replacing the starting material of step 5, 4-(difluoromethylene)piperidine hydrochloride, with piperidin-4-one O-methyl oxime (prepared using the method disclosed in "Molecules, 2016, vol. 21, # 12, art. no. 1674").

MS m/z (ESI): 700.3[M+1].

¹H NMR (500 MHz, CD₃OD): δ 7.67 (dt, 1H), 7.32-7.21 (m, 2H), 7.12-6.98 (m, 1H), 5.42 (ddd, 1H), 4.65-4.41 (m, 4H), 4.12 (t, 1H), 3.78 (d, 4H), 3.66 (s, 1H), 3.27-3.18 (m, 1H), 2.68-2.67 (m, 2H), 2.60-2.54 (m, 4H), 2.52-2.44 (m, 2H), 2.30 (q, 2H), 2.24-2.17 (m, 1H), 2.15-2.07 (m, 1H), 1.91 (d, 1H), 1.88-1.78 (m, 2H), 1.59 (dd, 3H), 0.99-0.95 (m, 3H), 0.76 (t, 2H), 0.54 (s, 2H).

### Example 9

### 4-((1-((((5S,5aS,6S,9R)-2-(8-Ethyl-7-fluoro-3-hydroxynaphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptalen-12-yl)oxy)methyl)cyclopropyl)methyl)-1-(methylimino)-1λ⁶-thiomorpholine 1-oxide 9

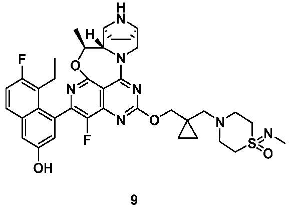

Title compound **9** (5 mg, yield: 6%) was obtained by using the synthesis scheme of Example 6 and replacing the starting material of step 5, 4-(difluoromethylene)piperidine hydrochloride, with 1-(methylimino)-1λ⁶-thiomorpholine-1-oxide (prepared using the method for Intermediate S disclosed on page 239 of the specification in the patent application "WO2022/23772, 2022, A1").

MS m/z (ESI): 720.3[M+1].

¹H NMR (500 MHz, CD₃OD): δ 7.68-7.64 (m, 1H), 7.29-7.22 (m, 2H), 7.13-7.01 (m, 1H), 5.40-5.37 (m, 1H), 4.61-4.39 (m, 3H), 4.13-4.10 (m, 1H), 3.77-3.66 (m, 2H), 3.25-2.85 (m, 9H), 2.82-2.46 (m, 6H), 2.23-1.80 (m, 6H), 1.58-1.57 (m, 3H), 1.00-0.97 (m, 1H), 0.83-0.77 (m, 3H), 0.56-0.54 (s, 2H).

### Example 10

### 4-((1-((((5S,5aS,6S,9R)-2-(8-Ethyl-7-fluoro-3-hydroxynaphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptalen-12-yl)oxy)methyl)cyclopropyl)methyl)-1-imino-1λ⁶-thiomorpholine 1-oxide 10

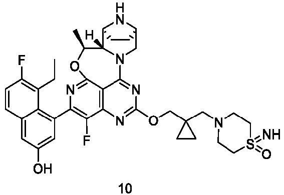

Title compound **10** (5 mg, yield: 6%) was obtained by using the synthesis scheme of Example 6 and replacing the starting material of step 5, 4-(difluoromethylene)piperidine hydrochloride, with the compound 1-imino-1λ⁶-thiomorpholine-1-oxide (prepared using the method disclosed in "Tetrahedron, 2014, vol. 70, # 37, p. 6613-6622").

MS m/z (ESI): 706.3[M+1].

¹H NMR (500 MHz, CD₃OD): δ 7.69-7.65 (m, 1H), 7.30-7.22 (m, 2H), 7.12-7.01 (m, 1H), 5.44-5.42 (m, 1H), 4.61-4.39 (m, 3H), 4.14-4.12 (m, 1H), 3.85-3.72 (m, 2H),3.27-2.90 (m, 9H), 2.70-2.64 (m, 4H), 2.25-1.74 (m, 7H), 1.59-1.57 (m, 3H), 1.00-0.97 (m, 1H), 0.84-0.71 (m, 3H).

### Example 11

### 5-Ethyl-6-fluoro-4-((5S,5aS,6S,9R)-1-fluoro-12-((1-(((E)-3-(fluoromethylene)pyrrolidin-1-yl)methyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalen-2-yl)naphthalen-2-ol 11

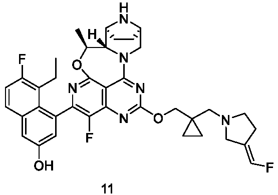

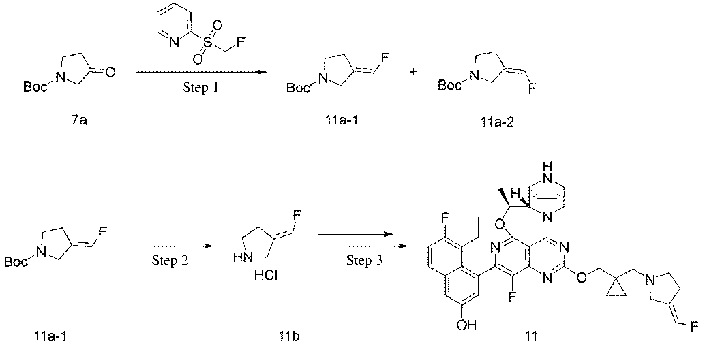

### Step 1

### tert-Butyl (E)-3-(fluoromethylene)pyrrolidine-1-formate 11a-1

### tert-Butyl (Z)-3-(fluoromethylene)pyrrolidine-1-formate 11a-2

2-((Fluoromethyl)sulfonyl)pyridine (4.7 g, 26.8 mmol, Shanghai Accela) was dissolved in tetrahydrofuran (100 mL), and a 1 M solution of potassium bis(trimethylsilyl)amide in tetrahydrofuran (33 mL) was added at -78 °C. The mixture was left to react at that temperature for 30 min, and 1-tert-butoxycarbonyl-3-pyrrolidone **7a** (5 g, 27 mmol) was then added. The mixture was left to react at that temperature for 3 h, then warmed to room temperature, and left to react for 1 h. The reaction mixture was quenched with a saturated ammonium chloride solution. 3 N hydrochloric acid (50 mL) was added, and the mixture was stirred for 1 h and extracted with ethyl acetate (50 mL × 3). The organic phases were combined, washed with a saturated sodium chloride solution, and dried over anhydrous sodium sulfate. After the drying agent was removed by filtration, the filtrate was concentrated under reduced pressure, and the residue was purified by silica gel column chromatography using eluent system B to give title compounds **11a-1** (800 mg, yield: 14.7%) and **11a-2** (800 mg, yield: 14.7%).

MS m/z (ESI): 146.2[M-55].

**11a-1:** ¹H NMR (500 MHz, CDCl₃): δ 6.70 (s, 0.5H), 6.54 (s, 0.5H), 3.93 (s, 2H), 3.48 (t, 2H), 2.66 (t, 2H), 1.48 (s, 9H).

**11a-2:** ¹H NMR (500 MHz, CDCl₃): δ 6.61 (s, 0.5H), 6.45 (s, 0.5H), 4.07 (s, 2H), 3.49 (t, 2H), 2.49 (t, 2H), 1.48 (s, 9H).

### Step 2

### (E)-3-(Fluoromethylene)pyrrolidine hydrochloride 11b

Compound **11a-1** (700 mg, 3.5 mmol) was dissolved in a 4 M solution of hydrogen chloride in 1,4-dioxane (10 mL). After 1 h of stirring, the reaction mixture was concentrated under reduced pressure to give crude title compound **11b** (480 mg). The product was directly used in the next step without purification.

MS m/z (ESI): 101.2[M+1].

### Step 3

### 5-Ethyl-6-fluoro-4-((5S,5aS,6S,9R)-1-fluoro-12-((1-(((E)-3-(fluoromethylene)pyrrolidin-1-yl)methyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalen-2-yl)naphthalen-2-ol 11

Title compound **11** (5 mg, yield: 12.1%) was obtained by using the synthesis scheme of Example 6 and replacing the starting material of step 5, 4-(difluoromethylene)piperidine hydrochloride, with compound **11b.**

MS m/z (ESI): 673.3[M+1].

¹H NMR (500 MHz, CD₃OD): δ 7.67 (dt, 1H), 7.33-7.20 (m, 2H), 7.11 (d, 1H), 6.70 (d, 1H), 5.40 (ddd, 1H), 4.58 (dd, 1H), 4.50-4.36 (m, 2H), 4.10 (t, 1H), 3.71 (d, 1H), 3.62 (s, 1H), 3.22-3.17 (m, 4H), 2.75 (s, 2H), 2.62-2.51 (m, 5H), 2.25-2.06 (m, 2H), 1.85-1.76 (m, 3H), 1.58 (dd, 3H), 1.31 (s, 2H), 0.98 (t, 1H), 0.81 (t, 2H), 0.74-0.58 (m, 3H).

### Example 12

### 2-((1-((((5S,5aS,6S,9R)-2-(8-Ethyl-7-fluoro-3-hydroxynaphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptalen-12-yl)oxy)methyl)cyclopropyl)methyl)hexahydrocyclopenta[c]pyrrol-5(1H)-one O-methyl oxime 12

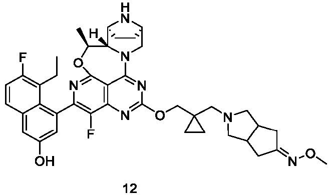

MS m/z (ESI): 726.3[M+1].

### Example 13

### 1-((1-((((5S,5aS,6S,9R)-2-(8-Ethyl-7-fluoro-3-hydroxynaphthalen-1-yl)-1-fluoro-5-methyl-5a,6,7,8,9,10-hexahydro-5H-4-oxa-3,10a,11,13,14-pentaaza-6,9-methanonaphtho[1,8-ab]heptalen-12-yl)oxy)methyl)cyclopropyl)methyl)azepan-4-one O-methyl oxime 13 (a mixture of cis-trans isomers)

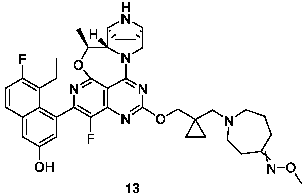

Title compound **13** was obtained by using the synthesis scheme of Example 6 and replacing the starting material of step 5, 4-(difluoromethylene)piperidine hydrochloride, with azepan-4-one O-methyl oxime trifluoroacetate (prepared using the method disclosed in "RSC Advances, 2017, vol. 7, # 3, p. 1480-1483").

MS m/z (ESI): 714.3[M+1].

### Example 14

### 4-((5S,5aS,6S,9R)-12-(1-((5-(Difluoromethylene)hexahydrocyclopenta[c]pyrrol-2(1H)-yl)methyl)cyclopropyl)methoxy)-1-fluoro-5-methyl-5a,6,7,8,9,10-hexahydro-5H-4-oxa-3,10a,11,13,14-pentaaza-6,9-methanonaphthol[1,8-ab]heptalen-2-yl)-5-ethyl-6-fluoronaphthalen-2-ol 14

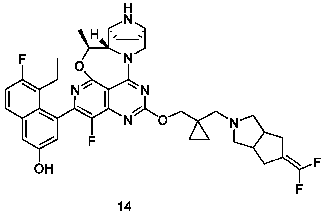

Title compound **14** (10 mg, yield: 39.9%) was obtained by using the synthesis scheme of Example 7 and replacing the starting material of step 1, compound **7a,** with *N*-tert-butoxycarbonyl-hexahydro-5-oxocyclopenta[*c*]pyrrole (Shanghai Bide).

MS m/z (ESI): 731.3[M+1].

¹H NMR (500 MHz, CD₃OD): δ 7.68-7.66 (m, 1H), 7.29-7.26 (m, 2H), 7.12-7.01 (m, 1H), 5.41-5.39 (m, 1H), 4.58-4.39 (m, 3H), 4.11-4.10 (m, 1H), 3.71-3.47 (m,3H), 3.19-3.18 (m, 3H), 2.73-2.08 (m, 12H), 1.82-1.79 (m,3H), 1.59-1.57 (m, 3H), 0.99-0.92 (m, 2H), 0.72-0.54 (m, 5H).

### Example 15

### 4-((5S,5aS,6S,9R)-12-((1-((4-(Difluoromethylene)azepan-1-yl)methyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]heptalen-2-yl)-5-ethyl-6-fluoronaphthalen-2-ol 15

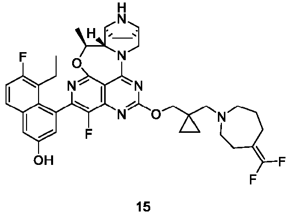

Title compound **15** (5 mg, yield: 19.3%) was obtained by using the synthesis scheme of Example 7 and replacing the starting material of step 1, compound **7a,** with the compound tert-butyl 4-oxoazepane-1-carboxylate (Shanghai Bide).

MS m/z (ESI): 719.3[M+1].

¹H NMR (500 MHz, CD₃OD): δ 7.68-7.65 (m, 1H), 7.29-7.24 (m, 1H), 7.12-7.11 (m, 1H), 6.98-6.95 (m, 1H), 5.39-5.36 (m, 1H), 4.59-4.57 (m, 1H), 4.46-4.42 (m, 2H), 4.14-4.10 (m, 1H), 3.76-3.74 (m, 1H), 3.68-3.63 (m, 1H), 3.25-3.20 (m, 1H), 2.98-2.81 (m, 6H), 2.56-2.42 (m, 4H), 2.33-2.30 (m, 2H), 2.23-2.20 (m, 1H), 2.18-2.06 (m, 2H), 1.92-1.81 (m, 4H), 1.62-1.59 (m, 3H), 0.99-0.95 (m, 1H), 0.93-0.91 (m, 1H), 0.80-0.79 (m, 2H), 0.62-0.60 (m, 2H).

### Example 16

### 5-Ethyl-6-fluoro-4-((5S,5aS,6S,9R)-1-fluoro-12-(((Z)-2-(fluoromethylene)tetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalen-2-yl)naphthalen-2-ol 16 (a mixture of diastereomers)

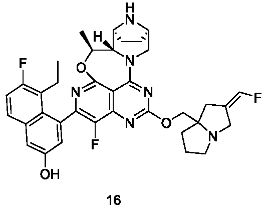

### Example 16-p1 and 16-p2

### 5-Ethyl-6-fluoro-4-((5S,5aS,6S,9R)-1-fluoro-12-(((S,Z)-2-(fluoromethylene)tetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalen-2-yl)naphthalen-2-ol 16-p1

### 5-Ethyl-6-fluoro-4-((5S,5aS,6S,9R)-1-fluoro-12-(((R,Z)-2-(fluoromethylene)tetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalen-2-yl)naphthalen-2-ol 16-p2

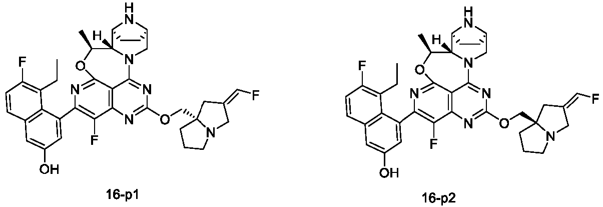

### Example 17

### 4-((5S,5aS,6S,9R)-12-((2,6-Dimethylenetetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalen-2-yl)-5-ethyl-6-fluoronaphthalen-2-ol 17

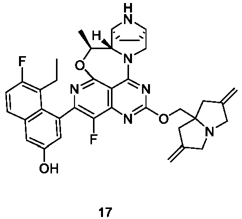

Title compound **17** (20 mg, yield: 24.4%) was obtained by using the synthesis scheme of Example 5 and replacing the starting material of step 5, compound **5b,** with (2,6-dimethylenetetrahydro-1*H*-pyrrolizin-7*a*(5*H*)-yl)methanol (prepared using the method disclosed in Example 4 on page 30 of the specification in the patent application "WO2022247757").

MS m/z (ESI): 653.3[M+1].

¹H NMR (500 MHz, CD₃OD): δ 7.72-7.56 (m, 1H), 7.37-7.18 (m, 2H), 7.12-6.91 (m, 1H), 5.43-5.27 (m, 1H), 5.11-4.95 (m, 2H), 4.62-4.49 (m, 1H), 4.44-4.21 (m, 2H), 4.13-4.01 (m, 1H), 3.85-3.54 (m, 4H), 3.50-3.37 (m, 4H), 3.23-3.08 (m, 2H), 2.87-2.72 (m, 2H), 2.70-2.39 (m, 3H), 2.27-1.97(m, 2H), 1.97-1.66 (m, 3H), 1.60-1.46(m, 2H), 1.02-0.90(m, 1H), 0.84-0.64 (m, 2H).

### Example 18

### 4-((5S,5aS,6S,9R)-12-((2-(Difluoromethylene)tetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalen-2-yl)-5-ethyl-6-fluoronaphthalen-2-ol 18 (a mixture of diastereomers)

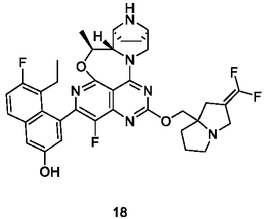

Title compound **18** (50 mg, yield: 24.2%) (a mixture of diastereomers) was obtained by using the synthesis scheme of Example 5 and replacing the starting material of step 5, compound **5b,** with (2-(difluoromethylene)tetrahydro-1*H*-pyrrolizin-7*a*(5H)-yl)methanol (prepared using the method disclosed in Example 11 on page 54 of the specification in the patent application "WO2022247757").

MS m/z (ESI): 677.3[M+1].

¹H NMR (500 MHz, CD₃OD): δ 7.67 (dt, 1H), 7.32-7.21 (m, 2H), 7.12-6.99 (m, 1H), 5.39 (ddd, 1H), 4.59 (p, 1H), 4.37 (dd, 1H), 4.29 (d, 1H), 4.15-4.08 (m, 1H), 3.81 (dt, 1H), 3.71 (d, 1H), 3.62 (d, 1H), 3.48-3.40 (m, 1H), 3.26-3.12 (m, 2H), 2.88-2.77 (m, 1H), 2.73 (q, 1H), 2.64-2.45 (m, 2H), 2.24-2.11 (m, 2H), 2.04 (dddd, 2H), 1.97-1.76 (m, 5H), 1.58 (dd, 3H), 0.99-0.79 (m, 3H).

### Example 18-p1 and 18-p2

### 4-((5S,5aS,6S,9R)-12-(((S)-2-(Difluoromethylene)tetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalen-2-yl)-5-ethyl-6-fluoronaphthalen-2-ol 18-p1

### 4-((5S,5aS,6S,9R)-12-(((R)-2-(Difluoromethylene)tetrahydro-1H-pyrrolizin-7a(5H)-yl)methoxy)-1-fluoro-5-methyl-5a,6,7,8,9,10-hexahydro-5H-4-oxa-3,10a,11,13,14-pentaaza-6,9-methanonaphtho[1,8-ab]heptalen-2-yl)-5-ethyl-6-fluoronaphthalen-2-ol 18-p1

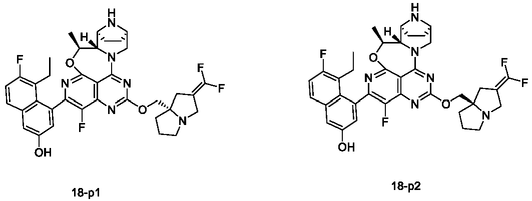

Isomer mixture **18** (30 mg, 44.3 µmol) was resolved on a chiral column (Gilson 281, chromatography column: CHIRALPAK IG, 20 × 250 mm, 5 µm; mobile phase A: n-hexane, mobile phase B: ethanol (0.5% NH3 in MeOH), gradient ratio: A:B: 75:25, flow rate: 20 mL/min) to give the title compounds (10 mg, 10 mg, yields: 33% and 33%).

The chiral HPLC analysis method for the isomer mixture: the retention times were 10.274 min and 14.616 min (chromatography column: CHIRALPAK IG, 150 × 4.6 mm, 5 µm; mobile phase A: 90% ethanol (containing 0.1% diethylamine) + 10% dichloromethane; mobile phase B: n-hexane, gradient ratio: A:B: 30:70, flow rate: 1.0 mL/min).

A single-configuration compound (shorter retention time): 10.274 min (10 mg, yield: 33%).

MS m/z (ESI): 677.3[M+1].

HPLC analysis: retention time 1.45 min, purity: 90% (chromatography column: ACQUITY UPLC^{®}BEH, C18, 1.7 µm, 2.1 × 50 mm; mobile phase: water (10 mM ammonium bicarbonate), acetonitrile, gradient ratio: acetonitrile 10%-95%).

¹H NMR (500 MHz, CD₃OD): δ 7.71-7.64 (m, 1H), 7.33-7.22 (m, 2H), 7.12-6.98 (m, 1H), 5.54-5.47 (m, 1H), 4.68 (ddd, 1H), 4.56-4.49 (m, 1H), 4.44 (d, 1H), 4.24 (t, 1H), 4.03 (s, 1H), 3.93 (s, 1H), 3.69 (d, 1H), 3.36 (s, 2H), 2.92 (d, 2H), 2.67 (d, 1H), 2.59-2.43 (m, 2H), 2.32-1.87 (m, 9H), 1.60 (d, 3H), 0.89 (dt, 3H).

A single-configuration compound (longer retention time): 14.616 min (10 mg, yield: 33%).

MS m/z (ESI): 677.3[M+1].

HPLC analysis: retention time 1.45 min, purity: 90% (chromatography column: ACQUITY UPLC^{®}BEH, C18, 1.7 µm, 2.1 × 50 mm; mobile phase: water (10 mM ammonium bicarbonate), acetonitrile, gradient ratio: acetonitrile 10%-95%).

¹H NMR (500 MHz, CD₃OD): δ 7.68 (dt, 1H), 7.33-7.22 (m, 2H), 7.12-6.89 (m, 1H), 5.53-5.46 (m, 1H), 4.68 (td, 1H), 4.54 (d, 1H), 4.45 (d, 1H), 4.24 (t, 1H), 3.99 (d, 3H), 3.70 (d, 1H), 3.37 (s, 1H), 3.01-2.88 (m, 2H), 2.67 (d, 1H), 2.52 (ddt, 2H), 2.32-1.87 (m, 9H), 1.61 (dd, 3H), 0.89 (dt, 3H).

### Example 19

### 5-Ethyl-6-fluoro-4-((5S,5aS,6S,9R)-1-fluoro-12-(((E)-2-(fluoromethylene)tetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalen-2-yl)naphthalen-2-ol 19 (a mixture of diastereomers)

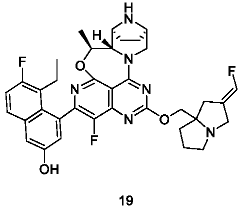

### Example 19-p1 and 19-p2

### 5-Ethyl-6-fluoro-4-((5S,5aS,6S,9R)-1-fluoro-12-(((S,E)-2-(fluoromethylene)tetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalen-2-yl)naphthalen-2-ol 19-p1

### 5-Ethyl-6-fluoro-4-((5S,5aS,6S,9R)-1-fluoro-12-(((R,E)-2-(fluoromethylene)tetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalen-2-yl)naphthalen-2-ol 19-p2

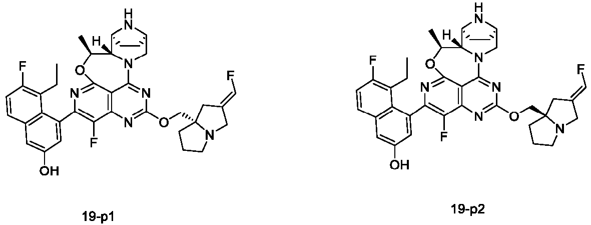

### Example 20

### 4-((5aS,6S,9R)-12-((2,6-Dimethylenetetrahydro-1H-pyrrolizin-7a(5H)-yl)methoxy)-1-fluoro-5a,6,7,8,9,10-hexahydro-5H-4-oxa-3,10a,11,13,14-pentaaza-6,9-methanonaphtho[1,8-ab]heptalen-2-yl)-5-ethyl-6-fluoronaphthalen-2-ol 20

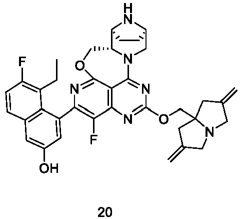

Title compound **20** (7 mg, yield: 8.5%) was obtained by using steps 1 to 15 and step 18 of the synthesis scheme of Example 1 and replacing the starting material of step 13, the compound 1,1-bis(hydroxymethyl)cyclopropane, with 2,6-dimethylenetetrahydro-1*H-*pyrrolizin-7*a*(5*H*)-yl)methanol.

MS m/z (ESI): 639.2[M+1].

¹H NMR (500 MHz, CD₃OD): δ 7.69 (ddd, 1H), 7.34-7.24 (m, 2H), 7.08 (dd, 1H), 5.11-5.02 (m, 4H), 4.67-4.61 (m, 1H), 4.51 (ddd, 1H), 4.40-4.34 (m, 2H), 4.17 (dd, 1H), 3.82 (d, 2H), 3.78-3.74 (m, 1H), 3.70-3.66 (m, 1H), 3.50-3.39 (m, 1H), 3.32-3.19 (m, 3H), 2.84 (d, 2H), 2.63 (d, 2H), 2.54-2.44 (m, 1H), 2.29-2.20 (m, 1H), 2.01-1.79 (m, 4H), 0.90 (dt, 3H).

### Example 21

### 4-((5aS,6S,9R)-12-((2-(Difluoromethylene)tetrahydro-1H-pyrrolizin-7a(5H)-yl)methoxy)-1-fluoro-5a,6,7,8,9,10-hexahydro-5H-4-oxa-3,10a,11,13,14-pentaaza-6,9-methanonaphtho[1,8-ab]heptalen-2-yl)-5-ethyl-6-fluoronaphthalen-2-ol 21 (a mixture of diastereomers)

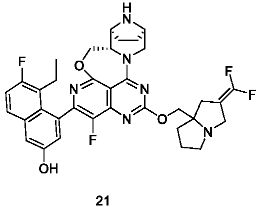

Title compound **21** (a mixture of diastereomers) was obtained by using steps 1 to 15 and step 18 of the synthesis scheme of Example 1 and replacing the starting material of step 13, the compound 1,1-bis(hydroxymethyl)cyclopropane, with (2-(difluoromethylene)tetrahydro-1*H*-pyrrolizin-7*a*(5*H*)-yl)methanol (prepared using the method disclosed in Example 11 on page 54 of the specification in the patent application "WO2022247757").

MS m/z (ESI): 663.2[M+1].

### Example 21-p1 and 21-p2

### 4-((5aS,6S,9R)-12-(((S)-2-(Difluoromethylene)tetrahydro-1H-pyrrolizin-7a(5H)-yl)methoxy)-1-fluoro-5a,6,7,8,9,10-hexahydro-5H-4-oxa-3,10a,11,13,14-pentaaza-6,9-methanonaphtho[1,8-ab]heptalen-2-yl)-5-ethyl-6-fluoronaphthalen-2-ol 21-p1

### 4-((5aS,6S,9R)-12-(((R)-2-(Difluoromethylene)tetrahydro-1H-pyrrolizin-7a(5H)-yl)methoxy)-1-fluoro-5a,6,7,8,9,10-hexahydro-5H-4-oxa-3,10a,11,13,14-pentaaza-6,9-methanonaphtho[1,8-ab]heptalen-2-yl)-5-ethyl-6-fluoronaphthalen-2-ol 21-p2

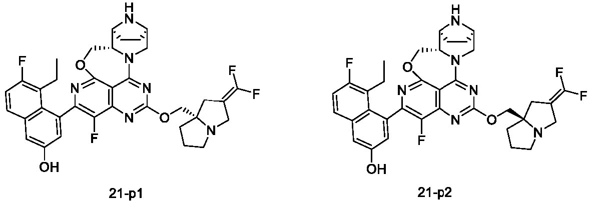

Compound **21** was resolved on a chiral column to give the title compounds.

MS m/z (ESI): 663.2[M+1].

### Example 22

### 4-((5S,5aS,6S,9R)-12-((1-((3-(Difluoromethylene)azetidin-1-yl)methyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]heptalen-2-yl)-5-ethyl-6-fluoronaphthalen-2-ol 22

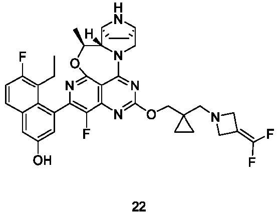

Title compound **22** (5 mg, yield: 19.3%) was obtained by using the synthesis scheme of Example 7 and replacing the starting material of step 1, compound **7a,** with the compound tert-butyl 3-oxoazetidine-1-carboxylate (Shanghai Bide).

MS m/z (ESI): 677.2[M+1].

¹H NMR (500 MHz, CD₃OD): δ 7.67 (dt, 1H), 7.33-7.21 (m, 2H), 7.11 (d, 1H), 5.40 (ddd, 1H), 4.59 (dd, 1H), 4.41-4.29 (m, 2H), 4.11 (t, 1H), 3.99-3.95 (m, 3H), 3.73 (d, 1H), 3.63 (s, 1H), 3.31 (t, 1H), 2.79-2.67 (m, 2H), 2.52 (dt, 2H), 2.15 (d, 2H), 1.97-1.78 (m, 3H), 1.58 (dd, 3H), 1.31 (s, 3H), 0.98 (t, 1H), 0.81 (t, 2H), 0.72-0.59 (m, 3H).

### Example 23

### 5-Ethyl-6-fluoro-4-((5S,5aS,6S,9R)-1-fluoro-12-((1-((4-(fluoromethylene)piperidin-1-yl)methyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalen-2-yl)naphthalen-2-ol 23

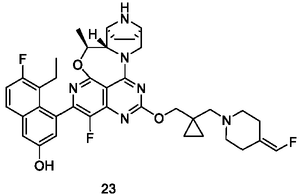

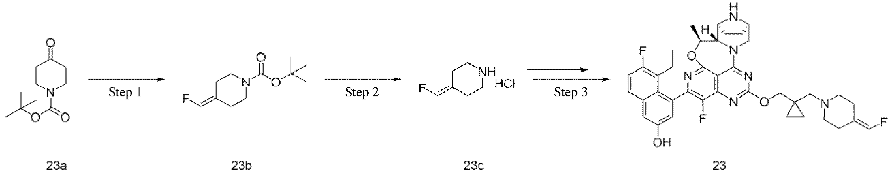

### Step 1

### tert-Butyl 4-(fluoromethylene)piperidine-1-formate 23b

2-((Fluoromethyl)sulfonyl)pyridine (4.2 g, 23.97 mmol) was dissolved in tetrahydrofuran (50 mL), and a 1 M solution of potassium bis(trimethylsilyl)amide in tetrahydrofuran (30 mL) was added at -78 °C. The mixture was left to react at that temperature for 30 min, and *N*-tert-butoxycarbonyl-4-piperidone **23a** (5 g, 25.09 mmol, Shanghai Accela) was then added. The mixture was left to react at that temperature for 3 h, then warmed to room temperature, and left to react for 1 h. The reaction mixture was quenched with a saturated ammonium chloride solution. 3 N hydrochloric acid (100 mL) was added, and the mixture was stirred for 1 h and then extracted with ethyl acetate (50 mL × 3). The organic phases were combined, washed with a saturated sodium chloride solution, and dried over anhydrous sodium sulfate. After the drying agent was removed by filtration, the filtrate was concentrated under reduced pressure, and the residue was purified by silica gel column chromatography using eluent system B to give title compound **23b** (2 g, yield: 37%).

### Step 2

### 4-(Fluoromethylene)piperidine hydrochloride 23c

Compound **23b** (1 g, 4.64 mmol) was dissolved in a 4 M solution of hydrogen chloride in 1,4-dioxane (20 mL). After 1 h of stirring, the reaction mixture was concentrated under reduced pressure to give crude title compound **23c** (700 mg). The product was directly used in the next step without purification.

MS m/z (ESI): 116.1[M+1].

### Step 3

### 5-Ethyl-6-fluoro-4-((5S,5aS,6S,9R)-1-fluoro-12-((1-((4-(fluoromethylene)piperidin-1-yl)methyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalen-2-yl)naphthalen-2-ol 23

Title compound **23** (15 mg, yield: 18.1%) was obtained by using the synthesis scheme of Example 6 and replacing the starting material of step 5, 4-(difluoromethylene)piperidine hydrochloride, with crude compound **23c.**

MS m/z (ESI): 687.2[M+1].

¹H NMR (500 MHz, CD₃OD): δ 7.67 (dt, 1H), 7.32-7.21 (m, 2H), 7.05 (dd, 1H), 6.52 (d, 1H), 5.45-5.38 (m, 1H), 4.62-4.39 (m, 5H), 4.11 (t, 2H), 3.73 (d, 1H), 3.63 (s, 1H), 3.50-3.44 (m, 1H), 3.28-3.17 (m, 3H), 2.66-2.43 (m, 5H), 2.35 (s, 2H), 2.25-2.15 (m, 1H), 2.10 (s, 2H), 1.94-1.72 (m, 3H), 1.58 (dd, 3H), 0.98 (t, 1H), 0.84-0.71 (m, 2H), 0.54 (s, 1H).

### Example 24

### 5-Ethyl-6-fluoro-4-((5S,5aS,6S,9R)-1-fluoro-12-((2-(fluoromethylene)tetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalen-2-yl)naphthalen-2-ol 24 (a mixture of isomers)

### 5-Ethyl-6-fluoro-4-((5S,5aS,6S,9R)-1-fluoro-12-(((Z)-2-(fluoromethylene)tetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalen-2-yl)naphthalen-2-ol 16 (a mixture of diastereomers)

### 5-Ethyl-6-fluoro-4-((5S,5aS,6S,9R)-1-fluoro-12-(((E)-2-(fluoromethylene)tetrahydro-1H-pyrrolizin-7a(5H)-yl)methoxy)-5-methyl-5a,6,7,8,9,10-hexahydro-5H-4-oxa-3,10a,11,13,14-pentaaza-6,9-methanonaphtho[1,8-ab]heptalen-2-yl)naphthalen-2-ol 19 (a mixture of diastereomers)

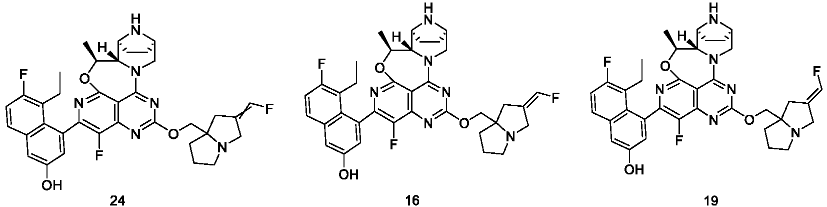

Crude title compound **24** (240 mg) was obtained by using the synthesis scheme of Example 5 and replacing the starting material of step 5, compound **5b,** with (2-(fluoromethylene)tetrahydro-1*H*-pyrrolizin-7*a*(5*H*)-yl)methanol (prepared using the method disclosed in Example 5 on page 33 of the specification in the patent application "WO2022247757") and purified by high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give compounds **16** and **19,** (10 mg, 10 mg, yields: 15% and 15%).

A mixture of diastereomers (shorter retention time): (10 mg, yield: 15%).

MS m/z (ESI): 659.2[M+1].

HPLC analysis: retention time 1.40 min, purity: 92% (chromatography column: ACQUITY UPLC^{®}BEH, C18, 1.7 µm, 2.1 × 50 mm; mobile phase: water (10 mM ammonium bicarbonate), acetonitrile, gradient ratio: acetonitrile 10%-95%).

¹H NMR (500 MHz, CD₃OD): δ 7.65 (dt, 1H), 7.30-7.18 (m, 2H), 7.03 (dd, 1H), 6.73 (d, 1H), 5.44-5.28 (m, 1H), 4.57 (t, 1H), 4.35 (d, 1H), 4.28 (dd, 1H), 4.09 (t, 1H), 3.71 (dd, 2H), 3.60 (s, 1H), 3.40 (d, 1H), 3.22-3.11 (m, 2H), 2.85 (d, 1H), 2.70 (q, 1H), 2.62-2.41 (m, 2H), 2.21-1.77 (m, 9H), 1.56 (dd, 3H), 0.85 (dt, 3H).

A mixture of diastereomers (longer retention time): (10 mg, yield: 15%).

MS m/z (ESI): 659.2[M+1].

HPLC analysis: retention time 1.41 min, purity: 96% (chromatography column: ACQUITY UPLC^{®}BEH, C18, 1.7 µm, 2.1 × 50 mm; mobile phase: water (10 mM ammonium bicarbonate), acetonitrile, gradient ratio: acetonitrile 10%-95%).

¹H NMR (500 MHz, CD₃OD): δ 7.66 (dt, 1H), 7.31-7.20 (m, 2H), 7.04 (dd, 1H), 6.75 (d, 1H), 5.43 (d, 1H), 4.67-4.56 (m, 1H), 4.55-4.39 (m, 2H), 4.15 (dd, 2H), 3.77 (s, 2H), 3.45 (s, 1H), 3.30-3.13 (m, 2H), 3.04-2.80 (m, 2H), 2.65-2.38 (m, 2H), 2.32-1.81 (m, 9H), 1.58 (d, 3H), 0.96-0.74 (m, 3H).

### Example 25

### 5-Ethyl-6-fluoro-4-((5S,5aS,6S,9R)-1-fluoro-12-((1-(((Z)-3-(fluoromethylene)pyrrolidin-1-yl)methyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalen-2-yl)naphthalen-2-ol 25

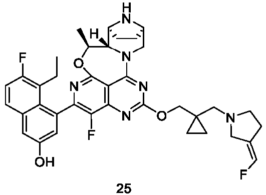

Title compound **25** (6 mg, yield: 14.5%) was obtained by using the synthesis scheme of Example 11 and replacing the starting material of step 2, compound **11a-1,** with compound **11a-2.**

MS m/z (ESI): 673.3[M+1].

¹H NMR (500 MHz, CD₃OD): δ 7.67 (dt, 1H), 7.42-7.17 (m, 2H), 7.05 (dd, 1H), 6.56 (dq, 1H), 5.40 (ddd, 1H), 4.57 (ddd, 1H), 4.48-4.32 (m, 2H), 4.10 (t, 1H), 3.86-3.60 (m, 2H), 3.35 (d, 3H), 3.29-3.07 (m, 1H), 2.78-2.71 (m, 2H), 2.70-2.45 (m, 4H), 2.42 (s, 2H), 2.26-2.05 (m, 2H), 1.99-1.76 (m, 3H), 1.58 (dd, 3H), 0.98 (t, 1H), 0.81 (t, 2H), 0.74 (d, 2H), 0.58 (d, 2H).

### Example 26

### 5-Ethyl-6-fluoro-4-((5S,5aS,6S,9R)-1-fluoro-12-((1-(((E)-3-(fluoromethylene)pyrrolidin-1-yl)methyl)cyclopropyl)methoxy)-5-methyl-5a,6,7,8,9,10-hexahydro-5H-4-oxa-3,10a,11,13,14-pentaaza-6,9-methanonaphthol[1,8-ab]heptalen-2-yl)naphthalen-2-amine 26

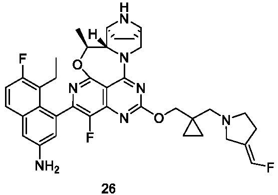

MS m/z (ESI): 672.3[M+1].

### Example 27

### 5-Ethyl-6-fluoro-4-((5S,5aS,6S,9R)-1-fluoro-12-((1-(((Z)-3-(fluoromethylene)pyrrolidin-1-yl)methyl)cyclopropyl)methoxy)-5-methyl-5a,6,7,8,9,10-hexahydro-5H-4-oxa-3,10a,11,13,14-pentaaza-6,9-methanonaphthol[1,8-ab]heptalen-2-yl)naphthalen-2-amine 27

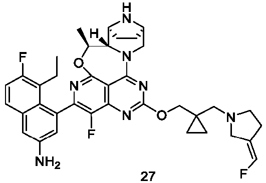

MS m/z (ESI): 672.3[M+1].

### Example 28

### (5S,5aS,6S,9R)-2-(8-Ethyl-7-fluoronaphthalen-1-yl)-1-fluoro-12-((1-(((Z)-3-(fluoromethylene)pyrrolidin-1-yl)methyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalene 28

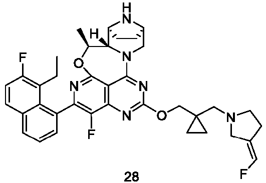

The title compound was obtained by using the synthesis scheme of Example 6, replacing the starting material of step 2, the compound 2-(8-ethyl-7-fluoro-3-(methoxymethoxy)naphthalen-1-yl)-4,4,5,5-tetramethyl-1,3,2-dioxaborolane, with 2-(8-ethyl-7-fluoronaphthalen-1-yl)-4,4,5,5-tetramethyl-1,3,2-dioxaborolane (prepared using the method for Intermediate L disclosed on page 93 of the specification in the patent application "WO2022232331"), and replacing the starting material of step 5, the compound 4-(difluoromethylene)piperidine hydrochloride, with compound **11a-2.**

MS m/z (ESI): 657.2[M+1].

### Example 29

### (5S,5aS,6S,9R)-2-(8-Ethyl-7-fluoronaphthalen-1-yl)-1-fluoro-12-((1-(((E)-3-(fluoromethylene)pyrrolidin-1-yl)methyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalene 29

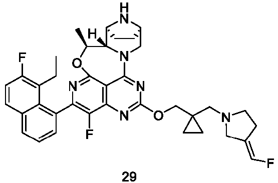

The title compound was obtained by using the synthesis scheme of Example 6, replacing the starting material of step 2, the compound 2-(8-ethyl-7-fluoro-3-(methoxymethoxy)naphthalen-1-yl)-4,4,5,5-tetramethyl-1,3,2-dioxaborolane, with 2-(8-ethyl-7-fluoronaphthalen-1-yl)-4,4,5,5-tetramethyl-1,3,2-dioxaborolane (prepared using the method for Intermediate L disclosed on page 93 of the specification in the patent application "WO2022232331"), and replacing the starting material of step 5, the compound 4-(difluoromethylene)piperidine hydrochloride, with compound **11a-1.**

MS m/z (ESI): 657.2[M+1].

### Example 30

### 5-Ethyl-6-fluoro-4-((5S,5aS,6S,9R)-1-fluoro-12-((1-(((Z)-3-(fluoromethylene)pyrrolidin-1-yl)methyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalen-2-yl)naphthalen-2-yl methylcarbamate 30

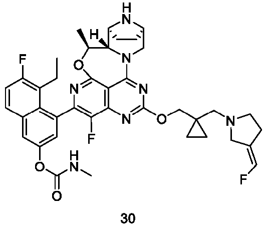

MS m/z (ESI): 730.2[M+1].

### Example 31

### 5-Ethyl-6-fluoro-4-((5S,5aS,6S,9R)-1-fluoro-12-((1-(((E)-3-(fluoromethylene)pyrrolidin-1-yl)methyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalen-2-yl)naphthalen-2-yl methylcarbamate 31

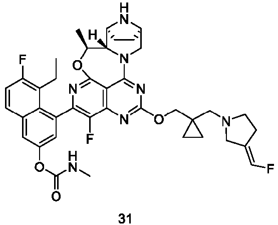

MS m/z (ESI): 730.2[M+1].

### Example 32

### 5-Ethyl-6-fluoro-4-((5S,5aS,6S,9R)-1-fluoro-12-((1-((4-(fluoromethylene)piperidin-1-yl)methyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalen-2-yl)naphthalen-2-amine 32

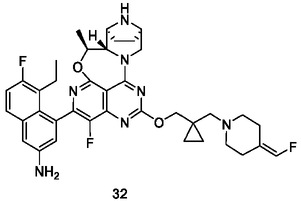

MS m/z (ESI): 686.2[M+1].

### Example 33

(5*S*,5*aS*,6*S*,9*R*)-2-(8-Ethyl-7-fluoronaphthalen-1-yl)-1-fluoro-12-((1-((4-(fluoromethylene)piperidin-1-yl)methyl)cyclopropyl)methoxy)-5-methyl-5*a*,6,7,8,9,10-hexahydro-5*H*-4-oxa-3,10*a*,11,13,14-pentaaza-6,9-methanonaphtho[1,8-*ab*]heptalene **33**

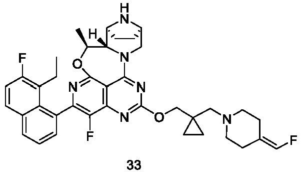

The title compound was obtained by using the synthesis scheme of Example 6, replacing the starting material of step 2, the compound 2-(8-ethyl-7-fluoro-3-(methoxymethoxy)naphthalen-1-yl)-4,4,5,5-tetramethyl-1,3,2-dioxaborolane, with 2-(8-ethyl-7-fluoronaphthalen-1-yl)-4,4,5,5-tetramethyl-1,3,2-dioxaborolane (prepared using the method for Intermediate L disclosed on page 93 of the specification in the patent application "WO2022232331"), and replacing the starting material of step 5, the compound 4-(difluoromethylene)piperidine hydrochloride, with compound **23c.**

MS m/z (ESI): 671.2[M+1].

### Example 34

### 5-Ethyl-6-fluoro-4-((5S,5aS,6S,9R)-1-fluoro-12-((1-((4-(fluoromethylene)piperidin-1-yl)methyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalen-2-yl)naphthalen-2-yl methylcarbamate 34

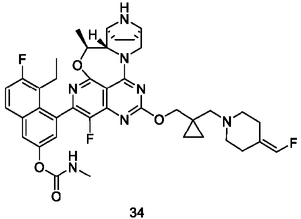

MS m/z (ESI): 744.2[M+1].

### Example 35

### 5-Ethyl-6-fluoro-4-((5S,5aS,6R,9R)-1-fluoro-12-((1-((5-(fluoromethylene)hexahydrocyclopenta[c]pyrrol-2(1H)-yl)methyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalen-2-yl)naphthalen-2-ol 35

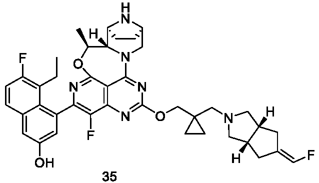

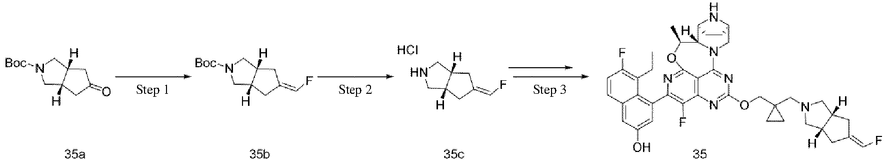

### Step 1

### tert-Butyl (3aR,6aS)-5-(fluoromethylene)hexahydrocyclopenta[c]pyrrole-2(1H)-carboxylate 35b

2-((Fluoromethyl)sulfonyl)pyridine (1.8 g, 4.7 mmol, Shanghai Accela) was dissolved in tetrahydrofuran (15 mL), and a 1 M solution of potassium bis(trimethylsilyl)amide in tetrahydrofuran (5.4 mL) was added at -78 °C. The mixture was left to react at that temperature for 30 min, and a solution of tert-butyl cis-5-oxohexahydrocyclopenta[*c*]pyrrole-2(1*H*)-carboxylate **35a** (1 g, 4.4 mmol, Nanjing PharmaBlock) in tetrahydrofuran (3 mL) was then added. The mixture was left to react at that temperature for 1 h, then warmed to room temperature, and left to react for 16 h. The reaction mixture was quenched with a saturated ammonium chloride solution and extracted with ethyl acetate (20 mL × 3). The organic phases were combined, washed with a saturated sodium chloride solution, and dried over anhydrous sodium sulfate. After the drying agent was removed by filtration, the filtrate was concentrated under reduced pressure, and the residue was purified by silica gel column chromatography using eluent system B to give title compound **35b** (760 mg, yield: 70.9%).

### Step 2

### (3aR,6aS)-5-(Fluoromethylene)octahydrocyclopenta[c]pyrrole hydrochloride 35c

Compound **35b** (760 mg, 3.1 mmol) was dissolved in acetonitrile (1.5 mL), and a 4 M solution of hydrogen chloride in 1,4-dioxane (6 mL) was added. After 1 h of stirring, the reaction mixture was concentrated under reduced pressure to give crude title compound **35c** (560 mg). The product was directly used in the next step without purification.

MS m/z (ESI): 142.3[M+1].

### Step 3

### 5-Ethyl-6-fluoro-4-((5S,5aS,6R,9R)-1-fluoro-12-((1-((5-(fluoromethylene)hexahydrocyclopenta[c]pyrrol-2(1H)-yl)methyl)cyclopropyl)methoxy)-5a,6,7,8,9,10-hexahydro-5H-4-oxa-3,10a,11,13,14-pentaaza-6,9-methanonaphtho[1,8-ab]heptalen-2-yl)naphthalen-2-ol 35

Title compound **35** (5 mg, yield: 10.3%) was obtained by using the synthesis scheme of Example 6 and replacing the starting material of step 5, 4-(difluoromethylene)piperidine hydrochloride, with compound **35c.**

MS m/z (ESI): 713.3[M+1].

¹H NMR (500 MHz, CD₃OD): δ 7.67-7.63 (m, 1H), 7.28-7.21 (m, 2H), 7.04 (d, 1H), 6.50 (d, 1H), 5.39-5.34 (m, 1H), 4.58-4.53 (m, 1H), 4.41 (t, 2H), 4.08 (t, 1H), 3.70 (d, 1H), 3.60 (s, 1H), 3.18 (t, 1H), 2.98 (s, 2H), 2.71-2.37 (m, 8H), 2.29-2.16 (m, 3H), 2.06-2.00 (m, 2H), 1.91-1.76 (m, 3H), 1.57 (d, 3H), 0.96 (t, 1H), 0.79 (t, 2H), 0.72 (s, 2H), 0.55 (s, 2H).

### Example 36

### 3-Chloro-5-((5S,5aS,6S,9R)-1-fluoro-12-((1-((4-(fluoromethylene)piperidin-1-yl)methyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalen-2-yl)-4-(trifluoromethyl)aniline 36

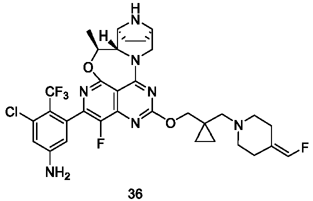

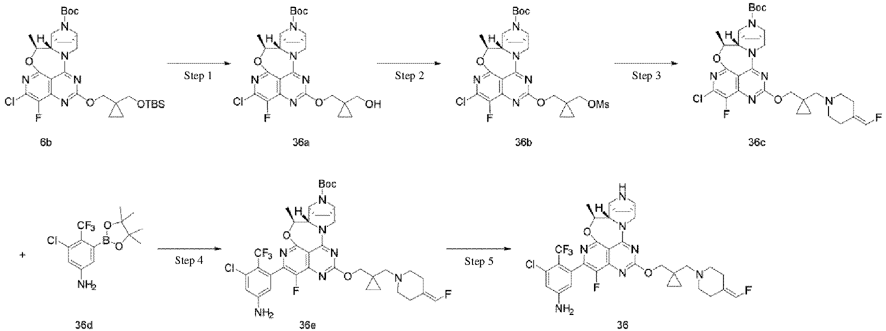

### Step 1

### tert-Butyl (5S,5aS,6S,9R)-2-chloro-1-fluoro-12-((1-(hydroxymethyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalene-4-carboxylate 36a

Compound **6b** (100 mg, 153.8 µmol) was dissolved in tetrahydrofuran (4 mL), and a 1 M solution of tetrabutylammonium fluoride in tetrahydrofuran (187 µL) was added. After 2 h of stirring, the reaction mixture was quenched with a saturated aqueous ammonium chloride solution and extracted with ethyl acetate (15 mL × 3). The organic phases were combined, washed with water and a saturated sodium chloride solution in sequence, and dried over anhydrous sodium sulfate. After the drying agent was removed by filtration, the filtrate was concentrated under reduced pressure to give crude title compound **36a** (82 mg). The product was directly used in the next step without purification.

MS m/z (ESI): 536.2[M+1].

### Step 2

### tert-Butyl (5S,5aS,6S,9R)-2-chloro-1-fluoro-5-methyl-12-((1-((methylsulfonyl)oxy)methyl)cyclopropyl)methoxy)-5a,6,7,8,9,10-hexahydro-5H-4-oxa-3,10a,11,13,14-pentaaza-6,9-methanonaphtho[1,8-ab]heptalene-14-carboxylate 36b

Crude compound **36a** (83 mg, 154.9 µmol) and N,N-diisopropylethylamine (60 mg, 464.2 µmol) were dissolved in dichloromethane (3 mL), and methanesulfonyl chloride (25 mg, 218.2 µmol) was added under an ice bath. The mixture was naturally warmed to room temperature and left to react for 30 min. The reaction mixture was quenched with a saturated aqueous ammonium chloride solution and extracted with ethyl acetate (10 mL × 3). The organic phases were combined, washed with water and a saturated sodium chloride solution in sequence, and dried over anhydrous sodium sulfate. After the drying agent was removed by filtration, the filtrate was concentrated under reduced pressure to give crude title compound **36b** (95 mg). The product was directly used in the next step without purification.

MS m/z (ESI): 614.2[M+1].

### Step 3

### tert-Butyl (5S,5aS,6S,9R)-2-chloro-1-fluoro-12-((1-((4-(fluoromethylene)piperidin-1-yl)methyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalene-14-formate 36c

Crude compound **36b** (95 mg, 154.7 µmol) and compound **23c** (35.5 mg, 234.5 µmol) were dissolved in acetonitrile (4 mL), and anhydrous potassium carbonate (64 mg, 463 µmol) and sodium iodide (70 mg, 467 µmol) were added. After 2 h of stirring at 80 °C, the reaction mixture was concentrated under reduced pressure, and the residue was purified by silica gel column chromatography using eluent system A to give title compound **36c** (80 mg, yield: 81.6%).

MS m/z (ESI): 633.2[M+1].

### Step 4

### tert-Butyl (5S,5aS,6S,9R)-2-(5-amino-3-chloro-2-(trifluoromethyl)phenyl)-1-fluoro-12-((1-(4-(fluoromethylene)piperidin-1-yl)methyl)cyclopropyl)methoxy)-5-methyl-5a,6,7,8,9,10-hexahydro-5H-4-oxa-3,10a,11,13,14-pentaaza-6,9-methanonaphthol[1,8-ab]heptalene-14-carboxylate 36e

Compound **36c** (20 mg, 31.6 µmol), 3-chloro-5-(4,4,5,5-tetramethyl-1,3,2-dioxaborolan-2-yl)-4-(trifluoromethyl)aniline **36d** (15 mg, 46.6 µmol, prepared using the method disclosed in Example 80 on page 257 of the specification in the patent application "WO2022148422"), tetrakis(triphenylphosphine)palladium(0) (7 mg, 6.1 µmol), and cesium carbonate (31 mg, 95.1 µmol) were mixed in 1,4-dioxane (1 mL) and water (0.2 mL). The mixture was left to react at 100 °C for 1 h in a nitrogen atmosphere. The reaction mixture was cooled to room temperature, then diluted with ethyl acetate, and dried over anhydrous sodium sulfate. After the drying agent was removed by filtration, the filtrate was concentrated under reduced pressure to give crude title compound **36e** (20 mg). The product was directly used in the next step without purification.

MS m/z (ESI): 792.2[M+1].

### Step 5

### 3-Chloro-5-((5S,5aS,6S,9R)-1-fluoro-12-((1-((4-(fluoromethylene)piperidin-1-yl)methyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalen-2-yl)-4-(trifluoromethyl)aniline 36

Crude compound **36e** (15 mg, 18.9 µmol) was dissolved in dichloromethane (1 mL), and a 4 M solution of hydrochloric acid in 1,4-dioxane (0.5 mL) was added under an ice bath. The mixture was left to react at that temperature for 0.5 h and then concentrated under reduced pressure, and the residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give title compound **36** (4 mg, yield: 30.5%).

MS m/z (ESI): 690.3[M-1].

¹H NMR (500 MHz, CD₃OD): δ 6.90 (s, 1H), 6.58 (d, 1H), 6.42 (s, 1H), 5.37 (dd, 1H), 4.58-4.39 (m, 3H), 4.07 (t, 1H), 3.70 (d, 1H), 3.60 (d, 1H), 3.20-3.14 (m, 1H), 2.61-2.42 (m, 6H), 2.33 (s, 2H), 2.08 (s, 3H), 1.92-1.74 (m, 3H), 1.58 (d, 3H), 0.74 (s, 2H), 0.52 (s, 2H).

### Example 37

### 2-Amino-7-fluoro-4-((5S,5aS,6S,9R)-1-fluoro-12-((1-((4-(fluoromethylene)piperidin-1-yl)methyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalen-2-yl)benzo[b]thiophene-3-carbonitrile 37

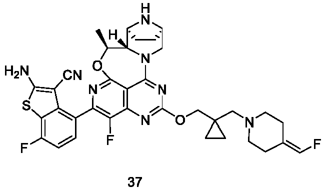

Title compound **37** (4 mg, yield: 34.4%) was obtained by using the synthesis scheme of Example 36 and replacing the starting material of step 4, compound 3**6d,** with the compound tert-butyl (3-cyano-4-(4,4,5,5-tetramethyl-1,3,2-dioxaborolan-2-yl)-7-fluorobenzo[b]thiophen-2-yl)carbamate (prepared using the method for preparation 15 disclosed on page 50 of the specification in the patent application "WO2021118877").

MS m/z (ESI): 687.3[M-1].

¹H NMR (500 MHz, CD₃OD): δ 7.39 (dd, 1H), 7.04 (t, 1H), 6.51 (d, 1H), 5.41 (dd, 1H), 4.53-4.46 (m, 2H), 4.40 (d, 1H), 4.10 (d, 1H), 3.70 (d, 1H), 3.60 (d, 1H), 3.19 (s, 1H), 2.62-2.31 (m, 8H), 2.09 (d, 3H), 1.92-1.75 (m, 3H), 1.61 (d, 3H), 0.75 (s, 2H), 0.52 (s, 2H).

### Example 38

### 2-Fluoro-3-((5S,5aS,6S,9R)-1-fluoro-12-((1-((4-(fluoromethylene)piperidin-1-yl)methyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalen-2-yl)-5-methyl-4-(trifluoromethyl)aniline 38

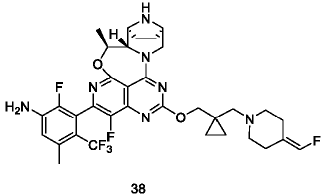

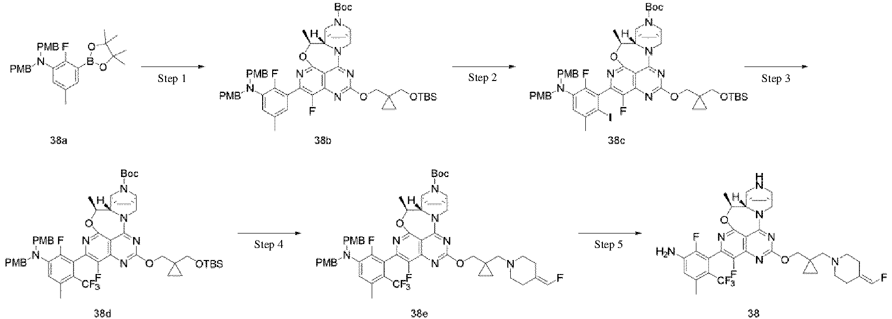

### Step 1

### tert-Butyl (5S,5aS,6S,9R)-2-(3-(bis(4-methoxybenzyl)amino)-2-fluoro-5-methylphenyl)-12-((1-(((tert-butyldimethylsilyl)oxy)methyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate 38b

Compound **6b** (200 mg, 307.6 µmol), 2-fluoro-*N*,*N*-bis(4-methoxybenzyl)-5-methyl-3-(4,4,5,5-tetramethyl-1,3,2-dioxaborolan-2-yl)aniline **38a** (200 mg, 407 µmol, prepared using the method disclosed in Example 27a and 27b on page 308 of the specification in the patent application "WO2020035031"), tetrakis(triphenylphosphine)palladium(0) (55 mg, 47.6 µmol), and cesium carbonate (300 mg, 920.7 µmol) were mixed in 1,4-dioxane (5 mL) and water (1 mL). The mixture was left to react at 100 °C for 1 h in a nitrogen atmosphere. The reaction mixture was cooled to room temperature, then diluted with ethyl acetate, and dried over anhydrous sodium sulfate. After the drying agent was removed by filtration, the filtrate was concentrated under reduced pressure to give crude title compound **38b** (280 mg). The product was directly used in the next step without purification.

MS m/z (ESI): 979.2[M+1].

### Step 2

### tert-Butyl (5S,5aS,6S,9R)-2-(3-(bis(4-methoxybenzyl)amino)-2-fluoro-6-iodo-5-methylphenyl)-12-((1-(((tert-butyldimethylsilyl)oxy)methyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate 38c

Crude compound **38b** (280 mg, 285.91 µmol) was dissolved in N,N-dimethylformamide (DMF) (4 mL), and silver acetate (120 mg, 718.9 µmol) and iodine (145 mg, 571.3 µmol) were sequentially added. The mixture was left to react at 15 °C for 1 h. The reaction mixture was diluted with water and extracted with ethyl acetate (20 mL × 2). The organic phases were combined and dried over anhydrous sodium sulfate. After the drying agent was removed by filtration, the filtrate was concentrated under reduced pressure, and the residue was purified by silica gel column chromatography using eluent system A to give title compound **38c** (180 mg, yield: 57%).

MS m/z (ESI): 1105.2[M+1].

### Step 3

### tert-Butyl (5S,5aS,6S,9R)-2-(3-(bis(4-methoxybenzyl)amino)-2-fluoro-5-methyl-6-(trifluoromethyl)phenyl)-12-((1-(((tertbutyldimethylsilyl)oxy)methyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate 38d

Compound **38c** (150 mg, 135.7 µmol) was dissolved in *N*,*N*-dimethylformamide (DMF) (1 mL), and cuprous iodide (125 mg, 656.3 µmol), methyl difluoro(fluorosulfonyl)acetate (260 mg, 1.4 mmol, Shanghai Bide) were sequentially added. The mixture was left to react at 100 °C for 1 h. The reaction mixture was diluted with water and extracted with ethyl acetate (20 mL × 2). The organic phases were combined and dried over anhydrous sodium sulfate. After the drying agent was removed by filtration, the filtrate was concentrated under reduced pressure, and the residue was purified by silica gel column chromatography using eluent system A to give title compound **38d** (80 mg, yield: 57.6%). MS m/z (ESI): 1047.2[M+1].

### Step 4

### tert-Butyl (5S,5aS,6S,9R)-2-(3-(bis(4-methoxybenzyl)amino)-2-fluoro-5-methyl-6-(trifluoromethyl)phenyl)-1-fluoro-12-(1-((4-(fluoromethylene)piperidin-1-yl)methyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate 38e

Crude title compound **38e** (40 mg) was obtained by using steps 1 to 3 of the synthesis scheme of Example 36 and replacing the starting material of step 1, compound **6b,** with compound **38d.**

MS m/z (ESI): 1030.2[M+1].

### Step 5

### 2-Fluoro-3-((5S,5aS,6S,9R)-1-fluoro-12-((1-((4-(fluoromethylene)piperidin-1-yl)methyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalen-2-yl)-5-methyl-4-(trifluoromethyl)aniline 38

Crude compound **38e** (40 mg, 38.8 µmol) was dissolved in trifluoroacetic acid (1 mL), and trifluoromethanesulfonic acid (0.1 mL) was added under an ice bath. After 1 h of stirring, the reaction mixture was concentrated under reduced pressure, and the pH was adjusted to about 7 with a small amount of a saturated sodium bicarbonate solution. The mixture was concentrated under reduced pressure. The residue was dissolved in methanol, and the resulting mixture was then filtered. The filtrate was concentrated under reduced pressure, and the residue was purified by high performance liquid preparative chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give title compound 38 (10 mg, yield: 37.3%).

MS m/z (ESI): 690.2[M+1].

¹H NMR (500 MHz, CD₃OD): δ 6.87 (d, 1H), 6.51 (d, 1H), 5.41-5.34 (m, 1H), 4.61-4.39 (m, 3H), 4.08 (t, 1H), 3.70 (d, 1H), 3.60 (s, 1H), 3.47 (q, 1H), 3.18 (d, 1H), 2.61-2.29 (m, 7H), 2.21 (t, 1H), 2.07 (s, 2H), 1.83 (dd, 3H), 1.59 (dd, 3H), 1.36 (s, 3H), 0.74 (s, 2H), 0.52 (s, 2H).

### Example 39

### 3-Chloro-5-((5S,5aS,6S,9R)-12-((1-((4-(difluoromethylene)piperidin-1-yl)methyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]heptalen-2-yl)-4-(trifluoromethyl)aniline 39

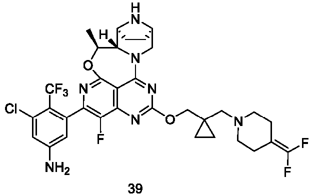

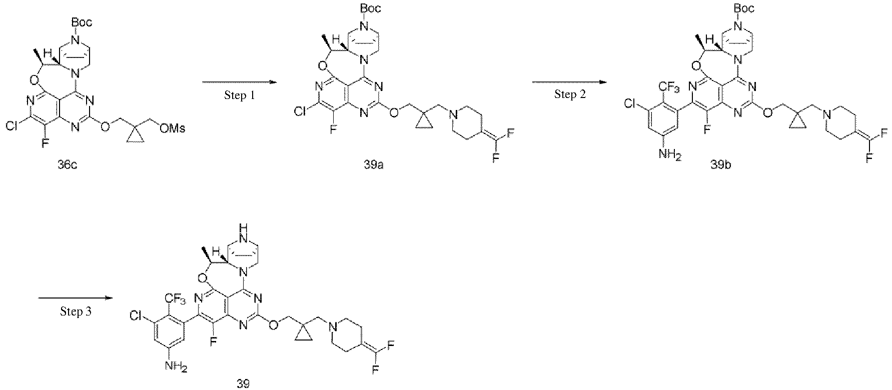

### Step 1

### tert-Butyl (5S,5aS,6S,9R)-2-chloro-12-((1-((4-(difluoromethylene)piperidin-1-yl)methyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-formate 39a

Crude compound **36c** (50 mg, 81.4 µmol) and 4-(difluoromethylene)piperidine hydrochloride (54.2 mg, 319 µmol) were dissolved in acetonitrile (5 mL), and anhydrous potassium carbonate (56.3 mg, 407 µmol) and sodium iodide (36.6 mg, 244 µmol) were added. After 2 h of stirring at 80 °C, the reaction mixture was concentrated under reduced pressure, and the residue was purified by silica gel column chromatography using eluent system A to give title compound **39a** (50 mg, yield: 94.3%).

MS m/z (ESI): 651.2[M+1].

### Step 2

### tert-Butyl (5S,5aS,6S,9R)-2-(5-amino-3-chloro-2-(trifluoromethyl)phenyl)-12-((1-(4-(difluoromethylene)piperidin-1-yl)methyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate 39b

Compound **39a** (50 mg, 76.8 µmol), compound **36d** (37 mg, 115 µmol), tetrakis(triphenylphosphine)palladium(0) (17.7 mg, 15.3 µmol), and cesium carbonate (75 mg, 230 µmol) were mixed in 1,4-dioxane (3 mL) and water (0.5 mL). The system was purged with nitrogen and left to react at 100 °C for 16 h. The reaction mixture was cooled to room temperature, then diluted with ethyl acetate, and dried over anhydrous sodium sulfate. After the drying agent was removed by filtration, the filtrate was concentrated under reduced pressure to give crude title compound **39b** (60 mg). The product was directly used in the next step without purification.

MS m/z (ESI): 810.2[M+1].

### Step 3

### 3-Chloro-5-((5S,5aS,6S,9R)-12-((1-((4-(difluoromethylene)piperidin-1-yl)methyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]heptalen-2-yl)-4-(trifluoromethyl)aniline 39

Crude compound **39b** (50 mg, 61.7 µmol) was dissolved in dichloromethane (3 mL), and a 4 M solution of hydrogen chloride in 1,4-dioxane (1 mL) was added under an ice bath. The mixture was naturally warmed to room temperature, left to react for 1 h, and then concentrated under reduced pressure, and the residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give title compound **39** (4 mg, yield: 9%).

MS m/z (ESI): 710.2[M+1]

¹H NMR (500 MHz, CD₃OD): δ 6.90 (d, 1H), 6.56 (s, 1H), 6.42 (s, 1H), 5.38 (d, 1H), 4.44 (s, 3H), 4.10 (s, 2H), 3.71 (d, 2H), 3.20 (d, 1H), 2.63 (s, 5H), 2.26-2.05 (m, 3H), 1.82 (s, 2H), 1.59 (d, 2H), 1.31 (s, 3H), 0.77 (s, 2H), 0.56 (s, 2H).

### Example 40

### 3-((5S,5aS,6S,9R)-12-((1-((4-(Difluoromethylene)piperidin-1-yl)methyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]heptalen-2-yl)-2-fluoro-5-methyl-4-(trifluoromethyl)aniline 40

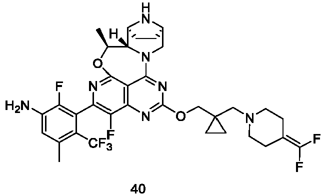

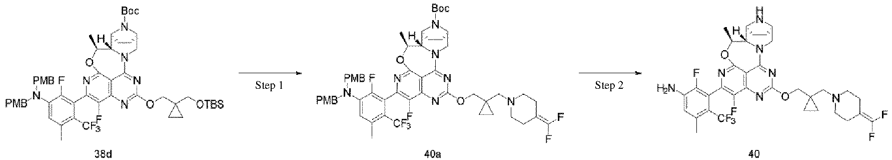

### Step 1

### tert-Butyl (5S,5aS,6S,9R)-2-(3-(bis(4-methoxybenzyl)amino)-2-fluoro-5-methyl-6-(trifluoromethyl)phenyl)-12-((1-((4-(difluoromethylene)piperidin- 1-yl)methyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-carboxylate 40a

Crude title compound **40a** (31 mg) was obtained by using steps 3 to 5 of the synthesis scheme of Example 6 and replacing the starting material of step 3, compound **6c,** with compound **38d.**

MS m/z (ESI): 1048.2[M+1].

### Step 2

### 3-((5S,5aS,6S,9R)-12-((1-((4-(Difluoromethylene)piperidin-1-yl)methyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]heptalen-2-yl)-2-fluoro-5-methyl-4-(trifluoromethyl)aniline 40

Crude compound **40a** (31 mg, 19.7 µmol) was dissolved in trifluoroacetic acid (1 mL), and trifluoromethanesulfonic acid (0.1 mL) was added under an ice bath. After 1 h of stirring, the reaction mixture was concentrated under reduced pressure, and the pH was adjusted to about 7 with a small amount of a saturated sodium bicarbonate solution. The mixture was concentrated under reduced pressure. The residue was dissolved in methanol, and the resulting mixture was then filtered. The filtrate was concentrated under reduced pressure, and the residue was purified by high performance liquid preparative chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give title compound **40** (5 mg, yield: 24.6%).

MS m/z (ESI): 708.2[M+1].

¹H NMR (500 MHz, CD₃OD): δ 6.87 (d, 1H), 5.38-5.36 (m, 1H), 4.61-4.39 (m, 3H), 4.08 (t, 1H), 3.70 (d, 1H), 3.60 (s, 1H), 3.47 (q, 1H), 3.18 (d, 1H), 2.61-2.29 (m, 7H), 2.21 (t, 1H), 2.07 (s, 2H), 1.83 (dd, 3H), 1.59 (dd, 3H), 1.35(s, 3H), 0.74 (s, 2H), 0.52 (s, 2H).

### Example 41

### 2-Amino-4-((5S,5aS,6S,9R)-12-((1-((4-(difluoromethylene)piperidin-1-yl)methyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]heptalen-2-yl)-7-fluorobenzo[b]thiophene-3-carbonitrile 41

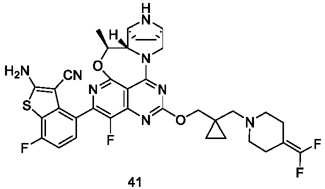

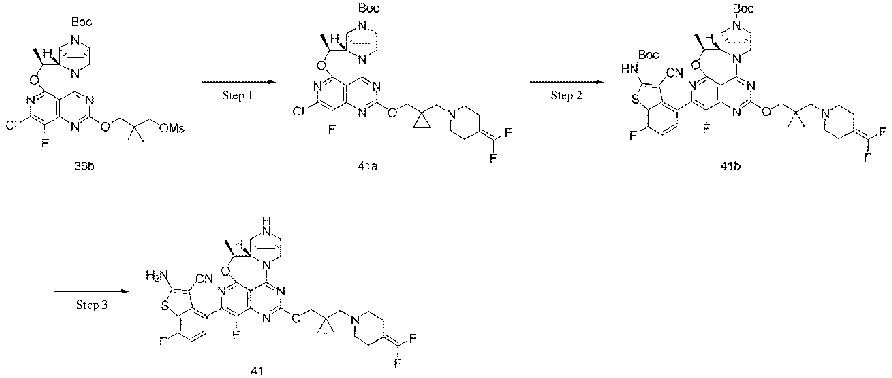

### Step 1

### tert-Butyl (5S,5aS,6S,9R)-2-chloro-12-((1-((4-(difluoromethylene)piperidin-1-yl)methyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-formate 41a

Crude compound **36b** (30 mg, 48.8 µmol) and 4-(difluoromethylene)piperidine hydrochloride (12.4 mg, 73.2 µmol) were dissolved in acetonitrile (4 mL), and anhydrous potassium carbonate (20.2 mg, 146.5 µmol) and sodium iodide (22 mg, 146.5 µmol) were added. After 1 h of stirring at 80 °C, the reaction mixture was cooled to room temperature and then filtered. The filtrate was diluted with water and extracted with ethyl acetate (5 mL × 3). The organic phases were combined, washed with water and a saturated sodium chloride solution in sequence, and dried over anhydrous sodium sulfate. After the dying agent was removed by filtration, the filtrate was concentrated under reduced pressure to give crude title compound **41a** (31 mg). The product was directly used in the next step without purification.

MS m/z (ESI): 651.2[M+1].

### Step 2

### tert-Butyl (5S,5aS,6S,9R)-2-(2-((tert-butoxycarbonyl)amino)-3-cyano-7-fluorobenzo[b]thiophen-4-yl)-12-(1-((4-(difluoromethylene)piperidin-1-yl)methyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]heptalene-14-formate 41b

Crude compound **41a** (31 mg, 47.6 µmol), tert-butyl (3-cyano-4-(4,4,5,5-tetramethyl-1,3,2-dioxaborolan-2-yl)-7-fluorobenzo[b]thiophen-2-yl)carbamate (26.9 mg, 66.6 µmol), tetrakis(triphenylphosphine)palladium(0) (11 mg, 9.5 µmol), and cesium carbonate (46.5 mg, 142.8 µmol) were mixed in *N*,*N*-dimethylformamide (1 mL). The mixture was left to react at 100 °C for 3 h in a nitrogen atmosphere. The reaction mixture was cooled to room temperature and then filtered, and the filtrate was concentrated under reduced pressure to give crude title compound **41b** (43 mg). The product was directly used in the next step without purification.

MS m/z (ESI): 907.2[M+1].

### Step 3

### 2-Amino-4-((5S,5aS,6S,9R)-12-((1-((4-(difluoromethylene)piperidin-1-yl)methyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]heptalen-2-yl)-7-fluorobenzo[b]thiophene-3-carbonitrile 41

Crude compound **41b** (40 mg, 44.1 µmol) was dissolved in dichloromethane (0.5 mL), and trifluoroacetic acid (0.5 mL) was added. The mixture was stirred for 1 h and then concentrated under reduced pressure, and the residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give title compound **41** (2 mg, yield: 6.4%). MS m/z (ESI): 707.2[M+1]. ¹H NMR (500 MHz, CD₃OD): δ 7.39 (dd, 1H), 7.04 (t, 1H), 5.41 (dd, 1H), 4.91 (s, 3H), 4.54 (d, 1H), 4.48 (d, 1H), 4.39 (d, 1H), 4.10 (d, 1H), 3.71 (d, 1H), 3.61 (s, 1H), 3.19 (d, 1H), 2.70-2.41 (m, 5H), 2.22 (s, 3H), 2.10 (q, 2H), 2.01-1.83 (m, 2H), 1.79 (d, 2H), 1.61 (d, 3H), 0.75 (s, 2H), 0.52 (s, 2H).

### Example 42

### 4-((5S,5aS,6S,9R)-1-Fluoro-12-((1-((4-(fluoromethylene)piperidin-1-yl)methyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalen-2-yl)-6-methyl-5-(trifluoromethyl)pyridin-2-amine 42

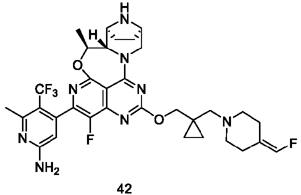

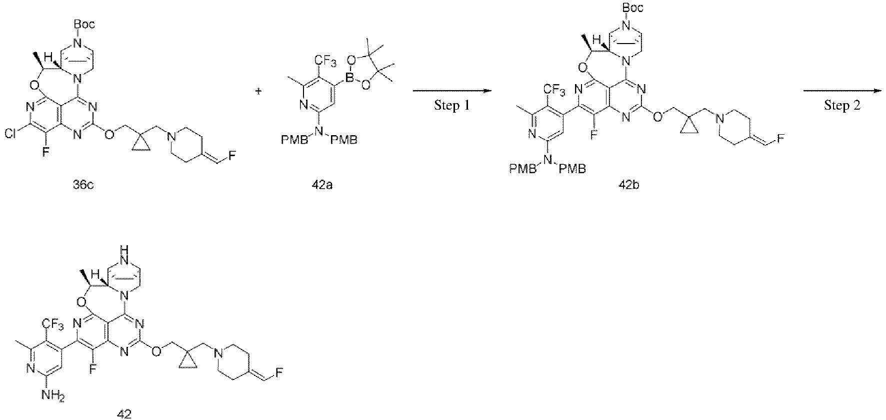

### Step 1

### tert-Butyl (5S,5aS,6S,9R)-2-(6-(bis(4-methoxybenzyl)amino)-2-methyl-3-(trifluoromethyl)pyridin-4-yl)-1-fluoro-12-(1-((4-(fluoromethylene)piperidin-1-yl)methyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalene-4-carboxylate 42b

Compound **36c** (100 mg, 157.9 µmol), *N*,*N*-bis(4-methoxybenzyl)-6-methyl-4-(4,4,5,5-tetramethyl-1,3,2-dioxaborolan-2-yl)-5-(trifluoromethyl)pyridin-2-amine **42a** (111.4 mg, 205.3 µmol, prepared using the method disclosed in Example 60 on page 244 of the specification in the patent application "WO2022042630"), tetrakis(triphenylphosphine)palladium(0) (36.5 mg, 31.6 µmol), and cesium carbonate (128.7 mg, 394.8 µmol) were mixed in 1,4-dioxane (2 mL) and water (0.4 mL). The mixture was left to react at 100 °C for 16 h in a nitrogen atmosphere. The reaction mixture was cooled to room temperature and then concentrated under reduced pressure, and the residue was purified by silica gel column chromatography using eluent system A to give title compound **42b** (160 mg, yield: 99.9%).

MS m/z (ESI): 1014.1[M+1].

### Step 2

### 4-((5S,5aS,6S,9R)-1-Fluoro-12-((1-((4-(fluoromethylene)piperidin-1-yl)methyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalen-2-yl)-6-methyl-5-(trifluoromethyl)pyridin-2-amine 42

Compound **42b** (15 mg, 157.9 µmol) was dissolved in trifluoroacetic acid (2 mL). The mixture was left to react at 100 °C for 5 h and concentrated under reduced pressure. The residue was dissolved in dichloromethane, and the resulting solution was washed with a saturated sodium bicarbonate solution. The organic phase was separated and concentrated under reduced pressure, and the residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give title compound **42** (30 mg, yield: 28.2%).

MS m/z (ESI): 673.2[M+1].

¹H NMR (500 MHz, CD₃OD): δ 6.49 (d, 1H), 5.35 (d, 1H), 4.51 (d, 1H), 4.41 (q, 2H), 4.06 (d, 1H), 3.69 (d, 1H), 3.59 (s, 1H), 3.15 (d, 1H), 2.57-2.42 (m, 8H), 2.31 (s, 2H), 2.19 (d, 1H), 2.06 (s, 3H), 1.77 (d, 2H), 1.67-1.51 (m, 4H), 0.89 (d, 1H), 0.72 (s, 2H), 0.50 (s, 2H).

### Example 43

### 2-Fluoro-5-((5S,5aS,6S,9R)-1-fluoro-12-((1-((4-(fluoromethylene)piperidin-1-yl)methyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalen-2-yl)-3-methyl-4-(trifluoromethyl)aniline 43

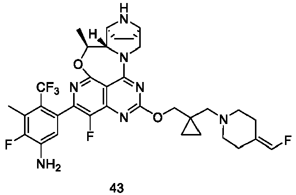

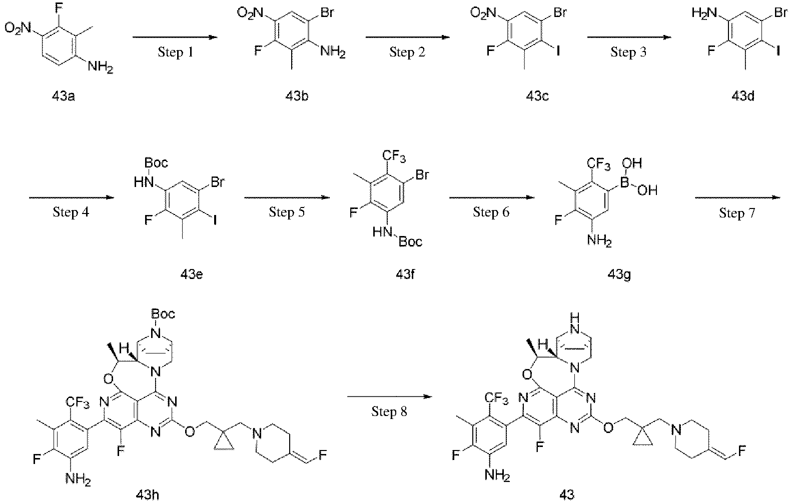

### Step 1

### 6-Bromo-3-fluoro-2-methyl-4-nitroaniline 43b

3-Fluoro-2-methyl-4-nitroaniline 43a (2.2 g, 12.9 mmol, prepared using the method disclosed in EXAMPLE 7 on page 10 of the specification in the patent application "US7626018") was dissolved in acetonitrile (80 mL), and N-bromosuccinimide (2.7 g, 15.5 mmol) was added. After 6 h of stirring at 80 °C, the reaction mixture was concentrated under reduced pressure, and the residue was purified by silica gel column chromatography using eluent system B to give title compound **43b** (2.9 g, yield: 90%).

MS m/z (ESI): 247.0[M-1].

### Step 2

### 1-Bromo-4-fluoro-2-iodo-3-methyl-5-nitrobenzene 43c

Concentrated hydrochloric acid (10 mL) was added to compound **43b** (1.5 g, 6.0 mmol) at 0 °C. After 10 min of stirring at that temperature, a 2 M aqueous sodium nitrite solution (9 mL) was added. After 1 h of stirring at 0 °C, a 2 M aqueous potassium iodide solution (12 mL) was added. After 10 min of stirring at that temperature, the mixture was heated to 80 °C and left to react for 1 h. The reaction mixture was cooled to room temperature, then quenched with saturated sodium thiosulfate, and extracted with ethyl acetate (20 mL × 3). The organic phases were combined and dried over anhydrous sodium sulfate. After the drying agent was removed by filtration, the filtrate was concentrated under reduced pressure to give crude title compound **43c** (2.1 g). The product was directly used in the next step without purification.

### Step 3

### 5-Bromo-2-fluoro-4-iodo-3-methylaniline 43d

Compound **43c** (2.1 g, 5.83 mmol) was mixed in ethanol (20 mL) and water (40 mL), and iron powder (1.3 g, 23.3 mmol) and ammonium chloride (6 g, 35 mmol) were added. After 3 h of stirring at 90 °C, the reaction mixture was cooled to room temperature and then filtered, and the filtrate was extracted with dichlorohexane (50 mL × 2). The organic phases were combined and dried over anhydrous sodium sulfate. After the drying agent was removed by filtration, the filtrate was concentrated under reduced pressure, and the residue was purified by silica gel column chromatography using eluent system B to give title compound **43d** (1.5 g, yield: 77.9%).

MS m/z (ESI): 327.9[M-1].

### Step 4

### tert-Butyl (5-bromo-2-fluoro-4-iodo-3-methylphenyl)carbamate 43e

Compound **43d** (0.8 g, 2.42 mmol) was dissolved in 1,4-dioxane (5 mL), and di-tert-butyl dicarbonate (BOC anhydride) (3.7 g, 16.9 mmol) was added. After 48 h of stirring at 100 °C, the reaction mixture was concentrated under reduced pressure, and the residue was purified by silica gel column chromatography using eluent system B to give title compound **43e** (1 g, yield: 95.9%).

MS m/z (ESI): 427.9[M-1].

### Step 5

### tert-Butyl (5-bromo-2-fluoro-3-methyl-4-(trifluoromethyl)phenyl)carbamate 43f

Compound **43e** (1.2 g, 2.79 mmol), cuprous iodide (1.32 g, 6.97 mmol), hexamethylphosphoric triamide (1.5 g, 8.37 mmol, Shanghai Accela), and methyl difluoro(fluorosulfonyl)acetate (1.6 g, 8.37 mmol, Shanghai Accela) were dissolved in N,N-dimethylformamide (DMF) (10 mL). The system was purged with nitrogen and stirred at 90 °C for 2 h. The reaction mixture was cooled to room temperature and then filtered, and the filtrate was diluted with ethyl acetate and washed with water. The organic phase was separated and concentrated under reduced pressure, and the residue was purified by silica gel column chromatography using eluent system B to give title compound **43f** (500 mg, yield: 48.1%).

MS m/z (ESI): 370.0[M-1].

### Step 6

### (5-Amino-4-fluoro-3-methyl-2-(trifluoromethyl)phenyl)boronic acid 43g

Compound **43f** (391 mg, 1.05 mmol), bis(pinacolato)diboron (400.2 mg, 1.57 mmol), [1,1'-bis(diphenylphosphino)ferrocene]palladium(II) dichloride (76.9 mg, 105.1 µmol), and potassium acetate (309.3 mg, 3.15 mmol) were mixed in dimethyl sulfoxide (5 mL). The system was purged with nitrogen three times and heated at 100 °C for 16 h. The reaction mixture was cooled to room temperature and then filtered. The filtrate was diluted with water and extracted with ethyl acetate (10 mL × 2). The organic phases were combined and dried over anhydrous sodium sulfate. After the drying agent was removed by filtration, the filtrate was concentrated under reduced pressure, and the residue was purified by silica gel column chromatography using eluent system B to give title compound **43g** (100 mg, yield: 40.1%).

MS m/z (ESI): 237.9[M+1].

### Step 7

### tert-Butyl (5S,5aS,6S,9R)-2-(5-amino-4-fluoro-3-methyl-2-(trifluoromethyl)phenyl)-1-fluoro-12-((1-((4-(fluoromethylene)piperidin-1-yl)methyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalene-14-carboxylate 43h

Compound **36c** (70 mg, 110.5 µmol), compound **43g** (26.2 mg, 110.5 µmol), tetrakis(triphenylphosphine)palladium(0) (25.5 mg, 25.2 µmol), and cesium carbonate (108 mg, 331.6 µmol) were mixed in 1,4-dioxane (1 mL) and water (0.2 mL). The system was purged with nitrogen and left to react at 100 °C for 16 h. The reaction mixture was concentrated under reduced pressure, and the residue was purified by silica gel column chromatography using eluent system A to give title compound **43h** (40 mg, yield: 45.8%). MS m/z (ESI): 790.2[M+1].

### Step 8

### 2-Fluoro-5-((5S,5aS,6S,9R)-1-fluoro-12-((1-((4-(fluoromethylene)piperidin-1-yl)methyl)cyclopropyl)methoxy)-5-methyl-5a,6,7,8,9,10-hexahydro-5H-4-oxa-3,10a,11,13,14-pentaaza-6,9-methanonaphtho[1,8-ab]heptalen-2-yl)-3-methyl-4-(trifluoromethyl)aniline 43

Compound **43h** (97.7 mg, 123.7 µmol) was dissolved in dichloromethane (1 mL), and trifluoroacetic acid (1 mL) was added. The mixture was stirred for 2 h and then concentrated under reduced pressure, and the residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give title compound **43** (5 mg, yield: 5.8%).

MS m/z (ESI): 690.2[M+1].

¹H NMR (500 MHz, CD₃OD): δ 6.87 (d, 1H), 6.52 (d, 1H), 5.38 (d, 1H), 4.57 (d, 2H), 4.53-4.31 (m, 2H), 4.09 (d, 1H), 3.71 (s, 1H), 3.61 (s, 1H), 3.47 (s, 1H), 3.17 (d, 2H), 2.55 (s, 5H), 2.36 (t, 4H), 2.10 (s, 3H), 1.90-1.76 (m, 3H), 1.60 (d, 3H), 1.33 (d, 2H), 0.75 (d, 2H), 0.53 (d, 2H).

### Example 44

### 5-((5S,5aS,6S,9R)-12-((1-((4-(Difluoromethylene)piperidin-1-yl)methyl)cyclopropyl)methoxy)-1-fluoro-5-methyl-5a,6,7,8,9,10-hexahydro-5H-4-oxa-3,10a,11,13,14-pentaaza-6,9-methanonaphtho[1,8-ab]heptalen-2-yl)-2-fluoro-3-methyl-4-(trifluoromethyl)aniline 44

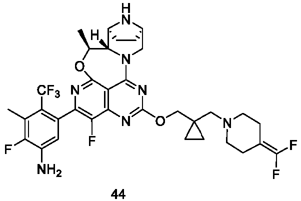

Title compound **44** (3 mg, yield: 3.4%) was obtained by using the synthesis scheme of Example 43 and replacing the starting material of step 7, compound 3**6c,** with compound **41a.**

MS m/z (ESI): 708.2[M+1].

¹H NMR (500 MHz, CD₃OD): δ 6.87 (d, 1H), 5.38 (d, 1H), 4.73-4.52 (m, 2H), 4.49-4.35 (m, 2H), 4.08 (d, 1H), 3.71 (s, 1H), 3.61 (s, 1H), 3.17 (d, 2H), 2.75-2.42 (m, 5H), 2.36 (s, 3H), 2.21 (s, 3H), 2.08 (s, 1H), 1.79 (d, 4H), 1.60 (d, 3H), 1.33 (d, 2H), 0.74 (d, 2H), 0.52 (d, 2H).

### Example 45

### 4-((5S,5aS,6S,9R)-12-((1-((4-(Difluoromethylene)piperidin-1-yl)methyl)cyclopropyl)methoxy)-1-fluoro-5-methyl-5a,6,7,8,9,10-hexahydro-5H-4-oxa-3,10a,11,13,14-pentaaza-6,9-methanonaphthol[1,8-ab]heptalen-2-yl)-6-methyl-5-(trifluoromethyl)pyridin-2-amine 45

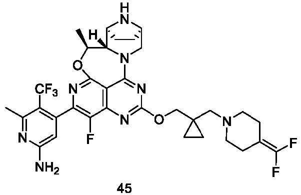

Title compound **45** (20 mg, yield: 25%) was obtained by using the synthesis scheme of Example 42 and replacing the starting material of step 1, compound **36c,** with compound **41a.**

MS m/z (ESI): 691.2[M+1].

¹H NMR (500 MHz, CD₃OD): δ 6.36 (s, 1H), 5.37 (dd, 1H), 4.60 (s, 1H), 4.53 (dt, 1H), 4.43 (s, 2H), 4.08 (d, 1H), 3.71 (d, 1H), 3.65-3.58 (m, 1H), 3.18 (d, 1H), 2.55 (d, 7H), 2.20 (d, 4H), 2.07 (d, 2H), 1.95-1.74 (m, 3H), 1.59 (d, 3H), 0.74 (s, 2H), 0.53 (s, 2H).

### Biological Evaluations

### Test Example 1: Biological Evaluation of Inhibition of ERK Phosphorylation in AGS Cells (HTRF Method)

### I. Objective

In the experiment, the inhibitory effects of the compounds of the present disclosure on the KRAS target were evaluated by assessing the inhibitory effects of the compounds on ERK phosphorylation in cells and based on IC₅₀ values.

### II. Experimental method

AGS cells were cultured in an RPMI1640 (Hyclone, SH30809.01) complete culture medium containing 10% fetal bovine serum. On the first day of the experiment, the AGS cells were seeded into a 96-well plate with a complete culture medium at a density of 40,000 cells/well, with each well containing 190 µL of a cell suspension. The plate was incubated overnight in a 37 °C, 5% CO₂ cell incubator.

The next day, the test compounds, serially diluted in a complete culture medium, were added at 10 µL/well. The final concentrations of the compounds were 9 concentration points obtained by 5-fold serial dilution from 10 µM. A blank control containing 0.5% DMSO was set. The plate was incubated in a 37 °C, 5% CO₂ cell incubator for 1 h. After the incubation, the 96-well cell culture plate was taken out, and the culture medium was removed using a pipette. The plate was washed once by adding 200 µL of PBS (Shanghai BasalMedia Technologies Co., Ltd., B320) to each well. The PBS was removed using a pipette, and 50 µL of a lysis buffer (Cisbio, 64KL1FDF) containing a blocking reagent (Cisbio, 64KB1AAC) was added to each well. The plate was shaken on a shaker at room temperature for 40 min for lysis. After lysis, the lysate was pipetted for good uniformity. 16 µL of the lysate in each well was transferred to two HTRF 96-well assay plates (Cisbio, 66PL96100), and then 4 µL of a premixed phosphorylated ERK1/2 antibody solution (Cisbio, 64AERPEG) or 4 µL of a premixed total ERK1/2 antibody solution (Cisbio, 64NRKPEG) was added to each well of the two plates. The plates were sealed with a plate sealer film, centrifuged for 1 min in a microplate centrifuge, and incubated overnight at room temperature in the dark.

On the third day, the fluorescence values at an excitation wavelength of 337 nm and emission wavelengths of 665 nm and 620 nm were obtained using a multi-mode microplate reader (PerkinElmer, EnVision).

### III. Data analysis

The ratio of the fluorescence signal at 665 nm to the fluorescence signal at 620 nm was calculated for phosphorylated ERK1/2 and total ERK1/2 for the compounds at various concentrations, and IC₅₀ values for the inhibitory activity of the compounds were calculated using Graphpad Prism software based on the concentrations of the compounds and the phosphorylated ERK1/2 ratios corrected using the total ERK1/2 ratios. The results are shown in Table 1 below.

**Table 1. Inhibitory activity data for ERK phosphorylation in AGS cells**

| Compound No. | AGS/IC₅₀ (nM) |
|---|---|
| 1 | 0.96 |
| 2 | 1.5 |
| 3 | 1.2 |

| | |
|---|---|
| Conclusion: The compounds of the present disclosure have relatively good inhibitory effects on ERK phosphorylation in AGS cells. | |

### Test Example 2: Biological Evaluation of Inhibition of 3D Proliferation of GP2d and AGS Cells

### I. Objective

The inhibitory effects of the compounds of the present disclosure on the KRAS target were evaluated by assessing the inhibitory effects of the compounds of the present disclosure on the 3D proliferation of GP2d and AGS cells.

### II. Experimental method

GP2d cells were cultured in a complete culture medium, namely a DMEM/high glucose culture medium (Hyclone, SH30243.01) containing 10% fetal bovine serum (Corning, 35-076-CV). On the first day of the experiment, the GP2d cells were seeded into a 96-well low-adsorption plate (Corning, CLS7007-24EA) with a complete culture medium at a density of 1000 cells/well, with each well containing 90 µL of a cell suspension. The plate was centrifuged at 2000 rpm for 5 min at room temperature and then incubated overnight in a 37 °C, 5% CO₂ cell incubator.

AGS cells were cultured in a complete culture medium, namely an RPMI1640 medium (Hyclone, SH30809.01) containing 10% fetal bovine serum (Corning, 35-076-CV). On the first day of the experiment, the AGS cells were seeded into a 96-well low-adsorption plate (Corning, CLS7007-24EA) with a complete culture medium at a density of 1000 cells/well, with each well containing 90 µL of a cell suspension. The plate was centrifuged at 2000 rpm for 5 min at room temperature and then incubated overnight in a 37 °C, 5% CO₂ cell incubator.

The next day, the test compounds, serially diluted in a complete culture medium, were added at 10 µL/well. The final concentrations of the compounds for GP2d cells were 9 concentration points obtained by 5-fold serial dilution from 1 µM, and the final concentrations of the compounds for AGS cells were 9 concentration points obtained by 5-fold serial dilution from 10 µM. A blank control containing 0.5% DMSO was set for both types of cells. The plates were incubated in a 37 °C, 5% CO₂ cell incubator for 5 days. On the seventh day, the 96-well cell culture plates were taken out, and 50 µL of the CellTiter-Glo^{®} 3D Cell Viability Assay reagent (Promega, G9682) was added to each well. The plates were shaken at room temperature in the dark for 25 min. The contents in the wells were pipetted for good uniformity, and 100 µL of the mixture was transferred from each well to a white impermeable 96-well plate (PerkinElmer, 6005290). The luminescence signal values were obtained using a multi-mode microplate reader (PerkinElmer, EnVision2105).

### III. Data analysis

IC₅₀ values for the inhibitory activity of the compound were calculated using Graphpad Prism software, and the results are shown in Table 2 below.

**Table 2. Inhibitory activity data for the 3D proliferation of AGS and GP2d cells**

| Compound No. | AGS /IC₅₀ (nM) | GP2d /IC₅₀ (nM) |
|---|---|---|
| 1 | 16.7 | 5.2 |
| 5 | 8.7 | 0.9 |
| 11 | - | 0.7 |
| 14 | - | 1.6 |
| Compound with longer retention time between 16 and 19 | 5.1 | 1.3 |
| 17 | - | 0.9 |
| 18 | - | 1.2 |
| Compound with shorter retention time between 18-p1 and 18-p2 | - | 1.7 |
| Compound with shorter retention time between 16 and 19 | 6.3 | 0.4 |
| 23 | - | 0.2 |
| 36 | 0.9 | 0.06 |
| 37 | 4.7 | 0.4 |
| 38 | 5.0 | 0.07 |
| 39 | 6.7 | 0.17 |
| 40 | - | 0.6 |
| 41 | - | 0.4 |
| 42 | 5.2 | 0.21 |

| | | |
|---|---|---|
| Conclusion: The compounds of the present disclosure have relatively good inhibitory effects on the 3D proliferation of AGS and GP2d cells. | | |

### Test Example 3: Biological Evaluation of Inhibition of 3D Proliferation of AsPC-1 Cells

On the first day of the experiment, well-grown AsPC-1 cells with 70%-80% confluency were digested and resuspended in an RPMI 1640 culture medium (Hyclone, SH30809.01) containing 10% FBS, and the cell density was adjusted to the desired level. The cell suspension was added to a U-shaped low-adsorption 96-well plate (Corning, CLS7007-24EA) at 90 µL/well, with a cell density of 1500 cells/well. The cell plate was centrifuged at 2500 rpm for 5 min and then incubated overnight in a 37 °C, 5% CO₂ incubator. The next day, 20 mM test compounds dissolved in DMSO were diluted to an initial concentration of 2 mM and then serially diluted 5-fold, and a total of 9 concentration points were obtained. Control wells were set using DMSO. The serially diluted compounds were then further diluted 20-fold with a culture medium. The test compounds, diluted with the culture medium, were added to the cell plate at 10 µL/well, with the final concentrations of the compounds being 9 concentration points obtained by 5-fold serial dilution from an initial concentration of 10 µM. Cell wells containing 0.5% DMSO were set as vehicle control wells, and wells containing only the culture medium and 0.5% DMSO were set as blank control wells. A duplicate well was set for each concentration of the compounds and the control wells, with the final concentration of DMSO in each well being 0.5%. The cell plate was centrifuged at 2500 rpm for 3 min and then incubated in a 37 °C, 5% CO₂ incubator for 5 days. On the seventh day, the 96-well cell culture plate was taken out, and 50 µL of the CellTiter-Glo^{®} Luminescent 3D Cell Viability Assay reagent (Promega, G9683) was added to each well. The plates were shaken at room temperature in the dark for 25 min. The contents in the wells were pipetted up and down for good uniformity, and then 100 µL of the mixture was transferred from each well to a white impermeable OptiPlate^{™}-96-well plate (PerkinElmer, 6005290). The luminescence signal values were obtained using a multi-mode microplate reader (PerkinElmer, EnVision2105).

The inhibition rate was calculated using the formula: inhibition rate = (luminescence value_{vehicle control well} - luminescence value_{test compound})/(luminescence value_{vehicle control well} - luminescence value_{blank control well}) × 100%. Curves were plotted using GraphPad Prism software based on the various concentrations of the compounds and the corresponding inhibition rates, and IC₅₀ values for the compounds were calculated.

**Table 3. Inhibitory activity data for the 3D proliferation of AsPC-1 cells**

| Example No. | AsPC-1/IC₅₀ (nM) |
|---|---|
| 5 | 5.7 |
| Compound with longer retention time between 16 and 19 | 7.9 |
| Compound with shorter retention time between 18-p1 and 18-p2 | 7.7 |
| Compound with shorter retention time between 16 and 19 | 6.6 |
| 23 | 7.9 |
| 36 | 0.2 |
| 37 | 2.4 |
| 38 | 1.7 |
| 39 | 2.6 |
| 41 | 3.7 |
| 42 | 1.8 |

| | |
|---|---|
| Conclusion: The compounds of the present disclosure have relatively good inhibitory effects on the 3D proliferation of AsPC-1 cells. | |

### Test Example 4: Pharmacokinetic Evaluation

### 1. Abstract

Balb/c nude mice were used as test animals. After intragastric (i.g.) administration of example compounds to balb/c nude mice, the plasma concentrations at different time points were determined by the LC/MS/MS method. The pharmacokinetic behavior of the compounds of the present disclosure in balb/c nude mice was studied, and their pharmacokinetic profiles were evaluated.

### 2. Method

### 2.1. Test compounds

Compounds 37 and 39.

### 2.2. Test animals

Eighteen female balb/c nude mice, provided by Vital River Laboratory Animal Technology Co., Ltd. with production license SCXK (Zhejiang) 2019-0001, were evenly divided into 2 groups.

### 2.3. Compound solution preparation

A certain amount of a test compound was weighed out, and 5% DMF + 45% PG + 50% (10% HS15-pH 7.4 buffer) + 400 mpk SNAC was added to prepare a 4 mg/mL colorless clear solution.

### 2.4. Administration

The dose administered was 40.0 mg/kg, and the volume was 10 mL/kg.

### 3. Procedure

0.1-mL blood samples were collected from the orbit before administration and 0.25 h, 0.5 h, 1.0 h, 2.0 h, 4.0 h, 6.0 h, 8.0 h, 11.0 h, and 24.0 h after administration. The blood samples were placed into EDTA-K2 anticoagulation test tubes and centrifuged at 10,000 rpm for 1 min (4 °C), and plasma was separated within 1 h and stored at -20 °C before analysis. The procedure starting from the blood collection to the centrifugation was performed under ice-bath conditions.

After administration of different concentrations of the test compounds, the plasma concentration of the test compounds in balb/c nude mice was determined: A 25-µL sample of the balb/c nude mouse plasma of each time point after administration was taken, and 200 µL of acetonitrile containing an internal standard (for compound 37, the internal standard was 100 ng/mL verapamil; for compound 39, the internal standard was 100 ng/mL diclofenac) was added. The mixture was vortexed for 5 min and centrifuged at 3700 rpm for 20 min, and the supernatant was mixed with water (1:1). 0.5-2 µL of the supernatant was taken and analyzed by LC/MS/MS.

### 4. Pharmacokinetic parameters

**Table 4. Pharmacokinetic parameters of the compounds of the present disclosure in balb/c nude mice**

| Compound No. | Plasma concentration Cmax (ng/mL) | Area under curve AUC₀₋ₜ (h*ng/mL) | Half-life T1/2 (h) | Clearance rate CL/F (mL/min/kg) | Apparent distribution volume Vz/F (mL/kg) |
|---|---|---|---|---|---|
| 37 | 699 | 5265 | 7.95 | 113 | 78033 |
| 39 | 578 | 7212 | 10.06 | 73.7 | 64216 |

| | | | | | |
|---|---|---|---|---|---|
| Conclusion: The compounds of the present disclosure have relatively good pharmacokinetic advantages in balb/c nude mice. | | | | | |

## Claims

1. A compound represented by general formula (IN) or a pharmaceutically acceptable salt thereof: 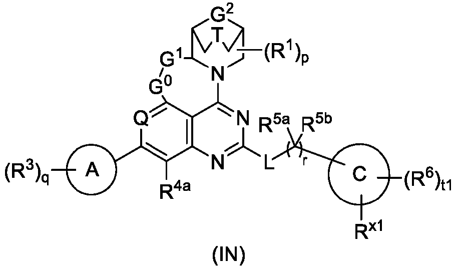 wherein:
ring C is heterocyclyl;
R^{x1} is a hydrogen atom or R^{x};
R^{x} is selected from the group consisting of =N-O-R⁶¹, =CR⁶²R⁶³, and =N-R⁶⁴;
G⁰ is selected from the group consisting of O, S, S(O), S(O)₂, CR^{G0a}R^{G0b}, and NR^{G0c};
G¹ is selected from the group consisting of CR^{G1a}R^{G1b}, CR^{G1a}R^{G1b}CR^{G1c}R^{G1d}, C=O, and C(O)CR^{G1a}R^{G1b};
G² is NR^{d};
T is a chemical bond or is selected from the group consisting of CR^{a}R^{b}, NR^{T}, and O;
Q is N or CR^{2a};
ring A is aryl or heteroaryl;
L is selected from the group consisting of a single bond, O, and NR^{e};
R^{a}, R^{b}, R^{G0a}, R^{G0b}, R^{G1a}, R^{G1b}, R^{G1e}, and R^{G1d} are identical or different and are each independently selected from the group consisting of a hydrogen atom, halogen, alkyl, alkoxy, haloalkyl, haloalkoxy, cyano, amino, nitro, hydroxy, hydroxyalkyl, cycloalkyl, and heterocyclyl; or, R^{G1a} and R^{G1b}, together with the carbon atom to which they are attached, form cycloalkyl; or, R^{G1c} and R^{G1d}, together with the carbon atom to which they are attached, form cycloalkyl;
each R¹ is identical or different and is independently selected from the group consisting of halogen, alkyl, alkenyl, alkynyl, alkoxy, haloalkyl, haloalkoxy, cyano, amino, -(CH₂)ᵤ-NR^{f}R^{g}, hydroxy, and hydroxyalkyl;
R^{2a} and R^{4a} are identical or different and are each independently selected from the group consisting of a hydrogen atom, halogen, alkyl, alkenyl, alkynyl, alkoxy, haloalkyl, haloalkoxy, cyano, amino, -(CH₂)ᵥ-NR^{h}Rⁱ, hydroxy, hydroxyalkyl, and cycloalkyl;
each R³ is identical or different and is independently selected from the group consisting of halogen, alkyl, alkenyl, alkynyl, alkoxy, haloalkyl, haloalkoxy, cyano, amino, -(CH₂)_{w}-NR^{j}R^{k}, -(CH₂)_{w1}-(O)ₓ₁-C(O)NR^{j1}R^{k1}, -(CH₂)_{w2}-(O)ₓ₂-C(O)OR^{j2}, nitro, hydroxy, hydroxyalkyl, cycloalkyl, heterocyclyl, aryl, and heteroaryl;
each R⁶ is identical or different and is independently selected from the group consisting of a hydrogen atom, halogen, alkyl, alkenyl, alkynyl, alkoxy, haloalkyl, haloalkoxy, cyano, amino, -(CH₂)_{y}-NR^{m}Rⁿ, -(CH₂)_{y1}-(O)_{z1}-C(O)NR^{m1}Rⁿ¹, -(CH₂)_{y2}-(O)_{z2}-C(O)OR^{m2}, =N-O-R⁶¹, =CR⁶²R⁶³, =N-R⁶⁴, nitro, hydroxy, hydroxyalkyl, oxo, cycloalkyl, heterocyclyl, aryl, and heteroaryl;
R⁶¹, R⁶², R⁶³, and R⁶⁴ are identical or different and are each independently selected from the group consisting of a hydrogen atom, halogen, alkyl, haloalkyl, hydroxyalkyl, and cycloalkyl;
R^{5a} and R^{5b} are identical or different and are each independently selected from the group consisting of a hydrogen atom, halogen, alkyl, haloalkyl, cyano, hydroxy, and hydroxyalkyl; or
R^{5a} and R^{5b}, together with the carbon atom to which they are attached, form cycloalkyl or heterocyclyl, and the cycloalkyl or heterocyclyl is independently and optionally substituted with one or more identical or different substituents selected from the group consisting of halogen, alkyl, haloalkyl, alkoxy, haloalkoxy, cyano, amino, hydroxy, and hydroxyalkyl;
R^{G0c}, R^{T}, R^{d}, R^{e}, R^{f}, R^{g}, R^{h}, Rⁱ, R^{j}, R^{k}, R^{j1}, R^{k1}, R^{j2}, R^{m}, Rⁿ, R^{m1}, Rⁿ¹, and R^{m2} are identical or different and are each independently selected from the group consisting of a hydrogen atom, alkyl, alkenyl, alkynyl, haloalkyl, hydroxyalkyl, cycloalkyl, heterocyclyl, aryl, and heteroaryl;
u, v, w, w1, w2, y, y1, and y2 are identical or different and are each independently selected from the group consisting of 0, 1, 2, and 3;
x1, x2, z1, and z2 are identical or different and are each independently selected from the group consisting of 0 and 1;
r is 0, 1, 2, or 3;
p is 0, 1, 2, 3, 4, or 5;
q is 0, 1, 2, 3, 4, or 5; and
t1 is 0, 1, 2, 3, 4, or 5.

2. The compound or the pharmaceutically acceptable salt thereof according to claim 1, wherein G¹ is CR^{G1a}H, and R^{G1a} is as defined in claim 1; preferably, G¹ is CR^{G1a}H, and R^{G1a} is methyl.

3. The compound or the pharmaceutically acceptable salt thereof according to claim 1 or 2, wherein G⁰ is O.

4. The compound or the pharmaceutically acceptable salt thereof according to any one of claims 1 to 3, wherein 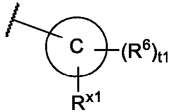 is selected from the group consisting of 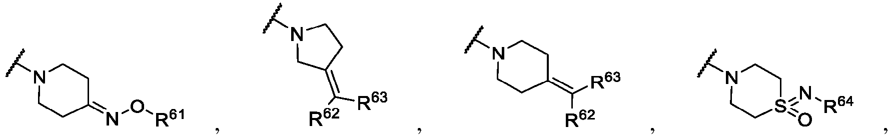 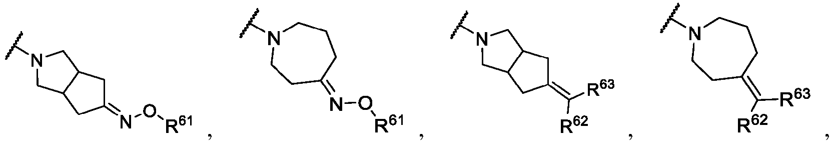 and 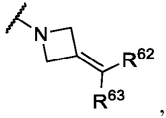 and R⁶¹, R⁶², R⁶³, and R⁶⁴ are as defined in claim 1.

5. The compound or the pharmaceutically acceptable salt thereof according to any one of claims 1 to 3, being a compound represented by general formula (II') or a pharmaceutically acceptable salt thereof: 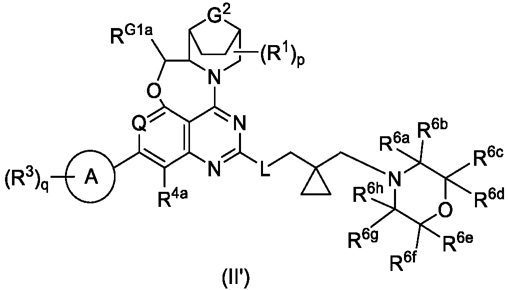 wherein:
R^{6a}, R^{6b}, R^{6c}, R^{6d}, R^{6e}, R^{6f}, R^{6g}, and R^{6h} are identical or different and are each independently selected from the group consisting of a hydrogen atom, halogen, alkyl, alkenyl, alkynyl, alkoxy, haloalkyl, haloalkoxy, cyano, amino, -(CH₂)_{y}-NR^{m}Rⁿ, -(CH₂)_{y1}-(O)_{z1}-C(O)NR^{m1}Rⁿ¹, -(CH₂)_{y2}-(O)_{z2}-C(O)OR^{m2}, nitro, hydroxy, hydroxyalkyl, cycloalkyl, heterocyclyl, aryl, and heteroaryl; or
R^{6a} and R^{6e}, together with the carbon atoms to which they are attached, R^{6a} and R^{6g}, together with the carbon atoms to which they are attached, R^{6c} and R^{6e}, together with the carbon atoms to which they are attached, or R^{6c} and R^{6g}, together with the carbon atoms to which they are attached, form a bridge; the bridge has 1, 2, 3, or 4 CH₂, any one of which may be optionally replaced with O, S, or NH, and the bridge is optionally substituted with one or more identical or different substituents selected from the group consisting of halogen, alkyl, haloalkyl, alkoxy, haloalkoxy, cyano, amino, hydroxy, and hydroxyalkyl; or
R^{6a} and R^{6b}, together with the carbon atom to which they are attached, R^{6c} and R^{6d}, together with the carbon atom to which they are attached, R^{6e} and R^{6f}, together with the carbon atom to which they are attached, or R^{6g} and R^{6h}, together with the carbon atom to which they are attached, form cycloalkyl or heterocyclyl, and the cycloalkyl or heterocyclyl is optionally substituted with one or more identical or different substituents selected from the group consisting of halogen, alkyl, haloalkyl, alkoxy, haloalkoxy, cyano, amino, hydroxy, and hydroxyalkyl;
ring A, G², Q, L, R^{G1a}, R¹, R³, R^{4a}, R^{m}, Rⁿ R^{m1}, Rⁿ¹, R^{m2}, y, y1, y2, z1, z2, p, and q are as defined in claim 1; preferably, R^{G1a} is C₁₋₆ alkyl.

6. The compound or the pharmaceutically acceptable salt thereof according to any one of claims 1 to 5, wherein Q is N.

7. The compound or the pharmaceutically acceptable salt thereof according to any one of claims 1 to 3, being a compound represented by general formula (IV') or (VV') or a pharmaceutically acceptable salt thereof: 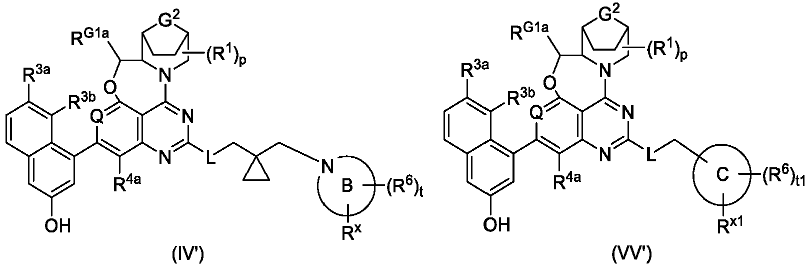 wherein:
R^{x} is selected from the group consisting of =N-O-R⁶¹, =CR⁶²R⁶³, and =N-R⁶⁴;
R⁶¹, R⁶², R⁶³, and R⁶⁴ are identical or different and are each independently selected from the group consisting of a hydrogen atom, halogen, alkyl, haloalkyl, hydroxyalkyl, and cycloalkyl;
t is 0, 1, 2, 3, or 4;
R^{3a} and R^{3b} are identical or different and are each independently selected from the group consisting of a hydrogen atom, halogen, alkyl, alkenyl, alkynyl, alkoxy, haloalkyl, haloalkoxy, cyano, amino, hydroxy, hydroxyalkyl, cycloalkyl, and heterocyclyl;
ring B is heterocyclyl;
R^{x1}, t1, ring C, G², Q, L, R^{G1a}, R¹, R^{4a}, R⁶, and p are as defined in claim 1; preferably, R^{G1a} is C₁₋₆ alkyl.

8. The compound or the pharmaceutically acceptable salt thereof according to any one of claims 1 to 6, wherein ring A is selected from the group consisting of naphthyl, phenyl, pyridinyl, benzothienyl, benzothiazolyl, and benzopyrazolyl.

9. The compound or the pharmaceutically acceptable salt thereof according to any one of claims 1 to 8, wherein R^{4a} is a hydrogen atom or halogen.

10. The compound or the pharmaceutically acceptable salt thereof according to any one of claims 1 to 9, wherein G² is NH.

11. The compound or the pharmaceutically acceptable salt thereof according to any one of claims 1 to 10, wherein L is O.

12. The compound or the pharmaceutically acceptable salt thereof according to any one of claims 1 to 11, wherein p is 0.

13. The compound or the pharmaceutically acceptable salt thereof according to any one of claims 1 to 6 and 8 to 12, wherein each R³ is identical or different and is independently selected from the group consisting of halogen, C₁₋₆ alkyl, C₂₋₆ alkynyl, C₁₋₆ haloalkyl, hydroxy, -O-C(O)NHC₁₋₆ alkyl, amino, cyano, C₁₋₆ hydroxyalkyl, and 3- to 8-membered cycloalkyl; preferably, each R³ is identical or different and is independently selected from the group consisting of halogen, C₁₋₆ alkyl, C₁₋₆ haloalkyl, hydroxy, -O-C(O)NHC₁₋₆ alkyl, amino, and cyano.

14. The compound or the pharmaceutically acceptable salt thereof according to any one of claims 1 to 13, being the following compounds:

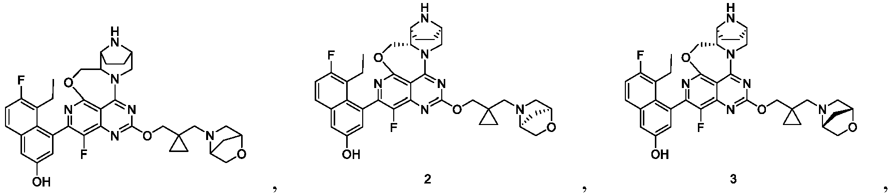

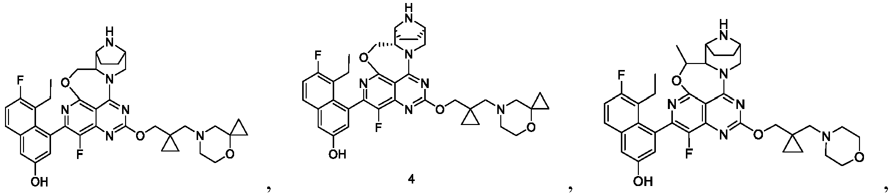

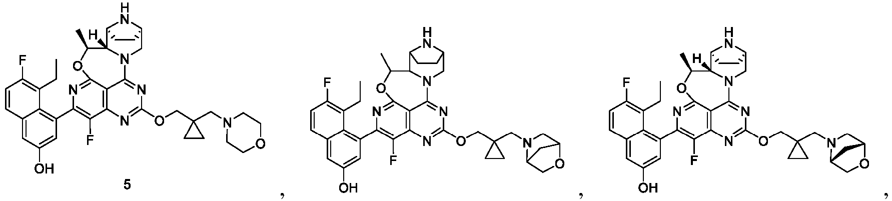

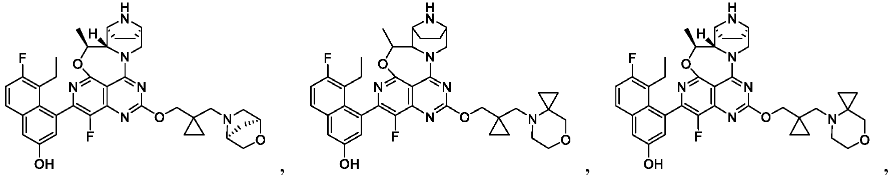

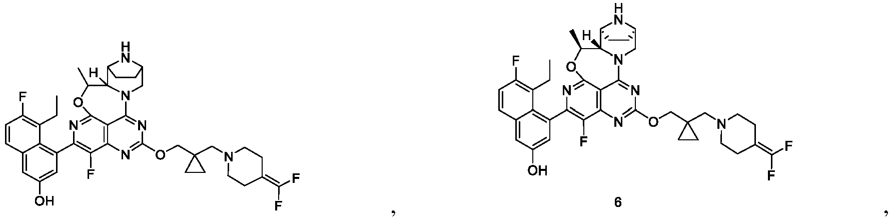

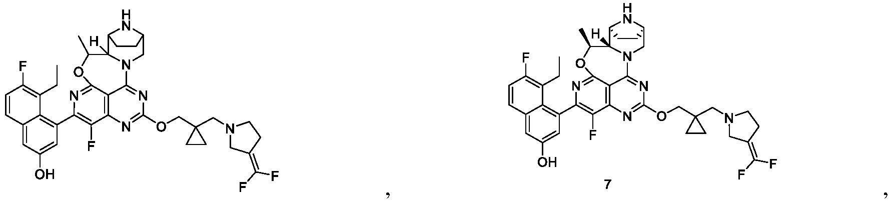

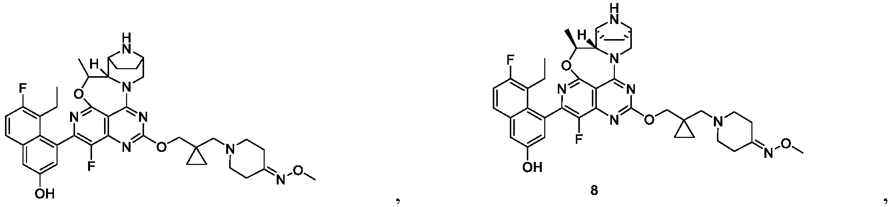

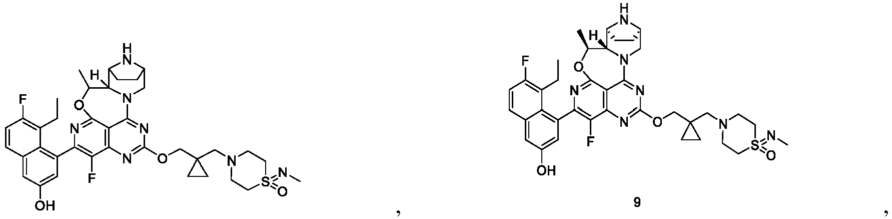

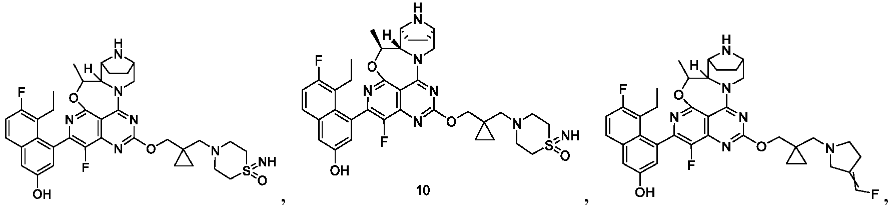

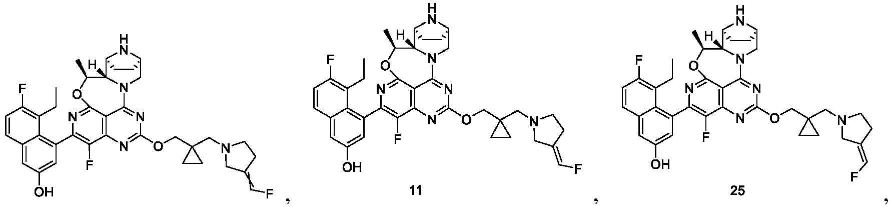

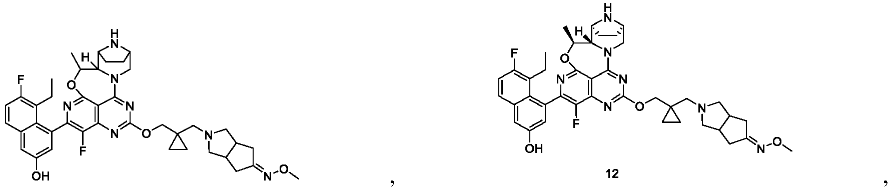

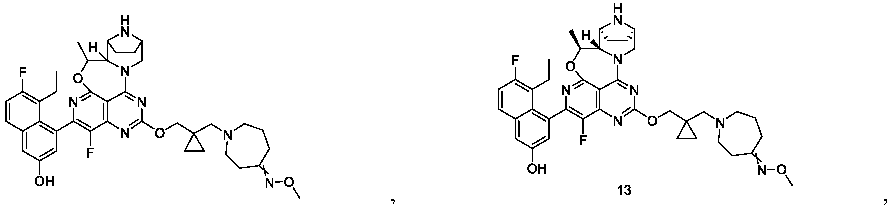

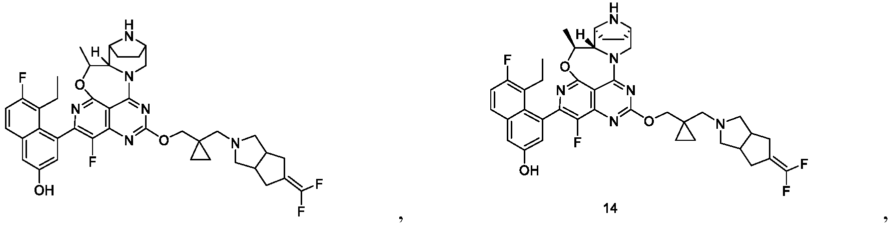

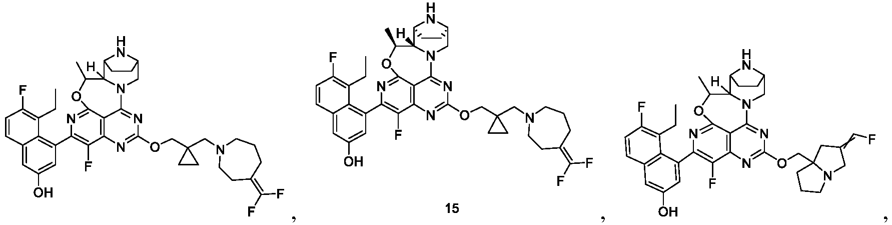

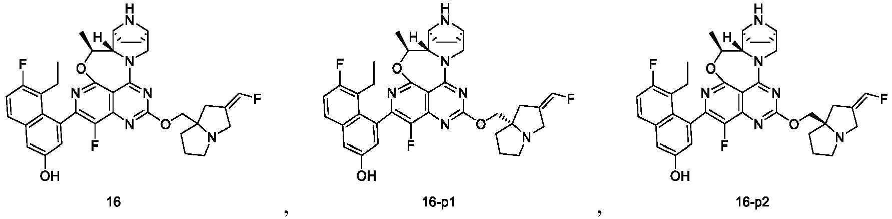

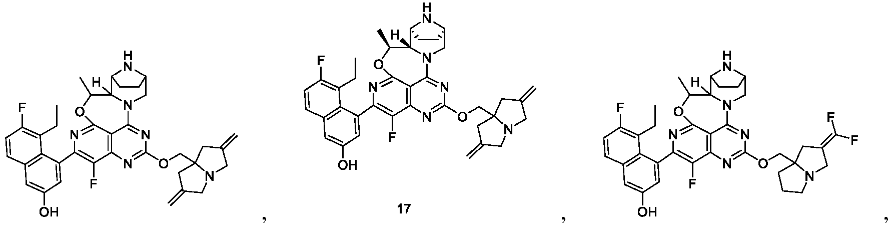

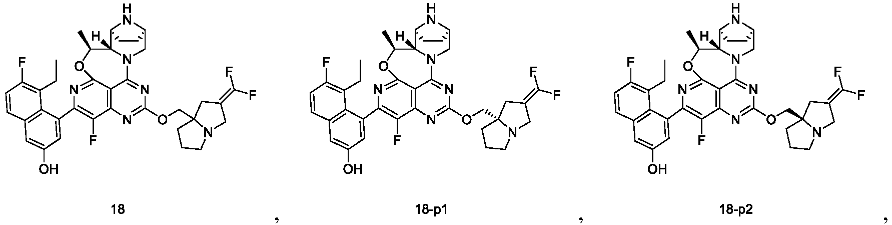

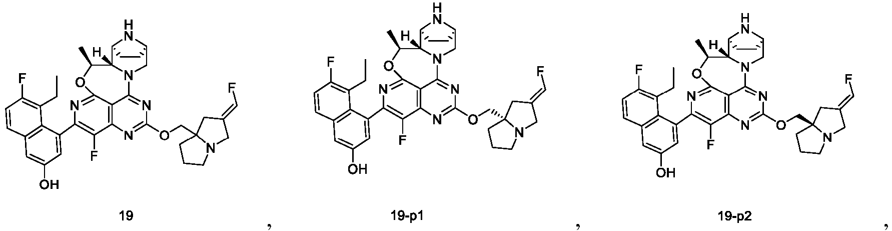

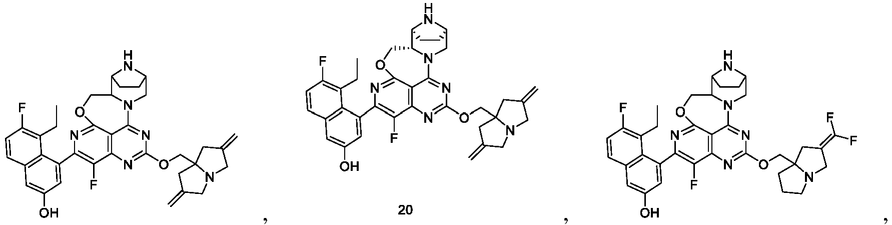

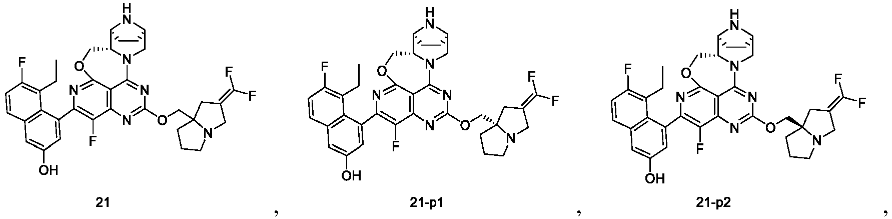

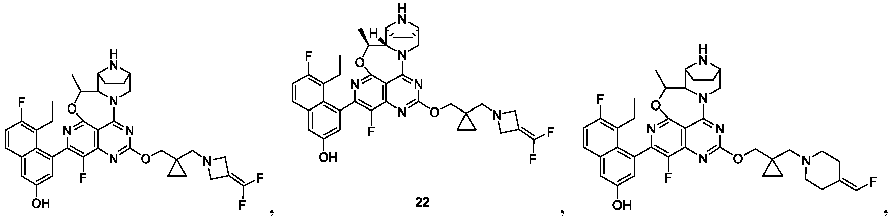

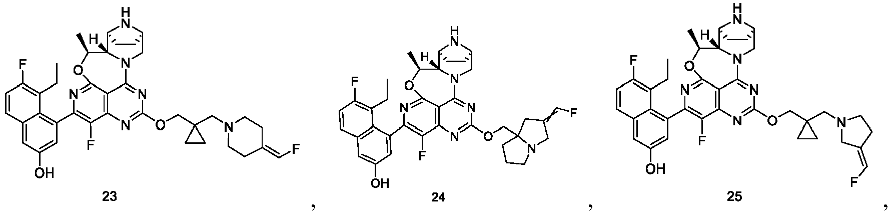

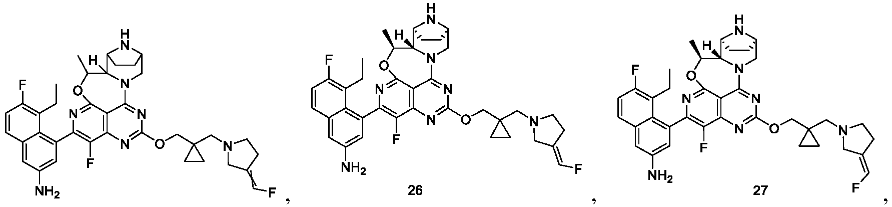

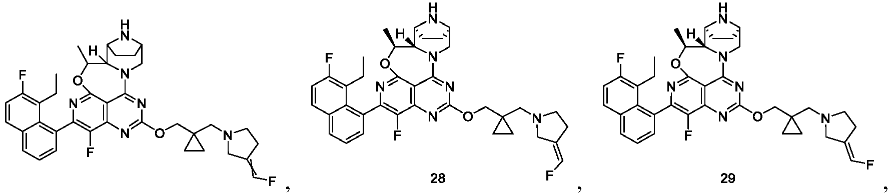

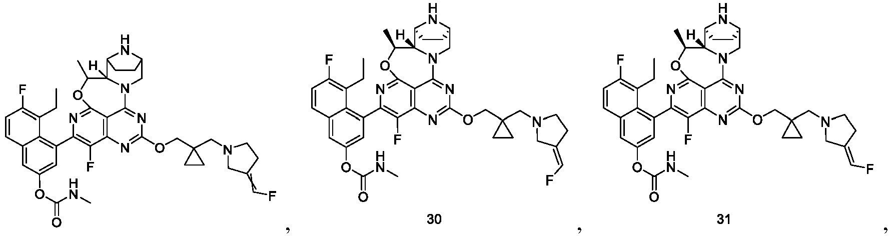

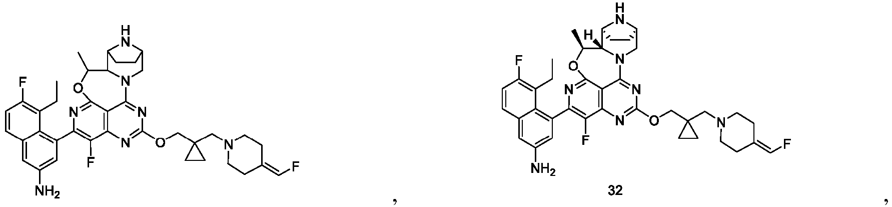

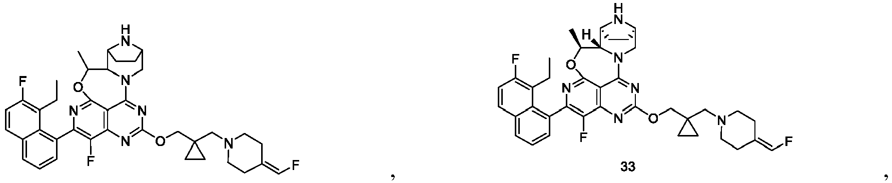

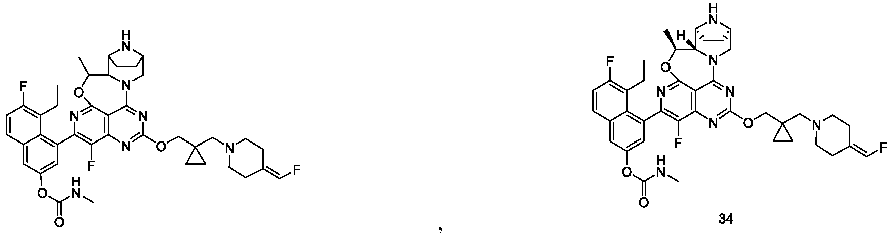

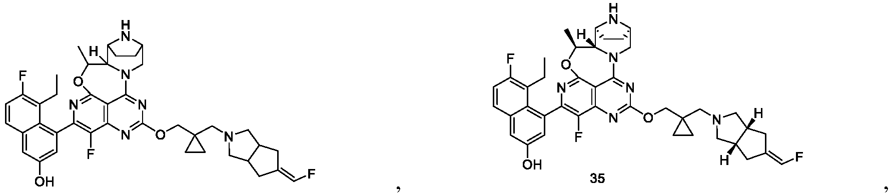

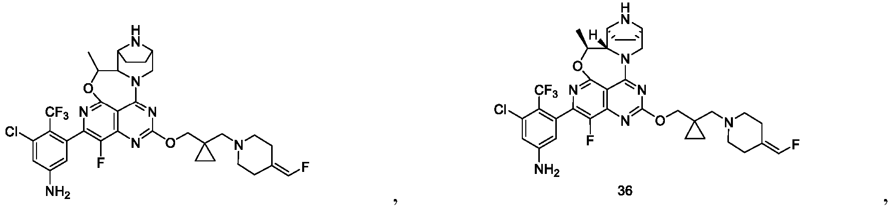

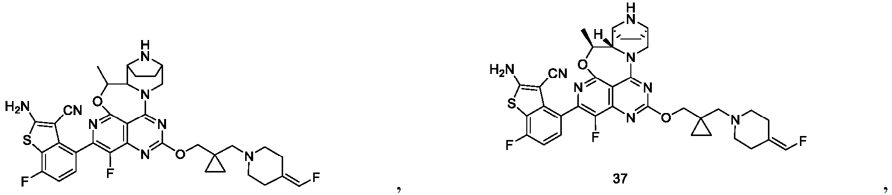

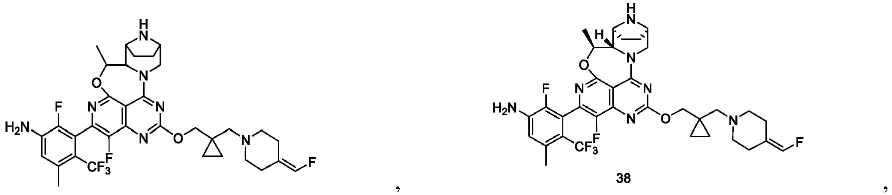

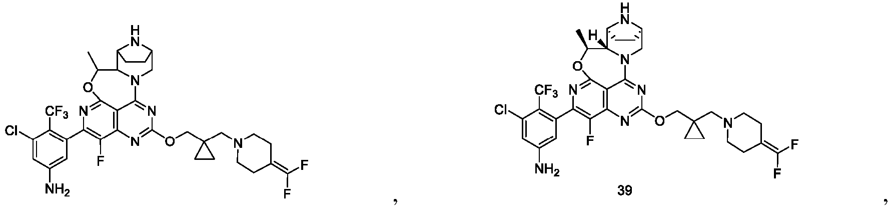

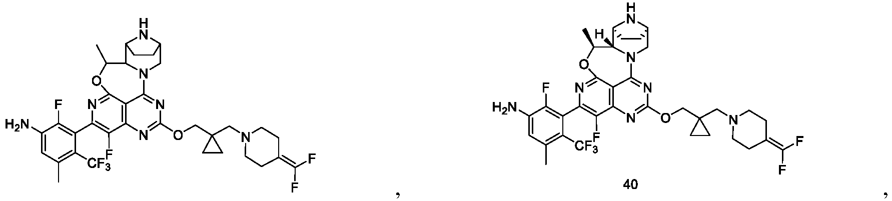

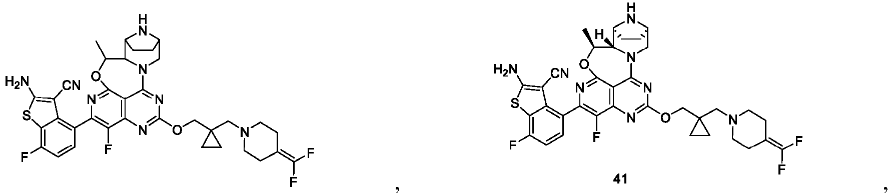

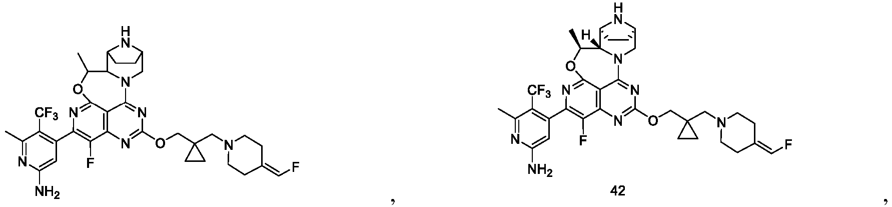

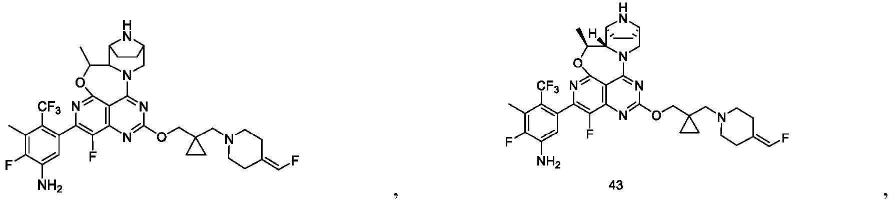

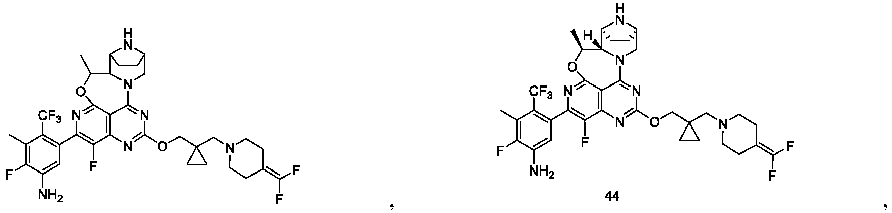

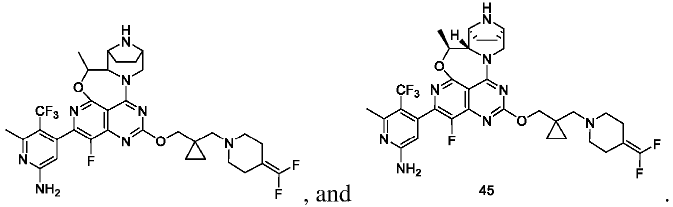

15. A compound represented by general formula (IN'A) or a salt thereof: 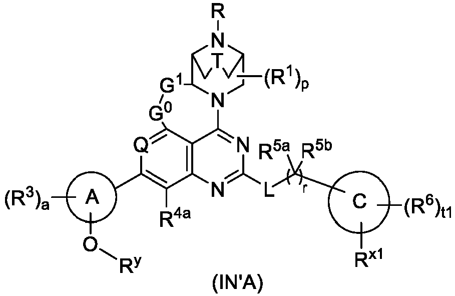 wherein:
R is an amino protecting group, preferably Boc;
R^{y} is a hydroxy protecting group, preferably MOM;
a is 0, 1, 2, 3, or 4;
G⁰, G¹, T, ring A, ring C, Q, L, R¹, R³, R^{4a}, R^{5a}, R^{5b}, R⁶, R^{x1}, p, t1, and r are as defined in claim 1.

16. A compound or a salt thereof, being the following compounds: 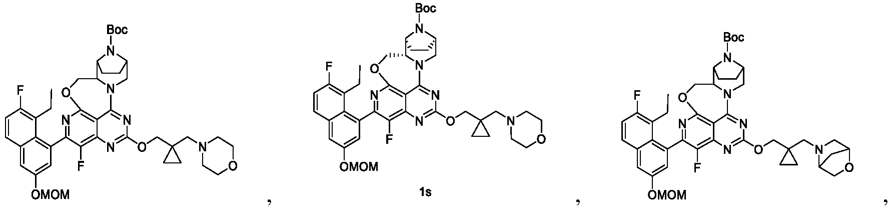 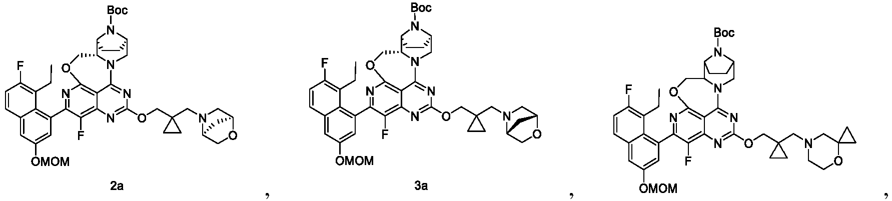 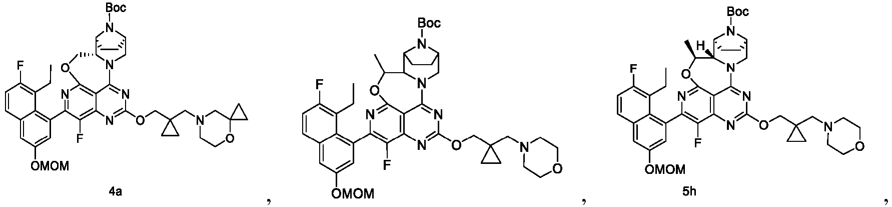 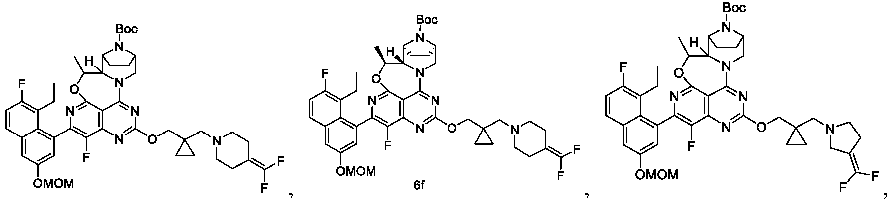 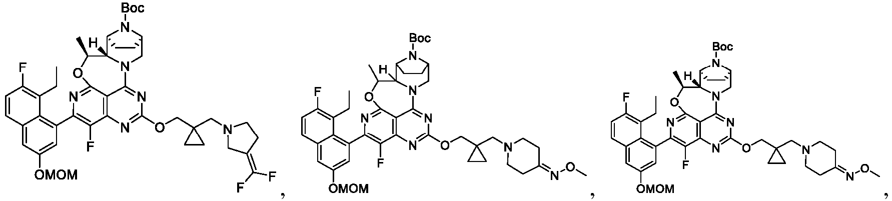 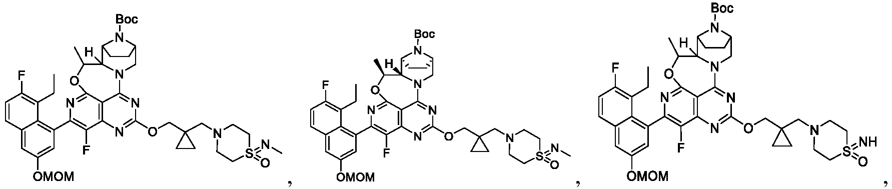 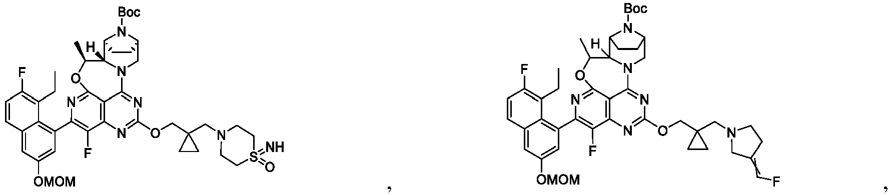 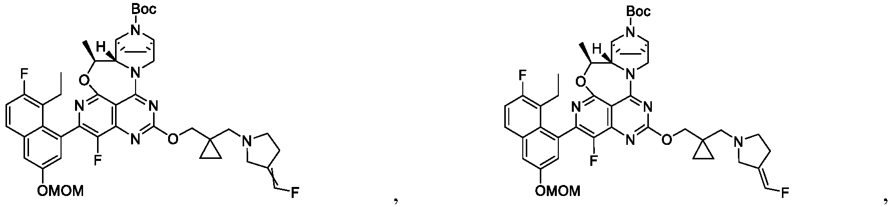 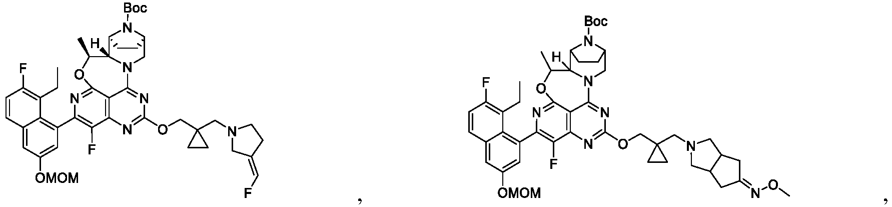 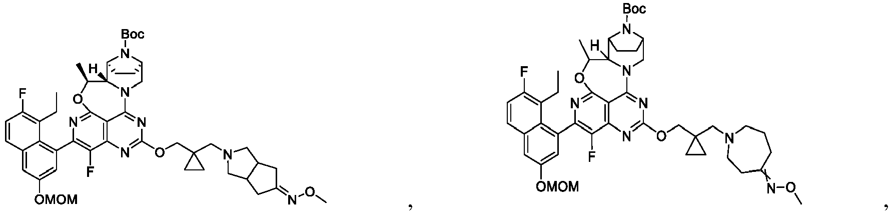 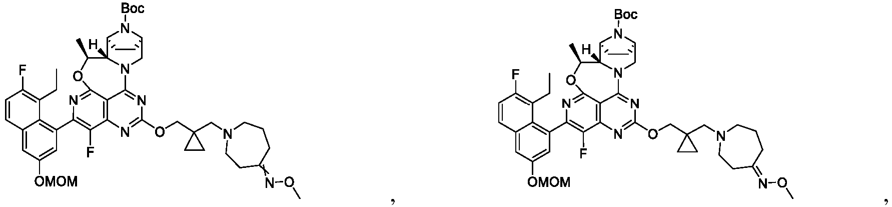 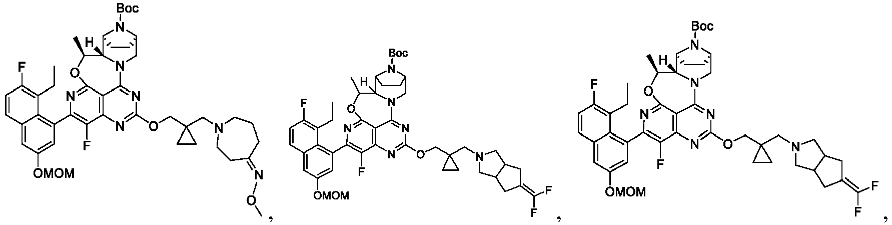 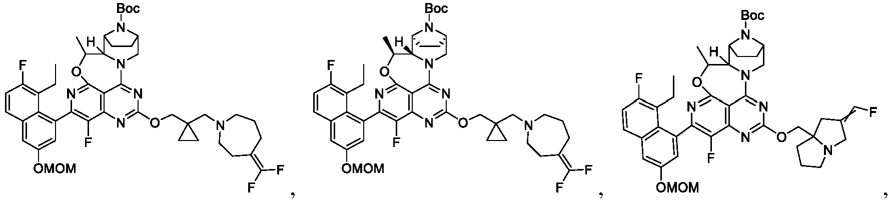 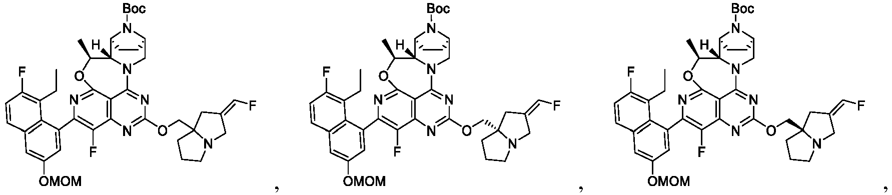 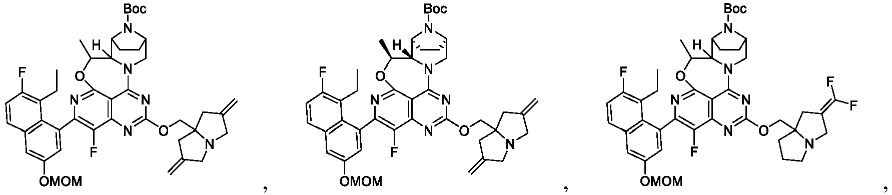 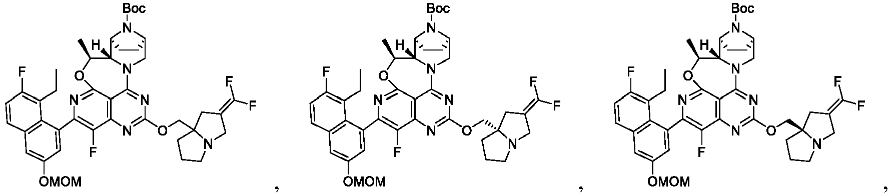 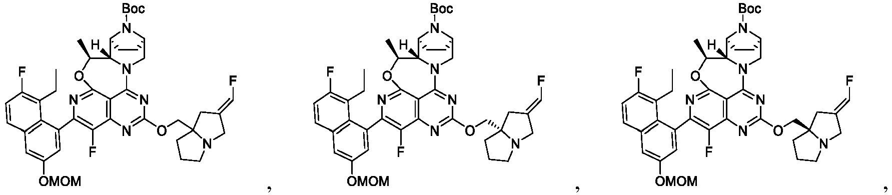 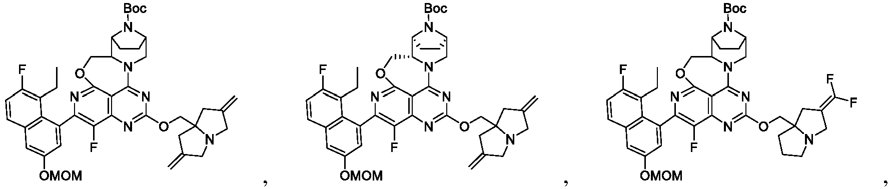 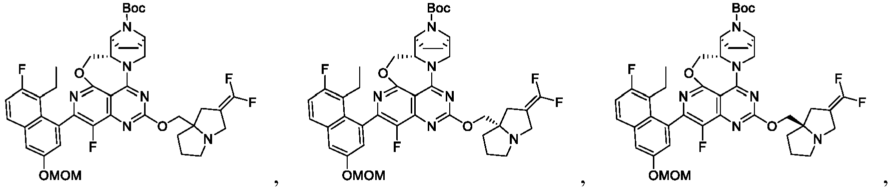 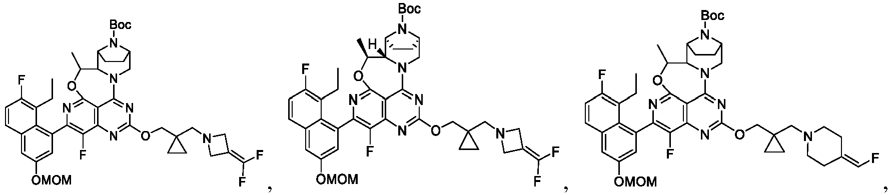 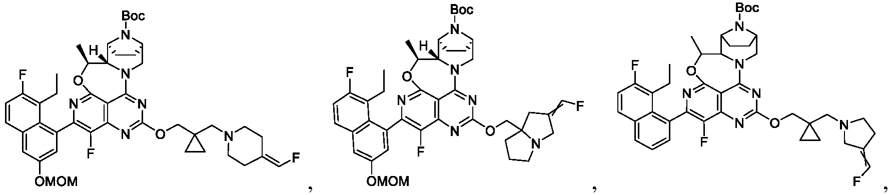 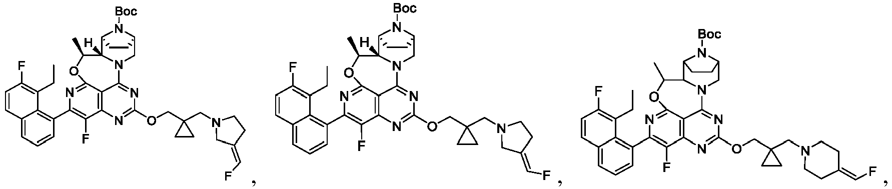 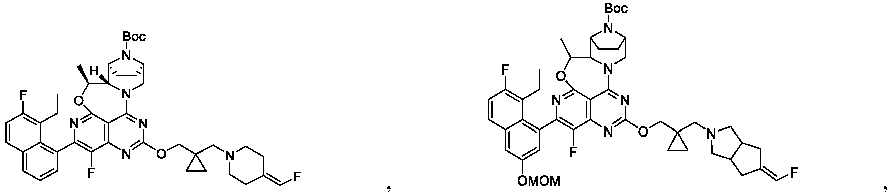 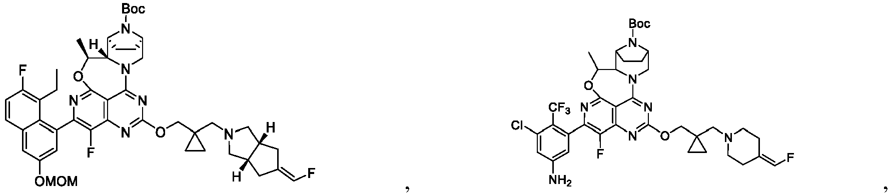 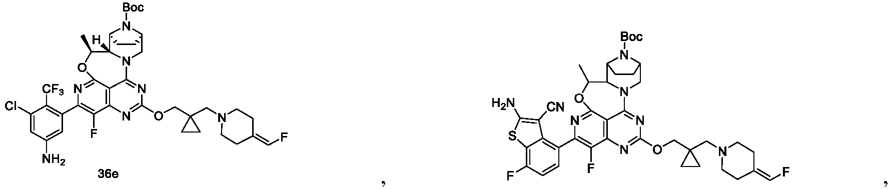 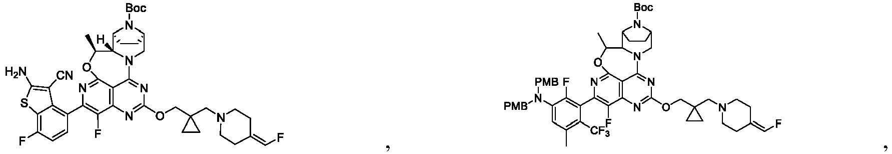 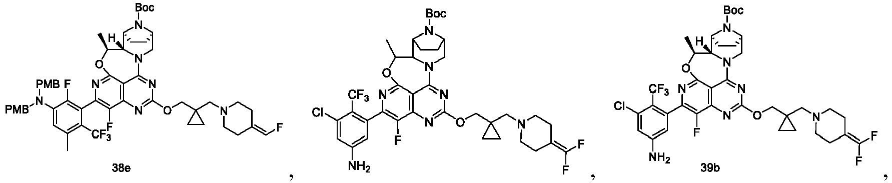 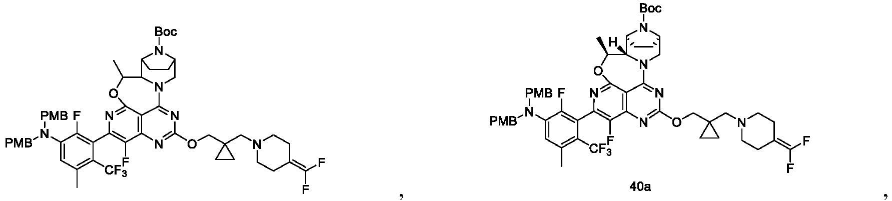 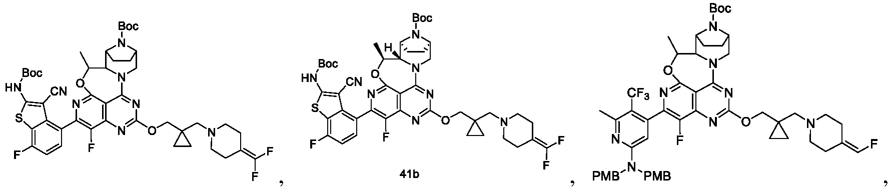 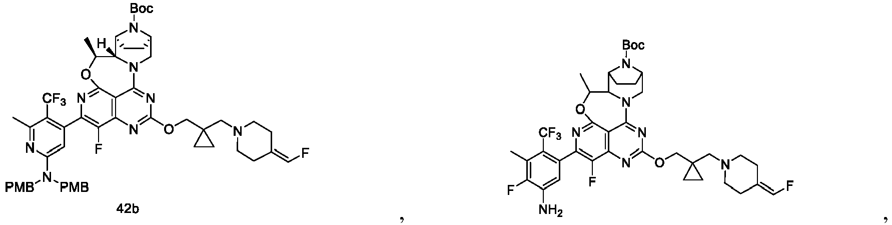 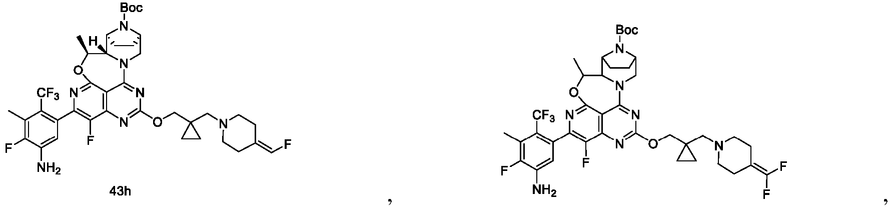 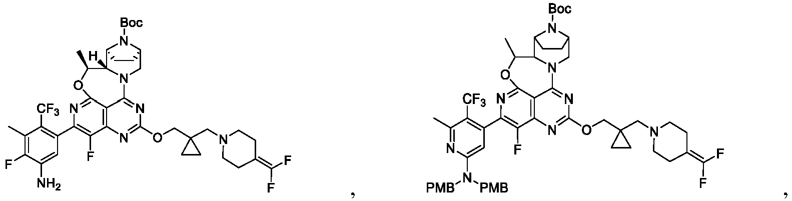 and

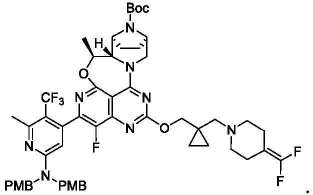

17. A method for preparing a compound represented by general formula (IN') or a pharmaceutically acceptable salt thereof, comprising: 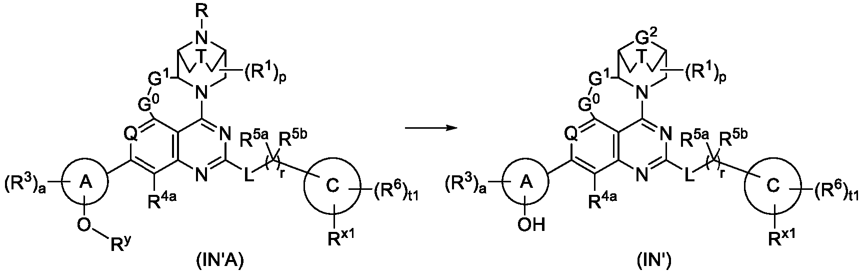
subjecting a compound of general formula (IN'A) or a salt thereof to a deprotection reaction to give the compound of general formula (IN') or the pharmaceutically acceptable salt thereof and, when group R³ has a protecting group thereon, optionally a step of removing the protecting group from group R³ before, concurrent with, or after the deprotection reaction,
wherein:
R is an amino protecting group, preferably Boc;
R^{y} is a hydroxy protecting group, preferably MOM;
a is 0, 1, 2, 3, or 4;
G² is NH;
G⁰, G¹, T, ring A, ring C, Q, L, R¹, R³, R^{4a}, R^{5a}, R^{5b}, R⁶, R^{x1}, p, t1, and r are as defined in claim 1.

18. A pharmaceutical composition, comprising the compound or the pharmaceutically acceptable salt thereof according to any one of claims 1 to 14, and one or more pharmaceutically acceptable carriers, diluents, or excipients.

19. Use of the compound or the pharmaceutically acceptable salt thereof according to any one of claims 1 to 14 or the pharmaceutical composition according to claim 18 in the preparation of a medicament for inhibiting KRAS G12D.

20. Use of the compound or the pharmaceutically acceptable salt thereof according to any one of claims 1 to 14 or the pharmaceutical composition according to claim 18 in the preparation of a medicament for treating and/or preventing a KRAS G12D-mediated disease or disorder.

21. Use of the compound or the pharmaceutically acceptable salt thereof according to any one of claims 1 to 14 or the pharmaceutical composition according to claim 18 in the preparation of a medicament for treating and/or preventing a tumor, wherein preferably, the tumor is selected from the group consisting of brain cancer, thyroid cancer, head and neck cancer, nasopharyngeal cancer, laryngeal cancer, oral cancer, salivary gland cancer, esophageal cancer, gastric cancer, lung cancer, liver cancer, renal cancer, pancreatic cancer, gallbladder cancer, bile duct cancer, colorectal cancer, small intestine cancer, gastrointestinal stromal tumor, urothelial cancer, urinary tract cancer, bladder cancer, breast cancer, vaginal cancer, ovarian cancer, endometrial cancer, cervical cancer, fallopian tube cancer, testicular cancer, prostate cancer, hemangioma, leukemia, lymphoma, myeloma, skin cancer, lipoma, bone cancer, soft tissue sarcoma, neurofibroma, neuroglioma, neuroblastoma, and glioblastoma; more preferably, the tumor is selected from the group consisting of pancreatic cancer, colorectal cancer, and non-small cell lung cancer.
